(19) Europäisches Patentamt / European Patent Office / Office européen des brevets

(11) **EP 4 592 286 A1**

(12) **EUROPEAN PATENT APPLICATION**
published in accordance with Art. 153(4) EPC

(43) Date of publication:
**30.07.2025 Bulletin 2025/31**

(21) Application number: **23867407.1**

(22) Date of filing: **15.09.2023**

(51) International Patent Classification (IPC):
***C07D 413/12*** (2006.01) ***C07D 487/04*** (2006.01)
***C07D 413/14*** (2006.01) ***C07F 9/6561*** (2006.01)
***C07F 9/6558*** (2006.01) ***C07D 473/16*** (2006.01)
***C07D 519/00*** (2006.01) ***C07D 487/08*** (2006.01)
***A61K 31/506*** (2006.01) ***A61K 31/519*** (2006.01)
***A61K 31/52*** (2006.01) ***A61K 31/517*** (2006.01)
***A61K 31/675*** (2006.01) ***A61K 31/4709*** (2006.01)
***A61P 25/00*** (2006.01) ***A61P 37/02*** (2006.01)
***A61P 35/00*** (2006.01) ***A61P 35/02*** (2006.01)
***A61P 25/16*** (2006.01) ***A61P 11/06*** (2006.01)
***A61P 17/00*** (2006.01)

(52) Cooperative Patent Classification (CPC):
**A61K 31/4709; A61K 31/506; A61K 31/517; A61K 31/519; A61K 31/52; A61K 31/675; A61P 11/06; A61P 17/00; A61P 25/00; A61P 25/16; A61P 35/00; A61P 35/02; A61P 37/02; C07D 413/12; C07D 413/14;** (Cont.)

(86) International application number:
**PCT/CN2023/119030**

(87) International publication number:
**WO 2024/061118 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.09.2022 CN 202211155875**

(71) Applicant: **Artivila Biopharma**
**Wuxi, Jiangsu 214000 (CN)**

(72) Inventors:
- **ZHU, Zhendong**
  **Wuxi, Jiangsu 214000 (CN)**
- **NIU, Deqiang**
  **Wuxi, Jiangsu 214000 (CN)**
- **FAN, Ming**
  **Wuxi, Jiangsu 214000 (CN)**
- **LI, Xuke**
  **Wuxi, Jiangsu 214000 (CN)**

(74) Representative: **Boult Wade Tennant LLP**
**Salisbury Square House**
**8 Salisbury Square**
**London EC4Y 8AP (GB)**

# (54) MACROCYCLIC NITROGEN-CONTAINING CROWN ETHER COMPOUND AND USE THEREOF AS PROTEIN KINASE INHIBITOR

(57) The present invention provides a macrocyclic nitrogen-containing crown ether compound represented by formula (I) and use thereof as a protein kinase inhibitor.

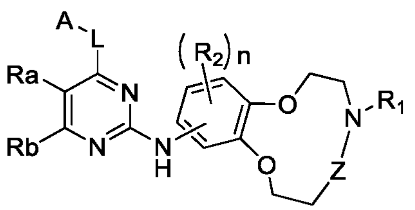

(I)

EP 4 592 286 A1

(52) Cooperative Patent Classification (CPC): (Cont.)
**C07D 473/16; C07D 487/04; C07D 487/08;**
**C07D 519/00; C07F 9/6558; C07F 9/6561**

## Description

This application claims the priority of Chinese Patent Application No.202211155875.X, filed with the China National Intellectual Property Administration on September 21, 2022 titled "MACROCYCLIC NITROGEN-CONTAINING CROWN ETHER COMPOUND AND USE THEREOF AS PROTEIN KINASE INHIBITOR", the disclosure of which is hereby incorporated by reference in its entirety.

## FIELD

The present disclosure relates to the field of medicinal chemistry, in particular to a kind of macrocyclic nitrogen-containing crown ether compounds and stereoisomers, tautomers or pharmaceutically acceptable salts thereof, preparation methods therefor and pharmaceutical compositions containing the compounds. The present disclosure also relates to use of the compounds as a protein kinase inhibitor.

## BACKGROUND

Protein kinases are enzyme proteins widely existing in cells and cell surfaces. Up to now, more than 500 kinds of protein kinases have been found and identified. They belong to a structurally related protein family, and their known members are related to almost all cell signal transduction activities. The catalytic function of protein kinase is to transfer $\gamma$-phosphate group in ATP (or GTP) molecule to the specific threonine, serine or tyrosine group on the target protein, so that the conformation of the target protein changes, so as to the function of the target protein changes from static to active state. Depending on the specificity of target amino acids of protein kinases, protein kinases are divided into two categories: serine/threonine protein kinases and tyrosine protein kinases.

The signal transduction and regulation participated by protein kinases play an extremely important role in the normal function of cells and organs, including cell growth, differentiation, proliferation, angiogenesis, apoptosis, cytoskeleton arrangement, regulation of metabolic reaction, membrane transport and cell movement, etc. In addition, the non-catalytic functions of protein kinases also play indispensable roles, including allosteric effect, subcellular targeting, protein complex scaffold, protein competitive interaction and DNA binding. On the other hand, when a protein kinase is mutated or overexpressed, the disordered protein kinase will lead to various pathological consequences, including cancer, inflammation, autoimmune diseases, cardiovascular and nervous system diseases. Therefore, protein kinases have become one of the most important targets in drug development today. In recent years, protein kinase inhibitors have been continuously successful in clinical treatment and approved by drug regulatory authorities around the world, which has brought a major breakthrough in clinical disease treatment.

The present disclosure specifically relates to a kind of macrocyclic nitrogen-containing crown ether compounds and use thereof as inhibitors of protein kinases, including but not limited to protein kinases such as LRRK2, JAK1, JAK2, JAK3, EGFR and CDK9.

Leucine-rich repetitive kinase 2 (LRRK2) is a protein product of LRRK2 gene, and belongs to serine/threonine protein kinases. Although the function of LRRK2 protein is not completely clear at present, LRRK2 gene mutation was found to be related to Parkinson's disease (PD) as early as 2004. LRRK2 gene mutation is common in G2019S and I2020T in the kinase active region and R1441G/C/H and Y1699C in GTP enzyme region. These mutations can all lead to the increase of LRRK2 protein kinase activity, and lead to various pathological changes, including increased phosphorylation of synuclein and TAU protein, nervous system inflammation and mitochondrial dysfunction of neurons.

PD is the second major neurodegenerative disease after Alzheimer's Disease, with more than 10 million patients worldwide, but no effective treatment has been found so far. 10% of PD patients have obvious family history, in which LRRK2 mutation is the most common cause. The mutation occurs in about 5% of familial and 1% of non-familial PD patients, and the clinical manifestations caused by these mutations are not different from those caused by sporadic PD. In addition, LRRK2 mutation can also lead to pathological changes of TAU protein, which is one major feature of Alzheimer's disease. Therefore, LRRK2 may be situated at the upstream stage of the pathogenesis of neurodegeneration, playing an important role in other neurodegenerative diseases. Therefore, the development of LRRK2 specific inhibitors has become one of the effective ways to treat PD and other neurodegenerative diseases.

Janus kinase (JAK) is a family of non-receptor tyrosine protein kinases, which includes four members: JAK1, JAK2, JAK3 and TYK2. It is a cytoplasmic tyrosine kinase that transduces cytokine signals from membrane receptors to STAT transcription factors. Diseases related to JAK-STAT include rheumatic arthritis (RA), asthma, ankylosing spondylitis, lupus erythematosus, psoriasis, vitiligo and other autoimmune diseases. Although pan-JAK inhibitors show good therapeutic efficacy in the treatment of inflammation and tumor diseases, their off-target effects at higher doses can cause serious side effects and adverse reactions. Therefore, it is still necessary to develop new inhibitors targeting different JAK subtypes for the treatment of autoimmune diseases. For example, highly selective JAK1 inhibitors are being studied in clinical treatment of rheumatic arthritis (RA), asthma and dermatitis, which may have better safety margines.

Cyclin-dependent kinases (CDK), belonging to serine/threonine kinases, are a family of protein kinases that regulate cell cycle and gene transcription. At different stages of cell cycle, CDK phosphorylates specific cyclin to regulate cell activities. CDK9 is one of the most important CDKs, which can cooperate with four kinds of cyclins, including cyclin T1, cyclin K, cyclin T2a and cyclin T2b. CDK9 binds to cyclin to form a heterodimer of positive transcription elongation factor b (P-TEFb). P-TEFb phosphorylates the C-terminal domain (CTD) of RNA polymerase II, which makes transcription extend from the initial site and is the core molecule of transcription extension. The disorder of CDK9 kinase activity will lead to many diseases, including highly proliferative diseases (such as cancer), viral infectious diseases or cardiovascular diseases.

In view of the key role of CDK9 in diseases, down-regulation of CDK9 provides an excellent opportunity to develop targeted treatment for cancer and other diseases. Although many small molecular CDK inhibitors have been reported, most of them lack selectivity for specific CDK, so they show low efficacy and high adverse event rate in clinic. Therefore, it is very important to develop targeted inhibitors with CDK9 specificity for the treatment of human diseases.

The existing protein kinase compounds are still not satisfactory in enzyme inhibition activity, or their water solubility is not good enough to meet the requirements of drug development. Therefore, curreatly there is still an urgent need to develop compounds with good protein kinase inhibition activity and/or good water solubility.

**SUMMARY**

The inventors discovered that the heterocyclic nitrogen-containing compounds comprising macrocyclic crown ether of the present disclosure showed good inhibitory activity against protein kinase; moreover, the compounds also showed good water solubility.

Based on the above findings, in the first aspect, the present disclosure provides a compound having a structure represented by formula (I),

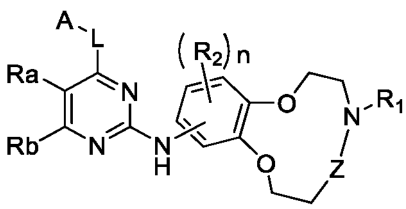

(I)

wherein,

Z represents absence (covalent bond) or $-OCH_2CH_2-$;

L represents absence (covalent bond), -O-, -NH- or -N($C_{1-4}$ alkyl)-;

A is aryl, 5-12 membered heteroaryl, $C_{1-10}$ alkyl, $C_{3-8}$ cycloalkyl, 3-8 membered heterocyclyl or 5-18 membered bridged ring group optionally substituted with one or two substituents, wherein the substituent is selected from the group consisting of deuterium, $C_{1-4}$ alkyl, $C_{1-10}$ alkoxy, $C_{3-6}$ cycloalkyl, 3-8 membered heterocyclyl, $C_{1-4}$ alkylphosphoryl, $C_{1-4}$ alkylsulfonyl, aminosulfonyl, $C_{1-4}$ alkylaminosulfonyl, cyano $C_{3-6}$ cycloalkyl, $C_{2-4}$ alkenylformyl, $C_{2-4}$ alkenylformamido, cyano $C_{1-4}$ alkylcarbamoyl, cyano $C_{1-4}$ alkyl, $C_{1-4}$ alkylformyl-3-8 membered heterocyclyl, $C_{3-6}$ cycloalkylformyl, 3-8 membered heterocyclyl-sulfonyl, $C_{1-4}$ alkylsulfonyl-3-8 membered heterocyclyl, amino $C_{3-6}$ cycloalkylformylamino, $C_{1-4}$ alkylphosphoramido, $C_{1-4}$ alkylsulfonamido, cyano, hydroxyl, oxo, mercapto, amino, $C_{2-4}$ alkenyl and halogen;

$R_1$ is hydrogen, or optionally substituted $C_{1-4}$ alkyl, $C_{3-6}$ cycloalkyl, $C_{1-4}$ alkyl-CO-, -CO or $C_{1-4}$ alkylsulfonyl, wherein the substituent is selected from the group consisting of deuterium, $C_{1-4}$ alkyl, $C_{1-10}$ alkoxy, $C_{3-6}$ cycloalkyl, oxo, cyano, hydroxyl, amino, dimethylamino, and hydroxylamino;

$R_2$ is independently selected from the group consisting of hydrogen, $C_{1-3}$ alkyl, trifluoromethyl, $C_{1-3}$ alkoxy, cyano and halogen;

n is 1 or 2;

Ra is selected from the group consisting of hydrogen, amino, trifluoromethyl, halogen, cyano, $C_{1-3}$ alkyl, acetyl, $C_{1-3}$ alkyl phosphoryl and $C_{1-3}$ alkyl sulfonyl;

Rb is selected from the group consisting of hydrogen, amino and $C_{1-3}$ alkylamino; and

Ra, Rb and the carbonatom to which they are linked can together form a 5-6 membered aromatic ring, 5-12 membered heteroaromatic ring or 5-8 membered heterocyclic ring;

or a stereoisomer, tautomer or pharmaceutically acceptable salt thereof.

More specifically, in some embodiments, the compound represented by formula (I) has structures represented by formula (II) or formula (III),

(II) 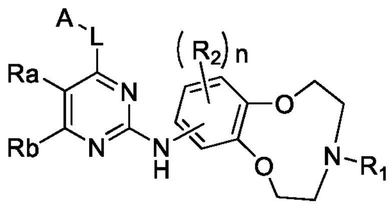 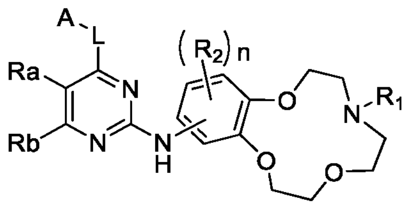 (III)

wherein L, A, $R_1$, $R_2$, Ra and Rb are defined as above.

In other embodiments, the compound represented by formula (I) has a structure represented by formula (IV),

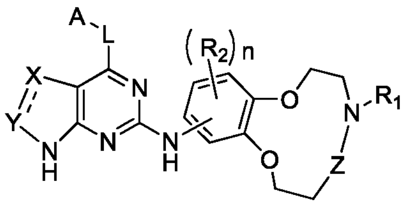 (IV)

wherein X and Y are each independently selected from the group consisting of C and N;

Z represents absence (covalent bond) or $-OCH_2CH_2-$; and

L, A, $R_1$, $R_2$ and n are as defined above.

In the present disclosure, for the above compound represented by formula (I) or compounds represented by formulas (II)-(IV),

It is preferable that A has one of the following structures:

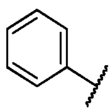 , 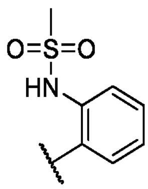 , 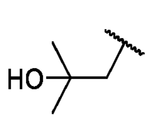 , 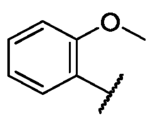 , 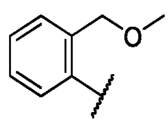 , 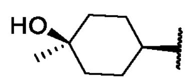 ,

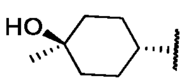 , 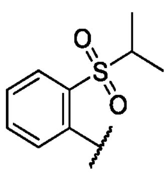 , 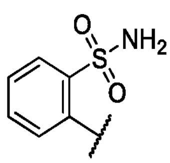 , 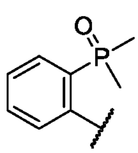 , 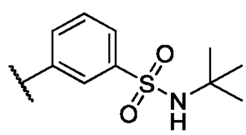 , 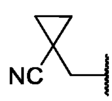 ,

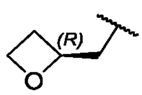 , 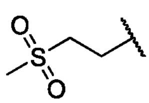 , 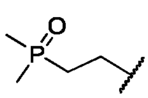 , 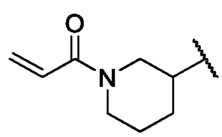 , 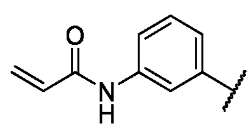 ,

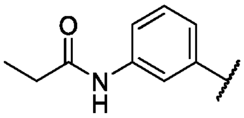, 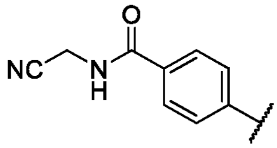, 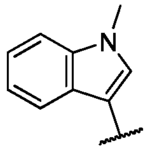, 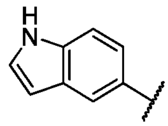, 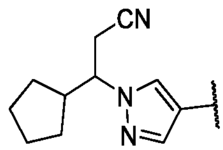,

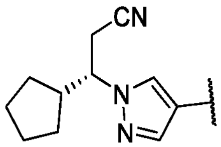, 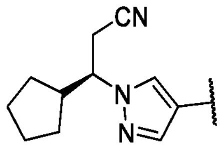, 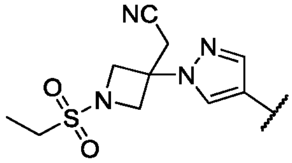, 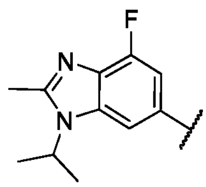,

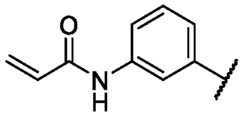, 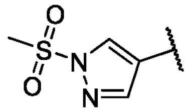, 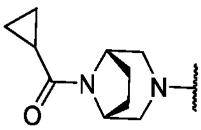, 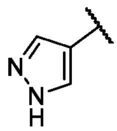, 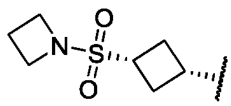,

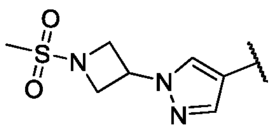, 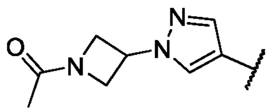, 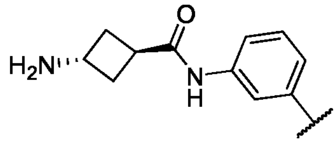,

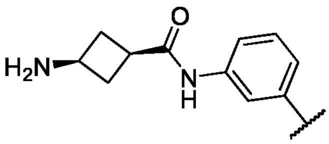, 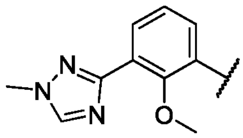, 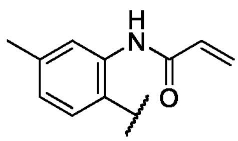, 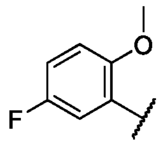,

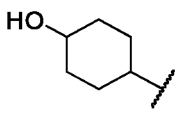, and 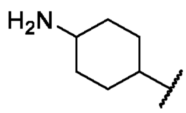.

The following compounds or the pharmaceutically acceptable salts thereof are preferred in the present disclosure:

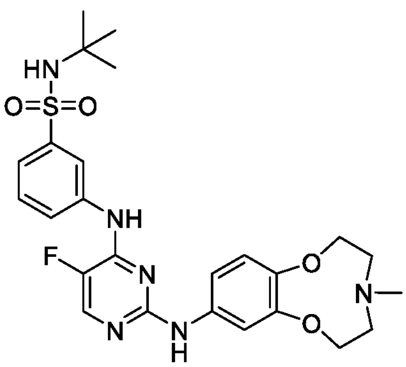

1

,

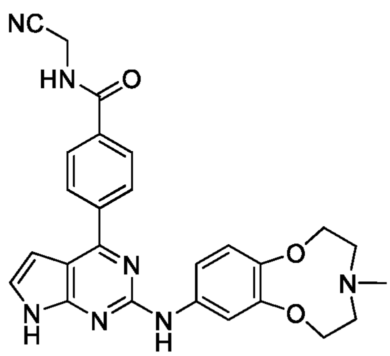

2

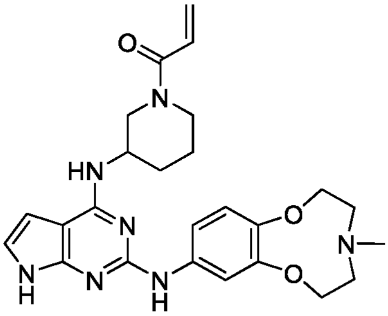

**3**

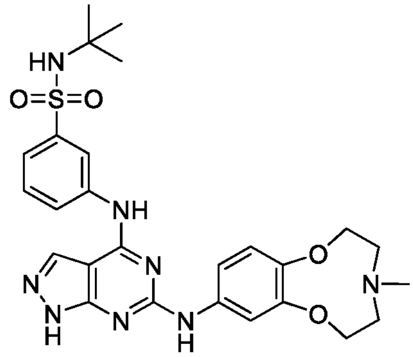

**4**

,

,

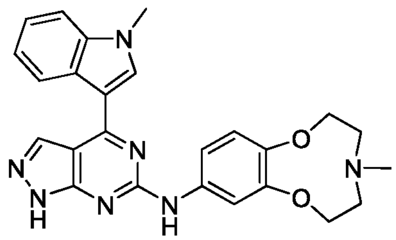

**5**

,

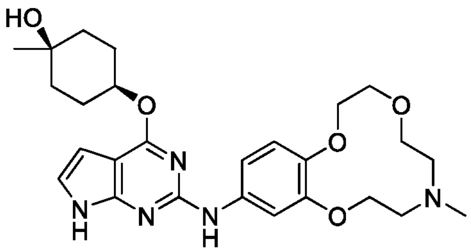

**6**

,

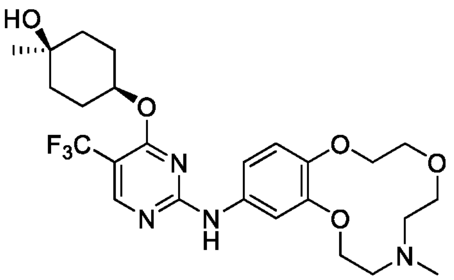

**7**

,

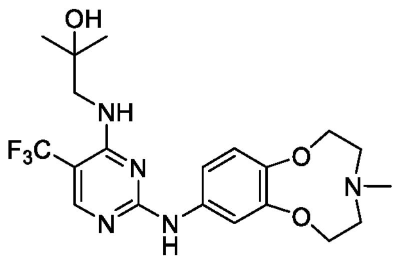

**8**

,

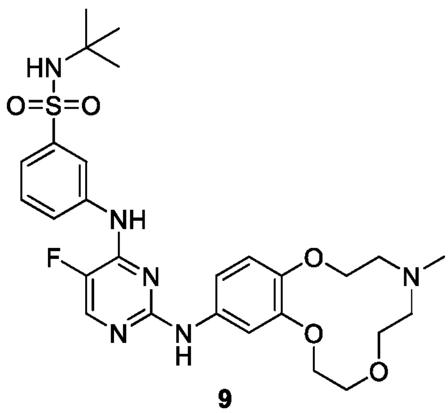

**9**

,

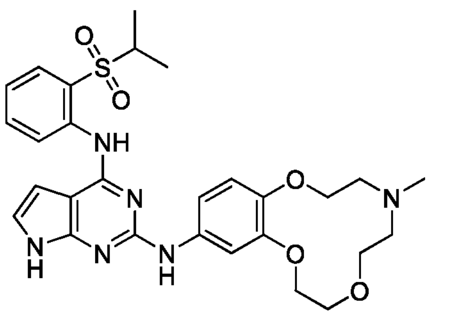

**10**

,

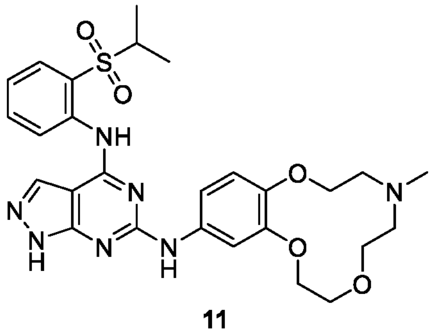
**11**
,

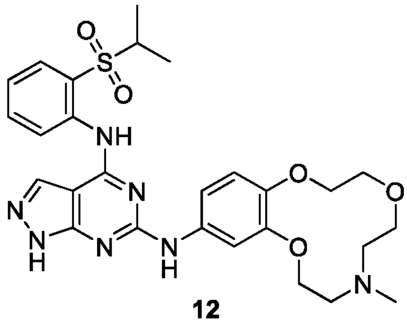
**12**
,

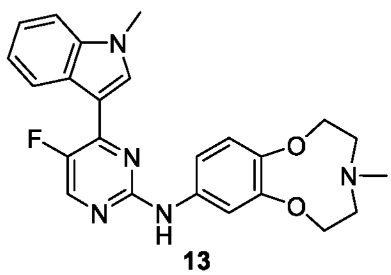
**13**
,

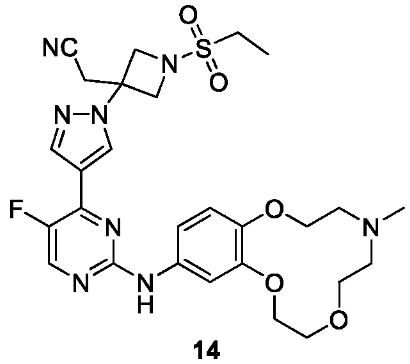
**14**
,

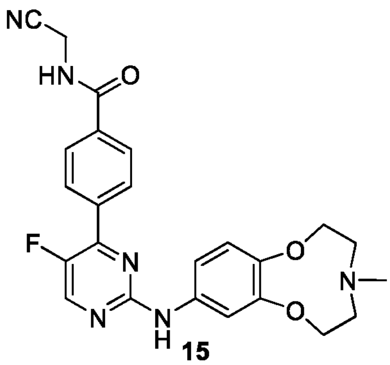
**15**
,

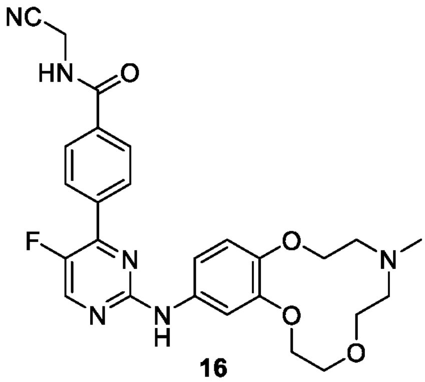
**16**
,

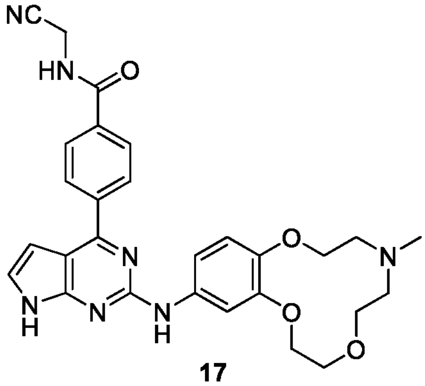
**17**
,

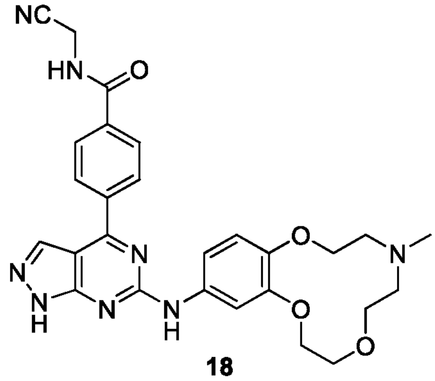
**18**
,

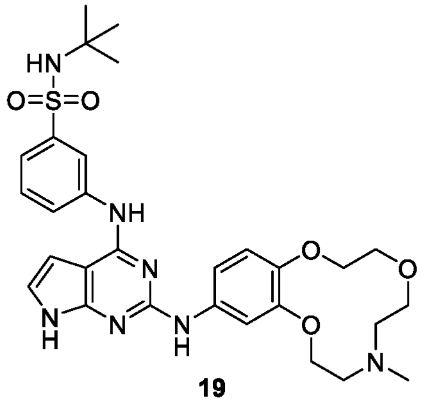

**19**

,

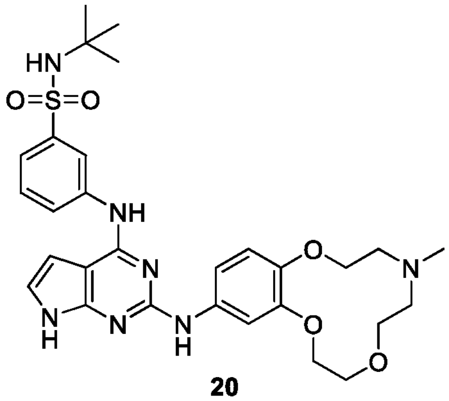

**20**

,

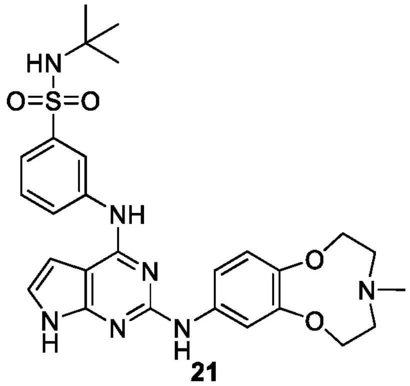

**21**

,

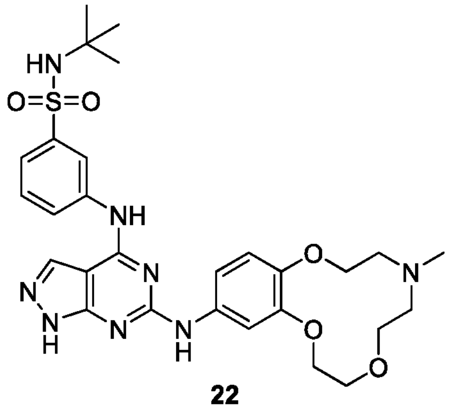

**22**

,

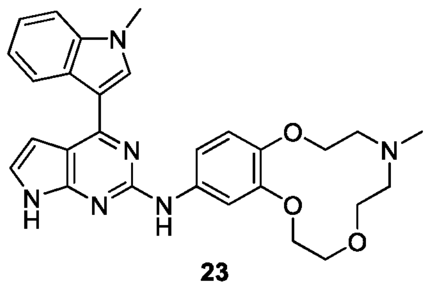

**23**

,

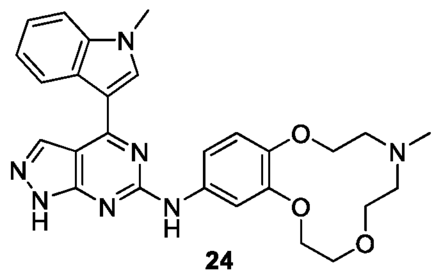

**24**

,

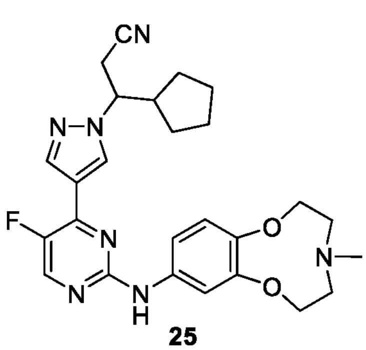

**25**

,

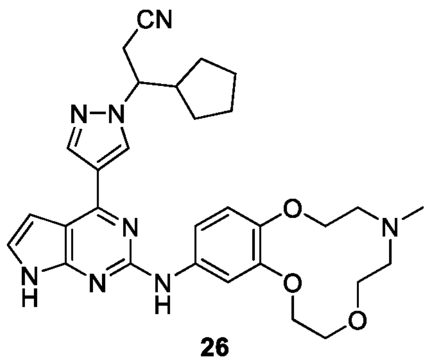

**26**

,

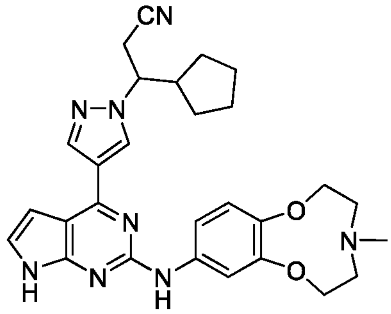

27

,

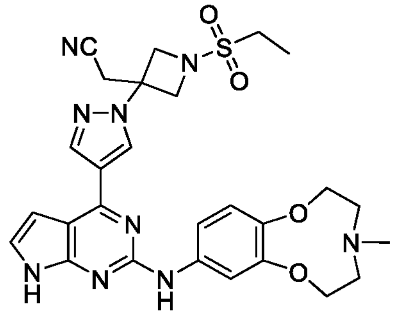

28

,

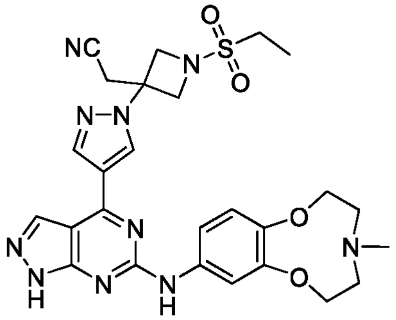

29

,

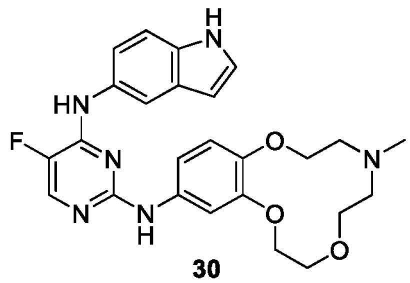

30

,

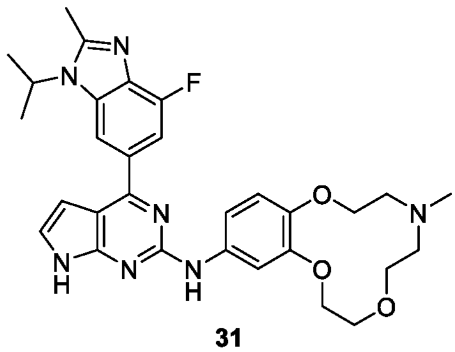

31

,

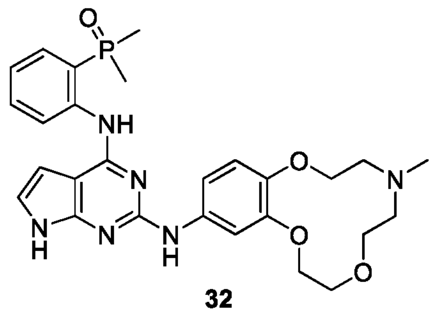

32

,

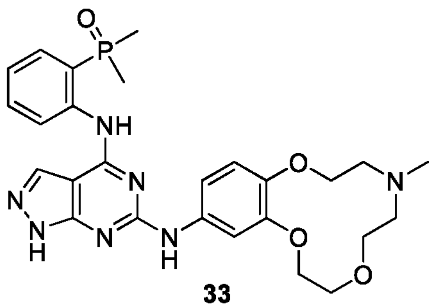

33

,

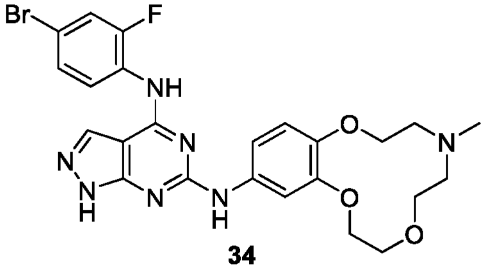

34

,

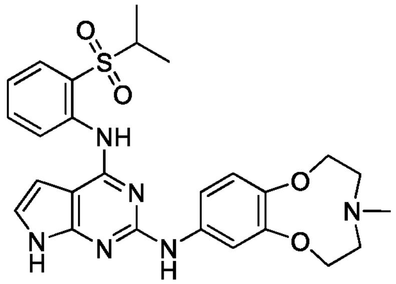

35

,

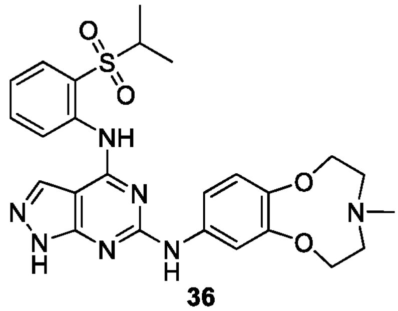

36

,

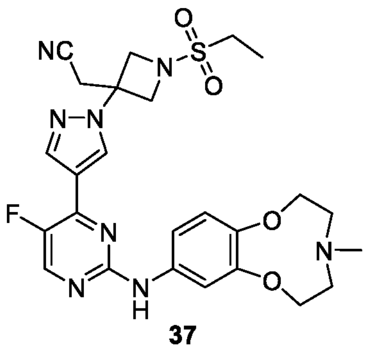

37

,

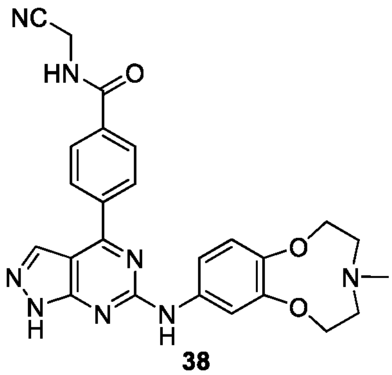

38

,

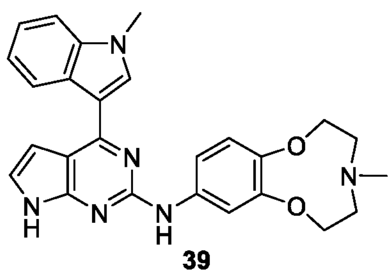

39

,

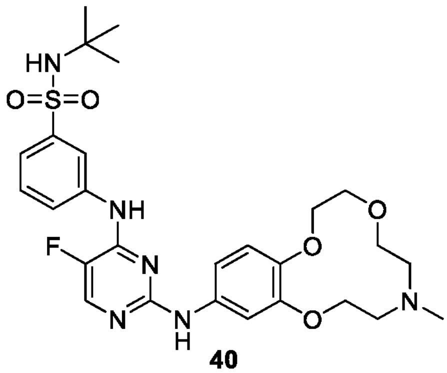

40

,

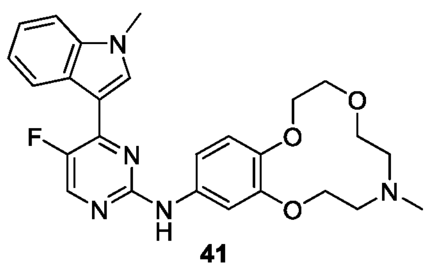

41

,

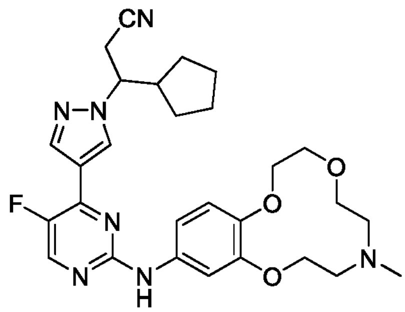

42

,

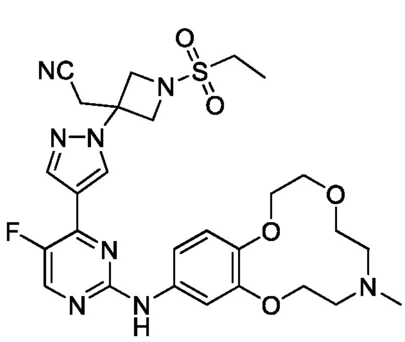

43 ,

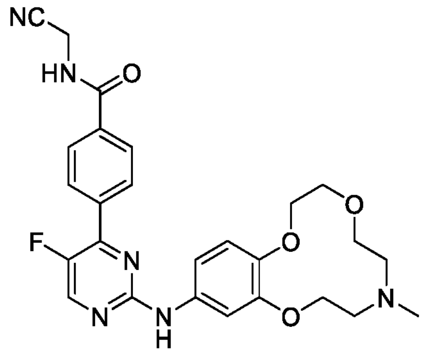

44 ,

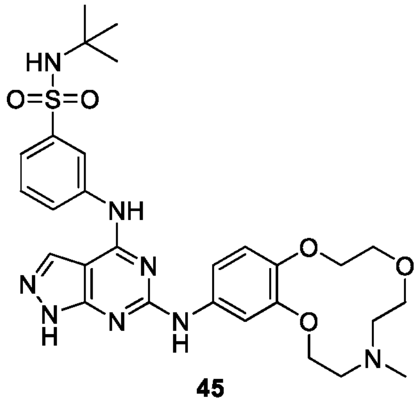

45 ,

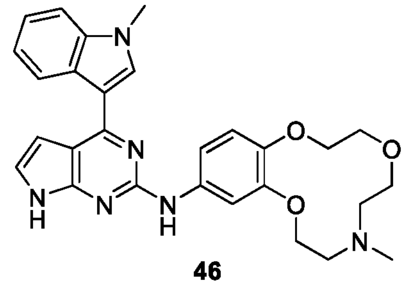

46 ,

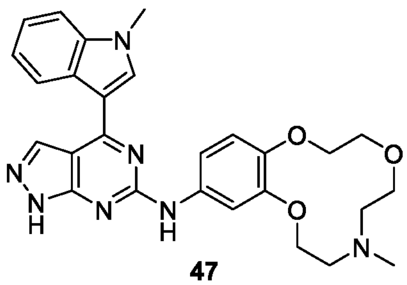

47 ,

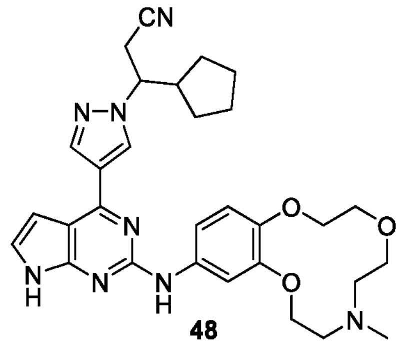

48 ,

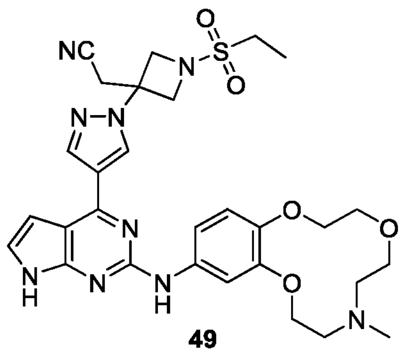

49 ,

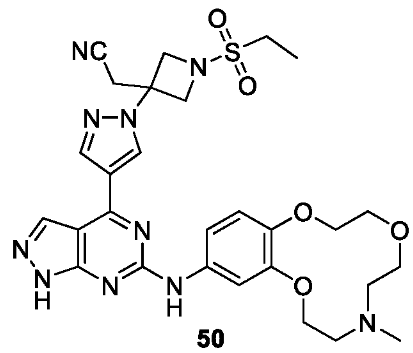

50 ,

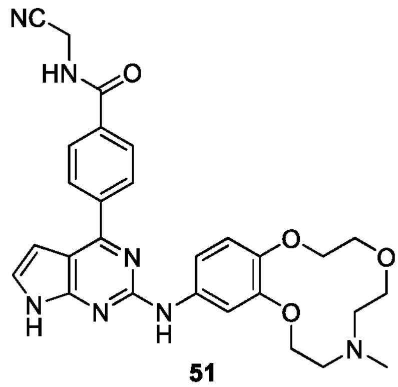

51

,

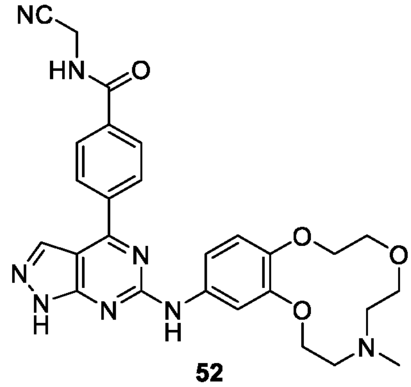

52

,

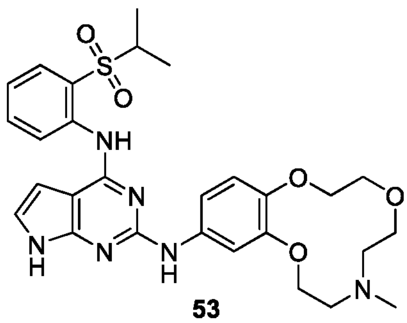

53

,

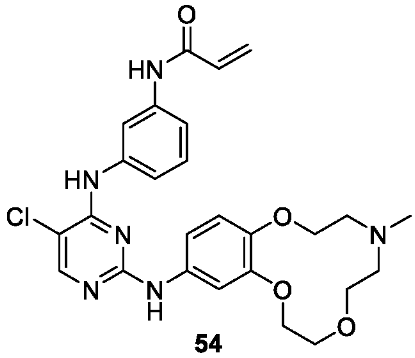

54

,

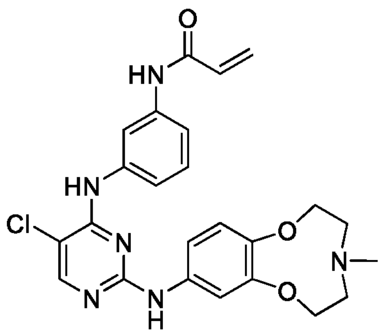

55

,

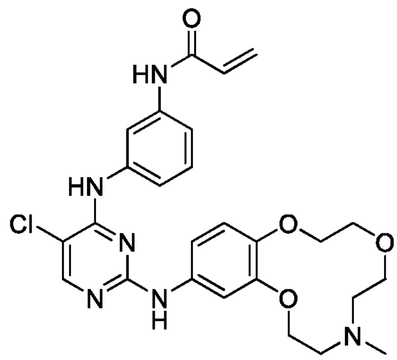

56

,

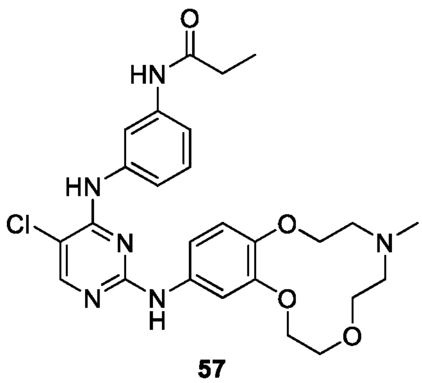

57

,

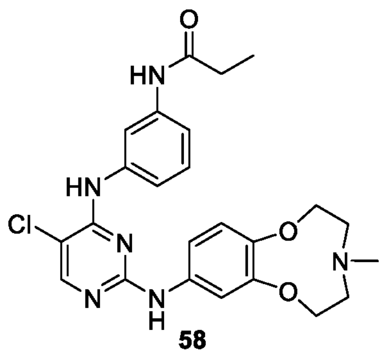

58

,

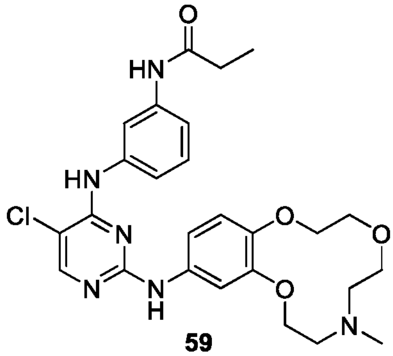

**59**

,

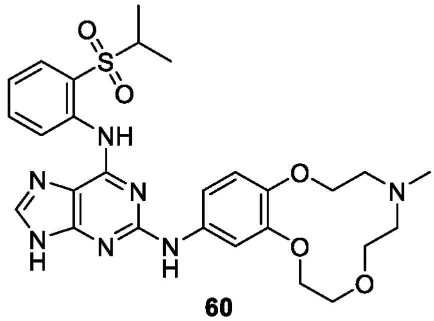

**60**

,

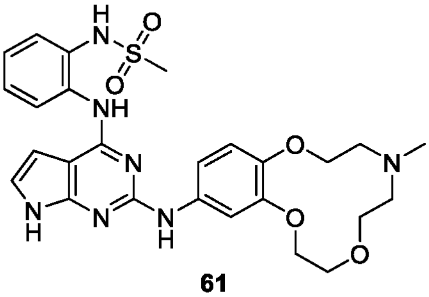

**61**

,

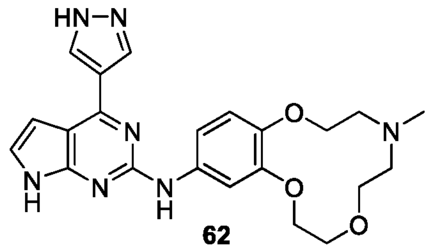

**62**

,

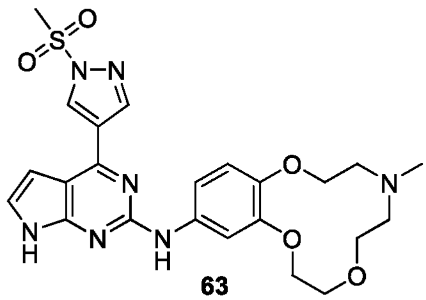

**63**

,

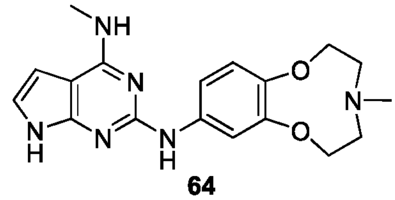

**64**

,

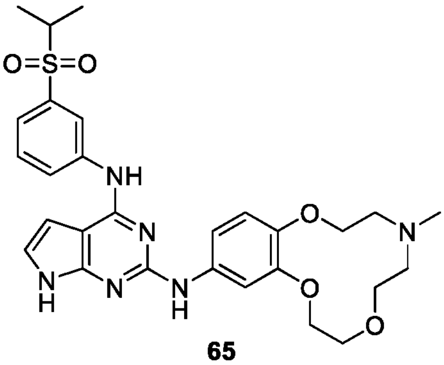

**65**

,

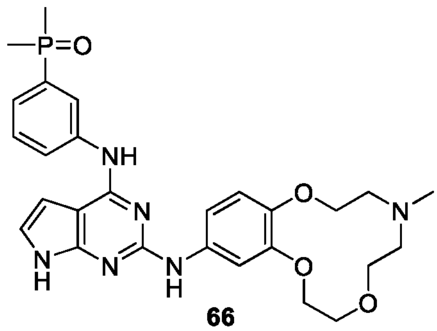

**66**

,

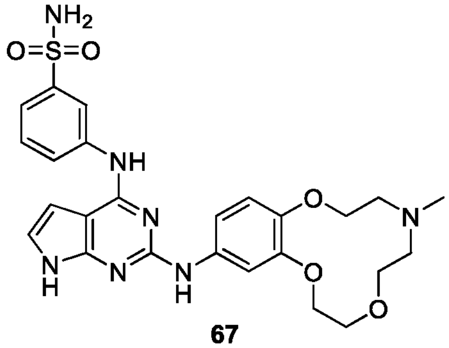

67

,

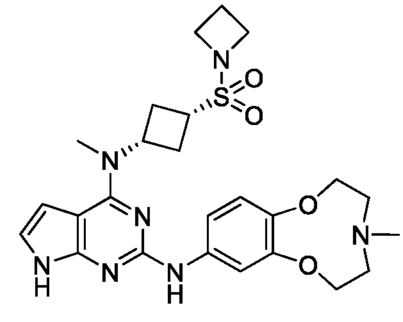

68

,

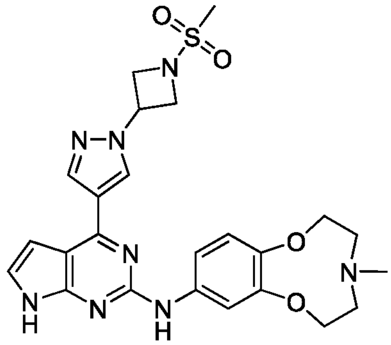

69

,

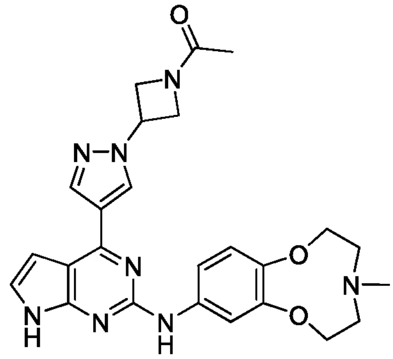

70

,

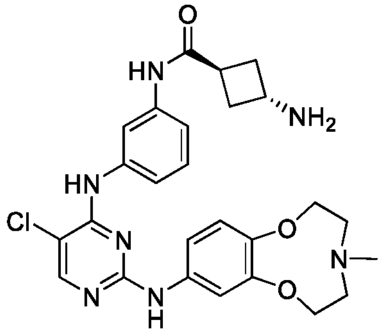

71

,

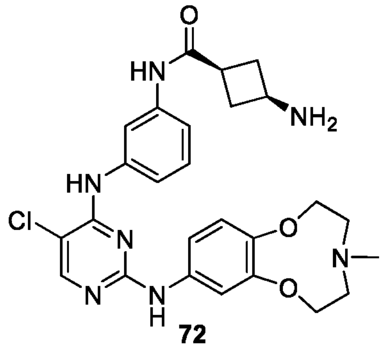

72

,

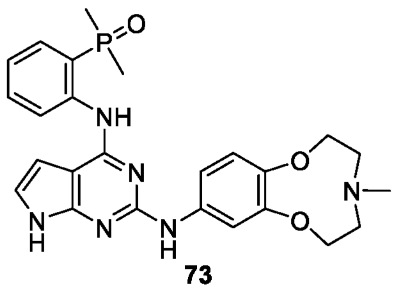

73

,

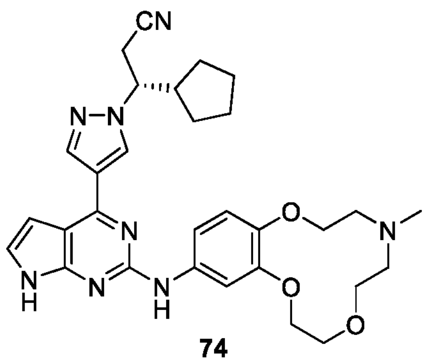

74

,

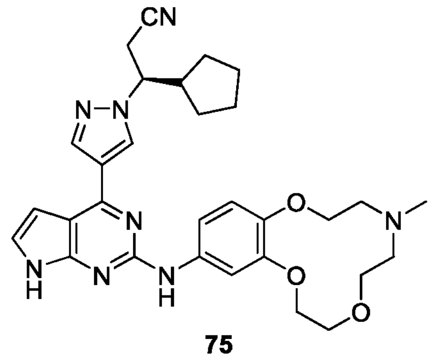

**75**

,

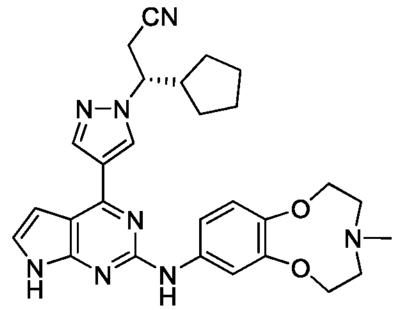

**76**

,

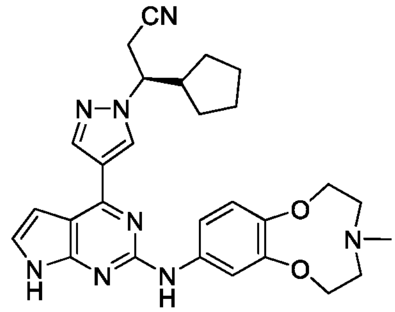

**77**

,

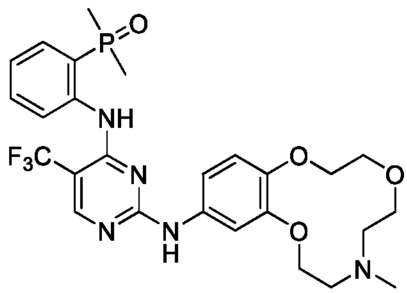

**78**

,

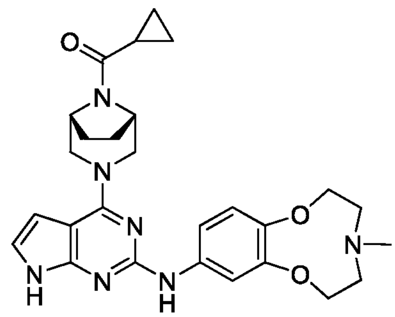

**79**

,

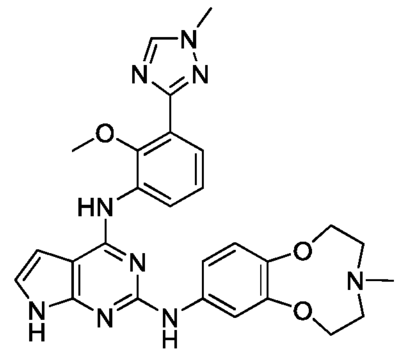

**80**

,

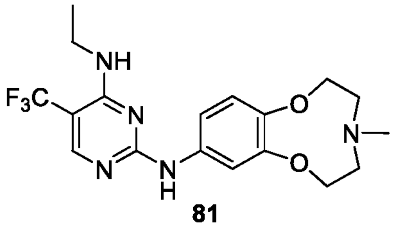

**81**

,

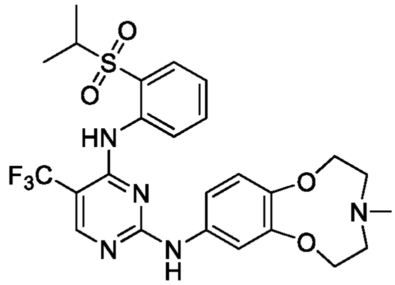

**82**

,

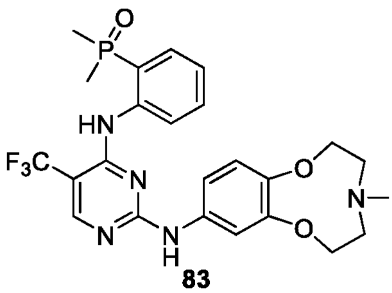
**83** ,

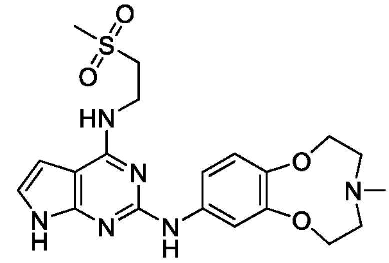
**84** ,

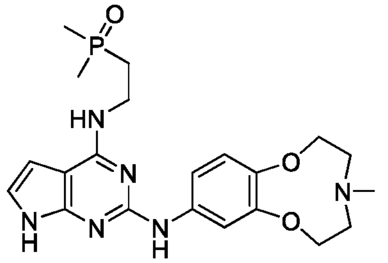
**85** ,

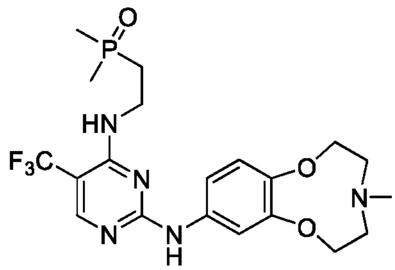
**86** ,

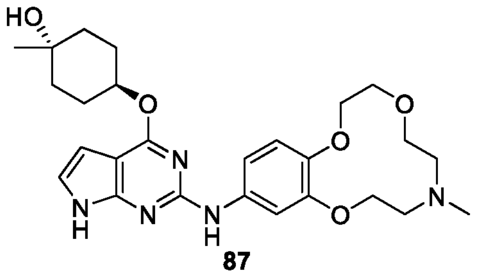
**87** ,

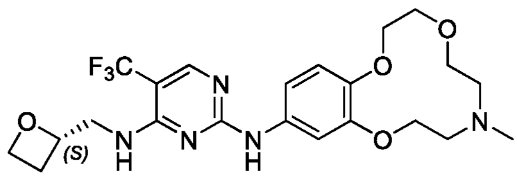
**88** ,

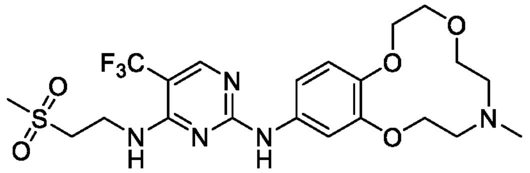
**89** ,

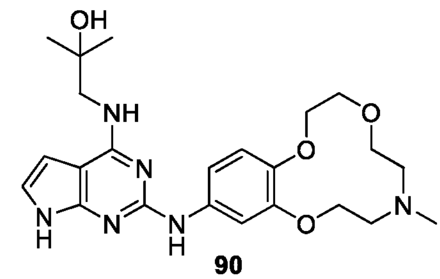
**90** ,

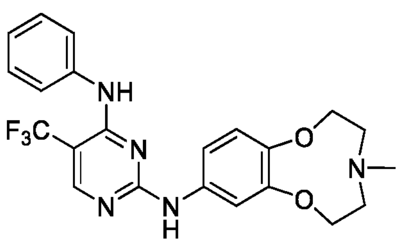
**91** ,

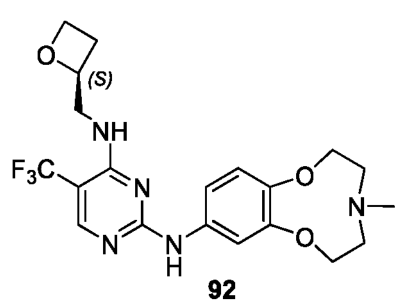
**92** ,

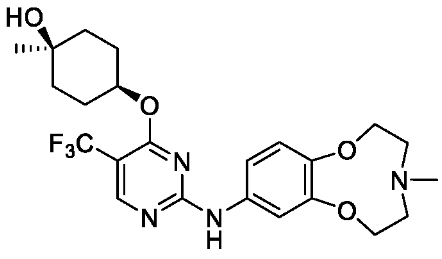

93

,

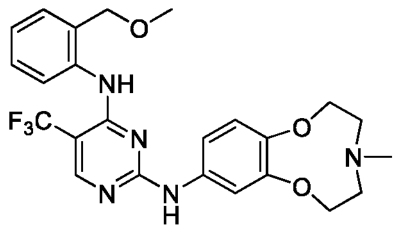

94

,

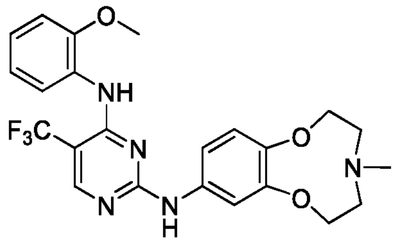

95

,

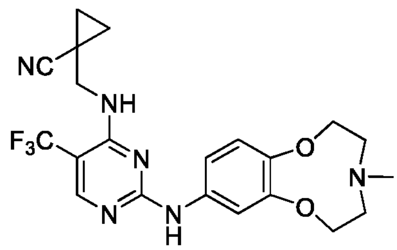

96

,

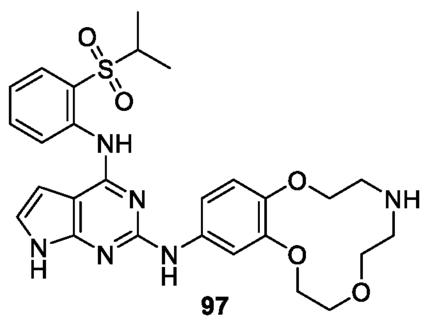

97

,

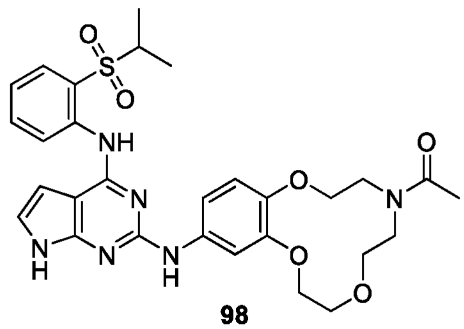

98

,

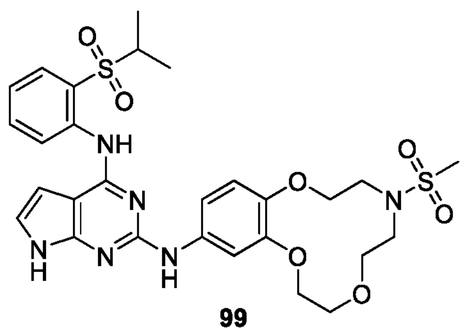

99

,

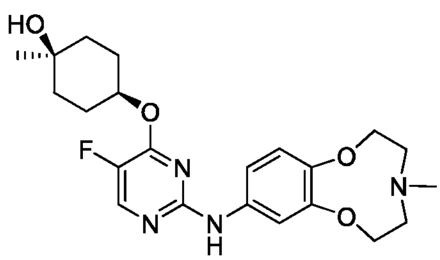

100

,

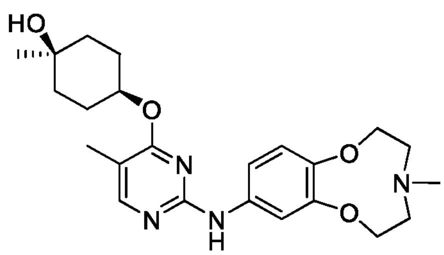

101

,

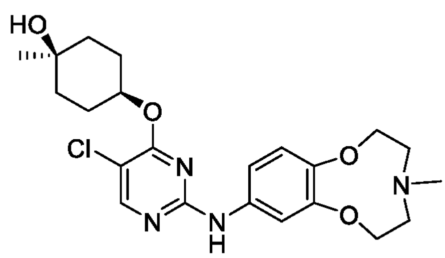

102

,

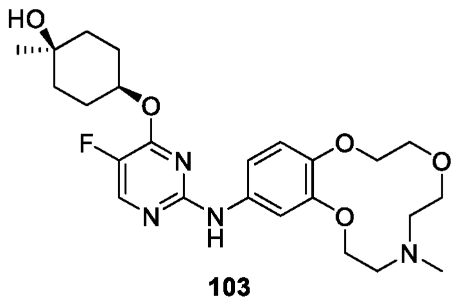

103

,

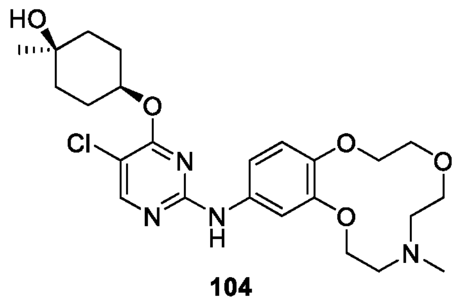

104

,

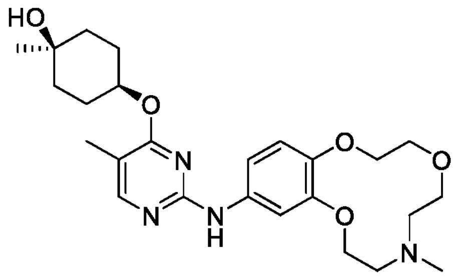

105

,

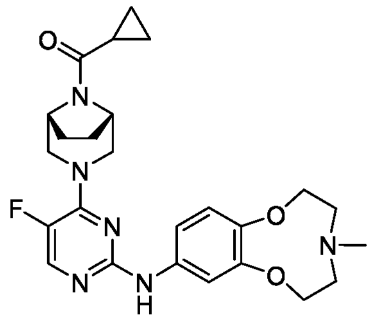

106

,

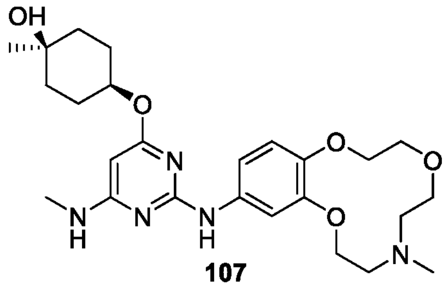

107

,

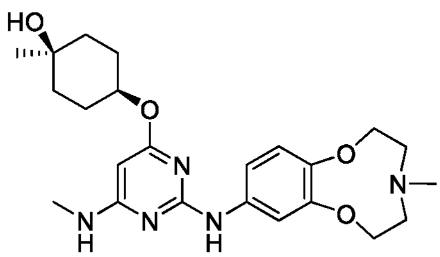

108

,

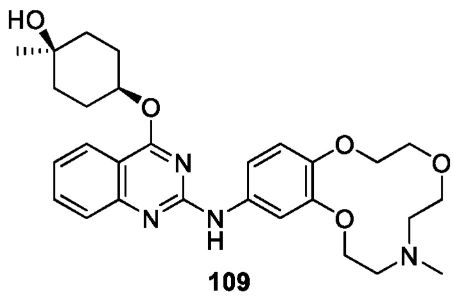

109

,

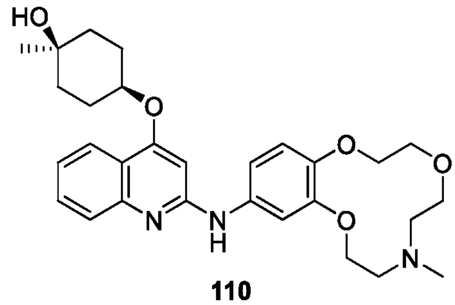

110

,

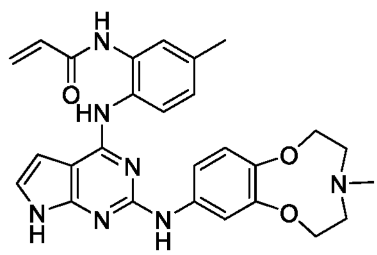

111

,

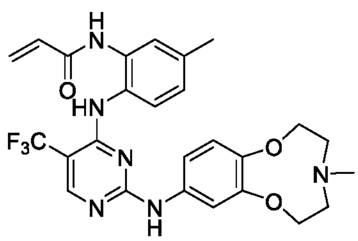

112

,

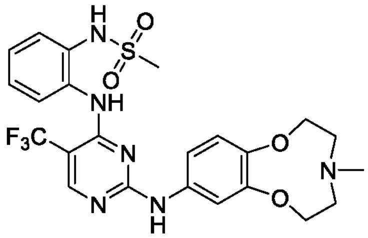

113 ,

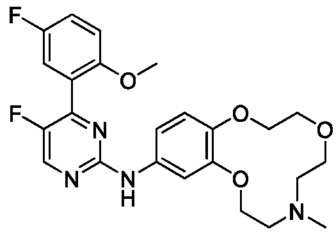

114 ,

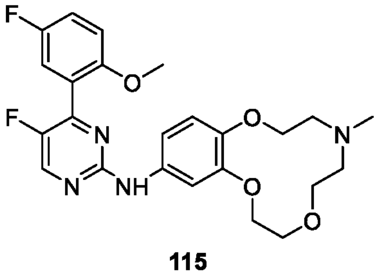

115 , and

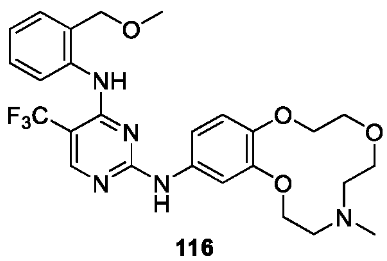

116 .

In another aspect, the present disclosure provides a pharmaceutical composition comprising the compound represented by formula (I) or a stereoisomer, tautomer or pharmaceutically acceptable salt thereof of the present disclosure, and optionally a pharmaceutically acceptable carrier. In some embodiments, the compound represented by formula (I) has the structures (II)-(IV) as described above. In some other embodiments, the compound represented by formula (I) is the specific compound described above.

In yet another aspect, the present disclosure provides use of the compound or the stereoisomer, tautomer or pharmaceutically acceptable salt thereof or the pharmaceutical composition thereof of the present disclosure described above in the manufacture of a medicament as a protein kinase inhibitor.

In yet another aspect, the present disclosure provides use of the compound or the stereoisomer, tautomer or pharmaceutically acceptable salt thereof or the pharmaceutical composition thereof of the present disclosure described above in the manufacture of a medicament for the treatment or prevention of a disease related to protein kinase. The disease related to protein kinase is a disease in which development or symptom of the disease is related to signal transmission, mediation, regulation or adjustment of the protein kinase. The protein kinase is selected from the group consisting of LRRK2, JAK1, JAK2, JAK3, EGFR and CDK9. Examples of the disease related to protein kinase include disease types of cancer, autoimmune diseases, metabolic diseases, inflammation, infection (bacteria, viruses, yeasts, fungi, etc.), central nervous system diseases, degenerative nervous system diseases, allergy/asthma, skin diseases, angiogenesis, neovascularization, angiogenesis and cardiovascular diseases.

In some embodiments, the disease related to protein kinase the present disclosure is selected from the group consisting of neurodegenerative diseases, autoimmune diseases and tumors. In some other preferred embodiments, the disease related to protein kinase of the present disclosure is selected from the group consisting of Parkinson's disease, asthma, dermatitis, non-small cell lung cancer, acute myeloid leukemia (AML) and liver cancer.

The compounds, compositions and methods described herein are useful for treating or preventing diseases or symptoms thereof, including transplant rejection (e.g., kidney, liver, heart, lung, pancreas (islet cells), bone marrow, cornea, small intestine, skin allograft or skin xenograft), graft versus host disease, osteoarthritis, rheumatoid arthritis, multiple sclerosis, diabetes, diabetic retinopathy, asthma, inflammatory intestinal diseases (Crohn's disease, ulcerative colitis), kidney diseases, cachexia, septic shock, lupus, diabetes, myasthenia gravis, psoriasis, dermatitis, eczema, seborrhea, Alzheimer's disease, Parkinson's disease, stem cell protection in chemotherapy, in vitro selection or purging during autologous or allogeneic bone marrow transplantation, leukemia (acute myeloid leukemia, chronic myeloid leukemia, acute lymphoblastic leukemia, etc.), cancer (breast cancer, lung cancer, colorectal cancer, ovarian cancer, prostate cancer, renal cancer, squamous cell carcinoma, prostate cancer, glioblastoma, melanoma, pancreatic cancer, Kaposi's sarcoma, etc.), eye diseases, retinopathy (for example, macular degeneration, diabetic retinopathy), keratopathy, glaucoma, bacterial infection, viral infection, fungal infection and heart disease, including but not limited to restenosis. In one embodiment, the composition and method described herein are useful for treating or preventing cancer, eye diseases, or retinopathy. In another embodiment, the composition and method described herein are useful for treating or preventing rheumatoid arthritis, transplant rejection, asthma or allergy, or symptoms thereof. In other embodiments, the composition and method described herein are useful for treating or preventing diseases or disease symptoms associated with hyperproliferative diseases or associated with angiogenesis.

In the present disclosure, "optionally substituted" means that the groups such as A and $R_1$ may or may not be substituted by a substituent, i.e., they are not limited to the situation where they are substituted by the listed substituents, but also include the situation where they are not substituted by the listed substituents. This expression is the same as the expression "R is a substituted or unsubstituted $C_{1-10}$ alkyl, $C_{3-6}$ cycloalkyl or heterocycloalkyl, phenyl, naphthyl, or indolyl, where the substituent is $C_{1-4}$ alkyl, $C_{1-4}$ alkoxy, cyano, hydroxyl, mercapto, amino, or halogen." However, the limitation by term substituted or unsubstituted does not narrowly refer to $C_{1-10}$ alkyl, but also applies to all the groups mentioned. In the present disclosure, when referring to "optionally substituted", the substituent can be deuterium, $C_{1-4}$ alkyl, $C_{1-10}$ alkoxy, $C_{3-6}$ cycloalkyl, 3-8 membered heterocyclyl, $C_{1-4}$ alkylphosphoryl, $C_{1-4}$ alkylsulfonyl, aminosulfonyl, $C_{1-4}$ alkylaminosulfonyl, cyano $C_{3-6}$ cycloalkyl, $C_{2-4}$ alkenylformyl, $C_{2-4}$ alkenylformamino, cyano $C_{1-4}$ alkylcarbamoyl, cyano $C_{1-4}$ alkyl, $C_{1-4}$ alkylformyl-3-8 membered heterocyclyl, $C_{3-6}$ cycloalkylformyl, 3-8 membered heterocyclyl-sulfonyl, $C_{1-4}$ alkylsulfonyl-3-8 membered heterocyclyl, amino $C_{3-6}$ cycloalkylformylamino, $C_{1-4}$ alkylphosphoramino, $C_{1-4}$ alkylsulfonamino, cyano, hydroxyl, oxo (=O), mercapto, amino, dimethylamino, hydroxylamino, $C_{2-4}$ alkenyl and halogen; wherein the aryl as a substituent can be further substituted by a substituent such as deuterium, $C_{1-4}$ alkyl, $C_{1-10}$ alkoxy, $C_{3-6}$ cycloalkyl, cyano, hydroxyl, mercapto, amino and halogen.

The term "alkyl" is used to denote a linear or branched saturated hydrocarbon group. For example, $C_{1-3}$ alkyl means a saturated hydrocarbon group containing 1-3 carbon atoms, $C_{1-4}$ alkyl means a saturated hydrocarbon group containing 1-4 carbon atoms, and $C_{1-10}$ alkyl means a saturated hydrocarbon group containing 1-10 carbon atoms, including a linear, branched or cyclic alkyl.

The term "alkoxy" means alkyl-O-. "$C_{1-6}$ alkoxy" is intended to include $C_1$, $C_2$, $C_3$, $C_4$, $C_5$, and $C_6$ alkoxy. Examples of alkoxy include, but are not limited to, methoxy, ethoxy, propoxy (such as n-propoxy and isopropoxy), and tert-butoxy. Similarly, "$C_{1-10}$ alkoxy" means -O-alkyl groups, and alkyl includes linear, branched and cyclic alkyl having 1-10 carbon atoms. Examples of alkoxy include methoxy, ethoxy, propoxy (such as n-propoxy, isopropoxy, and cyclopropoxy), and tert-butoxy. Preferred alkoxy herein is $C_{1-6}$ alkoxy, $C_{1-4}$ alkoxy.

The term "cycloalkyl" means non-aromatic carbocyclic groups, including cyclized alkyl groups. Cycloalkyl can include monocyclic, bicyclic or polycyclic ring systems. Examples of cycloalkyl include cyclopropyl, cyclobutyl, cyclopentyl, cyclohexyl, and cycloheptyl, and the $C_{3-8}$ cycloalkyl means a cycloalkyl group containing 3-8 carbon atoms. Preferred cycloalkyl herein is $C_{3-8}$ cycloalkyl, $C_{3-6}$ cycloalkyl.

The term "heterocycloalkyl" or "heterocyclyl" means a non-aromatic heterocycloalkyl in which one, two or three ring-forming carbon atoms are replaced by heteroatoms such as O, N and S atoms. The heterocycloalkyl preferably has 3, 4, 5, 6 or 7 ring-forming atoms. Preferred heterocycloalkyl herein is $C_{3-8}$ heterocycloalkyl.

"Aryl" refers to an aromatic carbocyclic group, including monocyclic, bicyclic, tricyclic or polycyclic aromatic hydrocarbon groups such as phenyl, naphthyl, anthryl, phenanthryl and the like. Aryl is preferably a monocyclic, bicyclic or tricyclic ring system having 6 to 14, or 6 to 12 ring members, where at least one ring in the system is aromatic and each ring in the system contains 3 to 7 ring members. "Substituted aryl" means that at least one hydrogen atom on the benzene ring of the aryl group is substituted with a non-hydrogen portion, and the substituent of the aryl may be halogen, $C_{1-10}$ alkoxy, -CN, -OH, -SH, $-NH_2$, or $C_{1-6}$ alkyl. Preferred aryl includes phenyl, biphenyl, indanyl, 1-naphthyl, 2-naphthyl and tetrahydronaphthyl. The term "aralkyl" or "arylalkyl" means an alkyl residue linked to an aromatic ring. Non-limiting examples include benzyl, phenylethyl, and the like. Fused aryl may be linked to another group at a suitable position on the cycloalkyl ring or aromatic ring.

The term "heteroaryl" means a stable 5-12 membered aromatic monocyclic or aromatic bicyclic or aromatic polycyclic heterocyclic ring, which is fully unsaturated, partially unsaturated, and which contains carbon atoms and 1, 2, 3 or 4 heteroatoms independently selected from the group consisting of N, O, and S. It includes 5-, 6-, or 7-membered aromatic monocyclic or 8-, 9-, 10-, 11-, 12-membered aromatic bicyclic or aromatic polycyclic heterocyclic rings. Preferably, any heterocyclic ring as defined above is fused with a benzene ring. Nitrogen and sulfur atoms may be optionally oxidized. Nitrogen atom is substituted or unsubstituted (i.e. N or NR, where R is H or another substituent if defined). A heterocycle may be linked to its side group at any heteroatom or carbon atom that results in a stable structure. If the resulting compound is stable, the heterocyclyl described herein may be substituted on carbon or nitrogen atoms. The nitrogen in the heterocycle may optionally be quaternized. Preferably, in the case that the total number of S and O atoms in the heterocycle exceeds 1, these heteroatoms are not adjacent to each other. Examples of heteroaryl include, but are not limited to, acridinyl, azetidinyl, azocinyl, benzimidazolyl, benzofuryl, benzothiofuryl, benzothienyl, benzoxazolyl, benzoxazolinyl, benzothiazolyl, benzotriazolyl, benzotetrazolyl, benzisoxazolyl, benzisothiazolyl, benzimidazolinyl, carbazolyl, 4aH-carbazolyl, carbolinyl, chromanyl, chromenyl, quinolinyl, decahydroquinolyl, dihydrofuro[2,3-b]tetrahydrofuryl, furyl, furazanyl, imidazolidinyl, imidazolinyl, imidazolyl, 1H-indazolyl, imidazopyridyl, dihydroindolyl, indolizinyl, indolyl, 3H-indolyl, isobenzofuryl, isochromanyl, isoindazolyl, isodihydroindolyl, isoindolyl, isoquinolinyl, isothiazolyl, isothiazolopyridyl, isoxazolyl, isoxazolopyridyl, methylenedioxyphenyl, morpholinyl, diazanaphthyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridyl, oxazolidinyl, perimidinyl, hydroxyindolyl, pyrimidinyl, phenanthridinyl, phenanthrolinyl, phenazinyl, phenothiazinyl, phenoxathiyl, phenoxazinyl, phthalazinyl, piperazinyl, piperidinyl, piperidinonyl, 4-piperidinonyl, piperonyl, pteridinyl, purinyl,

pyranyl, pyrazinyl, pyrazolidinyl, pyrazolinyl, pyrazolopyridyl, pyrazolyl, pyridazinyl, pyridoxazolyl, pyridimidazolyl, pyridothiazolyl, pyridyl, pyrimidinyl, pyrrolidinyl, pyrrolinyl, 2-pyrrolidonyl, 2H-pyrrolyl, pyrrolyl, quinazolinyl, quinolinyl, 4H-quinazinyl, quinoxalinyl, quinuclidinyl, tetrazolyl, tetrahydrofuryl, tetrahydroisoquinolyl, tetrahydroquinolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthryl, thiazolyl, thienyl, thiazolopyridyl, thienothiazolyl, thienooxazolyl, thienoimidazolyl, thienyl, triazinyl, 1,2,3-triazolyl, 1,2,4-triazolyl, 1,2,5-triazolyl, 1,3,4-triazolyl, xanthenyl, quinolinyl, isoquinolyl, phthalazinyl, quinazolinyl, indolyl, 1*H*-indazolyl, benzimidazolyl, 1,2,3,4-tetrahydroquinolinyl, 1,2,3,4-tetrahydroisoquinolinyl, 5,6,7,8-tetrahydroquinolinyl, 2,3-dihydrobenzofuryl, 1,2,3,4-tetrahydroquinoxalinyl and 1,2,3,4-tetrahydroquinazolinyl. The term "heteroaryl" may also include a biaryl structure formed by an "aryl" as defined above with a monocyclic "heteroaryl", for example, but not limited to, "-phenyl-pyridyl-", "-phenyl-pyrimidinyl", "-pyridyl-phenyl", "-pyridyl-pyrimidinyl", and "-pyrimidinyl-phenyl-".

The term "$C_{1-4}$ alkyl phosphoryl" means $C_{1-4}$ alkyl substituted phosphoryl, and the two alkyl on the phosphoryl can be the same or different.

The term "$C_{1-4}$ alkyl sulfonyl" means $C_{1-4}$ alkyl substituted sulfonyl.

The term "halogen" includes fluorine, chlorine, bromine and iodine.

"Bridged ring group" means a polycyclic group with 5 to 18 members, preferably 5 to 14 members, containing two or more cyclic structures and sharing two carbon atoms that are not directly connected to each other. 6 to 14 membered bridged ring group is more preferred, and 7 to 10 membered bridged ring group is still more preferred. Depending on the number of constituent rings, it can be divided into bicyclic, tricyclic, pyridone or polycyclic bridged heterocyclic groups, preferably bicyclic, tricyclic or pyridone, more preferably bicyclic or tricyclic. The bridging ring group herein allows one, two or three ring atoms to be heteroatoms selected from the group consisting of nitrogen, oxygen or $S(O)_n$ (where n is selected from the group consisting of 0, 1 and 2).

In the present disclosure, the group or substituent is linked to the core at the rightmost end in the customary order from left to right, unless otherwise specified. For example, for the cyano $C_{1-4}$ alkyl carbamoyl, the position where it as a substituent is linked to the core of the compound is the rightmost "formyl".

The term "pharmaceutically acceptable salt" used herein means certain salts of the compound of the present disclosure that can maintain the original biological activity and are suitable for medical use. The pharmaceutically acceptable salt of the compound represented by formula (I) may be a salt formed through reacting carboxyl or amino (mainly amino) with a suitable base or acid, for example, a salt formed with a suitable base (including metal salts and ammonium salts), or a salt formed with suitable acids. In the present disclosure, the compound preferably forms a salt with a suitable acid, the acid is selected from the group consisting of hydrochloric acid, hydrobromic acid, hydrofluoric acid, sulfuric acid, phosphoric acid, nitric acid, formic acid, acetic acid, propionic acid, oxalic acid, malonic acid, succinic acid, fumaric acid, maleic acid, lactic acid, malic acid, tartaric acid, citric acid, picric acid, methanesulfonic acid, ethanesulfonic acid, toluenesulfonic acid, benzenesulfonic acid, naphthalenesulfonic acid, trifluoroacetic acid and aspartic acid, preferably methanesulfonic acid or hydrochloric acid.

The term "treatment" used herein includes any effects that result in improvement of a condition, disease, disorder and the like, such as alleviation, reduction, regulation, amelioration or elimination, or amelioration of the symptoms.

The phrase "pharmaceutically acceptable carrier" used herein means a pharmaceutical substance, composition or vehicle, such as a liquid or solid filler, diluent, excipient, manufacturing auxiliary (e.g. a lubricant, talc, magnesium stearate, calcium stearate, zinc stearate or stearic acid) or a solvent-encapsulating substance, which is involved in carrying or transporting an subject compound from one organ or body part to another organ or body part. Each carrier must be "acceptable" in terms of being compatible with the other ingredients of the formulation and harmless to the patient.

The term "pharmaceutical composition" means a composition comprising the compound of the present disclosure and at least one other pharmaceutical carrier. "Pharmaceutical carrier" means the vehicle generally accepted in the art for delivering bioactive agents to animals (specifically mammals), including (i.e.) adjuvants, excipients or vehicles, such as diluents, preservatives, fillers, flow regulators, disintegrants, wetting agents, emulsifiers, suspending agents, sweeteners, flavoring agents, fragrances, antibacterial agents, antifungal agents, lubricants and dispersants, depending on the mode of administration and the nature of dosage forms.

The term "Brettphos Pd G3" means methanesulfonic acid(2-dicyclohexylphosphine) -3,6-dimethoxy-2',4',6'-triisopropyl-1,1'-biphenyl)(2'-amino-1,1'-biphenyl-2-yl)palladium(II); "TLC" means thin layer chromatography;

In order to better illustrate the technical means adopted by the present disclosure and effects thereof, the present disclosure will be further illustrated in conjunction with the following non-limiting examples. Examples of the present disclosure, including descriptions provided in the examples, are intended to illustrate embodiments of the present disclosure, and are not intended to limit the scope of any claims. According to the present disclosure, those skilled in the art will understand that many changes can be made to the disclosed specific embodiments and still obtain the same or similar results without departing from the spirit and scope of the present disclosure.

All materials/reagents are obtained from commercial suppliers and can be used without further purification, unless otherwise specified. The structures of the compounds in the following examples were characterized and confirmed by nuclear magnetic resonance (NMR) and/or liquid chromatography-mass spectrometry (LC_MS).

$^1H$ NMR spectrum was recorded by Bruker Avance 400 MHz spectrometer at room temperature, and the measuring solvent was deuterated dimethyl sulfoxide (DMSO-d6), deuterated chloroform ($CDCl_3$), deuterated methanol ($CD_3OD$) or deuterated water ($D_2O$). The chemical shift value ($\delta$) was in ppm, tetramethylsilane (TMS) or residual solvent peak was used as internal standard, and the coupling constant (J) was in Hz. The abbreviations of the multiplicity of the peak types in the $^1H$ NMR spectrum are as follows: s (single peak), d (double peak), t (triple peak), q (quadruple peak), qn (quintuple peak), m (multiple peak), and br (broad peak).

The instrument used in liquid chromatography-mass spectrometry (LC_MS) was Shimadzu LCMS-2020, and the instrument used in preparative high performance liquid chromatography (Prep-HPLC) was Bonna-Agela FLEXA FL-H100G. The thin-layer chromatography silica gel plate used in thin-layer chromatography (TLC) was the Yantai Huanghai HSGF254 thin-layer chromatography silica gel plate. The specification for reaction monitoring was 2.5×8 cm, the coating thickness was 0.2±0.03 mm, the specification for separation and purification was20×20 cm, and its coating thickness was 0.4-0.5 mm. In silica gel column chromatography, Qingdao marine silica gel with 100-200 mesh or 200-300 mesh was used as carrier.

All professional and scientific terms used herein have the same meanings as those familiar to those skilled in the art, unless otherwise defined. In addition, any method and material similar to or equal to the recorded content can be applied to the method of the present invention.

**Synthetic example**

**Fragment Amine-1**

**Implementation method**

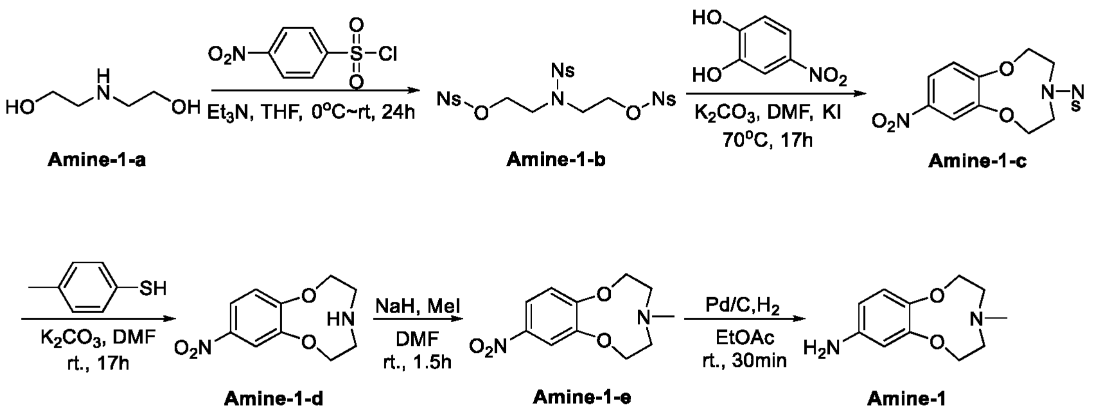


Step 1: synthesis of intermediate **Amine-1-b** (((4-nitrophenyl)sulfonyl)azadiyl)bis(ethan-2,1-diyl)bis(4-nitrobenzenesulfonate)

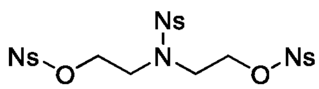

**Amine-1-b**

To a solution of diethanolamine (1.0 g, 9.51 mmol) and triethylamine (3.4 g, 33.28 mmol) in tetrahydrofuran (5 ml) was slowly added dropwise a solution of 4-nitrobenzenesulfonyl chloride (7.0 g, 31.38 mmol) in tetrahydrofuran (10 ml) at 0 °C. The resulting mixture was heated to room temperature and stirred for 16 hours. The completion of the reaction was monitored by TLC. The reaction solution was concentrated, and the residue was purified by recrystallization in methanol (30 ml) to obtain (((4-nitrophenyl)sulfonyl)aminodiyl) bis(ethan-2,1-diol)bis(4-nitrobenzenesulfonate) as a white solid (5.2 g, 83%).

$^1H$ NMR (400 MHz, DMSO-d6): δ 8.46 (d, *J* = 8.8Hz, 4H), 8.34 (d, *J* = 8.8Hz, 2H), 8.13 (d, *J* = 8.8Hz, 4H), 8.04 (d, *J* = 8.8Hz, 2H), 4.19 (t, *J* = 5.2Hz, 4H), 3.50 (t, *J* = 4.8Hz, 4H).

Step 2: synthesis of intermediate **Amine-1-c** 9-nitro-4-((4-nitrophenyl)sulfonyl)-3,4,5,6-tetrahydro-2*H*-benzo[*b*][1,4,7] dioxazoline

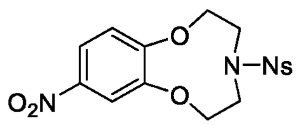

**Amine-1-c**

A solution of (((4-nitrophenyl)sulfonyl)azadiyl)bis(ethan-2,1-diyl)bis(4-nitrophenylsulfonate) (5.2 g, 7.9 mmol), 4-nitrocatechol (1.1 g, 7.2 mmol), potassium carbonate (2.1 g, 15.1 mmol) and potassium iodide (112 mg, 0.7 mmol) in *N,N*-dimethylformamide (20 ml) was reacted at 70°C for 12 hours. The completion of the reaction was monitored by TLC. The mixture was partitioned in ethyl acetate (150 ml) and water (200 ml). The organic phase was collected, washed with saturated sodium chloride solution (100 ml), dried over anhydrous sodium sulfate, and concentrated under reduced pressure. The obtained crude product was purified by silica gel column chromatography (an eluent of 33% dichloromethane in n-hexane solution) to obtain 9-nitro-4-((4-nitrophenyl)sulfonyl)-3,4,5,6-tetrahydro-2*H*-benzo[*b*][1,4,7]dioxazoline as a white solid (556 mg, 15%).

$^1$H NMR (400 MHz, DMSO-d6): δ 8.35 (d, *J* = 8.8Hz, 2H), 8.01-8.04 (m, 2H), 7.91 (d, *J* = 2.8Hz, 1H), 7.84-7.87 (m, 1H), 7.14 (d, *J* = 9.2Hz, 1H), 4.67 (t, *J* = 4.6Hz, 2H), 4.41 (t, *J* = 4.6Hz, 2H), 3.52-3.56 (m, 4H).

Step 3: synthesis of intermediate **Amine-1-d** 9-nitro-3,4,5,6-tetrahydro-2*H*-benzo[*b*][1,4,7]dioxazoline

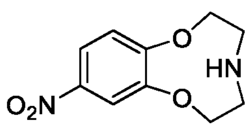

**Amine-1-d**

To a solution of 9-nitro-4-((4-nitrophenyl)sulfonyl)-3,4,5,6-tetrahydro-2*H*-benzo[*b*][1,4,7]dioxazoline (556.0 mg, 1.36 mmol) in *N,N*-dimethylformamide (10 ml) were added 4-methylthiophenol (203.0 mg, 1.63 mmol) and potassium carbonate (564.0 mg, 4.08 mmol). The resulting mixture was stirred at room temperature for 14 hours. The completion of the reaction was monitored by TLC. The reaction mixture was partitioned in ethyl acetate (100 ml) and water (100 ml). The organic phase was collected, washed with saturated sodium chloride solution (100 ml), dried over anhydrous sodium sulfate, and concentrated under reduced pressure. The obtained crude product was purified by silica gel column chromatography (eluted with 20%-33% ethyl acetate solution in n-hexane) to obtain 9-nitro-3,4,5,6-tetrahydro-2*H*-benzo[*b*][1,4,7]dioxazoline as a yellow solid (267 mg, 87%).

LC_MS: (ES$^+$): m/z 225.15 [M+H]$^+$.

$^1$H NMR (400 MHz, $CDCl_3$): δ 7.74-7.77 (m, 1H), 7.63 (d, *J* = 2.8Hz, 1H), 6.62 (d, *J* = 8.8Hz, 1H), 4.24 (t, *J* = 4.4Hz, 2H), 3.90-3.94 (m, 2H), 3.57-3.62 (m, 4H), 3.57-3.62 (m, 4H).

Step 4: synthesis of intermediate **Amine-1-e** 4-methyl-9-nitro-3,4,5,6-tetrahydro-2*H*-benzo[*b*][1,4,7]dioxazoline

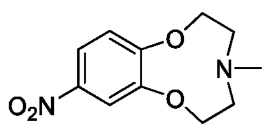

**Amine-1-e**

To a solution of 9-nitro-3,4,5,6-tetrahydro-2*H*-benzo[*b*][1,4,7]dioxazoline (600 mg, 2.68 mmol) in *N,N*-dimethylformamide (5 ml) were added sodium hydride (60%, 118 mg, 2.95 mmol) and iodomethane (457 mg, 3.22 mmol) at 0°C.

The resulting mixture was reacted at 0°C for 30 minutes, then warmed to room temperature and stirred for 3 hours. The completion of the reaction was monitored by TLC. The reaction mixture was partitioned in ethyl acetate (50 ml) and water (50 ml). The organic phase was collected, washed with saturated sodium chloride solution (30 ml×2), dried over anhydrous sodium sulfate, and concentrated under reduced pressure. The obtained crude product was purified by silica gel column chromatography (eluted with 15%-20% ethyl acetate solution in n-hexane) to obtain 4-methyl-9-nitro-3,4,5,6-tetrahydro-2*H*-benzo[*b*][1,4,7]dioxazoline as a yellow solid (583 mg, 91%).

LC_MS: (ES$^+$): m/z 239.30 [M+H]$^+$.

$^1$H NMR (400 MHz, $CDCl_3$): δ 7.78-7.81 (m, 1H), 7.67 (d, *J* = 2.4Hz, 1H), 6.59 (d, *J* = 8.8Hz, 1H), 4.21 (t, *J* = 4.4Hz, 2H), 3.56-3.63 (m, 6H), 3.36 (s, 3H).

Step 5: synthesis of intermediate **Amine-1** 4-methyl-3,4,5,6-tetrahydro-2*H*-benzo[*b*][1,4,7]dioxazoline-9-amine

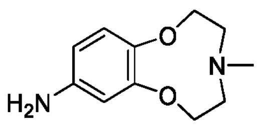

**Amine-1**

A solution of 4-methyl-9-nitro-3,4,5,6-tetrahydro-2*H*-benzo[*b*][1,4,7]dioxazoline (583 mg, 2.45 mmol) and Pd/C(10%, 60 mg) in methanol (10 ml) and ethyl acetate (10 ml) was reacted for 2 hours under hydrogen atmosphere (hydrogen balloon) at room temperature. The completion of the reaction was monitored by TLC. Pd/C was removed by filtration, the filter cake was washed with methanol (10 ml×2), and the combined filtrates were concentrated under reduced pressure to obtain crude product 4-methyl-3,4,5,6-tetrahydro-2*H*-benzo[*b*][1,4,7] dioxazolin-9-amine as a purple oily (497 mg, 97%), which can be used in the next step without further purification.

LC_MS: (ES$^+$): m/z 208.85 [M+H]$^+$.

$^1$H NMR (400 MHz, $CDCl_3$): δ 6.53-6.56 (m, 1H), 6.23-6.25 (m, 2H), 4.19 (t, *J* = 4.4Hz, 2H), 3.57 (t, *J* = 5.8Hz, 2H), 3.30-3.36 (m, 9H).

**Fragment Amine-2**

**Implementation Method**

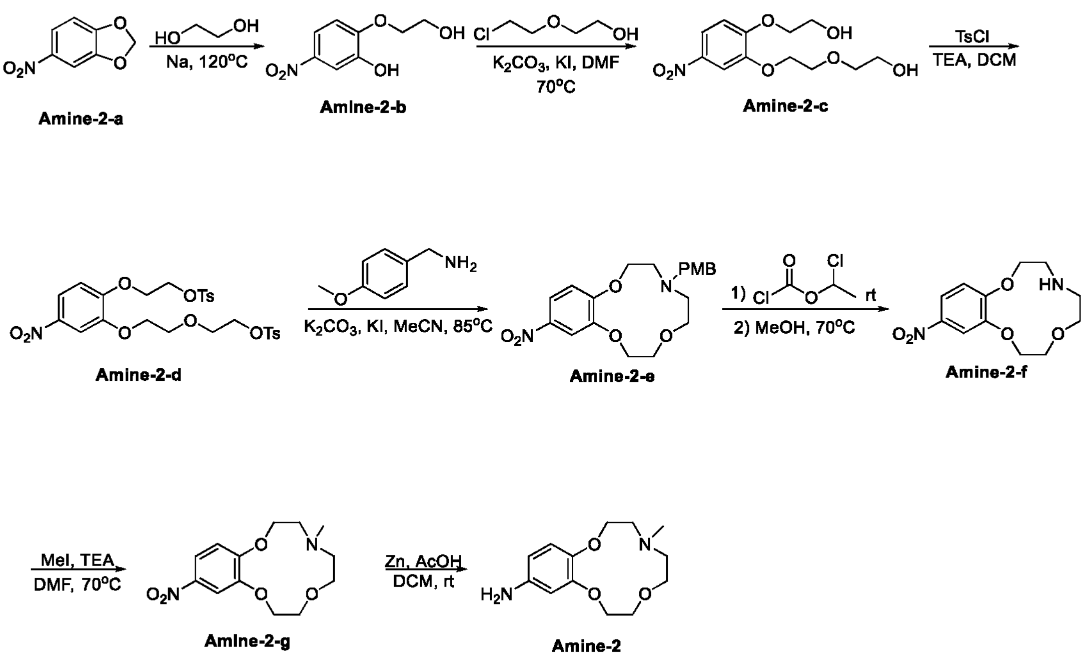


Amine-2-a, Amine-2-b, Amine-2-c, Amine-2-d, Amine-2-e, Amine-2-f, Amine-2-g, Amine-2

**Implementation Method**

Step 1: synthesis of intermediate **amine-2-b** 2-(2-hydroxyethoxy)-5-nitrophenol

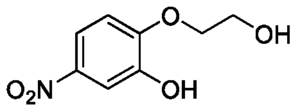

**Amine-2-b**

To a solution of metallic sodium (551 mg, 24 mmol) in ethylene glycol (10 ml) was added 5-nitrobenzo[d][1,3] dioxazole (2 g, 12 mmol) at 0°C. The resulting mixture was stirred at 120°C for 3 hours. The completion of the reaction was monitored by TLC. The reaction mixture was cooled to room temperature, acidified to pH=3-4 with 2N hydrochloric acid, the suspension was filtered, the solid was collected, washed with water and then dried under reduced pressure to obtain 2-(2-hydroxyethoxy)-5-nitrophenol as a brown solid (2.1 g, 88%).

LC_MS: (ES+): m/z 199.9 $[M+H]^+$.

$^1H$ NMR (400 MHz, DMSO-*d*6): δ 9.86 (s, 1H), 7.75-7.72 (m, 1H), 7.62-7.61 (m, 1H), 7.14-7.12 (m, 1H), 4.90 (s, 1H), 4.13-4.11 (m, 2H), 3.78-3.76 (m, 2H).

Step 2: synthesis of intermediate **Amine-2-c** 2-(2-(2-(2-hydroxyethoxy)-5-nitrophenoxy)ethoxy)ethanol

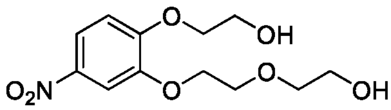

**Amine-2-c**

A solution of 2-(2-hydroxyethoxy)-5-nitrophenol (2.15 g, 10.75 mmol), 2-(2-chloroethoxy)ethan-1-ol (2 g, 16.13 mmol), potassium iodide (1.8 g, 10.75 mmol) and potassium carbonate (4.45 g, 32.25 mmol) in *N,N*-dimethylformamide (20 ml) was stirred at 70°C for 2 days. The completion of the reaction was monitored by TLC. The reaction mixture was cooled to room temperature and partitioned in water (200 ml) and ethyl acetate (100 ml). The organic layer was collected and the aqueous phase was extracted with ethyl acetate (50 ml). The organic layers were combined, washed with saturated sodium chloride solution (100 ml), dried over anhydrous sodium sulfate, and concentrated under reduced pressure to obtain 2-(2-(2-(2-hydroxyethoxy)-5-nitrophenoxy)ethoxy)ethanol as a orange oil (2.1 g, 67%), which was used in the next step without further purification.

LC_MS: (ES+): m/z 288.2 $[M+H]^+$.

$^1HNMR$ (400 MHz, $CDCl_3$): δ 7.95-7.92 (m, 1H), 7.77-7.76 (m, 1H), 6.94-6.92 (m, 1H), 4.24-4.22(m, 2H), 4.16-4.14 (m, 2H), 4.04-4.01 (m, 2H), 3.96-4.00 (m, 2H), 3.84-3.76 (m, 4H).

Step 3: synthesis of intermediate **Amine-2-d** 4-(4-nitro-2-(2-(2-(toluenesulfonyloxy)ethoxy)ethoxy) phenoxy)4-methylbenzenesulfonate

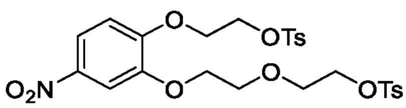

**Amine-2-d**

To a solution of 2-(2-(2-(2-hydroxyethoxy)-5-nitrophenoxy)ethoxy)ethanol (2.1 g, 7.3 mmol) and triethylamine (3.3 g, 21.9 mmol) in dichloromethane was added 4-toluenesulfonyl chloride (3.5 g, 18.25 mmol) at 0°C. The resulting mixture was stirred at room temperature for 14 hours. The completion of the reaction was monitored by TLC. The reaction mixture was partitioned in dichloromethane (10 ml) and water (20 ml). The organic layer was collected, washed with

saturated sodium chloride solution (20 ml×2), dried over anhydrous sodium sulfate, and concentrated under reduced pressure. The crude product was purified by silica gel column chromatography (eluent: 50% ethyl acetate solution in petroleum ether to 1.6% ethyl acetate solution in dichloromethane) to obtain 4-(4-nitro-2-(2-(2-(toluenesulfonyloxy) ethoxy)ethoxy)phenoxy)4-methylbenzenesulfonate as a brown oil (3.5 g, 80%).

LC_MS: (ES+): m/z 596.1 $[M+H]^+$.

$^1$H NMR (400 MHz, $CDCl_3$) δ 7.86-7.72 (m, 6H), 7.34-7.29 (m, 4H), 6.86-6.84 (m, 1H), 4.42-4.39 (m, 2H), 4.31-4.29 (m, 2H), 4.20-4.18 (m, 2H), 4.15-4.13 (m, 2H), 3.85-3.79 (m, 4H), 2.44-2.42 (d, *J* = 8.4 Hz, 6H).

Step 4: synthesis of intermediate **Amine-2-e** 7-(4-methoxybenzyl)-13-nitro-2,3,6,7,8,9-hexahydro-5*H*-benzo[*b*][1,4,7] trioxa[10]azacyclododec ane

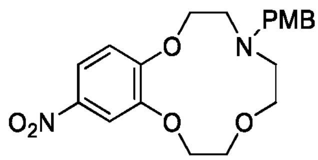

**Amine-2-e**

A solution of 4-methylbenzenesulfonic acid 2-(4-nitro-2-(2-(2-(tolylsulfonyloxyethoxy)ethoxy)phenoxy)ethyl) (3.5 g, 5.9 mmol), (4-methoxyphenyl)methylamine (806 mg, 5.9 mmol), potassium iodide (488 mg, 2.94 mmol) and potassium carbonate (4.05 g, 29.38 mmol) in acetonitrile (100 ml) was refluxed for 16 hours. The completion of the reaction was monitored by TLC. The reaction mixture was cooled to room temperature and partitioned in water (100 ml) and ethyl acetate (50 ml). The organic layer was collected and the aqueous layer was extracted with ethyl acetate (50 ml×2). The organic layers were combined, washed with saturated sodium chloride solution (80 ml), dried over anhydrous sodium sulfate, and concentrated under reduced pressure. The obtained crude product was separated and purified by silica gel column chromatography (an eluent of 33% ethyl acetate solution in petroleum ether) to obtain 7-(4-methoxybenzyl)-13-nitro-2,3,6,7,8,9-hexahydro-5*H*-benzo[*b*][1,4,7]trioxa[10]azacyclododec ane as a yellow solid (1.6 g, 70%).

LC_MS: (ES+): m/z 389.5 $[M+H]^+$.

Step 5: synthesis of intermediate **Amine-2-f** 13-nitro-2,3,6,7,8,9-hexahydro-5*H*-benzo[*b*][1,4,7] trioxa[10]azacyclododecane

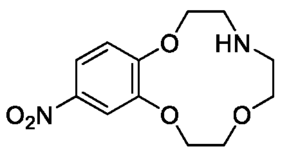

**Amine-2-f**

A solution of 7-(4-methoxybenzyl)-13-nitro-2,3,6,7,8,9-hexahydro-5*H*-benzo[*b*][1,4,7]trioxa[10]azacyclododec ane (1.6 g, 4.12 mmol) and 1-chloroethyl chloroformate (883 mg, 6.18 mmol) in 1,2-dichloroethane (30 ml) was stirred at room temperature for 13 hours. The completion of the reaction was monitored by TLC. The reaction mixture was concentrated and the residue was dissolved in methanol (8 ml). The obtained mixture was refluxed for 1 hour of reaction. The completion of the reaction was monitored by TLC. The reaction mixture was partitioned in water (20 ml) and dichloromethane (30 ml). The organic layer was collected and the aqueous layer was extracted with dichloromethane (10 ml×5). The organic layer was combined, washed with saturated sodium chloride solution (30 ml), dried over anhydrous sodium sulfate, and concentrated under reduced pressure. The obtained crude product was purified by silica gel column chromatography (eluted with 5%-10% methanol solution in dichloromethane) to obtain 13-nitro-2,3,6,7,8,9-hexahydro-5*H*-benzo[*b*][1,4,7]trioxa[10]azadodecane as a yellow solid (620 mg, 56%).

LC_MS: (ES+): m/z 269.2 $[M+H]^+$.

$^1$H NMR (400 MHz, $CDCl_3$): δ 7.93-7.91 (m, 1H), 7.86-7.85 (m, 1H), 7.12-7.10 (d, *J* = 8.8 Hz, 1H), 4.31-4.28 (m, 4H), 3.81-3.79 (m, 2H), 3.72-3.69 (m, 2H), 3.00-2.98 (m, 2H), 2.82-2.79 (m, 2H).

Step 6: synthesis of intermediate **Amine-2-g** 7-methyl-13-nitro-2,3,6,7,8,9-hexahydro-5*H*-benzo[*b*][1,4,7]trioxa[10] azacyclododecane

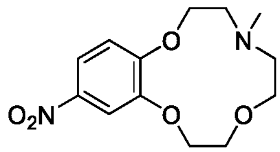

**Amine-2-g**

A solution of 13-nitro-2,3,6,7,8,9-hexahydro-5*H*-benzo[*b*][1,4,7]trioxa[10]azadodecane (515 mg, 1.9 mmol), triethylamine (567 mg, 3.8 mmol) and iodomethane (409 mg, 2.88 mmol) in *N,N*-dimethylformamide was reacted in a sealed tube for 3 hours at 70°C. The completion of the reaction was monitored by TLC. The reaction mixture was cooled to room temperature and partitioned in water (50 ml) and ethyl acetate (30 ml). The organic layer was collected and the aqueous layer was extracted with ethyl acetate (20 ml×2). The organic layer was combined, washed with saturated sodium chloride solution (30 ml), dried over anhydrous sodium sulfate, and concentrated under reduced pressure. The obtained crude product was purified by silica gel column chromatography (eluted with 5%-10% methanol solution in dichloromethane) to obtain 7-methyl-13-nitro-2,3,6,7,8,9-hexahydro-5*H*-benzo[*b*][1,4,7]trioxa[10]azacyclododecane as a yellow solid (267 mg, 49%).

LC_MS: (ES+): m/z 283.2 $[M+H]^+$.

$^1H$ NMR (400 MHz, DMSO-d6): δ 7.96-7.92 (m, 2H), 7.28-7.26 (d, *J* = 8.8 Hz, 1H), 4.34-4.25 (m, 4H), 3.71-3.70 (m, 4H), 3.05-2.84 (m, 4H), 2.48-2.40 (m, 3H).

Step 7: synthesis of **Amine-2** 7-methyl-2,3,6,7,8,9-hexahydro-5*H*-benzo[*b*][1,4,7]trioxa[10] azacyclododecane-13-amine

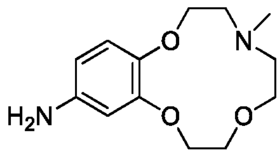

**Amine-2**

To a solution of 7-methyl-13-nitro-2,3,6,7,8,9-hexahydro-5*H*-benzo[*b*][1,4,7]trioxa[10]azacyclododecane (267 mg, 0.95 mmol) in dichloromethane (3 ml) was added zinc powder (870 mg, 13.3 mmol) and acetic acid (1.6 g, 26.6 mmol) at room temperature. The reaction mixture was stirred for 10 minutes under nitrogen atmosphere at room temperature. The completion of the reaction was monitored by TLC. The reaction mixture was basified with aqueous sodium bicarbonate solution (sat) till pH=7-8, and filtered. The filtrate was partitioned between 10% methanol solution in dichloromethane (20 ml) and water (10 ml). The organic layer was collected and the aqueous layer was extracted with 10% methanol solution in dichloromethane (20 ml×3). The organic layer was combined, washed with saturated sodium chloride solution(30 ml), dried over anhydrous sodium sulfate, and concentrated under reduced pressure to obtain crude product 7-methyl-2,3,6,7,8,9-hexahydro-5*H*-benzo[*b*][1,4,7]trioxa[10]azacyclododecane-13-amine as a yellow oil (230 mg, 96%).

LC_MS: (ES+): m/z 252.8 $[M+H]^+$.

**Fragment Amine-3**

**Implementation Method**

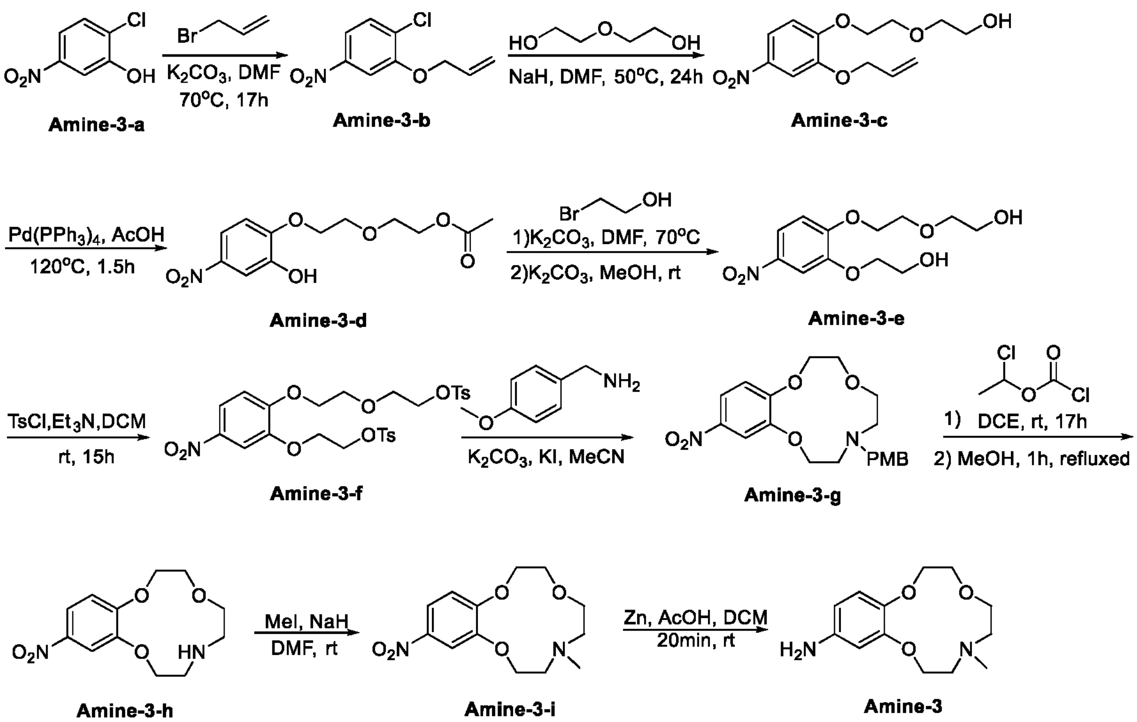


Amine-3-a Amine-3-b Amine-3-c Amine-3-d Amine-3-e Amine-3-f Amine-3-g Amine-3-h Amine-3-i Amine-3

Step 1: synthesis of intermediate **Amine-3-b** 2-(allyloxy)-1-chloro-4-nitrobenzene

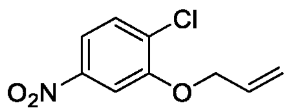

**Amine-3-b**

2-Chloro-5-nitrophenol (5.0 g, 28.8 mmol), 3-bromopropene (3.5 g, 28.8 mmol) and potassium carbonate (6 g, 43.2 mmol) were added into a reaction flask, 20 ml of *N,N*-dimethylformamide was added, and heated up to 70°C for 12 hours. The completion of the reaction was monitored by TLC. The reaction solution was cooled to room temperature, and extracted with water (200 ml) and ethyl acetate (100 ml). The water phase was extracted with ethyl acetate (50 ml), the combined organic phases was washed with saturated sodium chloride solution (100 ml), dried over anhydrous sodium sulfate and concentrated. The crude product was recrystallized to obtain 2-(allyloxy)-1-chloro-4-nitrobenzene as a brown solid (5.5 g, 90%).

$^1H$ NMR (400 MHz, $CDCl_3$) $\delta$ 7.78-7.82 (m, 2H), 7.53 (d, $J$ = 8.4 Hz, 1H), 6.03-6.12 (m, 1H), 5.49-5.54 (m, 1H), 5.37-5.40 (m, 1H), 4.71-4.73 (m, 2H).

Step 2: synthesis of the intermediate **Amine-3-c** 2-(2-(2-(allyloxy)-4-nitrophenoxy)ethoxy)ethan-1-ol

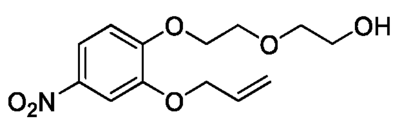

**Amine-3-c**

Diethylene glycol (10 g, 93.6 mmol) was added into a reaction flask containing 10 ml of N,N-dimethylformamide, and sodium hydride (60%, 562 mg, 14 mmol) was added at 0°C. The reaction solution was warmed to room temperature and reacted for 1 hour, then cooled to 0°C. 2-(Allyloxy)-1-chloro-4-nitrobenzene (2 g, 9.36 mmol) was added dropwise, and the reaction solution was stirred for 2 days under nitrogen protection at 50°C. The completion of the reaction was monitored

by TLC, and the reaction solution was extracted with ethyl acetate (100 ml) and water (100 ml). The organic phase was washed twice with saturated sodium chloride solution (100 ml), dried over anhydrous sodium sulfate, and concentrated. The crude product was separated and purified by column chromatography (eluted with 20%-50% ethyl acetate solution in n-hexane) to obtain 2-(2-(2-(allyloxy)-4-nitrophenoxy)ethoxy)ethan-1-ol as a brown oil (2 g, 75%).

$^1$H NMR (400 MHz, $CDCl_3$) δ 7.88-7.91 (m, 1H), 7.76 (d, *J* = 2.8 Hz, 1H), 6.94 (d, *J* = 8.8 Hz, 1H), 6.04-6.13 (m, 1H), 5.44-5.49 (m, 1H), 5.33-5.36 (m, 1H), 4.66-4.68 (m, 2H), 4.27 (t, *J* = 4.6 Hz, 2H), 3.96 (t, *J* = 4.8 Hz, 2H), 3.69-3.78 (m, 4H), 2.28 (s, 1H).

Step 3: synthesis of intermediate **Amine-3-d** 2-(2-(2-hydroxy-4-nitrophenoxy)ethoxy)ethyl acetate.

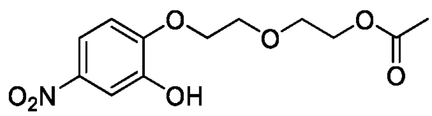

**Amine-3-d**

Tetrakis(triphenylphosphine)palladium (408 mg, 0.35 mmol) was added to a suspension of 2-(2-(2-(allyloxy)-4-nitrophenoxy)ethoxy)ethan-1-ol (1 g, 3.53 mmol) in acetic acid (5 ml) under the nitrogen protection at room temperature. The resulting mixture was replaced by nitrogen for three times, heated to 120°C and stirred for 2 hours. The completion of the reaction was monitored by TLC. The reaction mixture was concentrated under reduced pressure and partitioned in water (20 ml) and ethyl acetate (10 ml). The organic layer was collected and the aqueous phase was extracted with ethyl acetate (10 ml×2). The organic layers were combined, washed with saturated sodium chloride solution (20 ml), dried over anhydrous sodium sulfate, and concentrated under reduced pressure to obtain crude product 2-(2-(2-hydroxy-4-nitrophenoxy) ethoxy)ethyl acetate as a brown oil (860 mg, crude product), which was used in the next step without further purification.

LC_MS: ($ES^+$): m/z 286.10 $[M+H]^+$.

$^1$H NMR (400 MHz, $CDCl_3$) δ 7.77-7.80 (m, 2H), 6.92 (d, *J* = 9.6 Hz, 1H), 6.86 (s, 1H), 4.26-4.33 (m, 4H), 3.88-3.90 (m, 2H), 3.76-3.78 (m, 2H), 2.11 (s, 3H).

Step 4: synthesis of the intermediate **Amine-3-e** 2-(2-(2-(2-hydroxyethoxy)-4-nitrophenoxy)ethoxy)ethan-1-ol

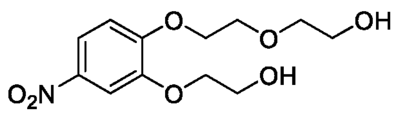

**Amine-3-e**

A solution of 2-(2-(2-hydroxy-4-nitrophenoxy)ethoxy)ethyl acetate (860 mg, 3 mmol), 2-bromoethanol (1.5 g, 12 mmol) and potassium carbonate (3.7 g, 27.1 mmol) in N,N-dimethylformamide (10 ml) was stirred at 70°C for 14 hours. The completion of the reaction was monitored by TLC. The reaction mixture was cooled to room temperature and partitioned in water (100 ml) and ethyl acetate (50 ml). The organic layer was collected and the aqueous phase was extracted with ethyl acetate (50 ml×2). The organic layers were combined, washed with saturated sodium chloride solution (100 ml), dried over anhydrous sodium sulfate, and concentrated under reduced pressure. The residue was dissolved in methanol (5 ml), potassium carbonate (416 mg, 3 mmol) was added at room temperature, and the resulting mixture was stirred at room temperature for 30 minutes of reaction. The completion of the reaction was monitored by TLC. The reaction mixture was partitioned in dichloromethane (30 ml) and water (20 ml). The organic layer was collected, washed with saturated sodium chloride solution (20 ml), dried over anhydrous sodium sulfate, and concentrated under reduced pressure. The obtained crude product was separated and purified by silica gel column chromatography (eluted with 2-5% methanol solution in dichloromethane) to obtain 2-(2-(2-(2-hydroxyethoxy)-4-nitrophenoxy)ethoxy)ethanol as light yellow oil (500 mg, 57% mg).

$^1$H NMR (400 MHz, $CDCl_3$) δ 7.91-7.94 (m, 1H), 7.78 (d, *J* = 2.4 Hz, 1H), 6.92 (d, *J* = 8.8 Hz, 1H), 4.75 (s, 1H), 4.24 (t, *J* = 4.2 Hz, 2H), 4.15 (t, *J* = 4.2 Hz, 2H), 4.02 (t, *J* = 4.2 Hz, 2H), 3.96 (s, 2H), 3.75-3.82 (m, 4H), 1.74 (s, 1H).

Step 5: synthesis of intermediate **Amine-3-f** 2-(5-nitro-2-(2-(2-(toluenesulfonyloxy)ethoxy)ethoxy)phenoxy)ethyl-4-toluenesulfonate

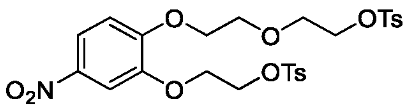

**Amine-3-f**

To a solution of 2-(2-(2-(2-hydroxyethoxy)-4-nitrophenoxy)ethoxy)ethan-1-ol (500 mg, 1.74 mmol), triethylamine (528 mg, 5.22 mmol) and 4-dimethylaminopyridine (21 mg, 0.17 mmol) in dichloromethane (5 ml) was added p-toluenesulfonyl chloride (730 mg, 3.83 mmol) at 0°C. The obtained mixture was heated to room temperature and stirred for 2 hours. The completion of the reaction was monitored by TLC. The reaction mixture was partitioned in dichloromethane (20 ml) and water (20 ml). The organic phase was collected, washed with saturated sodium chloride solution (10 ml×2), dried over anhydrous sodium sulfate, and concentrated under reduced pressure. The obtained crude product was separated and purified by silica gel column chromatography (eluted with 50%-100% ethyl acetate solution in hexane) to obtain 2-(5-nitro-2-(2-(2-(toluenesulfonyloxy)ethoxy)ethoxy)phenoxy)ethyl-4-toluenesulfonate as a brown oil (830 mg, 80%).

$^1H$ NMR (400 MHz, $CDCl_3$): δ 7.88-7.91 (m, 1H), 7.76-7.81 (m, 4H), 7.64 (d, *J* = 2.8 Hz, 1H), 7.30-7.35 (m, 4H), 6.91 (d, *J* = 9.2 Hz, 1H), 4.19 (t, *J* = 4.6 Hz, 4H), 4.38-4.40 (m, 2H), 4.23-4.26 (m, 2H), 3.86 (t, *J* = 4.6 Hz, 2H), 3.81 (t, *J* = 4.6 Hz, 2H), 2.43 (d, *J* = 8.0 Hz, 6H).

Step 6: synthesis of intermediate **Amine-3-g** 7-(4-methoxybenzyl)-12-nitro-2,3,6,7,8,9-hexahydro-5*H*-benzo[*b*][1,4,7]trioxa[10]azacyclododec ane

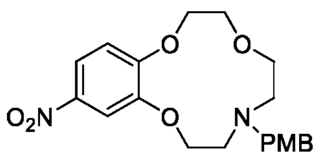

**Amine-3-g**

A solution of 2-(5-nitro-2-(2-(2-(toluenesulfonyloxy)ethoxy) ethoxy)phenoxy)ethyl-4-toluenesulfonate (30 ml, 1.06 mmol), 4-methoxybenzylamine (145 mg, 1.06 mmol), potassium carbonate (731 mg, 5.3mmol) and potassium iodide (88 mg, 0.53 mmol) in acetonitrile (30 ml) was refluxed for 14 hours. The completion of the reaction was monitored by TLC. The reaction mixture was cooled to room temperature and partitioned in water (30 mL) and ethyl acetate (10 ml). The organic layer was collected and the aqueous phase was extracted with ethyl acetate (10 ml×2). The organic phases were combined, washed with saturated sodium chloride solution (30 ml), dried over anhydrous sodium sulfate, and concentrated under reduced pressure. The obtained crude product was separated and purified by silica gel column chromatography (eluted with 20%-50% ethyl acetate solution in hexane) to obtain 7-(4-methoxybenzyl)-12-nitro-2,3,6,7,8,9-hexahydro-5*H*-benzo[*b*][1,4,7]trioxa[10]azacyclododec ane as yellow oil (210 mg, 51%).

$^1H$ NMR (400 MHz, $CDCl_3$): δ 7.90-7.93 (m, 1H), 7.75 (d, *J* = 2.0 Hz, 1H), 7.13 (s, 2H), 7.00 (d, *J* = 8.8 Hz, 1H), 6.78 (d, *J* = 7.2 Hz, 2H), 4.31 (t, *J* = 4.0 Hz, 2H), 4.17 (s, 2H), 3.87 (t, *J* = 3.8 Hz, 2H), 3.78 (s, 5H), 3.64 (s, 2H), 2.87 (d, *J* = 6.8 Hz, 4H).

Step 7: synthesis of intermediate **Amine-3-h** 12-nitro-2,3,6,7,8,9-hexahydro-5*H*-benzo[*b*][1,4,7]trioxa[10]azadodecane

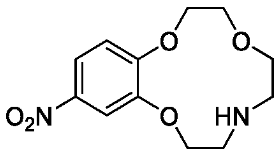

**Amine-3-h**

A solution of 7-(4-methoxybenzyl)-12-nitro-2,3,6,7,8,9-hexahydro-5*H*-benzo[*b*][1,4,7]trioxa[10]azacyclododecane (1.08 g, 2.78 mmol) and 1-chloroethyl chloroformate (596 mg, 4.17 mmol) in 1,2-dichloroethane (15 ml) was stirred at room temperature for 3 hours of reaction. The completion of the reaction was monitored by TLC. The reaction mixture was concentrated, methanol (3 ml) was added to the residue, and the resulting mixture was heated and refluxed for 1 hour. The completion of the reaction was monitored by TLC. The reaction mixture was partitioned in water (20 ml) and dichloromethane (30 ml). The organic layer was collected and the aqueous phase was extracted with dichloromethane (10 ml×2). The organic phase layers were combined, washed with saturated sodium chloride solution (20 ml), dried over anhydrous sodium sulfate, and concentrated under reduced pressure. The obtained crude product was separated and purified by silica gel column chromatography (eluted with 2%-5% methanol solution in dichloromethane) to obtain 12-nitro-2,3,6,7,8,9-hexahydro-5*H*-benzo[*b*][1,4,7]trioxa[10] azacyclododecane as a yellow solid (479 mg, 64%).

$^1$H NMR (400 MHz, DMSO-d6): δ 8.03 (t, *J* = 5.4 Hz, 2H), 7.44 (s, 1H), 7.35 (d, *J* = 8.8 Hz, 1H), 4.30-4.35 (m, 4H), 3.81 (t, *J* = 4.0 Hz, 2H), 3.70 (t, *J* = 4.6 Hz, 2H), 3.05-3.12 (m. 4H).

Step 8: synthesis of intermediate **Amine-3-i** 7-methyl-12-nitro-2,3,6,7,8,9-hexahydro-5*H*- benzo[*b*][1,4,7]trioxa[10] azacyclododecane

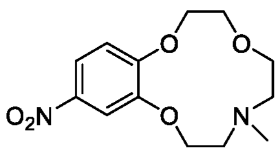

**Amine-3-i**

To a solution of 12-nitro-2,3,6,7,8,9-hexahydro-5*H*-benzo[*b*][1,4,7]trioxa[10]azacyclododecane (265 mg, 0.98 mmol) and iodomethane (167 mg, 1.18 mmol) in *N,N*-dimethylformamide (5 ml) was added sodium hydride ( 60%, 43 mg, 1.08 mmol) at 0°C. The reaction mixture was stirred at 0°C for 30 minutes of reaction, then heated to room temperature and stirred for another 3 hours. The completion of the reaction was monitored by TLC. The reaction mixture was partitioned in ethyl acetate (30 ml) and water (50 ml). The organic layer was collected and the aqueous phase was extracted with ethyl acetate (20 ml×4). The organic layers were combined, washed with saturated sodium chloride solution (50 ml), dried over anhydrous sodium sulfate, and concentrated under reduced pressure. The obtained crude product was separated and purified by silica gel column chromatography (eluted with 2%-10% methanol solution in dichloromethane) to obtain 7-methyl-12-nitro-2,3,6,7,8,9-hexahydro-5*H*-benzo[*b*][1,4,7]trioxa[10]azacyclododecane as a yellow oil (153 mg, 56%).

LC_MS: (ES$^+$): m/z 283.20 [M+H]$^+$.

$^1$H NMR (400 MHz, $CDCl_3$): δ 7.89-7.91 (m, 1H), 7.85 (d, *J* = 2.4Hz, 1H), 6.99 (d, *J* = 8.8Hz, 1H), 4.28 (t, *J* = 4.2Hz, 2H), 4.21 (t, *J* = 4.8 Hz, 2H), 3.88 (t, *J* = 4.2 Hz, 2H), 3.74 (t, *J* = 5Hz, 2H), 2.82 (t, *J* = 4.6 Hz, 2H), 2.68 (t, *J* = 4.8 Hz, 2H), 2.37 (s, 3H).

Step 9: synthesis of **Amine-3** 7-methyl-12-nitro-2,3,6,7,8,9-hexahydro-5*H*-benzo[*b*][1,4,7]trioxa[10]azacyclododecan-12-amin e

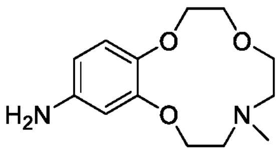

**Amine-3**

To a solution of 7-methyl-12-nitro-2,3,6,7,8,9-hexahydro-5*H*-benzo[*b*][1,4,7]trioxa[10]azadodecane (153 mg, 0.63 mmol) in dichloromethane (7 ml) were added zinc powder (574 mg, 8.78 mmol) and acetic acid (1.05 g, 17.55 mmol) at room temperature. The obtained mixture was stirred for 30 minutes of reaction under nitrogen protection at room temperature. The completion of the reaction was monitored by TLC. The reaction mixture was filtered and the filtrate was partitioned in dichloromethane (20 ml) and water (10 ml). The organic layer was collected and the aqueous phase was extracted with 10% methanol solution in dichloromethane (10 ml×3). The organic phases were combined, washed with saturated sodium chloride solution (20 ml), dried over anhydrous sodium sulfate, and concentrated under reduced pressure to obtain crude product 7-methyl-2,3,6,7,8,9-hexahydro-5H-benzo[b][1,4,7]trioxa[10]azacyclododecan-12-amine as a light brown oil (123 mg, 90%), which was used in the next step without further purification.

LC_MS: ($ES^+$): m/z 253.20 $[M+H]^+$.

**Example 1**

N-(tert-butyl)-3-((5-fluoro-2-((4-methyl-3,4,5,6-tetrahydro-2*H*-benzo[*b*][1,4,7]dioxazoli n-9-yl)amino)pyrimidin-4-yl)amino)benzsulfamide

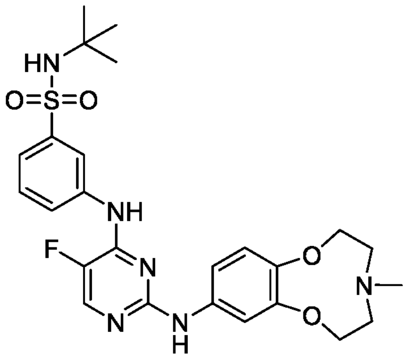

**1**

**Implementation steps (Implementation method 1)**

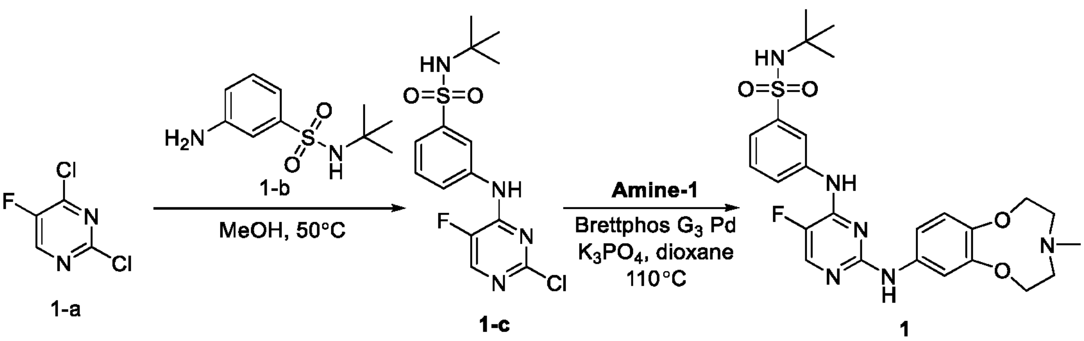


1-a

1-c

1

Step 1: Synthesis of fragment 1-c N-(tert-butyl)-3-((2-chloro-5-fluoropyrimidin-4-yl)amino)benzenesulfonamide

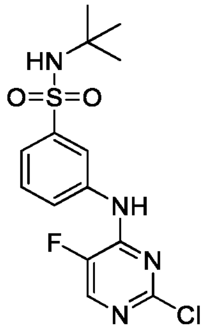

**1-c**

A solution of 2,4-dichloro-5-fluoropyrimidine (500 mg, 3 mmol) and 3-amino-N-(tert-butyl)benzenesulfonamide (685 mg, 3 mmol) in methanol (10 ml) was heated to 50°C and stirred for 3 hours. The completion of the reaction was monitored by TLC. The reaction solution was concentrated under reduced pressure, and the obtained crude product was separated and purified by silica gel column chromatography (eluted with 15%-25% ethyl acetate solution in n-hexane) to obtain *N*-(tert-butyl)-3-((2-chloro-5-fluoropyrimidin-4-yl)amino)benzenesulfonamide as a white solid (362 mg, 33%).
LC_MS: ($ES^+$): m/z 360.0 $[M+H]^+$.
$^1$H NMR (400 MHz, DMSO-d6): δ 10.27 (s, 1H), 8.38 (d, *J* = 3.6 Hz, 1H), 8.21 (t, *J* = 2.4 Hz, 1H), 7.88-7.91 (m, 1H), 7.55-7.59 (m, 3H), 1.14 (s, 9H),

Step 2: Compound 1 N-(tert-butyl)-3-((5-fluoro-2-((4-methyl-3,4,5,6-tetrahydro-2*H*-benzo[*b*][1,4,7]dioxazolin-9-yl)amino)pyrimidin-4-yl)amino)benzenesulfonamide

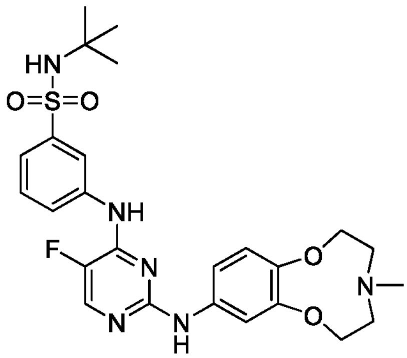

**1**

To a suspension of N-(tert-butyl)-3-((2-chloro-5-fluoropyrimidin-4-yl)amino)benzenesulfonamide (40 mg, 0.11 mmol), 4-methyl-3,4,5,6-tetrahydro-2*H*-benzo[*b*][1,4,7]dioxazolin-9-amine (23 mg, 0.11 mmol) and potassium phosphate (70 mg, 0.33 mmol) in 1,4-dioxane (1 ml) was added BrettPhos Pd G3 (10 mg, 0.011 mmol), and the obtained mixture was replaced by nitrogen gas for three times, heated to 110°C and stirred for 14 hours. The completion of the reaction was monitored by TLC. The reaction solution was cooled to room temperature and partitioned in water (10 ml) and ethyl acetate (10 ml). The organic layer was collected and the aqueous layer was extracted with ethyl acetate (10 ml). The organic layers were combined, washed with saturated sodium chloride solution (20 ml), dried over anhydrous sodium sulfate, and concentrated under reduced pressure. The obtained crude product was separated and purified by preparative TLC (eluted with 5% methanol solution in dichloromethane) to obtain target compound as a yellow solid (21.1 mg, 35%).
LC_MS: ($ES^+$): m/z 531.5 $[M+H]^+$.
$^1$H NMR (400 MHz, $CDCl_3$): δ 8.27 (s, 1H), 7.95 (d, *J* = 2.8 Hz, 1H), 7.76 (d, *J* = 8.0 Hz, 1H), 7.59 (d, *J* = 7.6 Hz, 1H), 7.41 (t, *J* = 8.0 Hz, 1H), 6.94-6.99 (m, 2H), 6.86-6.90 (m, 1H), 6.69 (d, *J* = 8.0 Hz, 1H), 4.65 (brs, 1H), 4.24 (t, *J* = 4.4 Hz, 2H), 3.62 (t, *J* = 5.6 Hz, 2H), 3.42-3.48 (m, 4H), 3.37 (s, 3H), 1.20 (s, 9H).

**Example 2**

N-(cyanomethyl)-4-(2-((4-methyl-3,4,5,6-tetrahydro-2*H*-benzo[*b*][1,4,7]dioxazolin-9-yl) amino)-7*H*- pyrrolo[2,3-*d*]pyrimidin-4-yl)benzamide

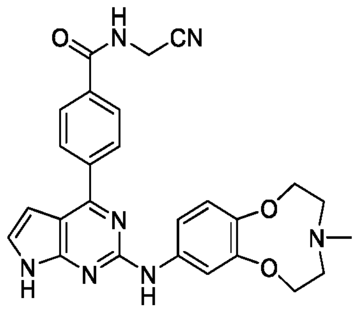

**2**

**Implementation steps (Implementation method 2)**

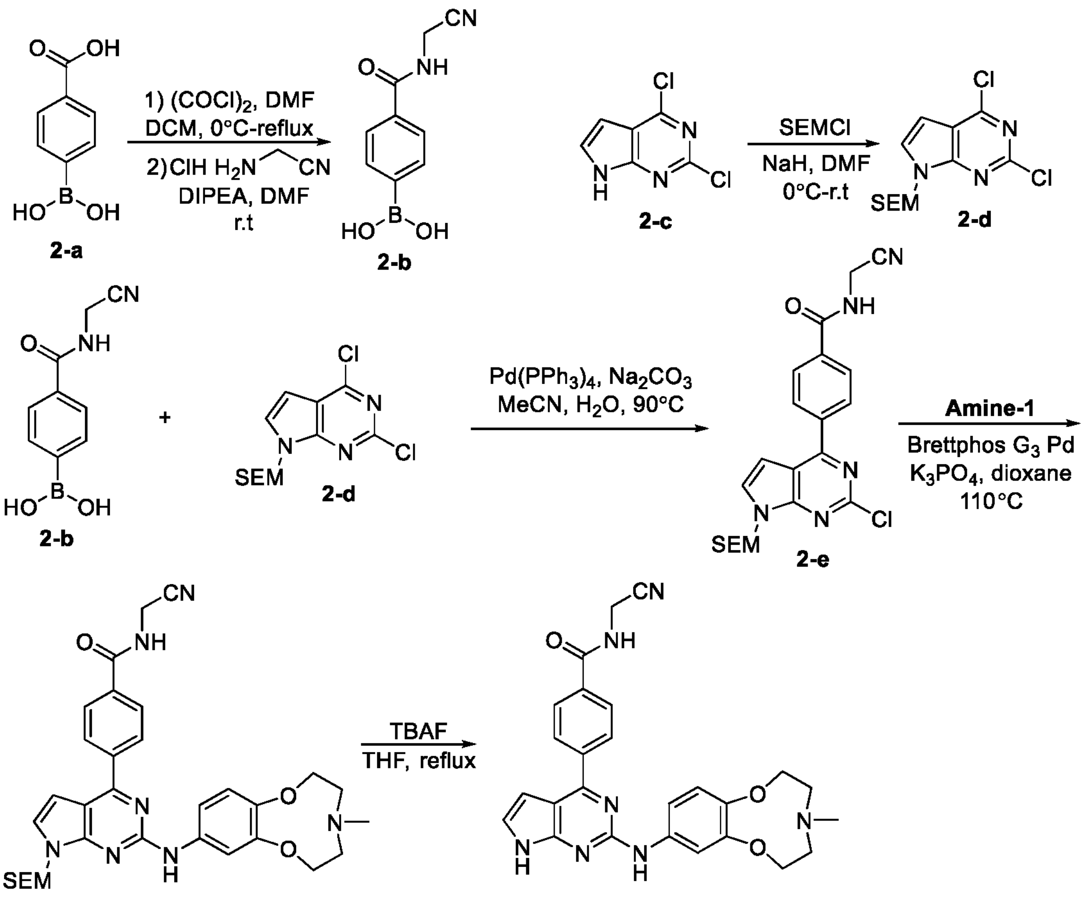


**2**

Step 1: synthesis of fragment 2-b (4-((cyanomethyl)carbamoyl)phenyl)boric acid

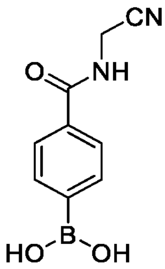

**2-b**

To a solution of 4-carboxyphenylboronic acid (2.0 g, 12.0 mmol) and *N,N*-dimethylformamide (2 ml) in dichloromethane (80 ml) was added oxalyl chloride (2.4 ml, 26.5 mmol) at 0°C. The resulting mixture was heated to reflux for 3 hours. The completion of the reaction was monitored by TLC. The reaction solution was concentrated, and the residue was dissolved in *N,N*-dimethylformamide (5 ml). Under nitrogen protection at 0°C, it was added dropwise to a solution of aminoacetonitrile hydrochloride (1.23 g, 13.2 mmol) and *N,N*-diisopropylethylamine (3.9 g, 30.1 mmol) in *N,N*-dimethylformamide (10 ml). The resulting mixture was stirred at room temperature for 12 hours of reaction. The completion of the reaction was monitored by TLC. To the reaction mixture was added water (150 ml) and extracted with ethyl acetate (50 ml×5). The organic layers were combined, dried over anhydrous sodium sulfate, filtered and concentrated under reduced pressure. The obtained crude product was recrystallized in ethyl acetate to obtain (4-((cyanomethyl)carbamoyl) phenyl)boric acid as a light yellow solid (950 mg, 38%).

$^1$H NMR (400 MHz, DMSO-d6): δ 9.18 (t, *J* = 5.4 Hz, 1H), 8.23 (s, 2H), 7.81-7.90 (m, 4H), 4.31 (d, *J* = 5.6 Hz, 2H).

Step 2: synthesis of fragment **2-d** 2,4-dichloro-7-((2-(trimethylsilyl)ethoxy)methyl)-7*H*-pyrrolo[2,3-*d*] pyrimidine

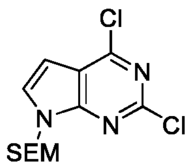

**2-d**

To a solution of 2,4-dichloro-7*H*-pyrrolo[2,3-*d*]pyrimidine (5 g, 26.6 mmol) in *N,N*-dimethylformamide (50 ml) was added sodium hydride (60%, 1.3 g, 31.9 mmol) at 0°C. The resulting mixture was stirred at room temperature for 1 hour of reaction, cooled to 0°C, and then 2-(trimethylsilyl)ethoxymethyl chloride (4.9 g, 29.3 mmol) was added dropwise. The obtained mixture was warmed to room temperature, stirred for 14 hours under nitrogen protection. The completion of the reaction was monitored by TLC. The reaction mixture was partitioned in ethyl acetate (150 ml) and water (300 ml). The organic layer was collected, washed with saturated sodium chloride solution (100 ml×2), dried over anhydrous sodium sulfate, and concentrated under reduced pressure. The obtained crude product was separated and purified by silica gel column chromatography (eluted with 1.5% ethyl acetate solution in n-hexane) to obtain 2,4-dichloro-7-((2-(trimethylsilyl) ethoxy)methyl)-7*H*-pyrrolo[2,3-*d*]pyrimidine as a colorless oil (2.3 g, 27%).

$^1$H NMR (400 MHz, $CDCl_3$): δ 7.37 (d, *J* = 3.6 Hz, 1H), 6.66 (d, *J* = 3.6 Hz, 1H), 5.60 (s, 2H), 3.53 (t, *J* = 8.2 Hz, 2H), 0.92 (t, *J* = 8.4 Hz, 2H), -0.04 (s, 9H).

Step 3: Synthesis of fragment **2-e** 4-(2-chloro-7-((2-(trimethylsilyl)ethoxy)methyl)-7*H*-pyrrolo[2,3-*d*]pyrimidin-4-yl)-N-(cyanomet hyl)benzamide

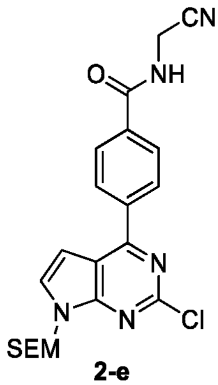

**2-e**

To a solution of (4-((cyanomethyl)carbamoyl)phenylboronic acid (200 mg, 0.98 mmol), 2,4-dichloro-7-((2-(trimethylsilyl)ethoxy)methyl)-7*H*-pyrrolo[2,3-*d*]pyrimidine (624 mg, 1.96 mmol) and aqueous sodium carbonate (1 ml, 2 N) in acetonitrile (2 ml) was added tetrakis(triphenylphosphine)palladium (113 mg, 0.1 mmol) under nitrogen atmosphere at room temperature. The resulting reaction mixture was replaced with nitrogen gas for three times, heated to 90°C and stirred for 12 hours. The completion of the reaction was monitored by TLC. The reaction mixture was partitioned in ethyl acetate (10 ml) and water (10 ml). The organic phase was collected, washed with saturated sodium chloride solution (10 ml), dried over anhydrous sodium sulfate, and concentrated under reduced pressure. The obtained crude product was separated and purified by silica gel column chromatography (eluted with 0.5%-2% methanol solution in dichloromethane) to obtain 4-(2-chloro-7-((2-(trimethylsilyl)ethoxy)methyl)-7*H*-pyrrolo[2,3-*d*]pyrimidin-4-yl)-N-(cyanomet hyl)benzamide as a white solid (300 mg, 34%).

LC_MS: ($ES^+$): m/z 442.1 $[M+H]^+$.

$^1H$ NMR (400 MHz, DMSO-d6): δ 9.41 (t, *J* = 5.4 Hz, 1H), 8.28 (d, *J* = 8.4 Hz, 2H), 8.08 (d, *J* = 8.4 Hz, 2H), 7.92 (d, *J* = 3.6 Hz, 1H), 7.10 (d, *J* = 3.6 Hz, 1H), 5.64 (s, 2H), 4.37 (d, *J* = 5.6 Hz, 2H), 3.56 (d, *J* = 8.0 Hz, 2H), 0.86 (t, *J* = 8.0 Hz, 2H), -0.08 (s, 9H).

Step 4: synthesis of fragment 2-f *N*-(cyanomethyl)-4-(2-((4-methyl-3,4,5,6-tetrahydro-2*H*-benzo[*b*][1,4,7]dioxazolin-9-yl)amino)-7-((2-(trimethylsilyl)ethoxy)methyl)-7H-pyrrolo[2,3-d]pyrimidin-4-yl)benzamide

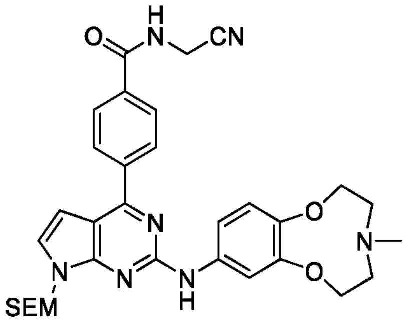

**2-f**

To a solution of 4-(2-chloro-7-((2-(trimethylsilyl)ethoxy)methyl)-7*H*-pyrrolo[2,3-*d*]pyrimidin-4-yl)-N-(cyanomet hyl)benzamide (60.0 mg, 0.1 mmol), 4-methyl-3,4,5,6-tetrahydro-2*H*-benzo[*b*][1,4,7]dioxazolidin-9-amine ( 35.4 mg, 0.2 mmol) and potassium phosphate (89.0 mg, 0.4 mmol) in 1,4-dioxane (1 ml) was added BrettPhos Pd G3 (9.1 mg, 0.01 mmol) under nitrogen atmosphere at room temperature. The resulting mixture was replaced with nitrogen gas for three times, heated to 110°C and stirred for 13 hours of reaction. The completion of the reaction was monitored by TLC. The reaction mixture was cooled to room temperature, partitioned in water (20 ml) and extracted with ethyl acetate (10 ml×2). The organic phases were combined, washed with saturated sodium chloride solution (20 ml), dried over anhydrous sodium sulfate, and concentrated under reduced pressure. The obtained crude product was separated and purified by preparative TLC (eluted with 5% methanol solution in dichloromethane) to obtain *N*-(cyanomethyl)-4-(2-((4-methyl-3,4,5,6-tetrahydro-2*H*-benzo[*b*][1,4,7] dioxazolin-9-yl)amino)-7-((2-(trimethylsilyl)ethoxy)methyl)-7*H*-pyrrolo [2,3-*d*]pyrimidin-4-yl)be nzamide as a yellow solid (62 mg, 72%).

LC_MS: ($ES^+$): m/z 614.4 $[M+H]^+$.

Step 5: synthesis of *N*-(cyanomethyl)-4-(2-((4-methyl-3,4,5,6-tetrahydro-2*H*-benzo[*b*][1,4,7]dioxazolin-9-yl)amino)-7*H*-pyrrolo[2,3-*d*]pyrimidin-4-yl)benzamide

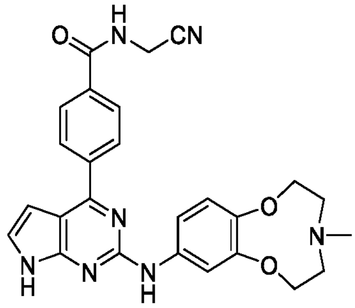

**2**

A solution of *N*-(cyanomethyl)-4-(2-((4-methyl-3,4,5,6-tetrahydro-2*H*-benzo[*b*][1,4,7]dioxazolidin-9-yl)amino) -7-((2-(trimethylsilyl)ethoxy)methyl)-7*H*-pyrrolo[2,3-*d*]pyrimidin-4-yl)benzamide (60 mg, 0.1 mmol) and tetrabutylammonium fluoride (0.5 ml, 1 M tetrahydrofuran solution) in tetrahydrofuran (0.5 ml) was heated to 70°C and stirred for 5 hours. The completion of the reaction was monitored by TLC. The reaction mixture was cooled to room temperature, and partitioned in water (10 ml). The organic phase was collected and the aqueous phase was extracted with ethyl acetate (10 ml×2). The organic phases were combined, washed with saturated sodium chloride solution (10 ml), dried over anhydrous sodium sulfate, and concentrated under reduced pressure. The obtained crude product was separated and purified by preparative TLC (eluted with 10% methanol solution in dichloromethane) to obtain target compound as a yellow solid (16.5 mg, 34%).

LC_MS: (ES$^+$): m/z 484.3 [M+H]$^+$.

$^1$H NMR (400 MHz, DMSO-d6): δ 11.63 (s, 1H), 9.35 (t, *J* = 5.6 Hz, 1H), 8.83-9.23 (m, 1H), 8.24 (d, *J* = 7.6 Hz, 2H), 8.05 (d, *J* = 8.4 Hz, 2H), 7.07-7.68 (m, 3H), 6.68 (s, 2H), 4.36 (d, *J* = 5.2 Hz, 2H), 3.92-4.28 (m, 2H), 3.49-3.59 (m, 2H), 3.32-3.41 (m, 2H), 3.29-3.31 (m, 2H), 3.28 (s, 3H).

**Example 3**

1-(3-((2-((4-methyl-3,4,5,6-tetrahydro-2*H*-benzo[*b*][1,4,7]dioxazolin-9-yl)amino)-7*H*-pyrrolo[2,3-*d*]pyrimidin-4-yl)amino) piperidin-1-yl)prop-2-en-1-one

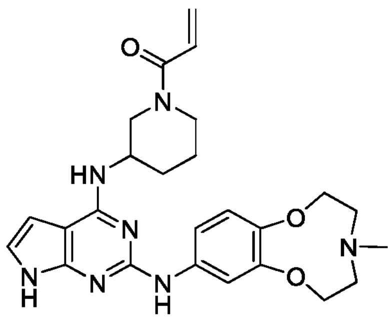

**3**

**Implementation steps (Implementation method 3)**

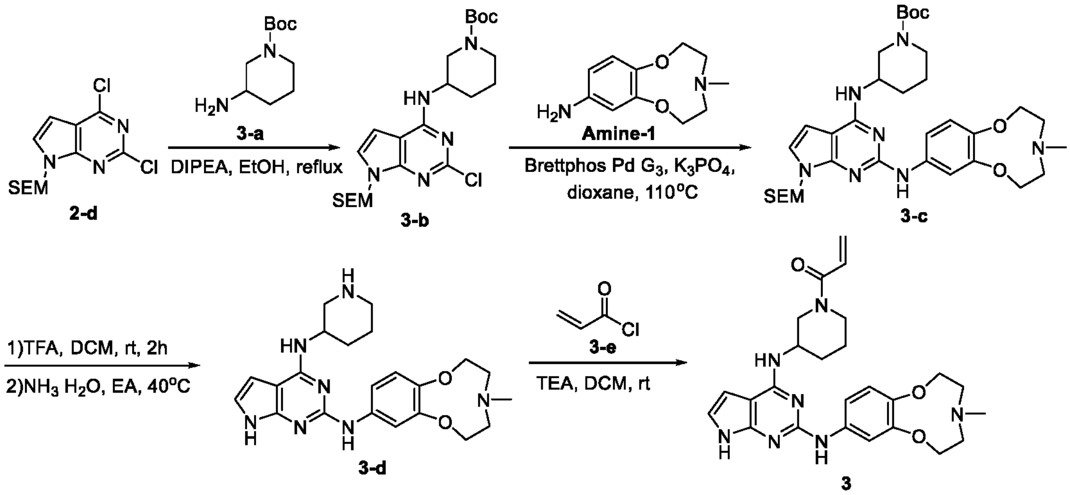


Step 1: synthesis of fragment **3-b** tert-butyl 3-((2-chloro-7-((2-(trimethylsilyl)ethoxy)methyl)-7*H*-pyrrolo[2,3-*d*]pyrimidin-4-yl)amino)piperi dine-1-carboxylate

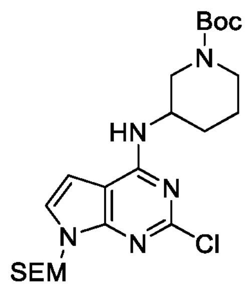

**3-b**

To a solution of fragment 2-d 2,4-dichloro-7-(((2-(trimethylsilyl)ethoxy)methyl)-7*H*-pyrrolo[2,3-*d*]pyrimidine (100 mg, 0.315 mmol), tert-butyl 3-aminopiperidine-1-carboxylate (94.6 mg, 0.473 mmol) and *N*-ethyl-*N*-isopropylpropan-2-amine (61 mg, 0.473 mmol) in ethanol (2 ml) was stirred at 90°C for 18 hours. The completion of the reaction was monitored by TLC. The reaction mixture was concentrated under reduced pressure, and the obtained crude product was separated and purified by silica gel column chromatography (eluted with 25% ethyl acetate in petroleum ether) to obtain tert-butyl 3-(((2-chloro-7-((2-(trimethylsilyl)ethoxy)methyl)-7*H*-pyrrolo[2,3-*d*] pyrimidin-4-yl)amino)piperidine-1-carboxylate as a white solid (130 mg, 85%).

LC_MS: ($ES^+$): m/z 482.2 $[M+H]^+$.

Step 2: synthesis of fragment 3-c tert-butyl 3-((2-((4-methyl-3,4,5,6-tetrahydro-2*H*-benzo[*b*][1,4,7]dioxazolin-9-yl)amino)-7-((2-(trimethylsil yl)ethoxy)methyl)-7*H*-pyrrolo[2,3-*d*]pyrimidin-4-yl)amino)piperidine-1-carboxylate

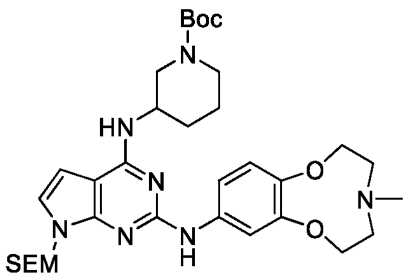

**3-c**

To a suspension of tert-butyl 3-(((2-chloro-7-((2-(trimethylsilyl)ethoxy)methyl)-7*H*-pyrrolo[2,3-*d*]pyrimidin-4-yl) amino)piperi din-1-carboxylate (84 mg, 0.17 mmol), 4-methyl-3,4,5,6-tetrahydro-2*H*-benzo[*b*][1,4,7]dioxazolin-9-amine (36 mg, 0.17 mmol) and potassium phosphate (111 mg, 0.53 mmol) in 1,4-dioxane (2 ml) was added BrettPhos Pd G3 (16 mg, 0.017 mmol). The reaction mixture was replaced with nitrogen gas for three times and stirred at 110°C for 20 hours of reaction. The completion of the reaction was monitored by TLC. The reaction mixture was cooled to room temperature and partitioned in water (10 ml) and ethyl acetate (10 ml). The organic layer was collected and the aqueous layer was extracted with ethyl acetate (10 ml×2). The organic layers were combined, washed with saturated sodium chloride solution (20 ml), dried over anhydrous sodium sulfate, and concentrated under reduced pressure. The obtained crude product was separated and purified by preparative TLC (eluted with 6% methanol and 50% ethyl acetate in petroleum ether) to obtain tert-butyl 3-((2-((4-methyl-3,4,5,6-tetrahydro-2*H*-benzo[*b*][1,4,7]dioxazolin-9-yl)amino)-7-((2-(trimethylsil yl)ethoxy) methyl)-7H-pyrrolo[2,3-d]pyrimidin-4-yl)amino)piperidin-1-carboxylic acid as a brown solid (60 mg, 47%).
LC_MS: ($ES^+$): m/z 654.9 $[M+H]^+$.

Step 3: synthesis of fragment 3-d *N*2-(4-methyl-3,4,5,6-tetrahydro-2*H*-benzo[*b*][1,4,7]dioxazolin-9-yl)-*N*4-(piperidin-3-yl)-7*H*-pyrr olo[2,3-*d*]pyrimidin-2,4-diamine

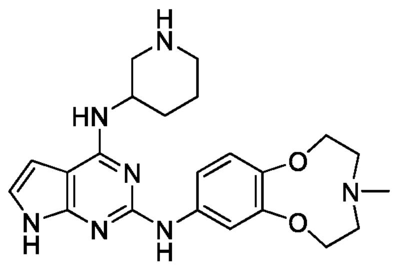

**3-d**

A solution of tert-butyl 3-((2-((4-methyl-3,4,5,6-tetrahydro-2*H*-benzo[*b*][1,4,7]dioxazolidin-9-yl)amino)-7-((2-(trimethyl silyl)ethoxy)methyl)-7*H*-pyrrolo[2,3-*d*]pyrimidin-4-yl)amino)piperidin-1-formate (75 mg, 0.11 mmol) and trifluoroacetic acid (1 ml) in dichloromethane (2 ml) was stirred for 5 hours at room temperature. The completion of the reaction was monitored by TLC. The reaction mixture was concentrated and the residue was added to ammonia (5 ml) and ethyl acetate (5 ml). The reaction mixture was stirred at 40°C for 16 hours. The completion of the reaction was monitored by TLC. The organic layer was collected, washed with saturated sodium chloride solution (10 ml), dried over anhydrous sodium sulfate, and concentrated under reduced pressure. The obtained crude product was separated and purified by preparative TLC (eluted with 10% methanol in dichloromethane) to obtain *N*2-(4-methyl-3,4,5,6-tetrahydro-2*H*-benzo[*b*][1,4,7]dioxazolin-9-yl)-*N*4-(piperidin-3-yl)-7*H*-pyrrolo[2,3-*d*]pyrimidin-2,4-diamine as a yellow solid (40 mg, 82%).
LC_MS: ($ES^+$): m/z 424.0 $[M+H]^+$.

Step 4: Synthesis of 1-(3-((2-((4-methyl-3,4,5,6-tetrahydro-2*H*-benzo[*b*][1,4,7]dioxazolin-9-yl)amino)-7*H*-pyrrolo[2,3 -d] pyrimidin-4-yl)amino)piperidin-1-yl)prop-2-en-1-one

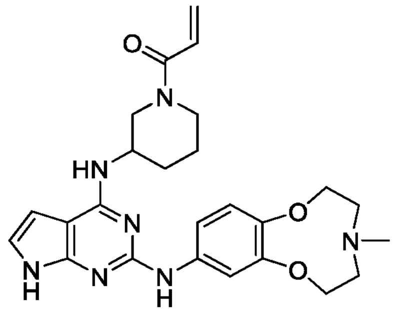

**3**

To a solution of *N*2-(4-methyl-3,4,5,6-tetrahydro-2*H*-benzo[*b*][1,4,7]dioxazolin-9-yl)-*N*4-(piperidin-3-yl)-7*H*-pyrrolo[2,3-*d*]pyrimidin-2,4-diamine (40 mg, 0.09 mmol) and triethylamine (28 mg, 0.28 mmol) in dichloromethane (2 ml) was added acryloyl chloride (8.5 mg, 0.09 mmol) at 0°C. The reaction mixture was stirred at room temperature for 4 hours. The completion of the reaction was monitored by TLC. The reaction mixture was partitioned in water (5 ml) and 10% methanol in dichloromethane (10 ml). The organic layer was collected, and the water layer was extracted with 10% methanol in dichloromethane (10 ml×3). The organic layers were combined, washed with saturated sodium chloride solution (20 ml), dried over anhydrous sodium sulfate, and concentrated under reduced pressure. The obtained crude product was separated and purified by preparative TLC (eluted with 10% methanol in dichloromethane) to obtain 1-(3-((2-((4-methyl-3,4,5,6-tetrahydro-2*H*-benzo[*b*][1,4,7]dioxazolin-9-yl)amino)-7*H*-pyrrolo[2,3 -d]pyrimidin-4-yl)amino)piperidin-1-yl)prop-2-en-1-one as a brown solid (12.1 mg, 26%).

LC_MS: (ES$^+$): m/z 478.5 [M+H]$^+$.

$^1$H NMR (400 MHz, DMSO-d6): δ 11.06 (s, 1H), 8.49 (brs, 1H), 7.23-7.35 (m, 2H), 7.10 (brs, 1H), 6.83-7.01 (m, 2H), 6.65-6.74 (m, 1H), 6.52 (s, 1H), 6.06-6.20 (m, 1H), 5.57-5.77 (m, 1H), 4.54 (d, *J* = 10.8 Hz, 1H), 4.04-4.17 (m, 5H), 3.57 (t, *J* = 5.6 Hz, 2H), 3.41 (brs, 2H), 3.32 (s, 3H), 3.20-3.26 (m, 1H), 3.08-3.16 (m, 1H), 2.73-2.98 (m, 1H), 2.05-2.11 (m, 1H), 1.88-1.90 (m, 1H), 1.52-1.70 (m, 2H).

**Example 4**

*N*-(tert-butyl)-3-((6-((4-methyl-3,4,5,6-tetrahydro-2*H*-benzo[*b*][1,4,7]dioxazolin-9-yl) amino)-1*H*-pyrazolo[3,4-*d*]pyrimidin-4-yl)amino)benzenesulfonamide

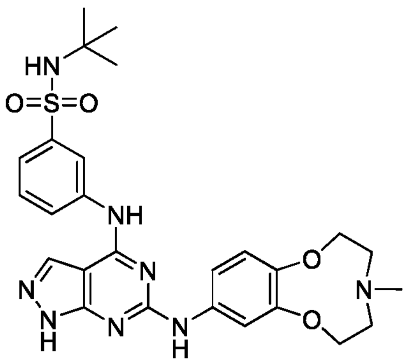

**4**

**Implementation steps (Implementation method 4)**

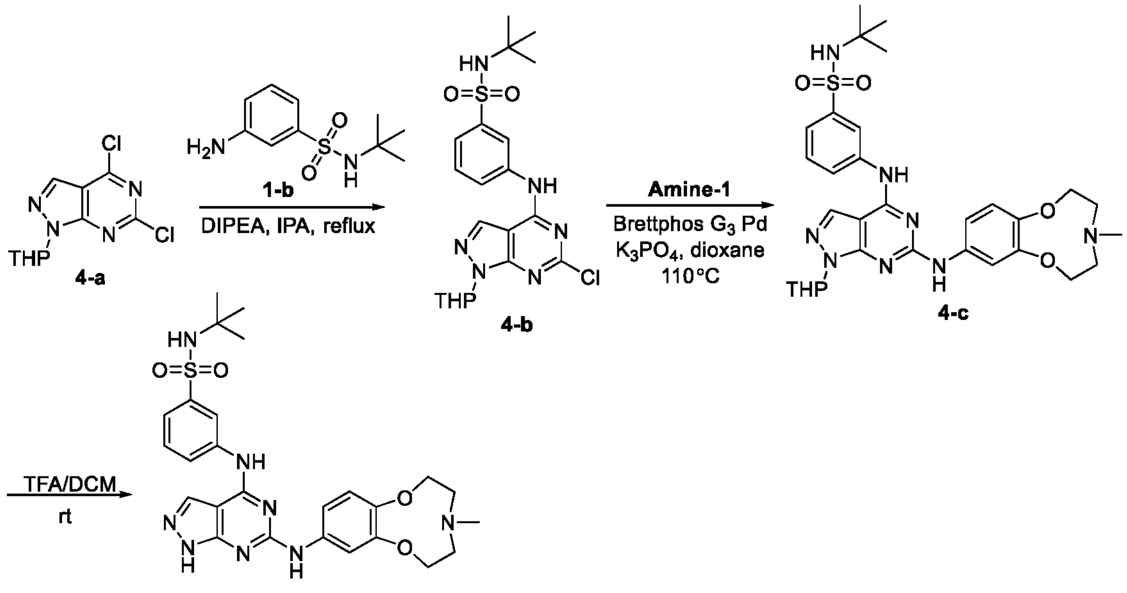


4

Step 1: synthesis of fragment 4-b *N*-(tert-butyl)-3-((6-chloro-1-(tetrahydro-2*H*-pyran-2-yl)-1*H*-pyrazolo[3,4-*d*]pyrimidin-4-yl) amino)benzenesulfonamide

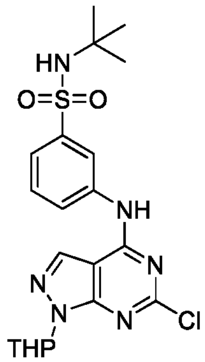

**4-b**

A solution of 4,6-dichloro-1-(tetrahydro-2*H*-pyran-2-yl)-1*H*-pyrazolo[3,4-*d*]pyrimidine (196 mg, 0.83 mmol), fragment **1-b** 3-amino-*N*-(tert-butyl)benzenesulfonamide (189 mg, 0.83 mmol) and *N,N*-diisopropylethylamine (128 mg, 0.99 mmol) in isopropanol ( 10 ml) was heated and refluxed with stirring for 6 hours. The completion of the reaction was monitored by TLC. The reaction solution was concentrated and the residue was partitioned in ethyl acetate (15 ml) and water (20 ml). The organic layer was collected, washed with saturated sodium chloride solution (20 ml), dried over anhydrous sodium sulfate, and concentrated under reduced pressure. The obtained crude product was separated and purified by silica gel column chromatography (eluted with 20%-33% ethyl acetate solution in n-hexane) to obtain *N*-(tert-butyl)-3-((6-chloro-1-(tetrahydro-2*H*-pyran-2-yl)-1*H*-pyrazolo[3,4-*d*]pyrimidin-4-yl) amino)benzenesulfonamide as a white solid (150 mg, 45%).

LC_MS: (ES$^+$): m/z 465.4 [M+H]$^+$.

$^1$H NMR (400 MHz, DMSO-d6): δ 10.77 (s, 1H), 8.34 (s, 1H), 8.19 (s, 1H), 8.02-8.11 (m, 1H), 7.61-7.66 (m, 3H), 5.81-5.84 (m, 1H), 3.94, 3.97 (two singles, 1H), 3.67-3.73 (m, 1H), 2.33-2.44 (m, 1H), 1.74-2.03 (m, 3H), 1.50-1.62 (m, 2H), 1.15 (s, 9H).

Step 2: synthesis of fragment **4-c** *N*-(tert-butyl)-3-((6-((4-methyl-3,4,5,6-tetrahydro-2*H*-benzo[*b*][1,4,7]dioxazolidin-9-yl)amino)-1 -(tetrahydro-2*H*-pyran-2-yl)-1*H*-pyrazolo[3,4-*d*]pyrimidin-4-yl)amino)benzenesulfonamide

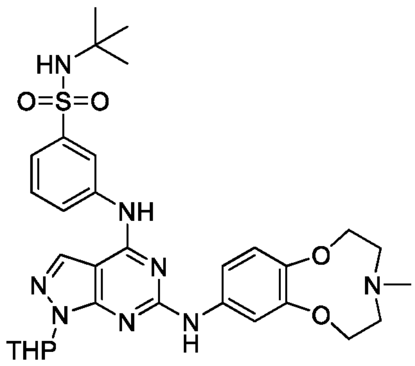

**4-c**

To a suspension of *N*-(tert-butyl)-3-((6-chloro-1-(tetrahydro-2H-pyran-2-yl)-1*H*-pyrazolo[3,4-*d*]pyrimidin-4-yl) amino)benzenesulfonamide (44. 6 mg, 0.096 mmol), 4-methyl-3,4,5,6-tetrahydro-2*H*-benzo[*b*][1,4,7]dioxazolin-9-amine (20 mg, 0.096 mmol) and potassium phosphate (61 mg, 0.29 mmol) in 1,4-dioxane (1 ml) was added BrettPhos Pd G3 (8.7 mg, 0.009 mmol) under nitrogen atmosphere at room temperature, the resulting mixture was replaced with nitrogen gas for three times, heated to 110°C and stirred for 16 hours of reaction. The completion of the reaction was monitored by TLC. The reaction solution was cooled to room temperature and partitioned in water (10 ml) and ethyl acetate (10 ml). The organic layer was collected and the aqueous layer was extracted with ethyl acetate (10 ml×2). The organic layers were combined, washed with saturated sodium chloride solution (20 ml), dried over anhydrous sodium sulfate, and concentrated under reduced pressure. The obtained crude product was separated and purified by preparative TLC (eluted with 5% methanol solution in dichloromethane) to obtain *N*-(tert-butyl)-3-((6-((4-methyl-3,4,5,6-tetrahydro-2*H*-benzo[*b*][1,4,7] dioxazolidin-9-yl)amino)-1-(tetrahydro-2*H*-pyran-2-yl)-1*H*-pyrazolo[3,4-*d*]pyrimidin-4-yl)amino )benzenesulfonamide as a yellow solid (52 mg, 80%).

LC_MS: ($ES^+$): m/z 637.2 $[M+H]^+$.

Step 3: synthesis of *N*-(tert-butyl)-3-((6-((4-methyl-3,4,5,6-tetrahydro-2*H*-benzo[*b*][1,4,7]dioxazolin-9-yl)amino)-1*H*-pyrazolo[3,4-*d*]pyrimidin-4-yl)amino)benzenesulfonamide

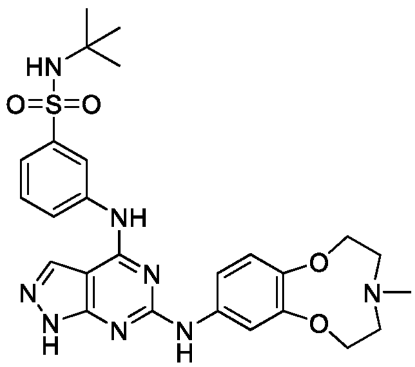

**4**

A solution of *N*-(tert-butyl)-3-((6-((4-methyl-3,4,5,6-tetrahydro-2*H*-benzo[*b*][1,4,7]dioxazolin-9-yl)amino)-1-(tetrahydro-2*H*-pyran-2-yl)-1*H*-pyrazolo[3,4-*d*]pyrimidin-4-yl)amino)benzenesulfonamide (52 mg, 0.082 mmol) and trifluoroacetic acid ( 0.5 ml) in dichloromethane (1.5 ml) was stirred at room temperature for 2 hours. The completion of the reaction was monitored by TLC. The organic solvent was concentrated under reduced pressure, and the residue was adjusted to pH=7-8 by adding sodium carbonate solution and then extracted with dichloromethane (10 ml×2). The organic layers were combined, washed with saturated sodium chloride solution (10 ml), dried over anhydrous sodium sulfate, and concentrated under reduced pressure. The obtained crude product was separated and purified by preparative TLC (eluted with 7.5% methanol solution in dichloromethane) to obtain *N*-(tert-butyl)-3-((6-((4-methyl-3,4,5,6-tetrahydro-2*H*-benzo[*b*][1,4,7]dioxazolin-9-yl)amino)-1*H*-pyrazolo[3,4-d]pyrimidin-4-yl)amino)benzenesulfonamide as a yellow solid (20 mg, 46%).

LC_MS: ($ES^+$): m/z 553.4 $[M+H]^+$.

$^1H$ NMR (400 MHz, DMSO-d6): δ 12.96 (s, 1H), 9.90 (s, 1H), 8.81 (s, 1H), 8.46 (d, *J* = 7.6 Hz, 1H), 8.28 (brs, 1H), 8.11 (s, 1H), 7.68 (s, 1H), 7.47-7.53 (m, 2H), 7.31 (brs, 1H), 7.06-7.09 (m, 1H), 6.66 (d, *J* = 8.8 Hz, 1H), 4.15 (t, *J* = 4.2 Hz, 2H), 3.53 (t, *J* = 5.6 Hz, 2H), 3.40 (t, *J* = 5.4 Hz, 2H), 3.33-3.35 (m, 1H), 3.29-3.30 (m, 1H), 3.27 (s, 3H), 1.14 (s, 9H).

**Example 5**

4-methyl-*N*-(4-(1-methyl-1*H*-indol-3-yl)-1*H*-pyrazolo[3,4-*d*]pyrimidin-6-yl)-3,4,5,6-tetrahydro-2*H*-benzo[*b*][1,4,7]dioxazolin-9-amine

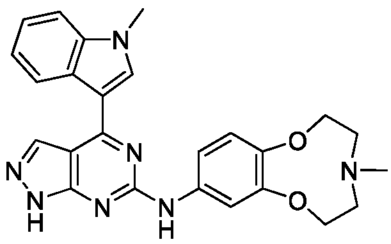

**5**

**Implementation steps (Implementation method 5)**

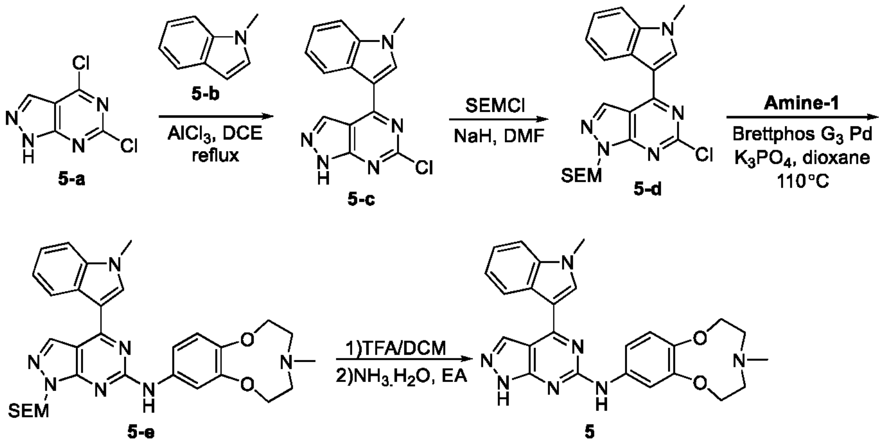


Step 1: synthesis of fragment **5-c** 6-chloro-4-(1-methyl-1*H*-indol-3-yl)-1*H*-pyrazolo[3,4-*d*]pyrimidine

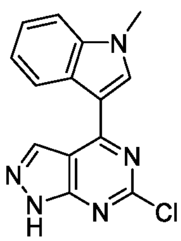

**5-c**

A solution of 4,6-dichloro-pyrazolo[3,4-d]pyrimidine (500 mg, 2.64 mmol), N-methylindole (347 mg, 2.64 mmol) and aluminum trichloride in 1,2-dichloroethane (10 ml) was heated to reflux for 12 hours. The completion of the reaction was monitored by TLC. The reaction mixture was partitioned in dichloromethane (10 ml) and water (20 ml). The organic layer was collected, washed with saturated sodium chloride solution (20 ml), dried over anhydrous sodium sulfate, and

concentrated under reduced pressure to obtain 6-chloro-4-(1-methyl-1*H*-indol-3-yl)-1*H*-pyrazolo[3,4-*d*]pyrimidine as a light yellow crude product (1.4 g), which was used in the next step without further purification.

Step 2: synthesis of fragment **5-d** 6-chloro-4-(1-methyl-1*H*-indol-3-yl)-1-(2-(trimethylsilyl)ethoxy)methyl)-1*H*-pyrazolo [3,4-*d*] pyrimidine

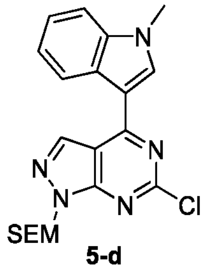

**5-d**

To a solution of 6-chloro-4-(1-methyl-1*H*-indole-3-yl)-1*H*-pyrazolo[3,4-*d*]pyrimidine (1.4 g, 4.9 mmol) in *N,N*-dimethylformamide (10 ml) was added sodium hydride (60%, 294 mg, 7.3mmol). The mixture was heated to room temperature and stirred for 1 hour , then cooled to 0°C. 2-(Trimethylsilyl)ethoxymethyl chloride (980 g, 5.8 mmol) was added dropwise to the reaction solution. The resulting mixture was stirred under nitrogen atmosphere at room temperature for 14 hours. The completion of the reaction was monitored by TLC. The reaction mixture was partitioned in ethyl acetate (100 ml) and water (100 ml). The organic layer was collected, washed with saturated sodium chloride solution (100 ml×2), dried over anhydrous sodium sulfate, and concentrated under reduced pressure. The obtained crude product was separated and purified by silica gel column chromatography (eluted with 0.5%-1% methanol solution in dichloromethane) to obtain 6-chloro-4-(1-methyl-1*H*-indol-3-yl)-1-(2-(trimethylsilyl)ethoxy)methyl)-1*H*-pyrazolo[3,4-*d*] pyrimidine as a white solid (245 mg, two-step 22%).

LC_MS: ($ES^+$): m/z 414.4 $[M+H]^+$.

$^1H$ NMR (400 MHz, DMSO-d6): δ 8.90 (d, *J* = 2.8 Hz, 2H), 8.62-8.64 (m, 1H), 7.61-7.63 (m, 1H), 7.31-7.38 (m, 2H), 5.70 (s, 2H), 3.98 (s, 3H), 3.64 (t, *J* = 8.0 Hz, 2H), 0.86 (t, *J* = 8.0 Hz, 2H), -0.08 (s, 9H).

Step 3: synthesis of fragment **5-e** 4-methyl-*N*-(4-(1-methyl-1*H*-indol-3-yl)-1-((2-(trimethylsilyl)ethoxy)methyl)-1*H*-pyrazolo[3,4-*d*] pyrimidin-6-yl)-3,4,5,6-tetrahydro-2*H*-benzo[*b*][1,4,7]dioxazolidin-9-amine

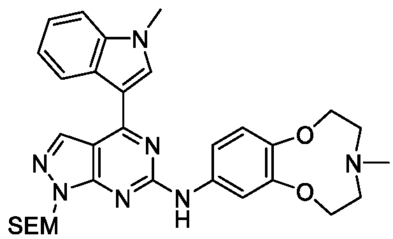

**5-e**

To a solution of 6-chloro-4-(1-methyl-1*H*-indol-3-yl)-1-(2-(trimethylsilyl)ethoxy)methyl)-1*H*-pyrazolo[3,4-*d*]pyri midine (100 mg, 0.24 mmol), 4-methyl-3,4,5,6-tetrahydro-2*H*-benzo[*b*][1,4,7]dioxazolin-9-amine (55 mg, 0.24 mmol) and potassium phosphate (154 mg, 0.72 mmol) in 1,4-dioxane (2 ml) was added BrettPhos Pd G3 (22 mg, 0.024 mmol). The resulting mixture was replaced with nitrogen for three times, heated to 110°C and stirred for 12 hours. The completion of the reaction was monitored by TLC. The reaction mixture was cooled to room temperature and partitioned in water (20 ml) and ethyl acetate (10 ml). The organic layer was collected and the aqueous phase was extracted with ethyl acetate (10 ml×2). The organic layers were combined, washed with saturated sodium chloride solution (20 ml), dried over anhydrous sodium sulfate, and concentrated under reduced pressure. The obtained crude product was separated and purified by preparative TLC (eluted with 5% methanol solution in dichloromethane) to obtain 4-methyl-*N*-(4-(1-methyl-1*H*-indol-3-yl)-1-((2-(trimethylsilyl)ethoxy)methyl)-1*H*-pyrazolo [3,4-*d*]pyrimidin-6-yl)-3,4,5,6-tetrahydro-2*H*-benzo[*b*][1,4,7]dioxazolidin-9-amine as a yellow solid (38 mg, 27%).

LC_MS: ($ES^+$): m/z 586.5 $[M+H]^+$.

Step 4: synthesis of 4-methyl-*N*-(4-(1-methyl-1*H*-indol-3-yl)-1*H*-pyrazolo[3,4-*d*]pyrimidin-6-yl)-3,4,5,6-tetrahydro-2 *H*-benzo[*b*][1,4,7]dioxazolin-9-amine

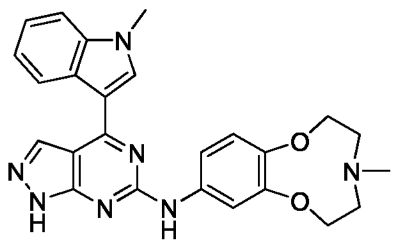

**5**

A solution of 4-methyl-*N*-(4-(1-methyl-1*H*-indol-3-yl)-1-(2-(trimethylsilyl)ethoxy)methyl)-1*H*-pyrazolo[3,4-*d*] pyrimidin-6-yl)-3,4,5,6-tetrahydro-2*H*-benzo[*b*][1,4,7]dioxazolin-9-amine (38 mg, 0.065 mmol) and trifluoroacetic acid (0.5 ml) in dichloromethane (0.5 ml) was stirred for 3 hours. The completion of the reaction was monitored by TLC. The reaction solution was concentrated, and the residue was added to a mixed solution containing ammonia hydroxide (2 ml) and ethyl acetate (4 ml). The resulting mixture was stirred at 30°C for 16 hours of reaction. The completion of the reaction was monitored by TLC. The organic layer was collected, washed with saturated sodium chloride solution (10 ml), dried over anhydrous sodium sulfate, and concentrated under reduced pressure. The obtained crude product was separated and purified by preparative TLC (eluted with 10% methanol solution in dichloromethane) to obtain 4-methyl-*N*-(4-(1-methyl-1*H*-indol-3-yl)-1*H*-pyrazolo[3,4-*d*]pyrimidin-6-yl)-3,4,5,6-tetrahydro-2 *H*-benzo[*b*][1,4,7]dioxazolin-9-amine as a yellow solid (18.5 mg, 62%).

LC_MS: (ES$^+$): m/z 456.2 [M+H]$^+$.

$^1$H NMR (400 MHz, $CDCl_3$): δ 8.62 (d, *J* = 7.6 Hz, 1H), 8.14 (s, 1H), 7.91 (s, 1H), 7.28-7.41 (m, 6H), 7.06-7.08 (m, 1H), 6.68 (d, *J* = 8.4 Hz, 1H), 4.26 (t, *J* = 4.2 Hz, 2H), 3.93 (s, 3H), 3.61 (t, *J* = 5.6 Hz, 2H), 3.42-3.55 (m, 4H), 3.38 (s, 3H).

**Example 6**

1-methyl-4-((2-((7-methyl-2,3,6,7,8,9-hexahydro-5*H*-benzo[*b*][1,4,7]trioxa[10]azacyclo dodecan-12-yl)amino)-7*H*-pyrrolo[2,3-*d*]pyrimidin-4-yl)oxy)cyclohexanol

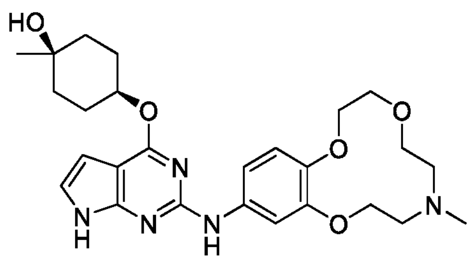

**6**

**Implementation steps (Implementation method 6)**

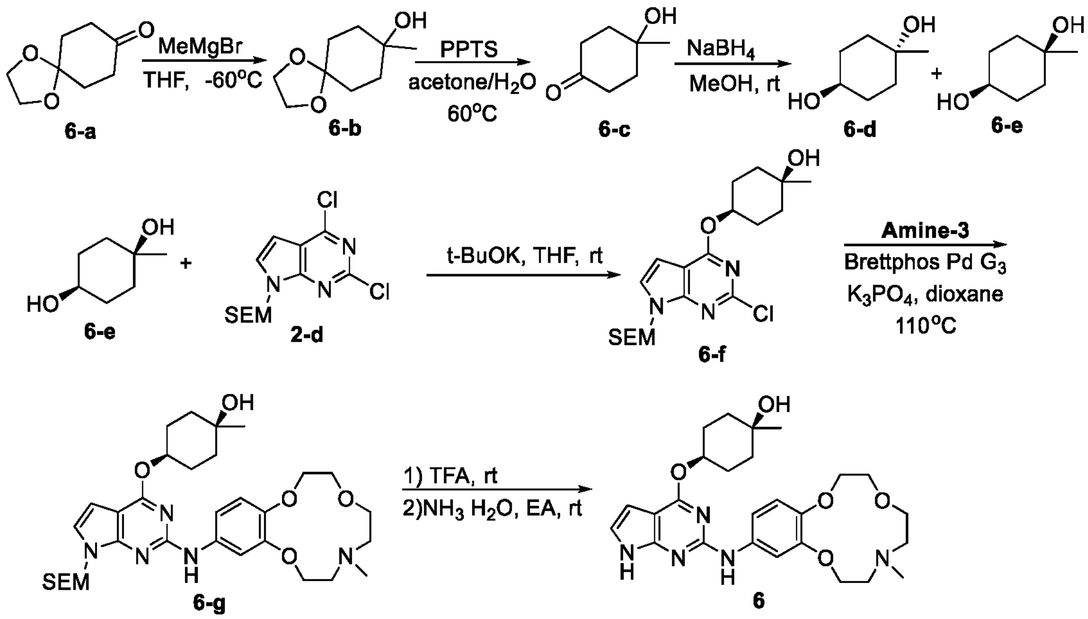


Step 1 : synthesis of fragment **6-b** 8-methyl-1,4-dioxaspiro[4.5]decan-8-ol

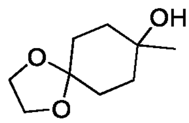

**6-b**

To a suspension of 1,4-cyclohexanedione monoethylene glycol ketal (2 g, 12.8 mmol) in anhydrous tetrahydrofuran (20 ml) was added methylmagnesium bromide (3M, dissolved in tetrahydrofuran, 7.7 ml, 23 mmol) at -60°C. The reaction mixture was stirred at -60°C for 20 minutes, then heated to -30°C and stirred for 30 minutes, and then stirred at 0°C for 30 minutes. The completion of the reaction was monitored by TLC. The reaction mixture was quenched with saturated ammonium chloride solution (10 ml) and extracted with ethyl acetate (20 ml). The organic layer was collected, washed with saturated sodium chloride solution (20 ml), dried over anhydrous sodium sulfate, and concentrated under reduced pressure to obtain 8-methyl-1,4-dioxaspiro[4.5]decan-8-ol (1.4 g, 63%) as a colorless oil, which was used directly in the next step without further purification.

Step 2 : synthesis of fragment **6-c** 4-hydroxy-4-methylcyclohexanone

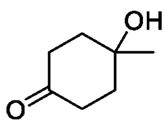

**6-c**

A suspension of 8-methyl-1,4-dioxaspiro[4.5]decan-8-ol (1.4 g, 8.1 mmol) and pyridinium p-toluenesulfonate (408.6 mg, 1.63 mmol) in acetone (16 ml) and water (8 ml) was stirred at 60°C for 13 hours under nitrogen protection. The completion of the reaction was monitored by TLC. The reaction mixture was cooled to room temperature, quenched by adding water (50 ml), and extracted with ethyl acetate (30 ml×2). The organic layers were combined, washed with saturated sodium chloride solution (20 ml), dried over anhydrous sodium sulfate, and concentrated under reduced pressure. The obtained crude product was separated and purified by silica gel column chromatography (eluted with 15% ethyl acetate in n-hexane) to obtain 4-hydroxy-4-methylcyclohexanone (630 mg, 57%) as a yellow oil.

Step 3 : synthesis of fragments **6-d** (1*r*,4*r*)-1-methylcyclohexan-1,4-diol **and 6-e** (1*s*,4*s*)-1-methylcyclohexan-1,4-diol

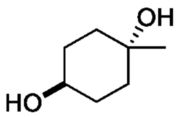

**6-d**

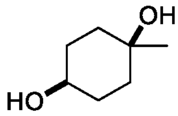

**6-e**

To a suspension of 4-hydroxy-4-methylcyclohexanone (630 mg, 4.9 mmol) in methanol (6 ml) was added sodium borohydride (372.1 mg, 9.5 mmol) at 0°C. The reaction mixture was stirred at room temperature for 12 hours. The completion of the reaction was monitored by TLC. The reaction mixture was quenched with water (0.5 ml) and concentrated under reduced pressure. The obtained crude product was separated and purified by silica gel column chromatography (eluted with 4% methanol in dichloromethane) to obtain 6-d (1*r* ,4*r*)-1-methylcyclohexan-1,4-diol as a white solid (80 mg, 12%),

$^1$H NMR (400 MHz, $CDCl_3$): δ 4.04 - 3.71 (m, 1H), 1.89 (dq, *J* = 16.0, 9.6, 6.7 Hz, 2H), 1.89 (dq, *J* = 16.0, 9.6, 6.7 Hz, 2H), 1.59 - 1.36 (m, 6H), 1.26 (s, 3H).

and 6-e (1*s* ,4*s*)-1-methylcyclohexan-1,4-diol as a white solid (400 mg, 60%);

$^1$H NMR (400 MHz, $CDCl_3$): δ 3.60 (tt, *J* = 9.7, 4.1 Hz, 1H), 1.81 - 1.72 (m, 2H), 1.72 - 1.58 (m, 4H), 1.55 - 1.37 (m, 4H), 1.22 (s, 3H).

Step 4: synthesis of fragment **6-f** (1*s*,4*s*)-4-((2-chloro-7-((2-(trimethylsilyl)ethoxy)methyl)-7*H*-pyrrolo[2,3-*d*]pyrimidin-4-yl)oxy)-1-methylcyclohexan-1-ol

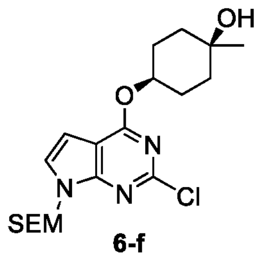

**6-f**

To a solution of (1s,4s)-1-methylcyclohexane-1,4-diol (40.4 mg, 0.3 mmol) and 2,4-dichloro-7-(((2-(trimethylsilyl) ethoxy)methyl)-7*H*-pyrrolo[2,3-*d*]pyrimidine (100 mg, 0.3 mmol) in anhydrous tetrahydrofuran (5 ml) was added potassium tert-butoxide (70.7 mg, 0.6 mmol) under nitrogen protection at 0°C, and the reaction mixture was stirred at room temperature for 13 hours of reaction. The completion of the reaction was monitored by TLC. The reaction mixture was partitioned in water (20 ml) and ethyl acetate (20 ml). The aqueous phase was extracted with ethyl acetate (10 ml×2). The organic layers were combined, washed with saturated sodium chloride solution (30 ml), dried over anhydrous sodium sulfate, and concentrated under reduced pressure. The obtained crude product was separated and purified by silica gel column chromatography (eluted with 25% ethyl acetate in n-hexane) to obtain (1*s*,4*s*)-4-((2-chloro-7-((2-(trimethylsilyl) ethoxy)methyl)-7*H*-pyrrolo[2,3-*d*]pyrimidin-4-yl)oxy)-1-methylcyclohexane-1-ol as a colorless oil (100 mg, 79%).

LC_MS: (ES$^+$): m/z 412.2 [M+H]$^+$.

Step 5 : synthesis of fragment **6-g** (1*s*,4*s*)-1-methyl-4-((2-((7-methyl-2,3,6,7,8,9-hexahydro-5*H*-benzo[*b*][1,4,7]trioxa [10]azacyclod odecan-12-yl)amino)-7-((2-(trimethylsilyl)ethoxy)methyl)-7*H*-pyrrolo[2,3-*d*]pyrimidin-4-yl)oxy) cyclohexan-1-ol

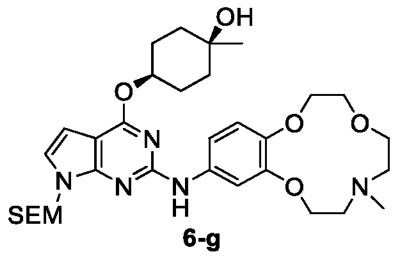

6-g

To a suspension of (1*s*,4*s*)-4-((2-chloro-7-(((2-(trimethylsilyl)ethoxy)methyl)-7*H*-pyrrolo[2,3-*d*]pyrimidin-4-yl) oxy)-1-methylcyclohexanol (100 mg, 0.24 mmol), **Amine-3** 6,7,8,9-hexahydro-5*H*-benzo[*b*][1,4,7]trioxa[10]azacyclododecan-12-amine (61.2 mg, 0.24 mmol) and potassium phosphate (154.8 mg, 0.73 mmol) in 1,4-dioxane (2 ml) was added BrettPhos Pd G3 (21.8 mg, 0.02 mmol) under nitrogen atmosphere at room temperature. The reaction mixture was replaced with nitrogen for three times and stirred at 110°C for 14 hours. The completion of the reaction was monitored by TLC. The reaction mixture was cooled to room temperature and partitioned in water (20 ml) and ethyl acetate (10 ml). The organic layer was collected and the aqueous layer was extracted with ethyl acetate (10 ml×2). The organic layers were combined, washed with saturated sodium chloride solution (20 ml), dried over anhydrous sodium sulfate, and concentrated under reduced pressure. The obtained crude product was subjected to preparative TLC (eluted with 10% methanol and 1% ammonia in dichloromethane) to obtain (1*s*,4*s*)-1-methyl-4-((2-((7-methyl-2,3,6,7,8,9-hexahydro-5*H*-benzo[*b*][1,4,7]trioxa[10]azacyclod odecan-12-yl)amino)-7-((2-(trimethylsilyl)ethoxy)methyl)-7*H*-pyrrolo[2,3-*d*]pyrimidin-4-yl) oxy) hexanol as a yellow solid (70 mg, 46%).

LC_MS: ($ES^+$): m/z 628.3 $[M+H]^+$.

Step 6 : synthesis of (1*s*,4*s*)-1-methyl-4-((2-((7-methyl-2,3,6,7,8,9-hexahydro-5*H*-benzo[*b*][1,4,7]trioxa[10]azacyclod odecyl-12-yl)amino)-7*H*-pyrrolo[2,3-*d*]pyrimidin-4-yl)oxy)cyclohexan-1-ol

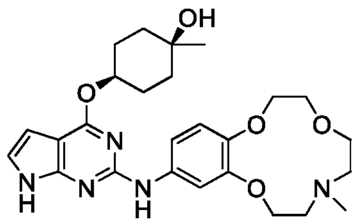

6

A solution of (1*s*,4*s*)-1-methyl-4-((2-((7-methyl-2,3,6,7,8,9-hexahydro-5*H*-benzo[*b*][1,4,7]trioxa[10]azacyclod odecan-12-yl)amino)-7-(((2-(trimethylsilyl)ethoxy)methyl)-7*H*-pyrrolo[2,3-*d*]pyrimidin-4-yl)oxy )hexanol (70 mg, 0.11 mmol) and trifluoroacetic acid (1 ml) in dichloromethane (1 ml) was stirred at 0°C for 3 hours. The completion of the reaction was monitored by TLC. The volatiles were removed under reduced pressure, and the residue was adjusted to pH=7-8 with saturated sodium carbonate solution and extracted with dichloromethane (10 ml×2). The organic layers were combined, washed with saturated sodium chloride solution (20 ml), dried over anhydrous sodium sulfate and concentrated. The residue was added to a solvent mixture containing ammonia (2 ml) and ethyl acetate (4 ml). The reaction mixture was stirred at 30°C for 12 hours. The completion of the reaction was monitored by TLC. The organic layer was collected, washed with saturated sodium chloride solution (10 ml), dried over anhydrous sodium sulfate, and concentrated under reduced pressure. The obtained crude product was separated and purified by preparative TLC (eluted with 10% methanol in dichloromethane) to obtain 1-methyl-4-((2-((7-methyl-2,3,6,7,8,9-hexahydro-5*H*-benzo[*b*][1,4,7]trioxa[10] azacyclododecan-12-yl)amino)-7*H*-pyrrolo[2,3-*d*]pyrimidin-4-yl)oxy)cyclohexanol as an orange solid (42.6 mg, 76%).

LC_MS: ($ES^+$): m/z 498.2 $[M+H]^+$.

$^1H$ NMR (400 MHz, DMSO-*d*6): δ 11.33 (s, 1H), 8.90 (s, 1H), 7.66 (s, 1H), 7.35 (brs, 1H), 6.95-6.98 (m, 2H), 6.25-6.27 (m, 1H), 5.16-5.22 (m, 1H), 4.08-4.22 (m, 5H), 3.72 (s, 4H), 3.29 (s, 3H), 2.82-3.05 (m, 4H), 1.86-1.87 (m, 4H), 1.66-1.69 (m, 2H), 1.41-1.48 (m, 2H), 1.17 (s, 3H).

**Example 7**

(1*s*,4*s*)-1-methyl-4-((2-((7-methyl-2,3,6,7,8,9-hexahydro-5*H*-benzo[*b*][1,4,7]trioxa[10]a zacyclododecan-12-yl)amino)-5-(trifluoromethyl)pyrimidin-4-yl)oxy)cyclohexan-1-ol

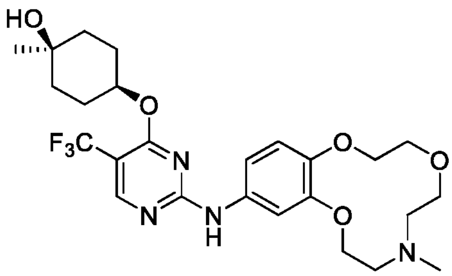

**7**

**Implementation steps (Implementation method 7)**

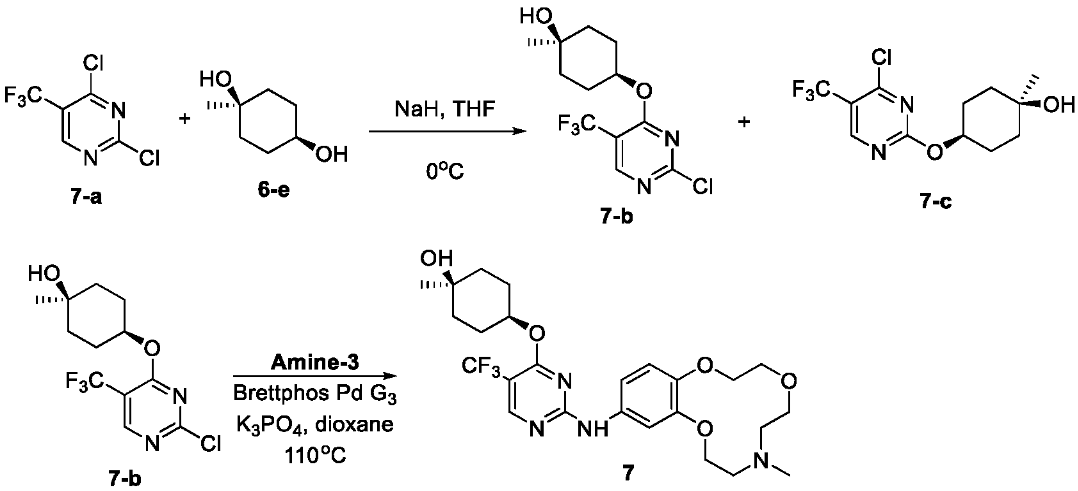


**7-a** **6-e** **7-b** **7-c** **7-b** **7**

Step 1: synthesis of fragment **7-b** (1*s*,4*s*)-4-((2-chloro-5-(trifluoromethyl)pyrimidin-4-yl)oxy)-1-methylcyclohexan-1-ol and fragment **7-c** (1*s*,4*s*)-4-((4-chloro-5-(trifluoromethyl)pyrimidin-2-yl)oxy)-1-methylcyclohexan-1-ol

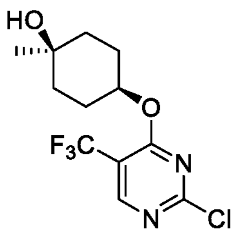

**7-b**

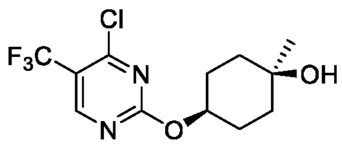

**7-c**

To a solution of (1s,4s)-1-methylcyclohexan-1,4-diol (100 mg, 0.77 mmol) in tetrahydrofuran (3 ml) was added sodium hydride (60%, 68 mg, 1.69 mmol) under nitrogen protection at 0°C. The mixture was slowly heated to room temperature and then stirred for 30 minutes. The reaction solution was then added dropwise to a solution of 2,4-dichloro-5-(trifluoromethyl)pyrimidine (334 mg, 1.54 mmol) in tetrahydrofuran (2 ml), and the reaction solution was stirred at room temperature for 16 hours. The completion of the reaction was monitored by TLC. The reaction mixture was quenched with saturated ammonium chloride solution (10 ml) and extracted with ethyl acetate (10 ml×2). The organic

layer was collected, washed with saturated sodium chloride solution (20 ml), dried over anhydrous sodium sulfate, and concentrated under reduced pressure. The obtained crude product was separated and purified by silica gel column chromatography (eluted with 6%-12.5% ethyl acetate in n-hexane) to obtain two groups of compounds: 7-b (1*s*,4*s*)-4-((2-chloro-5-(trifluoromethyl)pyrimidin-4-yl)oxy)-1-methylcyclohexan-1-ol as colorless oil (first eluting fraction) (28 mg, 12%);

LC_MS: ($ES^+$): m/z 311.0 $[M+H]^+$.

and 7-c (1*s*,4*s*)-4-((4-chloro-5-(trifluoromethyl)pyrimidin-2-yl)oxy)-1-methylcyclohexan-1-ol as colorless oil (second eluting fraction) (52 mg, 21%).

LC_MS: ($ES^+$): m/z 311.0 $[M+H]^+$.

Step 2 : synthesis of (1*s*,4*s*)-1-methyl-4-((2-((7-methyl-2,3,6,7,8,9-hexahydro-5*H*-benzo[*b*][1,4,7]trioxa[10]azacyclod odecan-12-yl)amino)-5-(trifluoromethyl)pyrimidin-4-yl)oxy)cyclohexan-1-ol

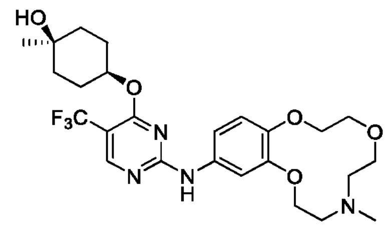

**7**

To a suspension of (1*s*,4*s*)-4-((2-chloro-5-(trifluoromethyl)pyrimidin-4-yl)oxy)-1-methylcyclohexan-1-ol (20.0 mg, 0.064 mmol), 6,7,8,9-hexahydro-5*H*-benzo[*b*][1,4,7]trioxa[10]azacyclododecan-12-amine (19.4 mg, 0.077 mmol) and potassium phosphate (154.8 mg, 0.73 mmol) in 1,4-dioxane (2 ml) was added BrettPhos Pd G3 (21.8 mg, 0.02 mmol) under nitrogen atmosphere at room temperature. The reaction mixture was replaced with nitrogen for three times and stirred at 110°C for 12 hours. The reaction was complete by TLC monitoring. The reaction mixture was cooled to room temperature and partitioned in water (10 ml) and ethyl acetate (10 ml). The organic layer was collected, and the aqueous layer was extracted with ethyl acetate (10 ml×2). The organic layers were combined, washed with saturated sodium chloride solution (20 ml), dried over anhydrous sodium sulfate, and concentrated under reduced pressure. The obtained crude product was purified by preparative TLC (eluted with 10% methanol and 1% ammonia in dichloromethane) to obtain (1*s*,4*s*)-1-methyl-4-((2-((7-methyl-2,3,6,7,8,9-hexahydro-5*H*-benzo[*b*][1,4,7]trioxa[10]azacyclod odecan-12-yl)amino)-5-(trifluoromethyl)pyrimidin-4-yl)oxy)cyclohexan-1-ol as a gray-white solid (10 mg, 30%).

LC_MS: ($ES^+$): m/z 527.5 $[M+H]^+$.

$^1$H NMR (400 MHz, $CD_3OD$): δ 8.34 (s, 1H), 7.54 (brs, 1H), 7.28-7.30 (m, 1H), 7.09 (d, *J* = 8.8 Hz, 1H), 5.18-5.22 (m, 1H), 4.35 (t, *J* = 4.8 Hz, 2H), 4.19 (t, *J* = 4 Hz, 2H), 3.82-3.97 (m, 4H), 3.43-3.53 (m, 2H), 2.91 (s, 3H), 1.90-1.96 (m, 4H), 1.77-1.81 (m, 2H), 1.50-1.57 (m, 2H), 1.25 (s, 3H).

## Example 8

2-methyl-1-((2-((4-methyl-3,4,5,6-tetrahydro-2*H*-benzo[*b*][1,4,7]dioxazolin-9-yl)amino )-5-(trifluoromethyl)pyrimidin-4-yl)amino)propan-2-ol

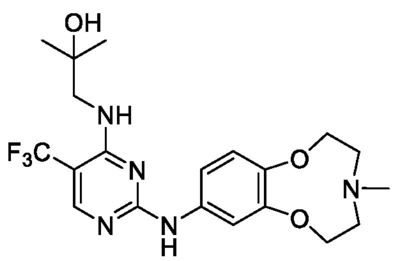

**8**

**Implementation steps (Implementation method 8)**

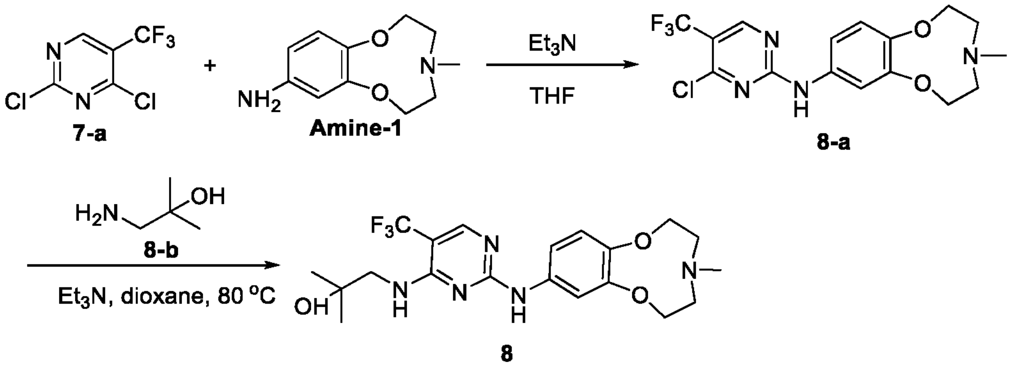


7-a Amine-1 8-a 8-b 8

Step 1: synthesis of fragment **8-a** *N*-(4-chloro-5-(trifluoromethyl)pyrimidin-2-yl)-4-methyl-3,4,5,6-tetrahydro-2*H*-benzo [*b*][1,4,7] dioxazolin-9-amine

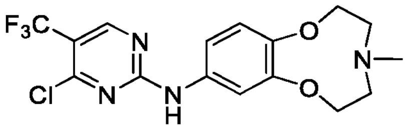

**8-a**

A solution of 2,4-dichloro-5-(trifluoromethyl)pyrimidine (800 mg, 3.69 mmol), 4-methyl-3,4,5,6-tetrahydro-2*H*-benzo[*b*][1,4,7]dioxazolin-9-amine (768 mg, 3.69 mmol) and triethylamine (750 mg, 7.40 mmol) in tetrahydrofuran (30 ml) was stirred at room temperature for 16 hours. The completion of the reaction was monitored by TLC. The reaction solution was concentrated and the residue was partitioned in ethyl acetate (15 ml) and water (20 ml). The organic layer was collected, washed with saturated sodium chloride solution (20 ml), dried over anhydrous sodium sulfate, and concentrated under reduced pressure. The obtained crude product was separated and purified by silica gel column chromatography (eluted with 20%-33% ethyl acetate solution in n -hexane) to obtain a yellow solid, which was then separated by preparative HPLC (mobile phase: 30%-90% acetonitrile solution in water in a gradient elution for 25 minutes) to obtain the first eluting component, which was *N*-(2-chloro-5-(trifluoromethyl)pyrimidin-4-yl)-4-methyl-3,4,5,6-tetrahydro-2*H*-benzo[*b*][1,4,7] dioxazolin-9-amine as a yellow solid(430 mg, 30%);

LC_MS: ($ES^+$): m/z 389.0 $[M+H]^+$.

$^1H$ NMR (400 MHz, $CDCl_3$): δ 8.34 (d, *J* = 1.2 Hz, 1H), 6.97 (d, *J* = 2.5 Hz, 1H), 6.93 (dd, *J*= 8.7, 2.6 Hz, 1H), 6.87 (s, 1H), 6.65 (d, *J* = 8.6 Hz, 1H), 4.27 - 4.18 (m, 2H), 3.60 (t, *J*= 5.6 Hz, 2H), 3.46 (dt, *J* = 8.9, 5.0 Hz, 4H), 3.37 (s, 3H).

The second eluting fraction was *N*-(4-chloro-5-(trifluoromethyl)pyrimidin-2-yl)-4-methyl-3,4,5,6-tetrahydro-2*H*-benzo[*b*][1,4,7]d ioxazolin-9-amine as a yellow solid (406 mg, 28%);

LC_MS: ($ES^+$): m/z 389.0 $[M+H]^+$.

$^1H$ NMR (400 MHz, $CDCl_3$):δ 8.48 (s, 1H), 7.35 (s, 1H), 7.03 (d, *J* = 2.5 Hz, 1H), 6.93 (d, *J*= 7.8 Hz, 1H), 6.64 (d, *J* = 8.7 Hz, 1H), 4.30 - 4.17 (m, 2H), 3.59 (t, *J* = 5.6 Hz, 2H), 3.45 (dq, *J* = 11.9, 6.1, 4.5 Hz, 4H), 3.36 (s, 3H).

Step 2 : synthesis of 2-methyl-1-((2-((4-methyl-3,4,5,6-tetrahydro-2*H*-benzo[*b*][1,4,7]dioxazolin-9-yl)amino)-5-(trifluoromethyl)pyrimidin-4-yl)amino)propan-2-ol

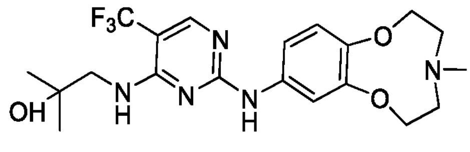

**8**

A solution of *N*-(4-chloro-5-(trifluoromethyl)pyrimidin-2-yl)-4-methyl-3,4,5,6-tetrahydro-2*H*-benzo[*b*][1,4,7]dioxazolin-9-amine (30 mg, 77.2 μmol), 1-amino-2-methylpropan-2-ol (14 mg, 155 μmol) and triethylamine (16 mg, 155 μmol) in 1,4-dioxane (1 ml) was stirred at 80°C for 16 hours. The completion of the reaction was monitored by TLC. The reaction solution was concentrated and the residue was partitioned in ethyl acetate (15 ml) and water (20 ml). The completion of the reaction was monitored by TLC. The organic layer was collected, washed with saturated sodium chloride solution (10 ml), dried over anhydrous sodium sulfate, and concentrated under reduced pressure. The obtained crude product was separated and purified by preparative TLC (eluted with 50% ethyl acetate in petroleum ether) to obtain 2-methyl-1-((2-((4-methyl-3,4,5,6-tetrahydro-2*H*-benzo[*b*][1,4,7]dioxazolin-9-yl)amino)-5-(trifluoromethyl)pyrimidin-4-yl)amino)propan-2-ol as an orange solid (18 mg, 52%).

LC_MS: ($ES^+$): m/z 442.1 $[M+H]^+$.

**Example 9**

***N*-(tert-butyl)-3-((5-fluoro-2-((7-methyl-2,3,6,7,8,9-hexahydro-5*H*-benzo[*b*][1,4,7]tr ioxa[10]azacyclododecan-13-yl)amino)pyrimidin-4-yl)amino)benzenesulfonamide**

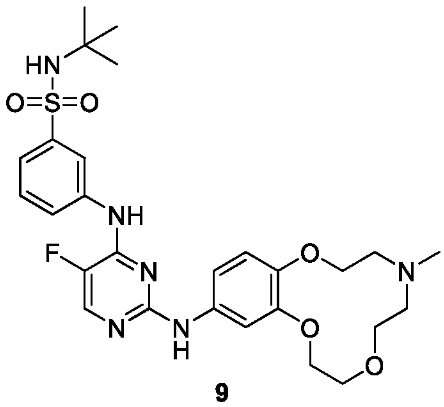

**9**

The implementation method was the same as method 1, except that fragment Amine-2 was used to replace fragment Amine-1 to obtain the title compound.

LC_MS: ($ES^+$): m/z 575.9 $[M+H]^+$.

$^1$H NMR (400 MHz, DMSO-d6): δ 9.67 (s, 1H), 9.10 (s, 1H), 8.19-8.14 (m, 3H), 7.60 (s, 1H), 7.52-7.49 (m, 2H), 7.43 (s, 1H), 7.22-7.19 (m, 1H), 6.96-6.94 (d, *J* = 8.4 Hz, 1H), 4.13-4.09 (m, 2H), 3.96 (s, 2H), 3.73 (s, 4H), 3.30 (s, 3H), 3.18-2.85 (m, 4H), 1.12 (s, 9H).

**Example 10**

*N*4-(2-(isopropylsulfonyl)phenyl)-*N*2-(7-methyl-2,3,6,7,8,9-hexahydro-5*H*-benzo[*b*][1,4, 7]trioxa[10]azacyclododecan-13-yl)-7*H*-pyrrolo[2,3-*d*]pyrimidin-2,4-diamine

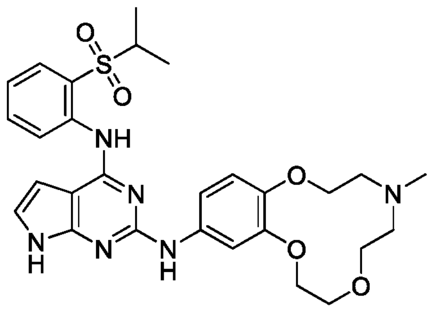

**10**

The implementation method was the same as method 4, except that fragment 2-d was used to replace fragment 4-a, fragment **10-a** 2-(isopropylsulfonyl)aniline was used to replace ragment 1-b, and fragment Amine-2 was used to replace fragment Amine-1 to obtain the title compound.
LC_MS: ($ES^+$): m/z 567.9 $[M+H]^+$.
$^1H$ NMR (400 MHz, DMSO-d6): δ 11.43 (s, 1H), 9.48 (s, 1H), 8.88-8.93 (m, 2H), 7.82 (d, *J* = 8.0 Hz, 1H), 7.73 (t, *J* = 7.8 Hz, 1H), 7.58 (s, 1H), 7.27-7.31 (m, 2H), 7.04 (d, *J* = 3.2 Hz, 1H), 6.91 (d, *J* = 8.4 Hz, 1H), 6.25 (d, *J* = 2.8 Hz, 1H), 4.01-4.12 (m, 4H), 3.61-3.77 (m, 4H), 3.42-3.51 (m, 1H), 3.29 (s, 3H), 2.72-2.87 (m, 2H), 2.26-2.38 (m, 2H), 1.18 (d, *J* = 6.8 Hz, 6H).

**Example 11**

*N*4-(2-(isopropylsulfonyl)phenyl)-*N*6-(7-methyl-2,3,6,7,8,9-hexahydro-5*H*-benzo[*b*][1,4, 7]trioxa[10]azacyclododecan-13-yl)-1*H*-pyrazolo[3,4-*d*]pyrimidin-4,6-diamine

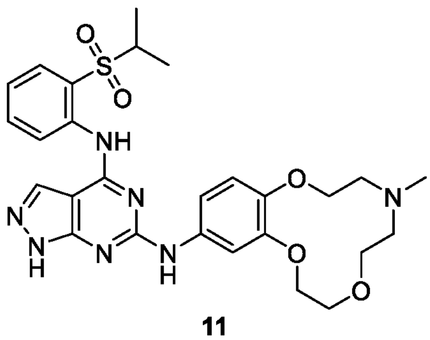

**11**

The implementation method was the same as method 4, except that fragment 2-d was used to replace fragment 4-a, fragment 2-(isopropylsulfonyl)aniline was used to replace fragment 1-b, and fragment Amine-2 was used to replace fragment Amine-1 to obtain the title compound.
LC_MS: ($ES^+$): m/z 568.6 $[M+H]^+$.
$^1H$ NMR (400 MHz, $CDCl_3$): δ 9.86 (s, 1H), 8.75-8.77 (m, 1H), 7.89-7.92 (m, 2H), 7.66-7.70 (m, 1H), 7.56 (s, 1H), 7.25-7.29 (m, 1H), 7.08-7.11 (m, 1H), 6.94-6.96 (m, 1H), 4.41 (s, 2H), 4.14-4.16 (m, 2H), 4.07 (s, 2H), 3.90 (s, 2H), 3.62 (s, 4H), 3.22-3.29 (m, 1H), 2.96 (s, 3H), 1.30-1.32 (d, *J* = 6.8 Hz, 6H).

**Example 12**

*N*4-(2-(isopropylsulfonyl)phenyl)-*N*6-(7-methyl-2,3,6,7,8,9-hexahydro-5*H*-benzo[*b*][1,4, 7]trioxa[10]azacyclododecan-12-yl)-1*H*-pyrazolo[3,4-*d*]pyrimidin-4,6-diamine

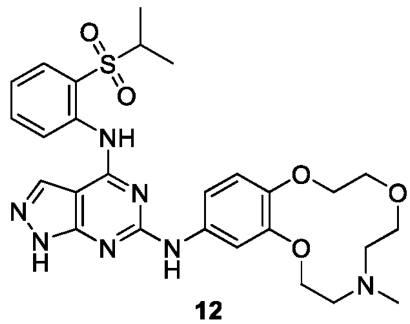

**12**

The implementation method was the same as method 4, except that fragment 2-d was used to replace fragment 4-a, fragment 2-(isopropylsulfonyl)aniline was used to replace fragment 1-b, and fragment Amine-3 was used to replace fragment Amine-1 to obtain the title compound.
LC_MS: ($ES^+$): m/z 568.5 $[M+H]^+$.
$^1H$ NMR (400 MHz, DMSO-d6): δ 13.11 (s, 1H), 9.65 (s, 1H), 9.15 (s, 1H), 8.34-8.50 (m, 1H), 7.90-7.92 (m, 1H), 7.78-7.82 (m, 2H), 7.44-7.48 (m, 2H), 7.25-7.28 (m, 1H), 6.90 (d, *J* = 8.4 Hz, 1H), 3.98-4.10 (m, 4H), 3.62-3.74 (m, 4H), 3.41-3.46 (m, 1H), 3.30 (s, 3H), 2.73-2.92 (m, 2H), 2.31-2.46 (m, 2H), 1.14 (d, *J* = 6.4 Hz, 6H).

**Example 13**

*N*-(5-fluoro-4-(1-methyl-*1H*-indol-3-yl)pyrimidin-2-yl)-4-methyl-3,4,5,6-tetrahydro-2*H* -benzo[*b*][1,4,7]bisoxazolin-9-amine

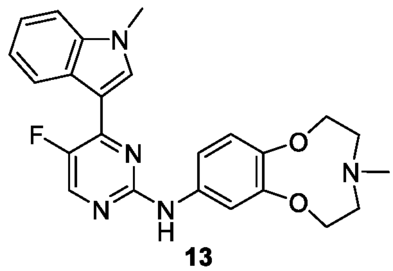

**13**

The implementation method was the same as method 5, except that fragment 1-a was used to replace fragment 5-a to obtain the title compound.
LC_MS: ($ES^+$): m/z 434.3 $[M+H]^+$.
$^1HNMR$ (400 MHz, $CDCl_3$): δ 8.69 (d, *J* = 8.0 Hz, 1H), 8.15 (d, *J* = 4.0 Hz, 1H), 7.93 (d, *J* = 2.4 Hz, 1H), 7.30-7.38(m, 2H), 7.23-7.26(m, 1H), 7.13 (d, *J* = 2.4 Hz, 1H), 7.02-7.04 (m, 1H), 6.83 (s, 1H), 6.68 (d, *J* = 8.4 Hz, 1H), 4.26 (t, *J* = 4.4 Hz, 2H), 3.88 (s, 3H), 3.62 (t, *J* = 5.6 Hz, 2H), 3.42-3.48 (m, 4H), 3.38(s, 3H).

**Example 14**

**2-(1-(ethylsulfonyl)-3-(4-(5-fluoro-2-((7-methyl-2,3,6,7,8,9-hexahydro-5*H*-benzo[*b*][ 1,4,7]trioxa[10]azacyclododecan-13-yl)amino)pyrimidin-4-yl)-1*H*-pyrazol-1-yl)azetidin-3-yl) acetonitrile**

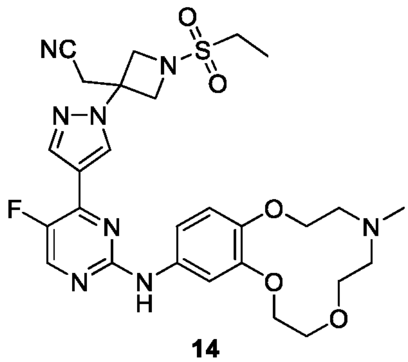

**14**

The implementation method was the same as method 2, except that fragment 1-a was used to replace fragment 2-d, fragment 14-e was used to replace fragment 2-b, and fragment Amine-2 was used to replace fragment Amine-1 to obtain the title compound.

LC_MS: ($ES^+$): m/z 601.6 $[M+H]^+$.

$^1$HNMR (400 MHz, DMSO-d6): δ 9.57 (s, 1H), 8.72 (s, 1H), 8.56 (d, *J* = 2.0 Hz, 1H), 8.25 (s, 1H), 7.59 (s, 1H), 7.33 (d, *J* = 8.8 Hz, 1H), 7.03 (d, *J* = 8.4 Hz, 1H), 4.53 (d, *J* = 9.2 Hz, 2H), 4.24 (d, *J* = 8.8 Hz, 2H), 4.21-4.10 (m, 4H), 3.89-3.74 (m, 4H), 3.69 (s, 2H), 3.30 (s, 3H), 3.26-3.21 (m, 2H), 3.14-2.91 (m, 2H), 2.67-2.56 (m, 2H), 1.24 (t, *J* = 7.2 Hz, 3H).

**Fragment 14-e was synthesized as follows:**

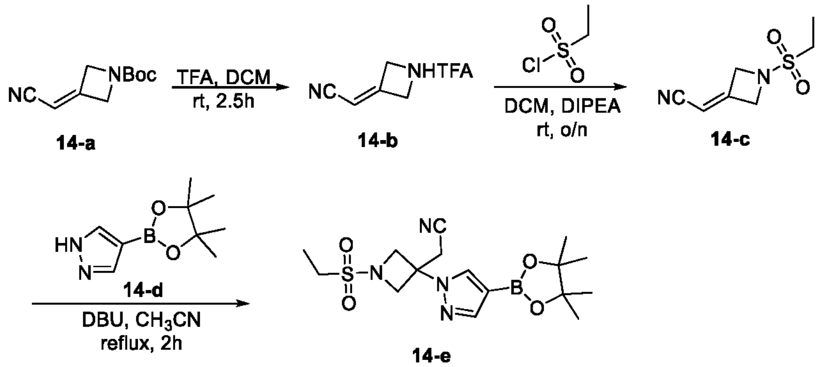


Step 1: synthesis of fragment **14-a** 2-(azetidin-3-ylidene)acetonitrile trifluoroacetate

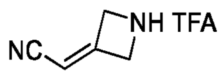

**14-b**

A solution of tert-butyl 3-(cyanomethylene)-azetidin-1-carboxylate (5 g, 25.7 mmol) and trifluoroacetic acid (20 ml) in dichloromethane (20 ml) was stirred at room temperature for 2.5 hours. The completion of the reaction was monitored by TLC. The reaction solution was concentrated under reduced pressure to obtain 2-(3- azetidinylidene) acetonitrile trifluoroacetate salt as a brown oil (5 g, crude product), which was used in the next step without further purification.

Step 2 : synthesis of fragment **14-c** 2-(1-(ethylsulfonyl)-3-azetidinylidene)acetonitrile

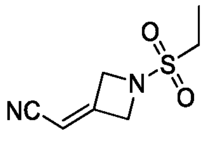

**14-c**

To a solution of 2-(3-azetidinylidene)acetonitrile trifluoroacetate salt (5 g, 26.2 mmol) and *N,N*-diisopropyldiamine (11.8 g, 91.6 mmol) in dichloromethane (50 ml) was added ethylsulfonyl chloride (4 g, 31.4 mmol) at 0°C. The resulting mixture was heated to room temperature and reacted for 14 hours. The completion of the reaction was monitored by TLC. The reaction mixture was partitioned in dichloromethane (50 ml) and water (100 ml). The organic layer was collected, washed with saturated sodium chloride solution (100 ml×2), dried over anhydrous sodium sulfate, and concentrated under reduced pressure. The crude product was separated and purified by silica gel column chromatography (eluted with 20%-33 % ethyl acetate solution in hexane) to obtain 2-(1-(ethylsulfonyl)-3-azetidinylidene) acetonitrile as a yellow solid (3 g, two-step combined yield 62%)

$^1$H NMR (400 MHz, DMSO-d6): δ 5.89-5.92 (m, 1H), 4.77-4.79 (m, 2H), 4.68-4.70 (m, 2H), 3.20-3.25 (q, 2H), 1.23 (t, *J* = 7.4 Hz, 3H).

Step 3 : synthesis of fragment **14-e** 2-(1-(ethylsulfonyl)-3-(4-(4,4,5,5-tetramethyl-1,3,2-dioxaborolan-2-yl)-1*H*-pyrazol-1-yl)azetidin-3-yl)acetonitrile

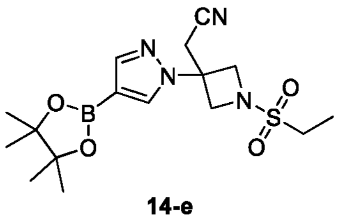

**14-e**

A solution of 2-(1-(ethylsulfonyl)-3-azetidinylidene)acetonitrile (2.9 g, 15.6 mmol), 4-pyrazoleboronic acid pinacol ester (3.02 g, 15.6 mmol) and 1,8-diazabicycloundec-7-ene (2.37 g, 15.6 mmol) in acetonitrile (40 ml) was heated to reflux for 2 hours. The completion of the reaction was monitored by TLC. The reaction mixture was concentrated under reduced pressure, and the obtained crude product was separated and purified by silica gel column chromatography (eluted with 30%-50% solution ethyl acetate in hexane) to obtain 2-(1-(ethylsulfonyl)-3-(4-(4,4,5,5-tetramethyl-1,3,2-dioxaborolan-2-yl)-1*H*-pyrazol-1-yl)azetidin-3-yl)acetonitrile as a white solid (3 g, 51%).

$^1$H NMR (400 MHz, DMSO-d6): δ 8.36 (s, 1H), 7.78 (s, 1H), 4.45 (d, *J* = 8.8 Hz, 2H), 4.15 (d, *J* = 9.2 Hz, 2H), 3.60 (s, 2H), 3.17-3.23 (q, 2H), 1.28 (s, 12H), 1.23 (t, *J* = 7.4 Hz, 3H).

**Example 15**

***N*-(Cyanomethyl)-4-(5-fluoro-2-((4-methyl-3,4,5,6-tetrahydro-2*H*-benzo[*b*][1,4,7] dioxazolin-9-yl)amino)pyrimidin-4-yl)benzamide**

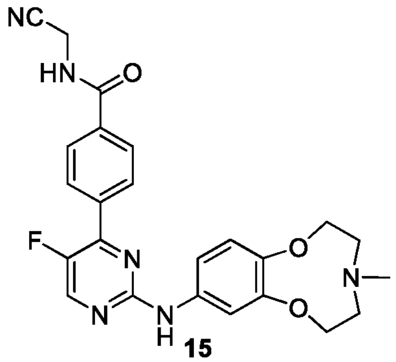

15

The implementation method was the same as method 2, except that fragment 1-a was used to replace fragment 2-d to obtain the title compound.

LC_MS: ($ES^+$): m/z 463.3 $[M+H]^+$.

$^1$H NMR (400 MHz, $CDCl_3$): δ 8.31 (d, *J* = 3.2 Hz, 1H), 8.18 (d, *J* = 8.4 Hz, 2H), 7.89 (d, *J* = 8.4 Hz, 2H), 7.14 (d, *J* = 2.4 Hz, 1H), 6.94-7.05 (m, 2H), 6.65-6.74 (m, 2H), 4.41 (d, *J* = 5.6 Hz, 2H), 4.24 (t, *J* = 4.4 Hz, 2H), 3.60 (t, *J* = 5.6 Hz, 2H), 3.41-3.48 (m, 4H), 3.37 (s, 3H).

## Example 16

***N*-(cyanomethyl)-4-(5-fluoro-2-((7-methyl-2,3,6,7,8,9-hexahydro-5*H*-benzo[*b*][1,4,7 ]trioxa[10]azacyclododecan-13-yl)amino)pyrimidin-4-yl)benzamide**

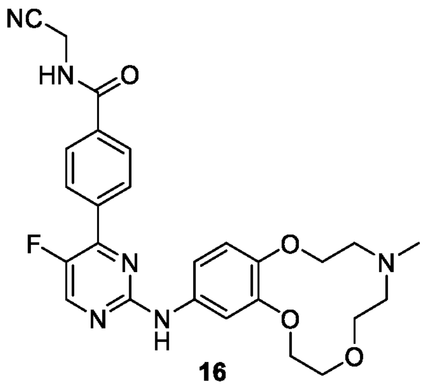

16

The implementation method was the same as method 2, except that fragment 1-a was used to replace fragment 2-d and fragment Amine-2 was used to replace fragment Amine-1 to obtain the title compound.

LC_MS: (ES+): m/z 507.8 $[M+H]^+$.

$^1$H NMR (400 MHz, DMSO-*d6*): δ 9.68 (s, 1H), 8.40-8.37 (m, 1H), 8.66-8.65 (m, 1H), 8.16-8.14 (m, 2H), 8.06-8.04 (m, 2H), 7.60 (m, 1H), 7.24-7.22 (m, 1H), 6.97-6.94 (m, 1H), 4.37-4.36 (m, 2H), 4.14-4.12 (m, 2H), 4.07-4.04 (m, 2H), 3.74 (m, 2H), 3.64-3.63 (m, 2H), 2.73-2.62 (m, 4H), 2.28 (s, 3H).

## Example 17

***N*-(cyanomethyl)-4-(2-((7-methyl-2,3,6,7,8,9-hexahydro-5*H*-benzo[*b*][1,4,7]trioxa [10]azacyclododecan-13-yl) amino)-7*H*-pyrrolo[2,3-*d*]pyrimidin-4-yl)benzamide**

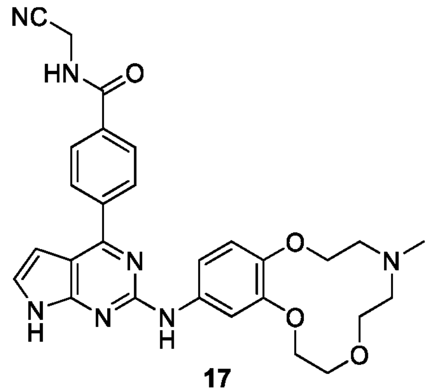

**17**

The implementation method was the same as method 2, except that fragment Amine-2 was used to replace fragment Amine-1 to obtain the title compound.

LC_MS: (ES$^+$): m/z 528.5 [M+H]$^+$.

$^1$H NMR (400 MHz, DMSO-d6): δ 11.71 (s, 1H), 9.41-9.38 (m, 1H), 9.27 (s, 1H), 8.28-8.26, (m, 2H), 8.08-8.06 (m, 2H), 7.81 (s, 1H), 7.33-7.32 (m, 2H), 6.96-6.94 (m, 1H), 6.73 (m, 1H), 4.38-4.36 (m, 2H), 4.17-4.08 (m, 4H), 3.78-3.68 (m, 4H), 3.31 (s, 3H), 2.71-2.62 (m, 2H), 2.37-2.30 (m, 2H).

**Example 18**

***N*-(cyanomethyl)-4-(2-((7-methyl-2,3,6,7,8,9-hexahydro-5*H*-benzo[*b*][1,4,7]trioxa [10]azacyclododecan-13-yl) amino)-7*H*-pyrrolo[2,3-*d*]pyrimidin-4-yl)benzamide**

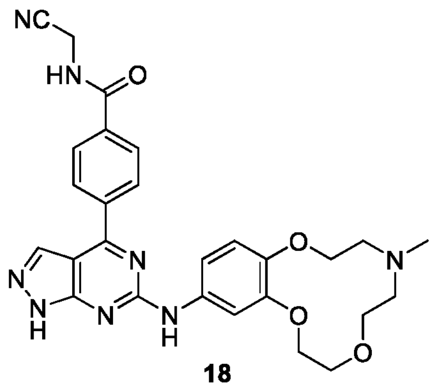

**18**

The implementation method was the same as method 2, except that fragment 4-a was used to replace fragment 2-d and fragment Amine-2 was used to replace fragment Amine-1 to obtain the title compound.

LC_MS: (ES$^+$): m/z 529.9 [M+H]$^+$.

$^1$H NMR (400 MHz, DMSO-d6): δ 9.74 (s, 1H), 9.44 (t, *J* = 1.6 Hz, 1H), 8.43-8.36 (m, 3H), 8.10 (d, *J* = 8.4 Hz, 2H), 7.73 (s, 1H), 7.39 (d, *J* = 8.8 Hz, 1H), 7.00 (d, *J* = 8.8 Hz, 1H), 4.37 (d, *J* = 4.8 Hz, 2H), 4.17-4.10 (m, 4H), 3.73 (d, *J* = 32.8 Hz, 4H), 2.82-2.68 (m, 4H), 2.37-2.33 (m, 3H).

**Example 19**

***N*-(tert-butyl)-3-((2-((7-methyl-2,3,6,7,8,9-hexahydro-5*H*-benzo[*b*][1,4,7]trioxa[10]a zacyclododecan-12-yl)amino)-7H-pyrrolo[2,3-*d*]pyrimidin-4-yl)amino)benzenesulfonamide**

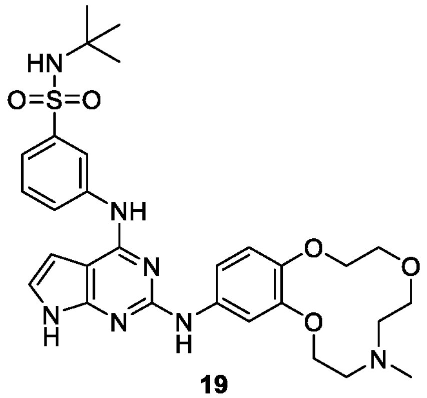

**19**

The implementation method was the same as method 4, except that fragment 2-d was used to replace fragment 4-a and fragment Amine-3 was used to replace fragment Amine-1 to obtain the title compound.
LC_MS: ($ES^+$): m/z 596.5 $[M+H]^+$.
$^1$H NMR (400 MHz, DMSO-d6): δ 11.27 (s, 1H), 9.42 (s, 1H), 8.71 (s, 1H), 8.44 (d, *J* = 7.2 Hz, 1H), 8.28 (s, 1H), 7.65 (s, 1H), 7.61 (brs, 1H), 7.51-7.42 (m, 2H), 7.35 (d, *J* = 8.0 Hz, 1H), 7.00-6.90 (m, 2H), 6.70 (s, 1H), 4.16-3.99 (m, 4H), 3.77-3.61 (m, 4H), 3.29 (s, 3H), 2.88-2.74 (m, 2H), 2.38-2.23 (m, 2H), 1.15 (s, 9H).

**Example 20**

***N*-(tert-butyl)-3-((2-((7-methyl-2,3,6,7,8,9-hexahydro-5*H*-benzo[*b*][1,4,7]trioxa[10]a zacyclododecan-13-yl)amino)-7H-pyrrolo[2,3-*d*]pyrimidin-4-yl)amino)benzenesulfonamide**

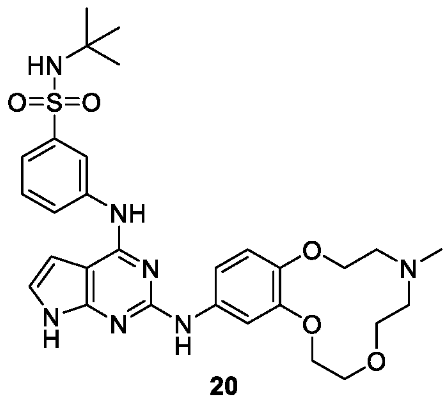

**20**

The implementation method was the same as method 4, except that fragment 2-d was used to replace fragment 4-a and fragment Amine-2 was used to replace fragment Amine-1 to obtain the title compound.
LC_MS: ($ES^+$): m/z 596.7 $[M+H]^+$.
$^1$H NMR (400 MHz, DMSO-d6): δ 11.31 (s, 1H), 9.48 (s, 1H), 8.82 (s, 1H), 8.44 (d, *J* = 8.0 Hz, 1H), 8.28 (s, 1H), 7.77 (s, 1H), 7.66 (s, 1H), 7.51-7.43 (m, 2H), 7.36-7.33 (m, 1H), 7.02-6.96 (m, 2H), 6.72-6.71 (m, 1H), 4.23 (s, 2H), 4.07 (s, 2H), 3.82 (brs, 4H), 3.32 (s, 3H), 2.80 (brs, 4H), 1.14 (s, 9H).

**Example 21**

***N*-(tert-butyl)-3-((2-((4-methyl-3,4,5,6-tetrahydro-2*H*-benzo[*b*][1,4,7]dioxazolin-9-y l)amino)-7*H*-pyrrolo[2,3-*d*] pyrimidin-4-yl)amino)benzenesulfonamide**

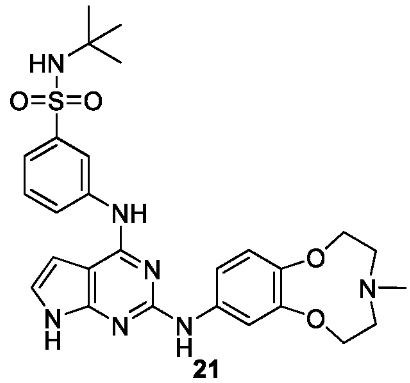

**21**

The implementation method was the same as method 4, except that fragment 2-d was used to replace fragment 4-a to obtain the title compound.

LC_MS: ($ES^+$): m/z 552.2 $[M+H]^+$.

$^1$H NMR (400 MHz, DMSO-d6): δ 11.24 (s, 1H), 9.42 (brs, 1H), 8.41-8.62 (m, 2H), 8.34 (s, 1H), 7.66 (s, 1H), 7.41-7.49 (m, 2H), 7.27-7.35 (m, 1H), 7.01-7.14 (m, 1H), 6.82-6.99 (m, 1H), 6.54-6.78 (m, 2H), 4.02-4.28 (m, 2H), 3.52-3.58 (m, 2H), 3.34-3.48 (m, 4H), 3.27 (s, 3H), 1.15 (s, 9H).

**Example 22**

***N*-(tert-butyl)-3-((6-((7-methyl-2,3,6,7,8,9-hexahydro-5*H*-benzo[*b*][1,4,7]trioxa[10]a zacyclododecan-13-yl)amino)-1*H*-pyrazolo[3,4-*d*]pyrimidin-4-yl)amino)benzenesulfonamide**

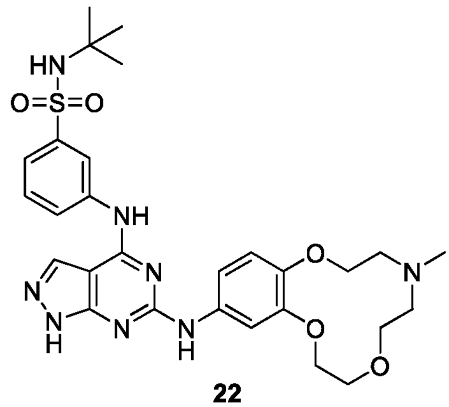

**22**

The implementation method was the same as method 4, except that fragment Amine-2 was used to replace fragment Amine-1 to obtain the title compound.

LC_MS: ($ES^+$): m/z 597.6 $[M+H]^+$.

$^1$H NMR (400 MHz, DMSO-*d*6): δ 13.04 (s, 1H), 10.00 (s, 1H), 9.08 (s, 1H), 8.50-8.48 (m, 1H), 8.22 (brs, 1H), 8.13 (s, 1H), 7.70 (s, 1H), 7.56-7.49 (m, 3H), 7.35-7.32 (m, 1H), 6.95 (d, *J* = 8.8 Hz, 1H), 4.09-4.05 (m, 4H), 3.72-3.65 (m, 4H), 3.32 (m, 3H), 2.77-2.74 (m, 1H), 2.64-2.60 (m, 1H), 2.33-2.29 (m, 2H), 1.14 (s, 9H).

**Example 23**

**7-methyl-*N*-(4-(1-methyl-1*H*-indol-3-yl)-7*H*-pyrrolo[2,3-*d*]pyrimidin-2-yl)-2,3,6,7,8,9-hexahydro-5*H*-benzo[*b*][1,4,7]trioxa[10]azacyclododecan-13-amine**

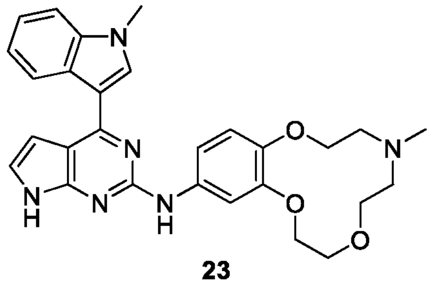

**23**

The implementation method was the same as method 5, except that fragment 2-c was used to replace fragment 5-a and fragment Amine-2 was used to replace fragment Amine-1 to obtain the title compound.

LC_MS: ($ES^+$): m/z 499.6 $[M+H]^+$.

$^1H$ NMR (400 MHz, DMSO-d6): δ 11.44 (s, 1H), 9.03 (brs, 1H), 8.87 (d, *J* = 8.0 Hz, 1H), 8.41 (s, 1H), 7.83 (brs, 1H), 7.53 (d, *J* = 8.0 Hz, 1H), 7.46-7.43 (m, 1H), 7.30-7.26 (m, 1H), 7.22-7.18 (m, 2H), 7.00 (d, *J* = 8.0 Hz, 1H), 6.87-6.86 (m, 1H), 4.22-4.10 (m, 4H), 3.95 (s, 3H), 3.85-3.70 (m, 4H), 3.31 (s, 3H), 3.13-2.93 (m, 2H), 2.76-2.54 (m, 2H).

**Example 24**

**7-methyl-*N*-(4-(1-methyl-1*H*-indol-3-yl)-1*H*-pyrazolo[3,4-*d*]pyrimidin-6-yl)-2,3,6,7, 8,9-hexahydro-5*H*-benzo[*b*][1,4,7]trioxa[10]azacyclododecan-13-amine**

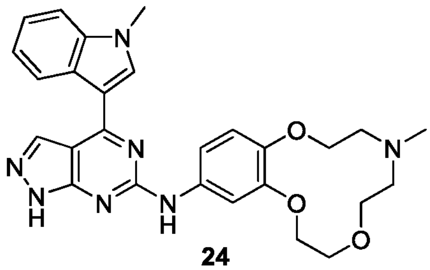

**24**

The implementation method was the same as method 5, except that fragment Amine-2 was used to replace fragment Amine-1 to obtain the title compound.

LC_MS: ($ES^+$): m/z 500.8 $[M+H]^+$.

$^1H$ NMR (400 MHz, DMSO-d6): δ 13.17 (s, 1H), 9.38 (s, 1H), 8.90 (d, *J* = 8.0 Hz, 1H), 8.66 (s, 1H), 8.50 (s, 1H), 7.69 (brs, 1H), 7.56 (d, *J* = 8.0 Hz, 1H), 7.46 (d, *J* = 8.4 Hz, 1H), 7.33-7.23 (m, 2H), 6.99 (d, *J* = 8.4 Hz, 1H), 4.21-4.06 (m, 4H), 3.97 (s, 3H), 3.81-3.63 (m, 4H), 3.29 (s, 3H), 2.85-2.71 (m, 2H), 2.39-2.30 (m, 2H).

**Example 25**

**3-cyclopentyl-3-(4-(5-fluoro-2-((4-methyl-3,4,5,6-tetrahydro-2*H*-benzo[*b*][1,4,7] dioxazolin-9-yl))amino)pyrimidin-4-yl)-1*H*-pyrazol-1-yl)propanenitrile**

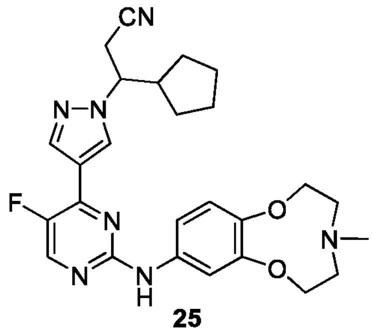

**25**

The implementation method was the same as method 2, except that fragment 25-b was used to replace fragment 2-b and fragment 1-a was used to replace fragment 2-d to obtain the title compound.

LC_MS: ($ES^+$): m/z 492.3 $[M+H]^+$.

$^1H$ NMR (400 MHz, $CDCl_3$): δ 8.26 (s, 1H), 8.21 (brs, 1H), 8.17 (s, 1H), 7.14 (brs, 1H), 6.84-7.02 (m, 2H), 6.58-6.73 (m, 1H), 4.19-4.25 (m, 3H), 3.60 (t, *J* = 5.6 Hz, 2H), 3.38-3.45 (m, 2H), 3.37 (s, 3H), 3.09-3.15 (m, 1H), 2.91-2.96 (m, 1H), 2.51-2.62 (m, 1H), 1.92-2.00 (m, 1H), 1.48-1.75 (m, 6H), 1.19-1.31 (m, 3H).

**Fragment 25-b was synthesized as follows:**

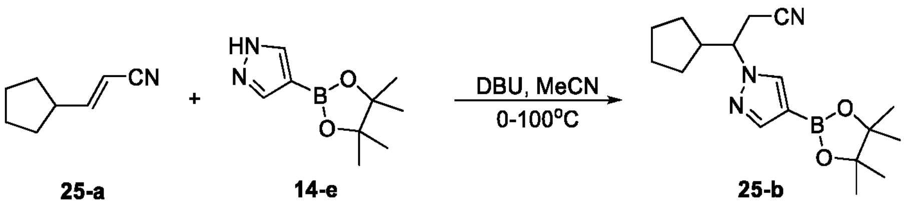


**25-a** **14-e** **25-b**

Step 1: synthesis of fragment **25-b** 3-cyclopentyl-3-(4-(4,4,5,5-tetramethyl-1,3,2-dioxaborolan-2-yl)-1*H*-pyrazol-1-yl) propionitrile

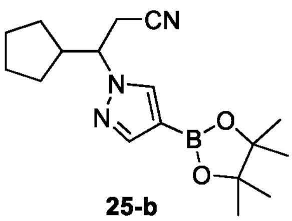

**25-b**

A solution of 3-cyclopentyl acrylonitrile (100 mg, 0.8 mmol) and 4-pyrazole boronic acid pinacol ester (161 mg, 0.8 mmol) in acetonitrile (1 ml) was stirred at 0°C for 10 minutes o, and then 1,8-diazabicycloundec-7-ene (126 mg, 0.8 mmol) was added at 0°C. The reaction system was heated to 100°C and stirred for 12 hours. The completion of the reaction was monitored by TLC. The reaction mixture was cooled to room temperature and concentrated under reduced pressure. The obtained crude product was separated and purified by silica gel column chromatography (eluted with 30% ethyl acetate solution in petroleum ether) to obtain 3-cyclopentyl-3-(4-(4,4,5,5-tetramethyl-1,3,2-dioxaborolan-2-yl)-1*H*-pyrazol-1-yl) propanenitrile as colorless oil (85 mg, 32%).

$^1H$ NMR (400 MHz, $CDCl_3$): δ 7.83 (s, 1H), 7.78 (s, 1H), 4.11-4.18 (m, 1H), 3.03-.309 (m, 1H), 2.85-2.91 (m, 1H), 2.45-2.56 (m, 1H), 1.88-1.96 (m, 1H), 1.61-1.72 (m, 2H), 1.56-1.59 (m, 1H), 1.43-1.54 (m, 2H), 1.32 (s, 12H), 1.25-1.29 (m, 1H), 1.08-1.16 (m, 1H).

**Example 26**

**3-cyclopentyl-3-(4-(2-((7-methyl-2,3,6,7,8,9-hexahydro-5*H*-benzo[*b*][1,4,7]trioxa[10 ]azacyclododecan-13-yl) amino)-7*H*-pyrrolo[2,3-*d*]pytimidin-4-yl)-1*H*-pyrazol-1-yl)propionit rile**

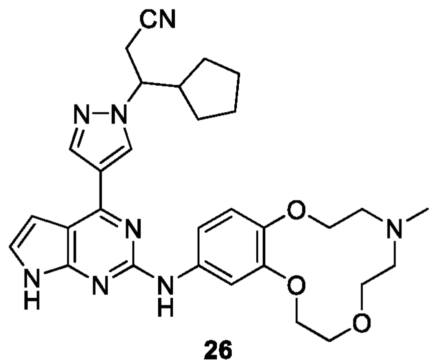

**26**

The implementation method was the same as method 2, except that fragment 25-b was used to replace fragment 2-b and fragment Amine-2 was used to replace fragment Amine-1 to obtain the title compound.

LC_MS: ($ES^+$): m/z 557.6 $[M+H]^+$.

$^1$HNMR (400 MHz, DMSO-d6): δ 11.50 (s, 1H), 9.02 (s, 1H), 8.68 (s, 1H), 8.29 (s, 1H), 7.77 (s, 1H), 7.38-7.36 (m, 1H), 7.23-7.22 (m, 1H), 6.98-6.96 (m, 1H), 6.79-6.78 (m, 1H), 4.58-4.52 (m, 1H), 4.16-4.12 (m, 4H), 3.79-3.72 (m, 4H), 3.29 (s, 3H), 3.25-3.16 (m, 2H), 2.97-2.81 (m, 2H), 2.43-2.42 (m, 2H), 1.85-1.82 (m, 1H), 1.25-1.23 (m, 8H).

**Example 27**

**3-cyclopentyl-3-(4-(2-((4-methyl-3,4,5,6-tetrahydro-2*H*-benzo[*b*][1,4,7]dioxazolin-9 -yl)amino)-7*H*-pyrrolo[2,3-*d*] pyrimidin-4-yl)-1*H*-pyrazol-1-yl)propanenitrile**

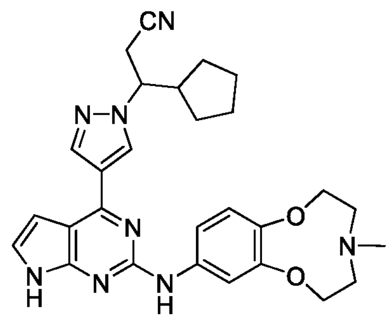

**27**

The implementation method was the same as method 2, except that fragment 25-b was used to replace fragment 2-b to obtain the title compound.

LC_MS: ($ES^+$): m/z 513.5 $[M+H]^+$.

$^1$H NMR (400 MHz, DMSO-d6): δ 11.45 (s, 1H), 8.72 (s, 1H), 8.66 (s, 1H), 8.26 (s, 1H), 7.40 (d, *J* = 2.4 Hz, 1H), 7.14-7.20 (m, 2H), 6.74 (d, *J* = 2.8 Hz, 1H), 6.65 (d, *J* = 8.8 Hz, 1H), 4.50-4.57 (m, 1H), 4.15 (t, *J* = 3.4 Hz, 2H), 3.53 (t, *J* = 5.6 Hz, 2H), 3.38 (t, *J* = 5.4 Hz, 2H), 3.28-3.30 (m, 1H), 3.27 (s, 3H), 3.24 (d, *J* = 9.2 Hz, 1H), 3.17-3.22 (m, 1H), 2.38-2.46 (m, 1H), 1.78-1.86 (m, 1H), 1.51-1.65 (m, 3H), 1.39-1.46 (m, 1H), 1.29-1.38 (m, 2H), 1.14-1.23 (m, 2H),

**Example 28**

**2-(1-(ethylsulfonyl)-3-(4-(2-((4-methyl-3,4,5,6-tetrahydro-2*H*-benzo[*b*][1,4,7] dioxazolin-9-yl)amino)-7*H*-pyrrolo [2,3-*d*]pytimidin-4-yl)-1*H*-pyrazol-1-yl)azetidin-3-yl) acetonitrile**

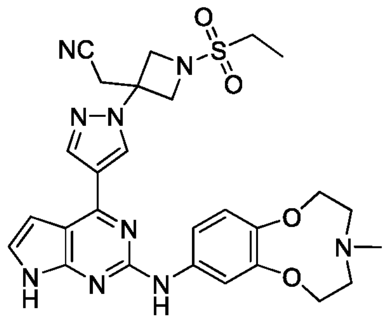

**28**

The implementation method was the same as method 2, except that fragment 14-a was used to replace fragment 2-b to obtain the title compound.

LC_MS: (ES$^+$): m/z 578.6 [M+H]$^+$.

$^1$H NMR (400 MHz, $CDCl_3$): δ 9.36 (s, 1H), 8.37 (s, 1H), 8.27 (s, 1H), 7.26-7.28 (m, 2H), 6.93-7.14 (m, 2H), 6.77-6.88 (m, 1H), 6.66 (d, *J* = 8.4 Hz, 1H), 6.47-6.59 (m, 1H), 4.62 (d, *J* = 9.2 Hz, 2H), 4.24 (d, *J* = 8.8 Hz, 4H), 3.60 (t, *J* = 5.4 Hz, 2H), 3.37-3.51 (m, 8H), 3.06-3.11 (q, 2H), 1.41 (t, *J* = 7.4 Hz, 3H).

**Example 29**

**2-(1-(ethylsulfonyl)-3-(4-(6-((4-methyl-3,4,5,6-tetrahydro-2*H*-benzo[*b*][1,4,7]dioxaz olin-9-yl)amino)-1*H*-pyrazolo [3,4-*d*]pyrimidin-4-yl)-1*H*-pyrazol-1-yl)azetidin-3-yl) acetonitrile**

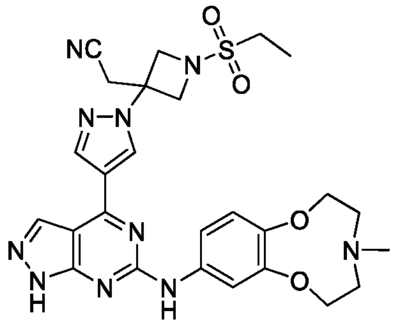

**29**

The implementation method was the same as method 2, except that fragment 14-a was used to replace fragment 2-b and fragment 4-a was used to replace fragment 2-d to obtain the title compound.

LC_MS: (ES$^+$): m/z 579.4 [M+H]$^+$.

$^1$H NMR (400 MHz, DMSO-d6): δ 13.26 (s, 1H), 9.31 (s, 1H), 8.94 (s, 1H), 8.44, 8.51 (two singles, 2H), 7.39 (brs, 1H), 7.19 (d, *J* = 7.2 Hz, 1H), 6.66-6.69 (m, 1H), 4.59 (d, *J* = 9.2 Hz, 2H), 4.26 (d, *J* = 8.8 Hz, 2H), 4.11-4.20 (m, 2H), 3.71 (brs, 2H), 3.49-3.57 (m, 2H), 3.37-3.45 (m, 2H), 3.23-3.32 (m, 7H), 1.25 (t, *J* = 7.0 Hz, 3H).

**Example 30**

**5-fluoro-*N*$^4$-(1*H*-indol-5-yl)-*N*$^2$-(7-methyl-2,3,6,7,8,9-hexahydro-5*H*-benzo[*b*][1,4,7] trioxa[10]azacyclododecan-13-yl)pyrimidin-2,4-diamine**

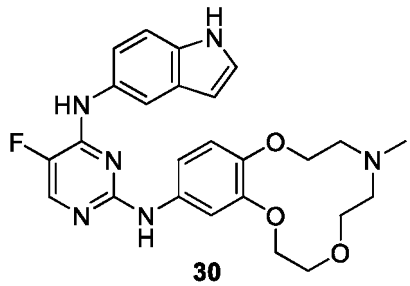

**30**

The implementation method was the same as method 1, except that fragment 1*H*-indole-5-amine was used to replace fragment 1-b, and fragment Amine-2 was used to replace fragment Amine-1 to obtain the title compound.

LC_MS: (ES$^+$): m/z 479.3 [M+H]$^+$.

$^1$H NMR (400 MHz, DMSO-d6): δ 11.07 (s, 1H), 9.14 (s, 1H), 8.96 (s, 1H), 8.00 (d, *J*= 4.0 Hz, 1H), 7.82 (s, 1H), 7.43 (s, 1H), 7.34-7.36 (m, 2H), 7.27-7.30 (m, 1H), 7.11-7.13 (m, 1H), 6.79 (d, *J* = 8.0 Hz, 1H), 6.39 (s, 1H), 4.01 (s, 2H), 3.62 (s, 4H), 3.47 (s, 2H), 2.67-2.95 (m, 4H), 2.33 (s, 3H).

**Example 31**

***N*-(4-(4-fluoro-1-isopropyl-2-methyl-1*H*-benzo[*d*]imidazol-6-yl)-7*H*-pyrrolo[2,3-*d*]p yrimidin-2-yl)-7-methyl-2,3,6,7,8,9-hexahydro-5*H*-benzo[*b*][1,4,7]trioxa[10] azacyclododecan-13-amine**

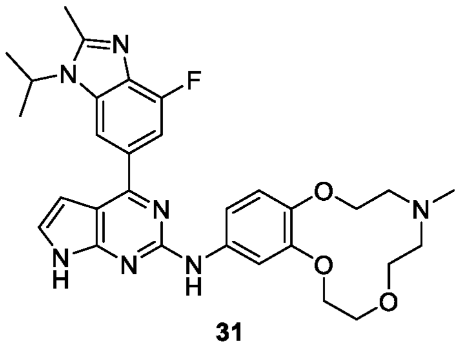

**31**

The implementation method was the same as method 2, except that fragment 31-b was used to replace fragment 2-b, and fragment Amine-2 was used to replace fragment Amine-1 to obtain the title compound.

LC_MS: (ES$^+$): m/z 560.5 [M+H]$^+$.

$^1$H NMR (400 MHz, DMSO-d6): δ 11.69 (s, 1H), 9.23 (s, 1H), 8.16 (s, 1H), 7.81 (s, 1H), 7.74 (d, *J* = 12.0 Hz, 1H), 7.38-7.41 (m, 1H), 7.32-7.33 (m, 1H), 6.97 (d, *J* = 8.4 Hz, 1H), 6.67-6.68 (m, 1H), 4.82-4.93 (m, 1H), 4.13-4.17 (m, 4H), 3.74-3.80 (m, 4H), 3.32 (s, 3H), 2.94 (brs, 4H), 2.65 (s, 3H), 1.64 (d, *J* = 7.2 Hz, 6H).

**The implementation method of 31-b was as follows:**

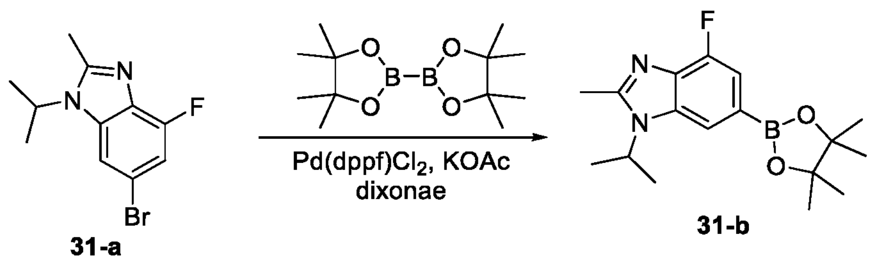


**31-a** **31-b**

Step 1: synthesis of fragment **31-b** 4-fluoro-1-isopropyl-2-methyl-6-(4,4,5,5-tetramethyl-1,3,2-dioxaborolan-2-yl)-1*H*-benzo[*d*] imidazole

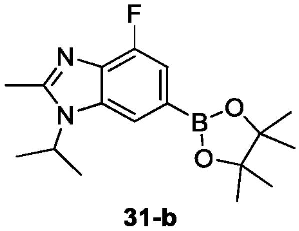

**31-b**

To a suspension of 6-bromo-4-fluoro-1-isopropyl-2-methyl-1*H*-benzo[*d*]imidazole (300 mg, 1.11 mmol), 4,4,4',4',5,5,5',5'-octamethyl-2,2'-bis(1,3,2-dioxaborolane) (338 mg, 1.33 mmol) and potassium acetate in dioxane (5 ml) was added 1,1'-bis(diphenylphosphino)ferrocenepalladium(II)chloride (80 mg, 0.11 mmol) under nitrogen atmosphere at room temperature. The reaction mixture was replaced with nitrogen gas for three times and stirred at 100°C for 14 hours. The completion of the reaction was monitored by TLC. The reaction mixture was concentrated, and the resulting crude product was separated and purified by silica gel column chromatography (eluted with 50% ethyl acetate in n-hexane) to obtain 4-fluoro-1-isopropyl-2-methyl-6-(4,4,5,5-tetramethyl-1,3,2-dioxaborolan-2-yl)-1*H*-benzo [*d*]imidazole (300 mg, 85%) as a white solid.

LC_MS: ($ES^+$): m/z 319.1 $[M+H]^+$.

**Example 32**

**dimethyl(2-((2-((7-methyl-2,3,6,7,8,9-hexahydro-5*H*-benzo[*b*][1,4,7]trioxa[10] azacyclododecan-13-yl)amino)-7*H*-pyrrolo[2,3-*d*]pyrimidin-4-yl)amino)phenyl)phosphine oxide**

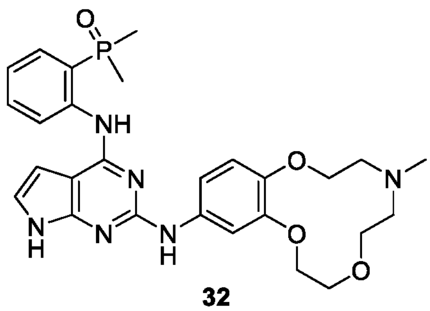

**32**

The implementation method was the same as method 4, except that fragment 2-d was used to replace fragment 4-a, fragment (2-aminophenyl)dimethylphosphine oxide was used to replace fragment 1-b, and fragment Amine-2 was used to replace fragment Amine-1 to obtain the title compound.

LC_MS: ($ES^+$): m/z 537.6 $[M+H]^+$.

$^1$H NMR (400 MHz, DMSO-d6): δ 11.62 (s, 1H), 11.27 (s, 1H), 9.10-9.13 (m, 1H), 8.81 (s, 1H), 7.48-7.59 (m, 3H), 7.32-7.35 (m, 1H), 7.06 (t, *J* = 7.2 Hz, 1H), 6.90-6.94 (m, 2H), 6.35-6.37 (m, 1H), 4.11 (t, *J* = 3.8 Hz, 2H), 4.04 (t, *J* = 5.0 Hz, 2H), 3.72 (t, *J* = 3.8 Hz, 2H), 3.64 (t, *J* = 4.8 Hz, 2H), 2.26 (s, 3H), 2.68-2.76 (m, 2H), 2.58-2.65 (m, 2H), 1.83 (d, *J* = 13.6 Hz, 6H).

**Example 33**

**dimethyl(2-((6-((7-methyl-2,3,6,7,8,9-hexahydro-5*H*-benzo[*b*][1,4,7]trioxa[10]azacy clododecan-13-yl)amino)-1*H*-pyrazolo[3,4-*d*]pyrimidin-4-yl)amino)phenyl)phosphine oxide**

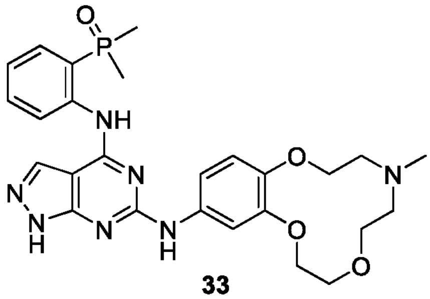

**33**

The implementation method was the same as method 4, except that fragment (2-aminophenyl)dimethylphosphine oxide was used to replace fragment 1-b, and fragment Amine-2 was used to replace fragment Amine-1 to obtain the title compound.

LC_MS: (ES+): m/z 538.2 [M+H]+.

$^1$H NMR (400 MHz, DMSO-$d_6$): δ 13.05 (s, 1H), 12.06 (s, 1H), 9.19 (s, 1H), 9.02 (s, 1H), 7.75 (s, 1H), 7.52-7.66(m, 3H), 7.35 (d, *J* = 8 Hz, 1H), 7.15 (t, *J* = 7.2 Hz, 1H), 6.96 (d, *J* = 8.8 Hz, 1H), 4.09-4.11 (m, 4H), 3.67-3.74 (m, 4H), 3.29(s, 3H), 2.67-2.82 (m, 2H), 2.32-2.33 (m, 2H), 1.85 (d, *J* = 13.6 Hz, 6H).

**Example 34**

***N*⁴-(4-bromo-2-fluorophenyl)-*N*⁶-(7-methyl-2,3,6,7,8,9-hexahydro-5*H*-benzo[*b*] [1,4,7]trioxa[10]azacyclododecan-13-yl)-1*H*-pyrazolo[3,4-*d*]pyrimidin-4,6-diamine**

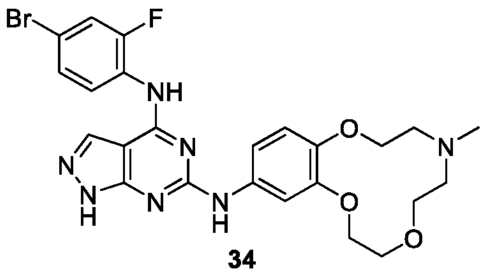

**34**

The implementation method was the same as method 4, except that fragment 4-bromo-2-fluoroaniline was used to replace fragment 1-b and fragment Amine-2 was used to replace fragment Amine-1 to obtain the title compound.

LC_MS: (ES+): m/z 558.4 [M+H]+.

$^1$H NMR (400 MHz, DMSO-*d*6): δ 12.99 (s, 1H), 9.76 (s, 1H), 9.05 (s, 1H), 8.07 (s, 1H), 7.80 (t, *J* = 8.2 Hz, 1H), 7.64-7.69 (m, 2H), 7.44 (d, *J* = 8.8 Hz, 1H), 7.27-7.29 (m, 1H), 6.97 (d, *J* = 8.8 Hz, 1H), 4.17-4.33 (m, 2H), 3.75-4.01 (m, 6H), 3.31 (s, 3H), 2.66-3.05 (m, 4H).

**Example 35**

***N*⁴-(2-(isopropylsulfonyl)phenyl)-*N*²-(4-methyl-3,4,5,6-tetrahydro-2*H*-benzo[*b*][1,4, 7]dioxazolin-9-yl)-7*H*-pyrrolo[2,3-*d*]pyrimidin-2,4-diamine**

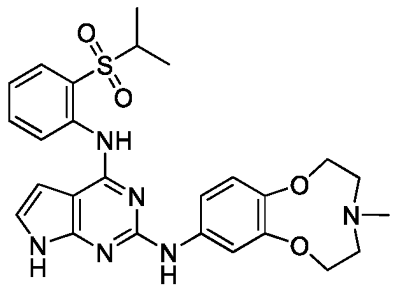

**35**

The implementation method was the same as method 4, except that fragment 2-d was used to replace fragment 4-a and fragment 2-(isopropylsulfonyl) aniline was used to replace fragment 1-b to obtain the title compound.

LC_MS: ($ES^+$): m/z 523.6 $[M+H]^+$.

$^1H$ NMR (400 MHz, $CDCl_3$): δ 9.58 (brs, 1H), 9.16 (brs, 1H), 8.87 (d, *J* = 8.4 Hz, 1H), 7.84-7.86 (m, 1H), 7.59-7.64 (m, 1H), 7.13-7.19 (m, 2H), 6.89-7.00 (m, 1H), 6.77-6.86 (m, 1H), 6.64-6.72 (m, 2H), 6.41 (d, *J* = 3.6 Hz, 1H), 4.24 (t, *J* = 4.2 Hz, 2H), 3.60 (t, *J* =5.8 Hz, 2H), 3.41-3.52 (m, 4H), 3.37 (s, 3H), 3.22-3.29 (m, 1H), 1.30 (d, *J* = 7.2 Hz, 6H).

**Example 36**

***N*[4]-(2-(isopropylsulfonyl)phenyl)-*N*[6]-(4-methyl-3,4,5,6-tetrahydro-2*H*-benzo[*b*] [1,4,7]dioxazolin-9-yl)-1*H*-pyrazolo[3,4-*d*]pyrimidin-4,6-diamine**

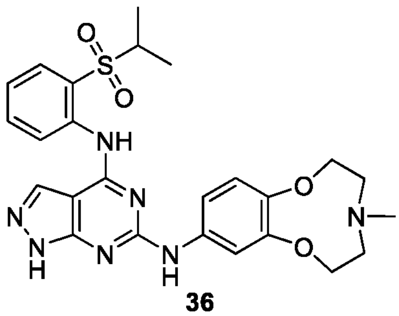

**36**

The implementation method was the same as method 4, except that fragment 2-(isopropylsulfonyl)aniline was used to replace fragment 1-b to obtain the title compound.

LC_MS: ($ES^+$): m/z 524.6 $[M+H]^+$.

$^1H$ NMR (400 MHz, $CDCl_3$): δ 9.83 (brs, 1H), 8.78 (d, *J* = 8.4 Hz, 1H), 7.87-7.91 (m, 2H), 7.64 (t, *J* = 7.8 Hz, 1H), 7.21-7.26 (m, 1H), 7.17 (brs, 1H), 6.85-7.05 (m, 1H), 6.52-6.78 (m, 1H), 4.25 (brs, 2H), 3.61 (t, *J* = 5.4 Hz, 2H), 3.41-3.56 (m, 4H), 3.38 (s, 3H), 3.20-3.27 (m, 1H), 1.31 (d, *J* = 6.8 Hz, 6H),

**Example 37**

**2-(1-(ethylsulfonyl)-3-(4-(5-fluoro-2-((4-methyl-3,4,5,6-tetrahydro-2*H*-benzo[*b*] [1,4,7]dioxazolin-9-yl)amino)pyrimidin-4-yl)-1*H*-pyrazol-1-yl)azetidin-3-yl)acetonitrile**

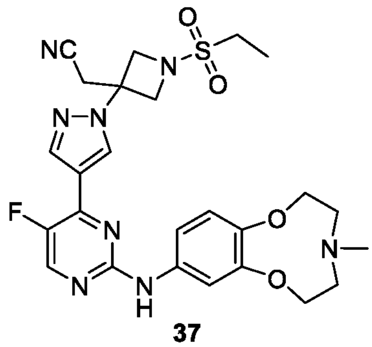

**37**

The implementation method was the same as method 2, except that fragment 14-a was used to replace fragment 2-b and fragment 1-a was used to replace fragment 2-d to obtain the title compound.

LC_MS: ($ES^+$): m/z 557.1 $[M+H]^+$.

$^1$H NMR (400 MHz, DMSO-d6): δ 8.22-8.30 (m, 3H), 7.17 (s, 1H), 4.59 (d, *J* = 9.2 Hz, 2H), 4.23 (d, *J* = 9.6 Hz, 4H), 3.60 (t, *J* = 5.2 Hz, 1H), 3.37, 3.38 (two singles, 5H), 3.05-3.11 (q, 2H), 1.53-1.70 (m, 2H), 1.41 (t, *J* = 7.4 Hz, 3H), 1.19-1.34 (m, 2H).

**Example 38**

***N*-(cyanomethyl)-4-(6-((4-methyl-3,4,5,6-tetrahydro-2*H*-benzo[*b*][1,4,7]dioxazolin-9-yl)amino)-1*H*-pyrazolo[3,4-*d*]pyrimidin-4-yl)benzamide**

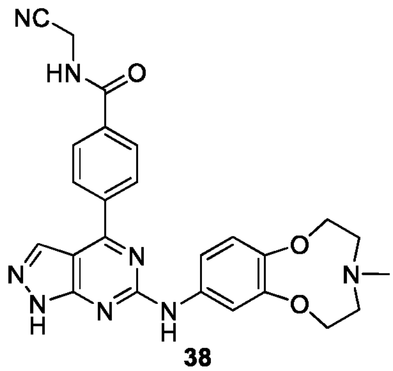

**38**

The implementation method was the same as method 2, except that fragment 4-a was used to replace fragment 2-d to obtain the title compound.

LC_MS: ($ES^+$): m/z 484.9 $[M+H]^+$.

$^1$H NMR (400 MHz, DMSO-d6): δ 13.39 (s, 1H), 9.51 (s, 1H), 9.43 (t, *J* = 5.6 Hz, 1H), 8.37 (s, 1H), 8.34 (d, *J* = 8.4 Hz, 2H), 8.10 (d, *J* = 8.4 Hz, 2H), 7.39 (d, *J* = 2.0 Hz, 1H), 7.18-7.20 (m, 1H), 6.69 (d, *J* = 8.8 Hz, 1H), 4.37 (t, *J* = 2.8 Hz, 2H), 4.16 (t, *J* = 4.2 Hz, 2H), 3.53 (t, *J* = 5.6 Hz, 2H), 3.41 (t, *J* = 5.6 Hz, 2H), 3.32-3.35 (m, 2H), 3.28 (s, 3H).

**Example 39**

**4-methyl-*N*-(4-(1-methyl-1*H*-indol-3-yl)-7*H*-pyrrolo[2,3-*d*]pyrimidin-2-yl)-3,4,5,6-tetrahydro-2*H*-benzo[*b*][1,4,7] dioxazolin-9-amine**

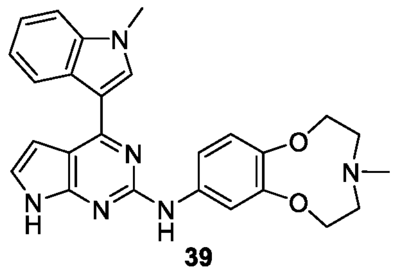

39

The implementation method was the same as method 5, except that fragment 2-c was used to replace fragment 5-a to obtain the title compound.

LC_MS: ($ES^+$): m/z 455.4 $[M+H]^+$.

$^1H$ NMR (400 MHz, $CDCl_3$): δ 9.21 (s, 1H), 8.51 (d, *J* = 4.8 Hz, 1H), 7.91 (s, 1H), 7.38 (d, *J* = 8.0 Hz, 1H), 7.30-7.34 (m, 1H), 7.27-7.29 (m, 2H), 7.07 (d, *J* = 6.8 Hz, 1H), 6.77 (brs, 1H), 6.66 (d, *J* = 8.4 Hz, 1H), 6.60 (d, *J* = 2.4 Hz, 1H), 4.24 (t, *J* = 4.4 Hz, 2H), 3.91 (s, 3H), 3.60 (t, *J* = 5.6 Hz, 2H), 3.38-3.51 (m, 4H), 3.36 (s, 3H).

**Example 40**

***N*-(tert-butyl)-3-((5-fluoro-2-((7-methyl-2,3,6,7,8,9-hexahydro-5*H*-benzo[*b*][1,4,7] trioxa[10]azacyclododecan-12-yl)amino)pyrimidin-4-yl)amino)benzenesulfonamide**

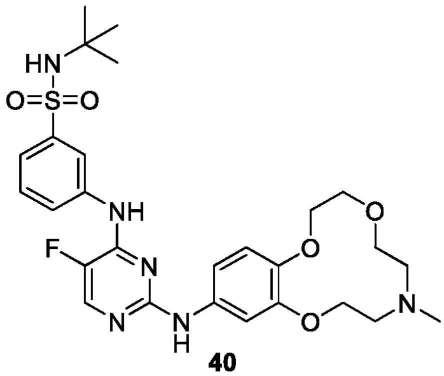

40

The implementation method was the same as method 1, except that fragment Amine-3 was used to replace fragment Amine-1 to obtain the title compound.

LC_MS: ($ES^+$): m/z 575.2 $[M+H]^+$.

$^1H$ NMR (400 MHz, DMSO-*d*6): δ 9.68 (s, 1H), 9.11(s, 1H), 8.20-8.16 (m, 3H), 7.63 (s, 1H), 7.52-7.41 (m, 3H), 7.23 (d, *J* = 8.8 Hz, 1H), 6.96 (d, *J* = 8.4 Hz, 1H), 4.07-4.03 (m, 4H), 3.70 (s, 4H), 3.32 (s, 3H), 3.17-2.67 (m, 4H), 1.12 (s, 9H).

**Example 41**

***N*-(5-fluoro-4-(1-methyl-1*H*-indol-3-yl)pyrimidin-2-yl)-7-methyl-2,3,6,7,8,9-hexahydro-5*H*-benzo[*b*][1,4,7]trioxa [10]azacyclododecan-12-amine**

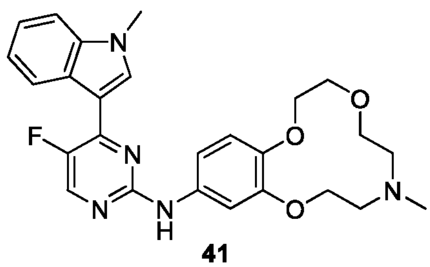

41

The implementation method was the same as method 5, except that fragment 1-a was used to replace fragment 5-a and fragment Amine-3 was used to replace fragment Amine-1 to obtain the title compound.

LC_MS: ($ES^+$): m/z 478.6 $[M+H]^+$.

$^1$H NMR (400 MHz, DMSO-d6): δ 9.38 (s, 1H), 9.74 (d, *J* = 8.0 Hz, 1H), 8.40 (d, *J* = 3.6 Hz, 1H), 8.23 (d, *J* = 2.8 Hz, 1H), 7.58-7.54 (m, 2H), 7.32 (t, *J* = 7.2 Hz, 2H), 7.22 (t, *J* = 7.6 Hz, 1H), 7.03 (d, *J* = 8.8 Hz, 1H), 4.23-4.04 (m, 4H), 3.93 (s, 3H), 3.83-3.64 (m, 4H), 3.31 (s, 3H), 3.18-2.70 (m, 4H).

**Example 42**

**3-cyclopentyl-3-(4-(5-fluoro-2-((7-methyl-2,3,6,7,8,9-hexahydro-5*H*-benzo[*b*][1,4,7] trioxa[10]azacyclododecan-12-yl)amino)pyrimidin-4-yl)-1*H*-pyrazol-1-yl)propanenitrile**

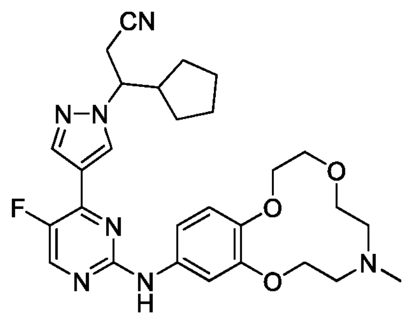

**42**

The implementation method was the same as method 2, except that fragment 25-b was used to replace fragment 2-b, fragment 1-a was used to replace fragment 2-d, and fragment Amine-3 was used to replace fragment Amine-1 to obtain the title compound.

LC_MS: ($ES^+$): m/z 536.9 $[M+H]^+$.

$^1$H NMR (400 MHz, DMSO-d6): δ 9.57(s, 1H), 8.57 (d, *J* = 1.6 Hz, 1H), 8.52 (d, *J* = 2.8 Hz, 1H), 8.18 (s, 1H), 7.59 (d, *J* = 1.6 Hz, 1H), 7.40 (d, *J* = 8.8 Hz, 1H), 7.08 (d, *J* = 8.8 Hz, 1H), 4.66-4.60 (m, 1H), 4.29 (s, 2H), 4.10 (brs, 2H), 3.82-3.72 (m, 4H), 3.32 (s, 3H), 3.23-3.20 (m, 2H), 2.79 (brs, 2H), 2.43-2.36 (m, 2H), 1.85-1.79 (m, 1H), 1.65-1.52 (m, 4H), 1.33-1.25 (m, 2H), 1.20-1.11 (m, 2H).

**Example 43**

**2-(1-(ethylsulfonyl)-3-(4-(5-fluoro-2-((7-methyl-2,3,6,7,8,9-hexahydro-5*H*-benzo[*b*][ 1,4,7]trioxa[10]azacyclododecan-12-yl)amino)pyrimidin-4-yl)-1*H*-pyrazol-1-yl)azetidin-3-yl) acetonitrile**

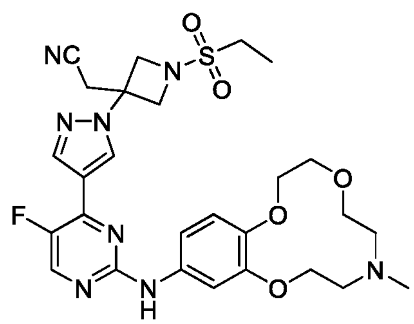

**43**

The implementation method was the same as method 2, except that fragment 14-a was used to replace fragment 2-b, fragment 1-a was used to replace fragment 2-d, and fragment Amine-3 was used to replace fragment Amine-1 to obtain the title compound.

LC_MS: ($ES^+$): m/z 601.6 $[M+H]^+$.

$^1$H NMR (400 MHz, DMSO-d6): δ 9.61 (s, 1H), 8.74 (s, 1H), 8.57 (d, *J* = 2.4 Hz, 1H), 8.26 (s, 1H), 7.59 (s, 1H), 7.36 (d, *J* = 7.6 Hz, 1H), 7.07 (d, *J* = 7.6 Hz, 1H), 4.54 (d, *J* = 9.2 Hz, 2H), 4.25 (d, *J* = 9.2 Hz, 4H), 4.10-4.09 (m, 2H), 3.74-3.69 (m, 6H), 3.31 (s, 3H), 3.26-3.21 (m, 2H), 2.82 (brs, 4H), 1.24 (t, *J* = 7.2 Hz, 3H).

**Example 44**

***N*-(cyanomethyl)-4-(5-fluoro-2-((7-methyl-2,3,6,7,8,9-hexahydro-5*H*-benzo[*b*] [1,4,7]trioxa[10]azacyclododecan-12-yl)amino)pyrimidin-4-yl)benzamide**

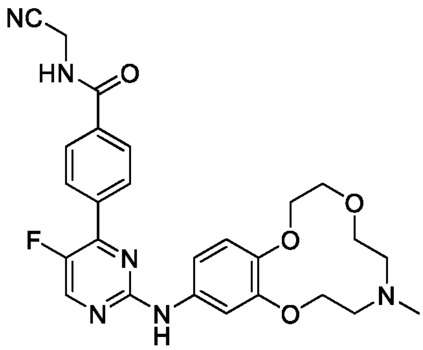

**44**

The implementation method was the same as method 2, except that fragment 1-a was used to replace fragment 2-d, and fragment Amine-3 was used to replace fragment Amine-1 to obtain the title compound.
LC_MS: (ES$^+$): m/z 507.8 [M+H]$^+$.
$^1$H NMR (400 MHz, DMSO-d6): δ 9.79 (s, 1H), 9.52-9.50 (m, 1H), 8.68 (d, *J* = 3.2 Hz, 1H), 8.17-8.15 (m, 2H), 8.10-8.07 (m, 2H), 7.65 (s, 1H), 7.33-7.31 (m, 1H), 7.08-7.06 (m, 1H), 4.37-4.35 (m, 2H), 4.28-4.22 (m, 2H), 4.17-4.09 (m, 2H), 3.83-3.70 (m, 4H), 3.32 (s, 3H), 2.92-2.66 (m, 4H).

**Example 45**

***N*-(tert-butyl)-3-((6-((7-methyl-2,3,6,7,8,9-hexahydro-5*H*-benzo[*b*][1,4,7]trioxa[10]a zacyclododecan-12-yl)amino)-1*H*-pyrazolo[3,4-*d*]pyrimidin-4-yl)amino)benzenesulfonamide**

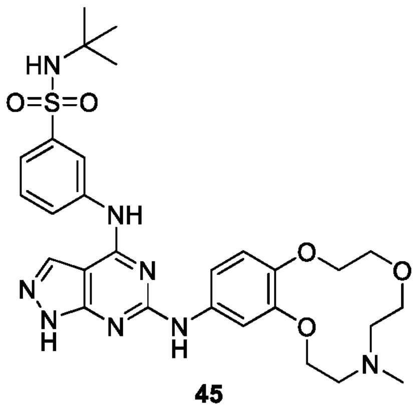

**45**

The implementation method was the same as method 4, except that fragment Amine-3 was used to replace fragment Amine-1 to obtain the title compound.
LC_MS: (ES$^+$): m/z 597.6 [M+H]$^+$.
$^1$H NMR (400 MHz, DMSO-d6): δ 13.08-13.06 (m, 1H), 10.04 (s, 1H), 9.13 (s, 1H), 8.50-8.48 (m, 1H), 8.22-8.16 (m, 2H), 7.70 (s, 1H), 7.61 (s, 1H), 7.56-7.50 (m, 2H), 7.40-7.39 (m, 1H), 7.05-7.03 (m, 1H), 4.17-4.11 (m, 4H), 3.73 (s, 4H), 3.32 (s, 3H), 2.94-2.62 (m, 4H), 1.14 (s, 9H).

**Example 46**

**7-methyl-N-(4-(1-methyl-1H-indol-3-yl)-7H-pyrrolo[2,3-d]pyrimidin-2-yl)-2,3,6,7,8, 9-hexahydro-5*H*-benzo[*b*][1,4,7]trioxa[10]azacyclododecan-12-amine**

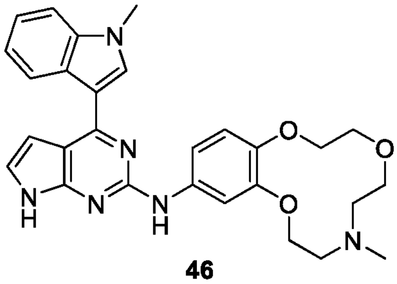

**46**

The implementation method was the same as method 5, except that fragment 2-c was used to replace fragment 5-a and fragment Amine-3 was used to replace fragment Amine-1 to obtain the title compound.

LC_MS: ($ES^+$): m/z 499.8 $[M+H]^+$.

$^1H$ NMR (400 MHz, DMSO-*d*6): δ 11.43 (s, 1H), 9.00 (s, 1H), 8.87 (d, *J* = 7.6 Hz, 1H), 8.41 (s, 1H), 7.76 (s, 1H), 7.30-7.18 (m, 3H), 7.00 (d, *J* = 8.8 Hz, 2H), 6.87 (s, 1H), 4.17-4.11 (m, 4H), 3.95 (s, 3H), 3.76-3.71 (m, 4H), 3.12-2.87 (m, 4H), 2.67 (s, 3H).

**Example 47**

**7-methyl-N-(4-(1-methyl-1H-indol-3-yl)-1H-pyrazolo[3,4-d]pyrimidin-6-yl)-2,3,6,7, 8,9-hexahydro-5*H*-benzo[*b*][1,4,7]trioxa[10]azacyclododecan-12-amine**

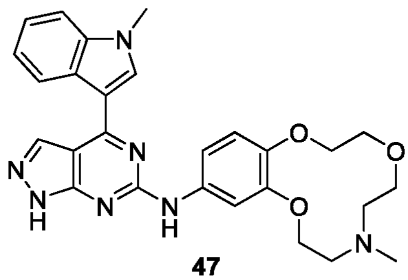

**47**

The implementation method was the same as method 5, except that fragment Amine-3 was used to replace fragment Amine-1 to obtain the title compound.

LC_MS: ($ES^+$): m/z 500.7 $[M+H]^+$.

$^1H$ NMR (400 MHz, DMSO-d6): δ 13.18 (s, 1H), 9.37(s, 1H), 8.91 (d, *J* = 7.6 Hz, 1H), 8.66 (s, 1H), 8.50 (s, 1H), 7.65-7.64 (m, 1H), 7.56 (d, *J* = 8.0 Hz, 1H), 7.47-7.45 (m, 1H), 7.34-7.23 (m, 2H), 7.00 (d, *J* = 8.8 Hz, 1H), 4.12-4.09 (m, 4H), 3.96 (s, 3H), 3.71-3.64 (m, 4H), 3.30 (s, 3H), 2.78 (brs, 2H), 2.63 (brs, 2H).

**Example 48**

**3-cyclopentyl-3-(4-(2-((7-methyl-2,3,6,7,8,9-hexahydro-5*H*-benzo[*b*][1,4,7]trioxa [10]azacyclododecan-12-yl)amino)-7*H*-pyrrolo[2,3-d]pyrimidin-4-yl)-1*H*-pyrazol-1-yl) propionitrile**

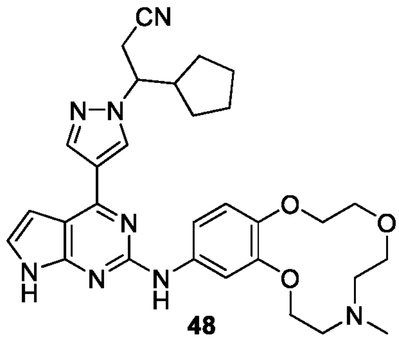

**48**

The implementation method was the same as method 2, except that fragment 25-b was used to replace fragment 2-b and fragment Amine-3 was used to replace fragment Amine-1 to obtain the title compound.

LC_MS: ($ES^+$): m/z 557.8 $[M+H]^+$.

$^1$HNMR (400 MHz, DMSO-d6): δ 11.51 (s, 1H), 9.04 (s, 1H), 8.69 (s, 1H), 8.30 (s, 1H), 7.74 (s, 1H), 7.73-7.40 (m, 1H), 7.24-7.22 (m, 1H), 7.00-6.98 (m, 1H), 6.80-6.79 (m, 1H), 4.59-4.53 (m, 1H), 4.26-4.09 (m, 4H), 3.71 (s, 4H), 3.31 (s, 3H), 3.26-3.21 (m, 2H), 2.95-2.75 (m, 2H), 2.46-2.40 (m, 2H), 1.85-1.81 (m, 1H), 1.63-1.51 (m, 3H), 1.47-1.43 (m, 1H), 1.35-1.17 (m, 4H).

**Example 49**

**2-(1-(ethylsulfonyl)-3-(4-(2-((7-methyl-2,3,6,7,8,9-hexahydro-5*H*-benzo[*b*][1,4,7]trio xa[10]azacyclododecan-12-yl)amino)-7*H*-pyrrolo[2,3-*d*]pyrimidin-4-yl)-1*H*-pyrazol-1-yl) azetidin-3-yl)acetonitrile**

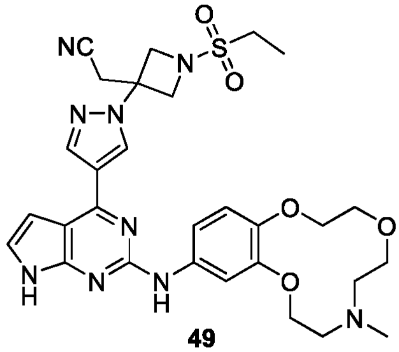

**49**

The implementation method was the same as method 2, except that fragment 14-a was used to replace fragment 2-b and fragment Amine-3 was used to replace fragment Amine-1 to obtain the title compound.

LC_MS: ($ES^+$): m/z 622.6 $[M+H]^+$.

$^1$H NMR (400 MHz, DMSO-d6): δ 11.55 (s, 1H), 9.08 (s, 1H), 8.82 (s, 1H), 8.39 (s, 1H), 7.72 (s, 1H), 7.40 (d, *J* = 8.0 Hz, 1H), 7.26-7.25 (m, 1H), 6.98 (d, *J* = 8.4 Hz, 1H), 6.89 (d, *J* = 3.2 Hz, 1H), 4.59 (d, *J* = 9.2 Hz, 2H), 4.25 (d, *J* = 8.8 Hz, 2H), 4.16-4.08 (m, 4H), 3.70 (s, 6H), 3.31 (s, 3H), 3.28-3.22 (m, 2H), 2.82 (brs, 2H), 2.39 (brs, 2H), 1.25 (t, *J* = 7.2 Hz, 3H).

**Example 50**

**2-(1-(ethylsulfonyl)-3-(4-(6-((7-methyl-2,3,6,7,8,9-hexahydro-5*H*-benzo[*b*][1,4,7] trioxa[10]azacyclododecan-12-yl)amino)-1*H*-pyrazolo[3,4-d]pyrimidin-4-yl)-1*H*-pyrazol-1-y 1)azetidin-3-yl)acetonitrile**

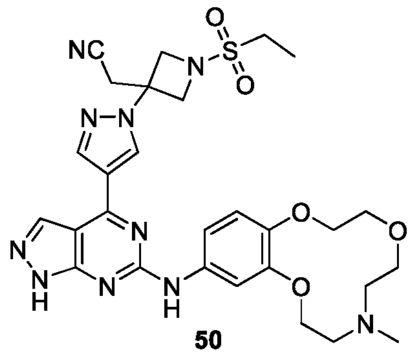

**50**

The implementation method was the same as method 2, except that fragment 14-a was used to replace fragment 2-b, fragment 4-a was used to replace fragment 2-d, and fragment Amine-3 was used to replace fragment Amine-1 to obtain the title compound.

LC_MS: ($ES^+$): m/z 623.6 $[M+H]^+$.

$^1H$ NMR (400 MHz, DMSO-d6): δ 13.37 (s, 1H), 9.62 (s, 1H), 8.98 (s, 1H), 8.58 (s, 1H), 8.47 (s, 1H), 7.74 (s, 1H), 7.50 (d, *J* = 8.0 Hz, 1H), 7.09 (d, *J* = 8.0 Hz, 1H), 4.60 (d, *J* = 9.2 Hz, 2H), 4.44-4.26 (m, 4H), 4.12 (brs, 2H), 3.92-3.72 (m, 6H), 3.32 (s, 3H), 3.28-3.23 (m, 2H), 2.85 (brs, 4H), 1.26 (t, *J* = 7.6 Hz, 3H).

**Example 51**

***N*-(cyanomethyl)-4-(2-((7-methyl-2,3,6,7,8,9-hexahydro-5*H*-benzo[*b*][1,4,7]trioxa [10]azacyclododecan-12-yl) amino)-7*H*-pyrrolo[2,3-*d*]pyrimidin-4-yl)benzamide**

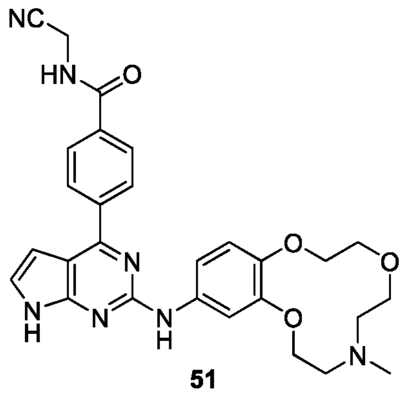

**51**

The implementation method was the same as method 2, except that fragment Amine-3 was used to replace fragment Amine-1 to obtain the title compound.

LC_MS: ($ES^+$): m/z 528.8 $[M+H]^+$.

$^1H$ NMR (400 MHz, DMSO-d6): δ 11.74 (s, 1H), 9.45 (t, *J* = 5.2 Hz, 1H), 9.34 (s, 1H), 8.27 (d, *J* = 8.4 Hz, 2H), 8.09 (d, *J* = 8.4 Hz, 2H), 7.84 (s, 1H), 7.43 (d, *J* = 8.0 Hz, 1H), 7.34 (d, *J* = 3.2 Hz, 1H), 7.04 (d, *J* = 8.4 Hz, 1H), 6.74 (d, *J* = 2.8 Hz, 1H), 4.37-4.29 (m, 4H), 4.13-4.10 (m, 2H), 3.81-3.74 (m, 4H), 3.31 (s, 3H), 3.02-2.62 (m, 4H).

**Example 52**

***N*-(cyanomethyl)-4-(6-((7-methyl-2,3,6,7,8,9-hexahydro-5*H*-benzo[*b*][1,4,7]trioxa[1 0]azacyclododecan-12-yl) amino)-1*H*-pyrazolo[3,4-*d*]pyrimidin-4-yl)benzamide**

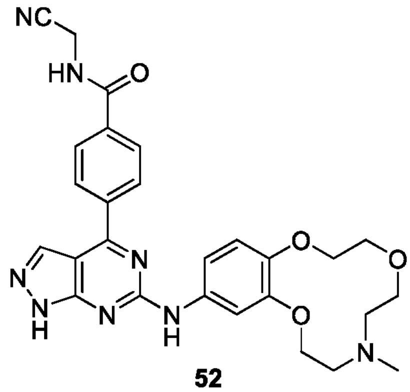

**52**

The implementation method was the same as method 2, except that fragment 4-a was used to replace fragment 2-d and fragment Amine-3 was used to replace fragment Amine-1 to obtain the title compound.

LC_MS: ($ES^+$): m/z 529.7 $[M+H]^+$.

$^1H$ NMR (400 MHz, DMSO-d6): δ 13.48 (s, 1H), 9.75 (s, 1H), 9.44 (t, *J* = 5.6 Hz, 1H), 8.43-8.36 (m, 3H), 8.11 (d, *J* = 8.4 Hz, 2H), 7.71 (d, *J* = 1.6 Hz, 1H), 7.42 (d, *J* = 8.4 Hz, 1H), 7.03 (d, *J* = 8.4 Hz, 1H), 4.38-4.37 (m, 2H), 4.17-4.09 (m, 4H), 3.71 (s, 4H), 3.30 (s, 3H), 2.97-2.79 (m, 2H), 2.45-2.33 (m, 2H).

**Example 53**

***N*⁴-(2-(isopropylsulfonyl)phenyl)-*N*²-(7-methyl-2,3,6,7,8,9-hexahydro-5*H*-benzo[*b*][ 1,4,7]trioxa[10]azacyclododecan-12-yl)-7*H*-pyrrolo[2,3-*d*]pyrimidin-2,4-diamine**

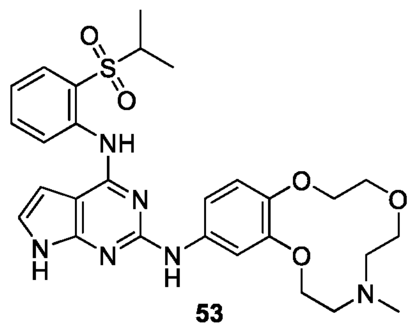

**53**

The implementation method was the same as method 4, except that fragment 2-(isopropylsulfonyl)aniline was used to replace fragment 1-b, fragment 2-d was used to replace fragment 4-a, and fragment Amine-3 was used to replace fragment Amine-1 to obtain the title compound.

LC_MS: ($ES^+$): m/z 567.6 $[M+H]^+$.

$^1H$ NMR (400 MHz, DMSO-*d*6): δ 11.45 (s, 1H), 9.48 (s, 1H), 8.89-8.93 (m, 2H), 7.81-7.84 (m, 1H), 7.72-7.76 (m, 1H), 7.56 (s, 1H), 7.28-7.31 (m, 2H), 7.04-7.05 (m, 1H), 6.92-6.94 (m, 1H), 6.25-6.26 (m, 1H), 4.03-4.08 (m, 4H), 3.67-3.69 (m, 4H), 3.44-3.48 (m, 1H), 3.31 (s, 3H), 2.75-2.90 (m, 2H), 2.33-2.36 (m, 2H), 1.17-1.19 (d, *J* = 6.8 Hz, 6H).

**Example 54**

***N*-(3-((5-chloro-2-((7-methyl-2,3,6,7,8,9-hexahydro-5*H*-benzo[*b*][1,4,7]trioxa[10] azacyclododecan-13-yl)amino) pyrimidin-4-yl)amino)phenyl)acrylamide**

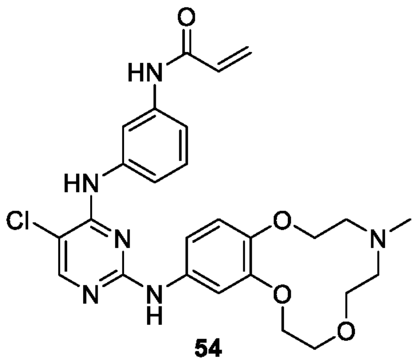

**54**

The implementation method was the same as method 3, except that fragment tert-butyl (3-aminophenyl) carbamate was used to replace fragment 3-a, fragment 1-a was used to replace fragment 2-d, and fragment Amine-2 was used to replace fragment Amine-1 to obtain the title compound.

LC_MS: (ES$^+$): m/z 525.2 [M+H]$^+$.

$^1$H NMR (400 MHz, DMSO-d6): δ 10.35 (s, 1H), 9.33 (s, 1H), 8.89 (s, 1H), 8.13 (s, 1H), 7.96 (s, 1H), 7.56-7.57 (m, 1H), 7.42(s, 1H), 7.20-7.29 (m, 3H), 6.81-6.83 (m, 1H), 6.48-6.55 (m, 1H), 6.22-6.27 (m, 1H), 5.73-5.76 (m, 1H), 4.06 (s, 2H), 3.68-3.81 (m, 6H), 3.32 (s, 3H), 2.91 (brs, 4H).

**Example 55**

***N*-(3-((5-chloro-2-((4-methyl-3,4,5,6-tetrahydro-2*H*-benzo[*b*][1,4,7]dioxazolin-9-yl) amino)pyrimidin-4-yl)amino) phenyl)acrylamide**

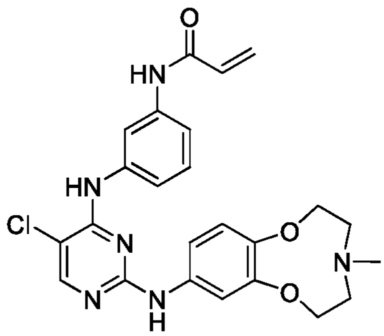

**55**

The implementation method was the same as method 3, except that fragment tert-butyl (3-aminophenyl) carbamate was used to replace fragment 3-a, and fragment 1-a was used to replace fragment 2-d to obtain the title compound.

LC_MS: (ES$^+$): m/z 481.2 [M+H]$^+$.

$^1$H NMR (400 MHz, DMSO-d6): δ 10.12 (s, 1H), 8.91 (s, 1H), 8.80 (s, 1H), 8.06 (s, 1H), 7.84 (s, 1H), 7.49 (d, *J* = 8.0 Hz, 1H), 7.36 (d, *J* = 7.6 Hz, 1H), 7.29-7.25 (m, 1H), 7.00-6.97 (m, 2H), 6.49-6.42 (m, 2H), 6.28-6.23 (m, 1H), 5.76-5.74 (m, 1H), 4.07 (s, 2H), 3.51-3.46 (m, 2H), 3.25 (brs, 7H).

**Example 56**

***N*-(3-((5-chloro-2-((7-methyl-2,3,6,7,8,9-hexahydro-5*H*-benzo[*b*][1,4,7]trioxa[10] azacyclododecan-12-yl)amino) pyrimidin-4-yl)amino)phenyl)acrylamide**

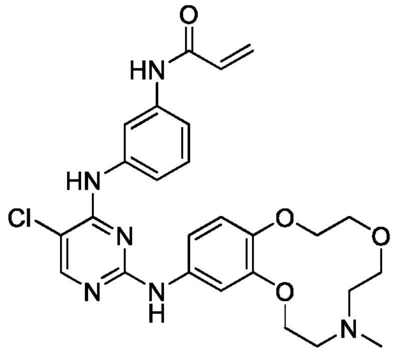

**56**

The implementation method was the same as method 3, except that fragment tert-butyl (3-aminophenyl) carbamate was used to replace fragment 3-a, fragment 1-a was used to replace fragment 2-d, and fragment Amine-3 was used to replace replacing fragment Amine-1 to obtain the title compound.

LC_MS: (ES+): m/z 526.0 [M+H]+.

$^1$H NMR (400 MHz, DMSO-*d*6): δ 10.37 (s, 1H), 9.34 (s, 1H), 8.89 (s, 1H), 8.77 (s, 1H), 8.13 (s, 1H), 7.97 (s, 1H), 7.59 (s, 1H), 7.43 (s, 1H), 7.29 (s, 2H), 6.56-6.48 (m, 1H), 6.27-6.22 (m, 1H), 5.76-5.73 (m, 1H), 4.14-3.95 (m, 4H), 3.77-3.69 (m, 4H), 3.31 (s, 3H), 2.77 (m, 4H).

**Example 57**

***N*-(3-((5-chloro-2-((7-methyl-2,3,6,7,8,9-hexahydro-5*H*-benzo[*b*][1,4,7]trioxa[10] azacyclododecan-13-yl)amino) pyrimidin-4-yl)amino)phenyl)propanamide**

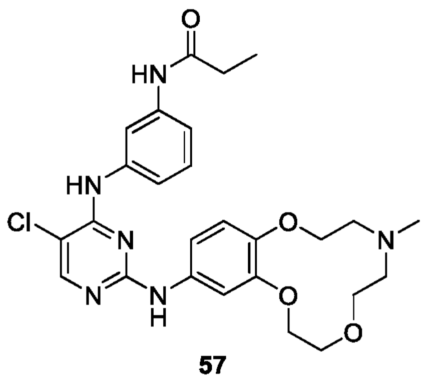

**57**

The implementation method was the same as method 3, except that fragment tert-butyl (3-aminophenyl) carbamate was used to replace fragment 3-a, fragment 1-a was used to replace fragment 2-d, fragment propionyl chloride was used to replace fragment 3-e, and fragment Amine-2 was used to replace fragment Amine-1 to obtain the title compound.

LC_MS: (ES+): m/z 527.2 [M+H]+.

$^1$H NMR (400 MHz, DMSO-d6): δ 10.01 (s, 1H), 9.34 (s, 1H), 8.87 (s, 1H), 8.13 (s, 1H), 7.87 (s, 1H), 7.46 (s, 2H), 7.19-7.25 (m, 3H), 6.86-6.88 (m, 1H), 4.14 (s, 2H), 3.70-3.79 (m, 6H), 3.32 (s, 3H), 2.50-2.79 (m, 4H), 2.29-2.35 (m, 2H), 1.07 (t, *J* = 7.6 Hz, 3H).

**Example 58**

***N*-(3-((5-chloro-2-((4-methyl-3,4,5,6-tetrahydro-2*H*-benzo[*b*][1,4,7]dioxazolin-9-yl) amino)pyrimidin-4-yl)amino) phenyl)propanamide**

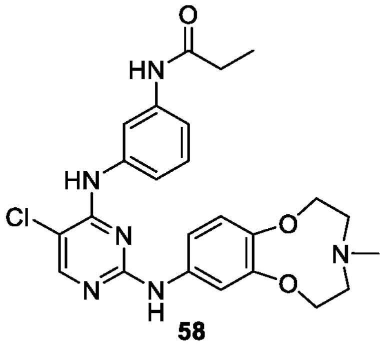

58

The implementation method was the same as method 3, except that fragment tert-butyl (3-aminophenyl) carbamate was used to replace fragment 3-a, fragment 1-a was used to replace fragment 2-d, and fragment propionyl chloride was used to replace fragment 3-e to obtain the title compound.
LC_MS: ($ES^+$): m/z 483.8 $[M+H]^+$.
$^1$HNMR (400 MHz, DMSO-*d*6): δ 9.83 (s, 1H), 8.89 (s, 1H), 8.75 (s, 1H), 8.06 (s, 1H), 7.78 (s, 1H), 7.38-7.34 (m, 2H), 7.25-7.21 (m, 1H), 7.02-6.97 (m, 2H), 6.49 (d, *J* = 8.4 Hz, 1H), 4.10-4.08 (m, 2H), 3.48 (t, *J* = 5.4 Hz, 2H), 3.41-3.34 (m, 2H), 3.29-3.28 (m, 2H), 3.25 (s, 3H), 2.34-2.29 (m, 2H), 1.07 (t, *J* = 7.4 Hz, 3H).

**Example 59**

***N*-(3-((5-chloro-2-((7-methyl-2,3,6,7,8,9-hexahydro-5*H*-benzo[*b*][1,4,7]trioxa[10] azacyclododecan-12-yl)amino) pyrimidin-4-yl)amino)phenyl)propanamide**

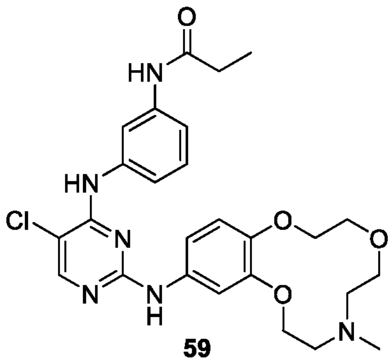

59

The implementation method was the same as method 3, except that fragment tert-butyl (3-aminophenyl) carbamate was used to replace fragment 3-a, fragment 1-a was used to replace fragment 2-d, fragment propionyl chloride was used to replace fragment 3-e, and fragment Amine-3 was used to replace fragment Amine-1 to obtain the title compound.
LC_MS: ($ES^+$): m/z 527.2 $[M+H]^+$.
$^1$HNMR (400 MHz, DMSO-*d*6): δ 9.87 (s, 1H), 9.17 (s, 1H), 8.86 (s, 1H), 8.11 (s, 1H), 7.77 (s, 1H), 7.42-7.40 (m, 1H), 7.32-7.25 (m, 3H), 7.15 (d, *J* = 8.8 Hz, 1H), 6.78 (d, *J* = 8.4 Hz, 1H), 4.05-3.98 (m, 2H), 3.83-3.73 (m, 2H), 3.69-3.56 (m, 4H), 3.33 (s, 3H), 2.67-2.59 (m, 2H), 2.33-2.28 (m, 4H), 1.07 (t, *J* = 7.6 Hz, 3H).

**Example 60**

***N*$^6$-(2-(isopropylsulfonyl)phenyl)-*N*$^2$-(7-methyl-2,3,6,7,8,9-hexahydro-5*H*-benzo[*b*][ 1,4,7]trioxa[10]azacyclododecan-13-yl)-9*H*-purin-2,6-diamine**

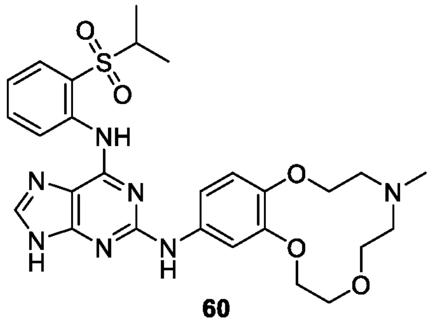

**60**

The implementation method was the same as method 4, except that fragment 2-(isopropylsulfonyl)aniline was used to replace fragment 1-b, fragment 60-b was used to replace fragment 4-a, and fragment Amine-2 was used to replace fragment Amine-1 to obtain the title compound.
LC_MS: (ES$^+$): m/z 568.3 [M+H]$^+$.
$^1$H NMR (400 MHz, DMSO-$d_6$): δ 12.80 (s, 1H), 9.84 (s, 1H), 9.14 (s, 1H), 9.02 (d, *J* = 8.4 Hz, 1H), 8.00 (s, 1H), 7.82-7.84 (m, 1H), 7.74 (t, *J* = 7.2 Hz, 1H), 7.56 (s, 1H), 7.28-7.32 (m, 2H), 6.96 (d, *J* = 8.8 Hz, 1H), 4.05-4.10 (m, 4H), 3.70-3.73 (m, 4H), 3.41-3.48 (m, 1H), 3.31 (s, 3H), 2.84 (s, 2H), 2.33-2.41 (m, 2H), 1.19 (d, *J* = 6.8 Hz, 6H).

**Fragment 60-b was synthesized as follows:**

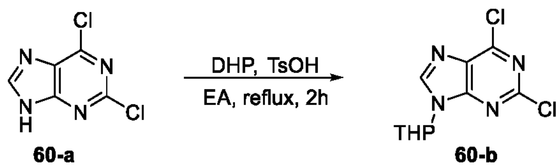


Step 1 : synthesis of fragment **60-b** 2,6-dichloro-9-(tetrahydro-2*H*-pyran-2-yl)-9*H*-purine

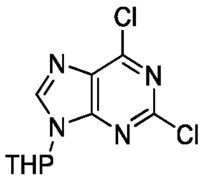

**60-b**

A solution of 2,6-dichloro-9*H*-purine (1 g, 5.32 mmol), 3,4-dihydro-2*H*-pyran (1.2 g, 14.36 mmol) and p-toluenesulfonic acid (27 mg, 0.16 mmol) in ethyl acetate was stirred at 50°C for 2 hours. The completion of the reaction was monitored by TLC. The reaction mixture was cooled to room temperature and concentrated under reduced pressure. The obtained crude product was separated and purified by silica gel column chromatography (eluted with 2% ethyl acetate in petroleum ether) to obtain 2,6-dichloro-9-(tetrahydro-2*H*-pyran-2-yl)-9*H*-purine (1 g, 68%) as a white solid .
LC_MS: (ES$^+$): m/z 272.9 [M+H]$^+$.
$^1$H NMR (400 MHz, $CDCl_3$): δ 8.33 (s, 1H), 5.75-5.78 (m, 1H), 4.17-4.21 (m, 1H), 3.75-3.82 (m, 1H), 2.15-2.19 (m, 1H), 2.08-2.11 (m, 1H), 1.92-2.02 (m, 1H), 1.66-1.85 (m, 3H).

**Example 61**

***N*-(2-((2-((7-methyl-2,3,6,7,8,9-hexahydro-5*H*-benzo[*b*][1,4,7]trioxa[10]azacyclodod ecan-13-yl)amino)-7*H*-pyrrolo[2,3-*d*]pyrimidin-4-yl)amino)phenyl)methanesulfonamide**

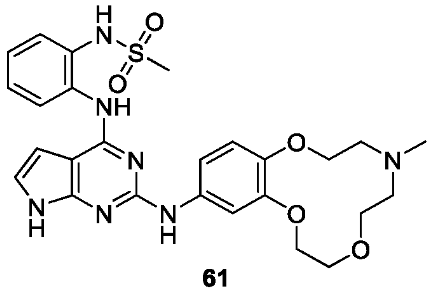

61

The implementation method was the same as method 4, except that fragment N-(2-aminophenyl) methanesulfonamide was used to replace fragment 1-b, fragment 2-d was used to replace fragment 4-a, and fragment Amine-2 was used to replace fragment Amine-1 to obtain the title compound.

LC_MS: ($ES^+$): m/z 554.6 $[M+H]^+$.

$^1$H NMR (400 MHz, DMSO-d6): δ 11.26 (s, 1H), 9.24 (s, 1H), 8.84 (s, 1H), 8.73 (s, 1H), 7.85-7.87(d, *J* = 8Hz, 1H), 7.61 (s, 1H), 7.39-7.41 (d, *J* = 8Hz, 1H), 7.28-7.32 (t, *J* = 8.0Hz, 1H), 7.18-7.23 (m, 2H), 6.95 (s, 1H), 6.85-6.87 (d, *J*= 8.0 Hz, 1H), 6.43 (s, 1H), 4.07 (s, 2H), 3.90 (s, 2H), 3.70 (s, 4H), 3.28 (s, 3H), 2.97 (s, 1H), 2.86 (s, 3H), 2.75 (s, 1H), 2.42 (s, 2H).

**Example 62**

***N*-(4-(1*H*-pyrazol-4-yl)-7*H*-pyrrolo[2,3-*d*]pyrimidin-2-yl)-7-methyl-2,3,6,7,8,9-hexahydro-5*H*-benzo[*b*][1,4,7]trioxa[10]azacyclododecan-13-amine**

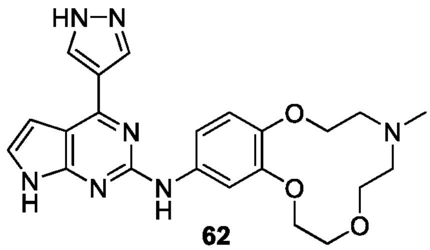

62

The implementation method was the same as method 2, except that fragment 14-e was used to replace fragment 2-b and fragment Amine-2 was used to replace fragment Amine-1 to obtain the title compound.

LC_MS: ($ES^+$): m/z 436.6 $[M+H]^+$.

$^1$H NMR (400 MHz, DMSO-d6): δ 13.30 (s, 1H), 11.45 (s, 1H), 8.96 (s, 1H), 8.26-8.53 (m, 2H), 7.72 (s, 1H), 7.36-7.38 (m, 1H), 7.17-7.19 (m, 1H), 6.93 (d, *J* = 8.8 Hz, 1H), 6.79 (d, *J* = 3.2 Hz, 1H), 4.06-4.16 (m, 4H), 3.66-3.76 (m, 4H), 3.30 (s, 3H), 2.67-2.77 (m, 2H), 2.31-2.33 (m, 2H).

Example 63

**7-methyl-*N*-(4-(1-(methylsulfonyl)-1*H*-pyrazol-4-yl)-7*H*-pyrrolo[2,3-*d*]pyrimidin-2-yl)-2,3,6,7,8,9-hexahydro-5*H*-benzo[*b*][1,4,7]trioxa[10]azacyclododecan-13-amine**

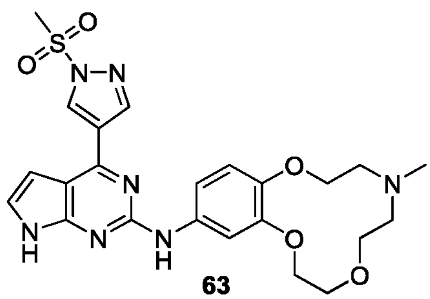

63

The implementation method was the same as method 2, except that fragment 63-a was used to replace fragment 2-b and fragment Amine-2 was used to replace fragment Amine-1 to obtain the title compound.
LC_MS: ($ES^+$): m/z 514.5 $[M+H]^+$.
$^1H$ NMR (400 MHz, DMSO-d6): δ 11.68 (s, 1H), 9.28 (s, 1H), 8.87 (s, 1H), 8.70 (s, 1H), 7.96-7.97 (m, 1H), 7.20-7.32 (m, 2H), 7.06-7.08 (m, 1H), 6.90-6.91 (m, 1H), 4.26-4.30 (m, 3H), 4.11-4.15 (m, 3H), 3.98-4.07 (m, 2H), 3.79-3.83 (m, 2H), 3.70 (s, 3H), 3.55-3.61 (m, 1H), 3.43-3.48 (m, 2H), 3.19-3.23 (m, 1H), 2.91-2.92 (m, 3H).

**Fragment 63-a was synthesized as follows:**

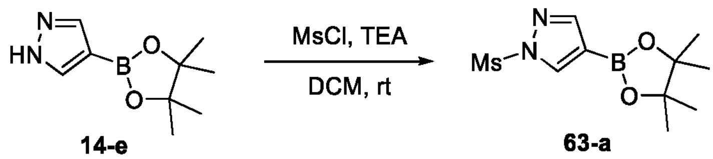


14-e 63-a

Step 1 : synthesis of fragment 63-a 1-(methylsulfonyl)-4-(4,4,5,5-tetramethyl-1,3,2-dioxaborolane-2-yl)-1*H*-pyrazole

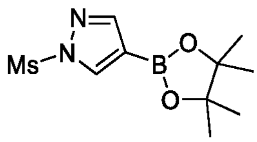

**63-a**

To a solution of 4-(4,4,5,5-tetramethyl-1,3,2-dioxaborolan-2-yl)-1*H*-pyrazole (300 mg, 1.55 mmol) and triethylamine (470 mg, 4.65 mmol) in dichloromethane (5 ml) was added methanesulfonyl chloride (212 mg, 1.86 mmol) at 0°C. The reaction mixture was warmed to room temperature and stirred for 3 hours. The completion of the reaction was monitored by TLC. The reaction mixture was partitioned in water (20 ml) and extracted with dichloromethane (10 ml×3). The organic layers were combined, dried over anhydrous sodium sulfate, and concentrated under reduced pressure. The obtained crude product was separated and purified by preparative TLC (using 30% ethyl acetate in petroleum ether) to obtain 1-(methylsulfonyl)-4-(4,4,5,5-tetramethyl-1,3,2-dioxaborolan-2-yl)-*1H*-pyrazole as a colorless oil (200 mg, 47%).
$^1H$ NMR (400 MHz, DMSO-d6): δ 8.35 (s, 1H), 8.04 (s, 1H), 3.57 (s, 3H), 1.29 (s, 12H).

**Example 64**

***N*⁴-methyl-*N*²-(4-methyl-3,4,5,6-tetrahydro-2*H*-benzo[*b*][1,4,7]dioxazolin-9-yl)-7*H*-pyrrolo[2,3-d]pyrimidin-2,4-diamine**

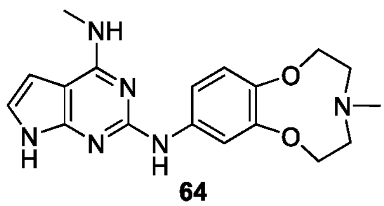

**64**

The implementation method was the same as method 4, except that fragment methylamine hydrochloride was used to replace fragment 1-b, and fragment 2-d was used to replace fragment 4-a to obtain the title compound.
LC_MS: ($ES^+$): m/z 355.1 $[M+H]^+$.
$^1H$ NMR (400 MHz, DMSO-d6): δ 10.96 (s, 1H), 8.36 (s, 1H), 7.38 (s, 1H), 7.22-7.26 (m, 1H), 7.10 (d, *J* = 8.0 Hz, 1H), 6.73 (s, 1H), 6.60 (d, *J* = 8.8 Hz, 1H), 6.36 (s, 1H), 4.11-4.13 (m, 2H), 3.51 (t, *J* = 5.6 Hz, 2H), 3.34-3.37 (m, 2H), 3.29 (s, 2H), 3.26 (s, 3H), 2.96 (d, *J* = 4.4 Hz, 3H).

**Example 65**

***N*[4]-(3-(isopropylsulfonyl)phenyl)-*N*[2]-(7-methyl-2,3,6,7,8,9-hexahydro-5*H*-benzo[*b*] [1,4,7]trioxa[10]azacyclododecan-13-yl)-7*H*-pyrrolo[2,3-*d*]pyrimidin-2,4-diamine**

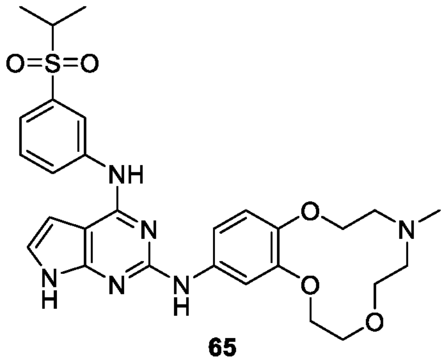

**65**

The implementation method was the same as method 4, except that fragment 3-(isopropylsulfonyl)aniline was used to replace fragment 1-b, fragment 2-d was used to replace fragment 4-a, and fragment Amine-2 was used to replace fragment Amine-1 to obtain the title compound.

LC_MS: ($ES^+$): m/z 567.6 $[M+H]^+$.

$^1H$ NMR (400 MHz, DMSO-d6): δ 11.28 (s, 1H), 9.53 (s, 1H), 8.90-8.92 (m, 1H), 8.76 (s, 1H), 7.94-7.95 (m, 1H), 7.57-7.61 (m, 2H), 7.43 (d, *J* = 8.0 Hz, 1H), 7.29-7.32 (m, 1H), 6.92-6.96 (m, 2H), 6.67-6.68 (m, 1H), 4.05-4.10 (m, 4H), 3.70-3.74 (m, 4H), 3.36-3.43 (m, 1H), 3.30 (s, 3H), 2.88 (brs, 2H), 2.39-2.46 (m, 2H), 1.19 (d, *J* = 6.8 Hz, 6H).

**Example 66**

**dimethyl(3-((2-((7-methyl-2,3,6,7,8,9-hexahydro-5*H*-benzo[*b*][1,4,7]trioxa[10] azacyclododecan-13-yl)amino)-7*H*-pyrrolo[2,3-*d*]pyrimidin-4-yl)amino)phenyl)phosphine oxide**

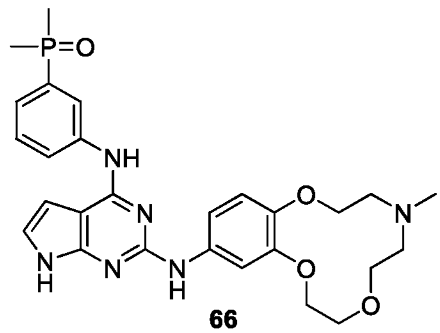

**66**

The implementation method was the same as method 4, except that fragment (3-aminophenyl)dimethylphosphine oxide was used to replace fragment 1-b, fragment 2-d was used to replace fragment 4-a, and fragment Amine-2 was used to replace fragment Amine-1 to obtain the title compound.

LC_MS: ($ES^+$): m/z 537.2 $[M+H]^+$.

$^1H$ NMR (400 MHz, DMSO-d6): δ 11.22 (s, 1H), 9.37 (s, 1H), 8.74-8.80 (m, 1H), 8.46-8.52 (m, 1H), 7.90 (d, *J* = 12.4 Hz, 1H), 7.65-7.73 (m, 1H), 7.44-7.48 (m, 1H), 7.29-7.38 (m, 2H), 6.93-6.96 (m, 2H), 6.68 (s, 1H), 4.14-4.18 (m, 2H), 3.94-4.03 (m, 2H), 3.65-3.80 (m, 4H), 3.31 (s, 3H), 2.74-3.08 (m, 4H), 1.64 (d, *J* = 13.2 Hz, 6H).

**Example 67**

**3-((2-((7-methyl-2,3,6,7,8,9-hexahydro-5*H*-benzo[*b*][1,4,7]trioxa[10]azacyclododeca n-13-yl)amino)-7*H*-pyrrolo [2,3-*d*]pyrimidin-4-yl)amino)benzenesulfonamide**

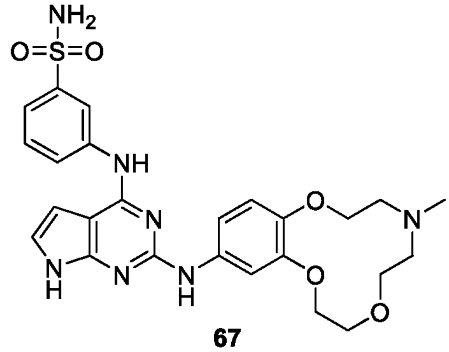

**67**

The implementation method was the same as method 4, except that fragment 3-aminobenzenesulfonamide was used to replace fragment 1-b, fragment 2-d was used to replace fragment 4-a, and fragment Amine-2 was used to replace fragment Amine-1 to obtain the title compound.

LC_MS: ($ES^+$): m/z 540.3 $[M+H]^+$.

$^1H$ NMR (400 MHz, DMSO-d6): δ 11.31 (s, 1H), 9.52 (s, 1H), 8.93-8.94 (m, 1H), 8.48 (s, 1H), 8.47 (d, *J* = 8.0 Hz, 1H), 8.17 (s, 1H), 7.78-7.79 (m, 1H), 7.35-7.52 (m, 4H), 7.03 (d, *J* = 8.8 Hz, 1H), 6.96-6.97 (m, 1H), 6.71-6.72 (m, 1H), 4.25-4.30 (m, 2H), 3.92-4.11 (m, 4H), 3.77-3.80 (m, 2H), 3.56 (brs, 1H), 3.43-3.46 (m, 1H), 3.16-3.23 (m, 2H), 2.91 (s, 3H).

**Example 68**

***N*^4-((1s,3s)-3-(azetidin-1-ylsulfonyl)cyclobutyl)-*N*^4-methyl-*N*^2-(4-methyl-3,4,5,6-tetrahydro-2*H*-benzo[*b*][1,4,7]dioxazolin-9-yl)-7*H*-pyrrolo[2,3-*d*]pyrimidin-2,4-diamine**

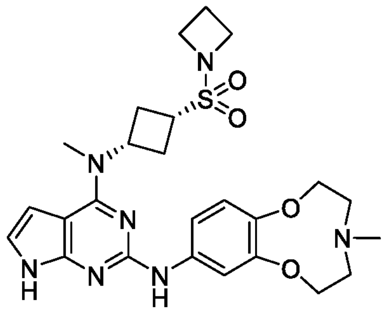

**68**

The implementation method was the same as method 4, except that fragment 68-h was used to replace fragment 1-b, and fragment 2-d was used to replace fragment 4-a to obtain the title compound.

LC_MS: ($ES^+$): m/z 528.3 $[M+H]^+$.

$^1H$ NMR (400 MHz, DMSO-d6): δ 11.19 (s, 1H), 8.43 (brs, 1H), 7.27 (brs, 1H), 6.99-7.14 (m, 1H), 6.81-6.92 (m, 1H), 6.47-6.73 (m, 2H), 5.14-5.23 (m, 1H), 4.09-4.17 (m, 1H), 3.87 (t, *J* = 7.6 Hz, 4H), 3.46-3.55 (m, 2H), 3.29-3.44 (m, 6H), 3.17-3.25 (m, 6H), 2.55-2.69 (m, 4H), 2.17-2.25 (m, 2H).

**Fragment 68-h was synthesized as follows:**

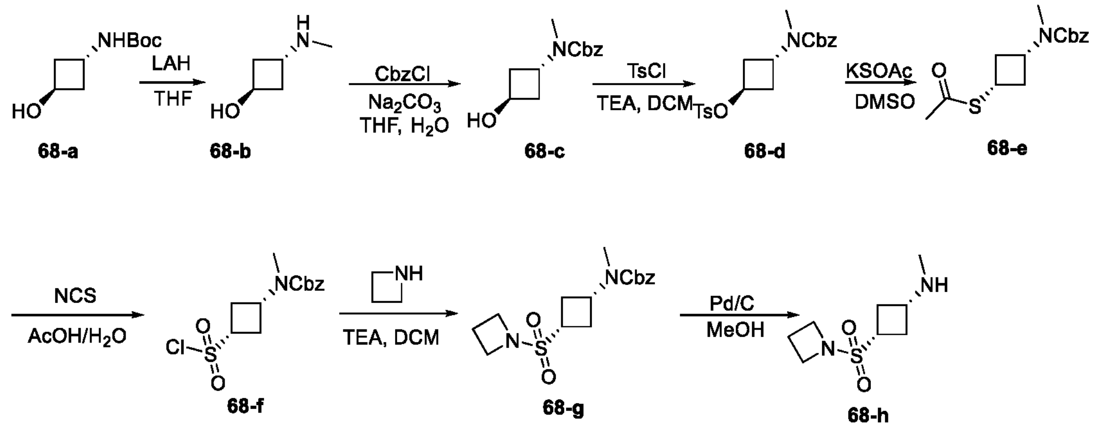


68-a 68-b 68-c 68-d 68-e 68-f 68-g 68-h

Step 1 : synthesis of fragment **68-b** (1*r*,3*r*)-3-(methylamino)cyclobutan-1-ol

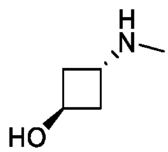

**68-b**

To a solution of tert-butyl((1*r*,3*r*)-3-hydroxycyclobutyl)carbamate (1 g, 5.34 mmol) in anhydrous tetrahydrofuran (15 ml) was added dropwise lithium aluminum hydride (812 mg, 21.36 mmol) at 0°C. The resulting mixture was stirred at 65 °C for 18 hours. The completion of the reaction was monitored by TLC. The reaction mixture solution was quenched by adding water (0.8 ml), sodium hydroxide (0.8 ml, 15% aqueous solution) and water (2.4 ml) at 0 °C, and the reaction was stired at room temperature for another 10 minutes. The solid was removed by filtration, and the filter cake was washed with 10% methanol in dichloromethane (20 ml×3). The filtrates were combined and concentrated under reduced pressure to obtain (1r,3r)-3-(methylamino)cyclobutan-1-ol as colorless oil (540 mg, crude product), which was directly used in the next step for reaction.

Step 2 : synthesis of fragment **68-c** benzyl ((1*r*,3*r*)-3-hydroxycyclobutyl)(methyl)carbamate

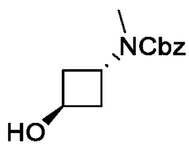

**68-c**

To a solution of crude product of (1*r*,3*r*)-3-(methylamino)cyclobutan-1-ol (540 mg, 5.34 mmol) and sodium carbonate (1.1 g, 10.7 mmol) in tetrahydrofuran (10 ml) and water (2.5 ml) was added benzyl chloroformate at 0°C. The resulting mixture was heated to room temperature and stirred for 15 hours. The completion of the reaction was monitored by TLC. The reaction mixture was partitioned in ethyl acetate (10 ml) and water (15 ml). The organic layer was collected, washed with saturated sodium chloride solution (10 ml), dried over anhydrous sodium sulfate, and concentrated under reduced pressure. The crude product was separated and purified by silica gel column chromatography (eluted with 20%-100% ethyl acetate in n-hexane) to obtain benzyl((1*r*,3*r*)-3-hydroxycyclobutyl)(methyl)carbamate as colorless oil (1.1 g, 87% for two steps).

LC_MS: (ES$^+$): m/z 236.1 [M+H]$^+$.

$^1$H NMR (400 MHz, DMSO-d6): δ 7.31-7.39 (m, 5H), 5.05 (s, 2H), 4.97 (d, *J* = 4.4 Hz, 1H), 4.71-4.80 (m, 1H), 4.15-4.17 (m, 1H), 2.82 (s, 3H), 2.30-2.37 (m, 2H), 1.95-2.01 (m, 2H).

Step 3 : synthesis of fragment **68-d** (1*r*,3*r*)-3-(((benzyloxy)carbonyl)(methyl)amino)cyclobutyl-4-methylbenzenesulfonic acid

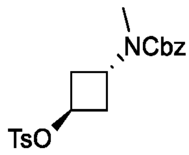

**68-d**

To a solution of benzyl((1*r*,3*r*)-3-hydroxycyclobutyl)(methyl)carbamate (1.1 g, 4.66 mmol), triethylamine (944 mg, 9.32 mmol), 4-dimethylaminopyridine (57 mg, 0.47 mmol) in dichloromethane (15 ml) at 0°C was added 4-toluenesulfonyl chloride (977 mg, 5.13 mmol). The resulting mixture was heated to room temperature and stirred for 16 hours. The completion of the reaction was monitored by TLC. The reaction mixture was diluted by adding dichloromethane (10 ml), washed with water (20 ml×2), washed with saturated sodium chloride solution (20 ml), dried over anhydrous sodium sulfate, and concentrated under reduced pressure. The obtained crude product was separated and purified by silica gel column chromatography (eluted with 10%-15% ethyl acetate in n-hexane) to obtain (1*r*,3*r*)-3-(((benzyloxy)carbonyl)(methyl)amino)cyclobutyl-4-methylbenzenesulfonic acid as a colorless oil (1.63 g, 89%) .

$^1$H NMR (400 MHz, $CDCl_3$): δ 7.76 (d, *J* = 8.4 Hz, 2H), 7.31-7.36 (m, 7H), 5.09 (s, 2H), 4.87-4.97 (m, 1H), 4.71-4.79 (m, 1H), 2.84 (s, 3H), 2.48-2.58 (m, 2H), 2.44 (s, 3H), 2.39-2.45 (m, 2H).

Step 4 : synthesis of fragment 68-e S-((1s,3s)-3-(((benzyloxy)carbonyl)(methyl)amino)cyclobutyl) ethanethioate

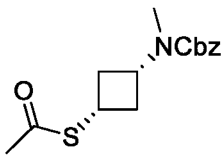

**68-e**

A solution of (1*r*,3*r*)-3-(((benzyloxy)carbonyl)(methyl)amino)cyclobutyl-4-methylbenzenesulfonic acid (1.63 g, 4.58 mmol) and potassium thioacetate (2.62 g, 22.93 mmol) in dimethyl sulfoxide (16 ml) was stirred at 60 °C for 17 hours. The completion of the reaction was monitored by TLC. The reaction mixture was partitioned in ethyl acetate (10 ml) and water (15 ml). The aqueous layer was extracted with ethyl acetate (10 ml×2). The organic layers were combined, washed with saturated sodium chloride solution (20 ml×3), dried over anhydrous sodium sulfate, and concentrated under reduced pressure. The obtained crude product was separated and purified by silica gel column chromatography (eluted with 10-20% ethyl acetate in n-hexane) to obtain *S*-((1*s*,3*s*)-3-(((benzyloxy)carbonyl)(methyl)amino)cyclobutyl)ethanethioate (1 g, 81%) as colorless oil.

LC_MS: ($ES^+$): m/z 294.1 $[M+H]^+$.

Step 5 : synthesis of fragment **68-f** benzyl ((1*s*,3*s*)-3-(chlorosulfonyl)cyclobutyl)(methyl)carbamate

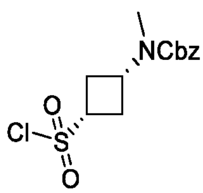

**68-f**

A solution of *S*-((1*s*,3*s*)-3-(((benzyloxy)carbonyl)(methyl)amino)cyclobutyl) ethanethioate (1 g, 3.4 mmol) in

acetonitrile (5 ml) was slowly added dropwise to a stirred reaction solution of *N*-chlorosuccinimide (983 mg, 7.15 mmol), acetic acid (0.12 ml), and water (0.12 ml) in acetonitrile (5 ml) at room temperature. The adding was kept constant and slow so that the temperature of the reaction solution did not exceed 30°C. The resulting mixture was stirred at room temperature for 15 minutes. The completion of the reaction was monitored by TLC. The reaction mixture was diluted by adding ethyl acetate (10 ml), washed with saturated sodium bicarbonate solution (20 ml), washed with saturated sodium chloride solution (20 ml), dried over anhydrous sodium sulfate, and concentrated under reduced pressure to obtain benzyl((1s,3s)-3-(chlorosulfonyl)cyclobutyl)(methyl)carbamate (622 mg, crude product) as yellow oil, which was used directly in the next step without purification.

Step 6 : synthesis of fragmet **68-g** benzyl ((1*s*,3*s*)-3-(azetidin-1-ylsulfonyl)cyclobutyl)(methyl)carbamate

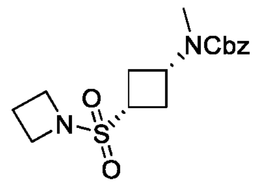

**68-g**

A solution of crude product benzyl((1*s*,3*s*)-3-(chlorosulfonyl)cyclobutyl)(methyl)carbamate (622 mg, 1.96 mmol), azetidine (112 mg, 1.96 mmol), *N,N*-diisopropylethylamine (505 mg, 3.91 mmol) in dichloromethane (3 ml) was stirred at room temperature for 16 hours. The completion of the reaction was monitored by TLC. The reaction mixture was diluted by adding dichloromethane (15 ml), washed with water (10 ml×2) , washed with saturated sodium chloride solution (20 ml), dried over anhydrous sodium sulfate, and concentrated under reduced pressure. The obtained crude product was separated and purified by silica gel column chromatography (eluted with 1%-1.5% methanol in dichloromethane) to obtain benzyl ((1*s*,3*s*)-3-(azetidin-1-ylsulfonyl)cyclobutyl)(methyl)carbamate (120 mg, 10% for two-steps) as colorless oil.

$^1$H NMR (400 MHz, $CDCl_3$): δ 7.32-7.37 (m, 5H), 5.12 (s, 2H), 4.69-4.81 (m, 1H), 3.96 (t, *J* = 7.6 Hz, 4H), 3.33-3.39 (m, 1H), 2.94 (s, 1H), 2.57-2.65 (m, 2H), 2.42-2.53 (m, 2H), 2.22-2.30 (m, 2H).

Step 7 : synthesis of fragment **68-h** (1s,3s)-3-(azetidin-1-ylsulfonyl)-*N*-methylcyclobutan-1-amine

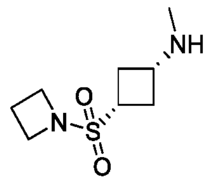

**68-h**

A solution of benzyl ((1*s*,3*s*)-3-(azetidin-1-ylsulfonyl)cyclobutyl)(methyl)carbamate (90 mg, 0.26 mmol) and palladium/carbon (100%, 90 mg) in methanol (4 ml) was stirred under hydrogen atmosphere (hydrogen balloon) at room temperature for 24 hours. The completion of the reaction was monitored by TLC. The palladium/carbon was removed by filtration and methanol washing was performed (5 ml×2). The filtrates were combined and concentrated under reduced pressure to obtain (1*s*,3*s*)-3-(azetidin-1-ylsulfonyl)-N-methylcyclobutan-1-amine as brown oil (55 mg, crude product), which was used directly in the next step without purification.

**Example 69**

**4-methyl-*N*-(4-(1-(1-(methylsulfonyl)azetidin-3-yl)-1*H*-pyrazol-4-yl)-7*H*-pyrrolo [2,3-*d*]pyrimidin-2-yl)-3,4,5,6-tetrahydro-2*H*-benzo[*b*][1,4,7]dioxazolin-9-amine**

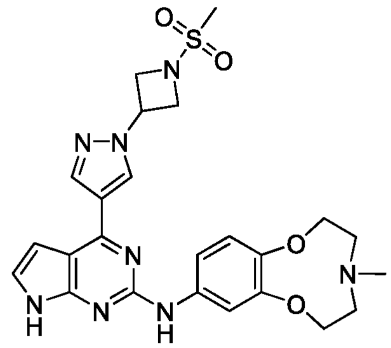

**69**

**Implementation method:**

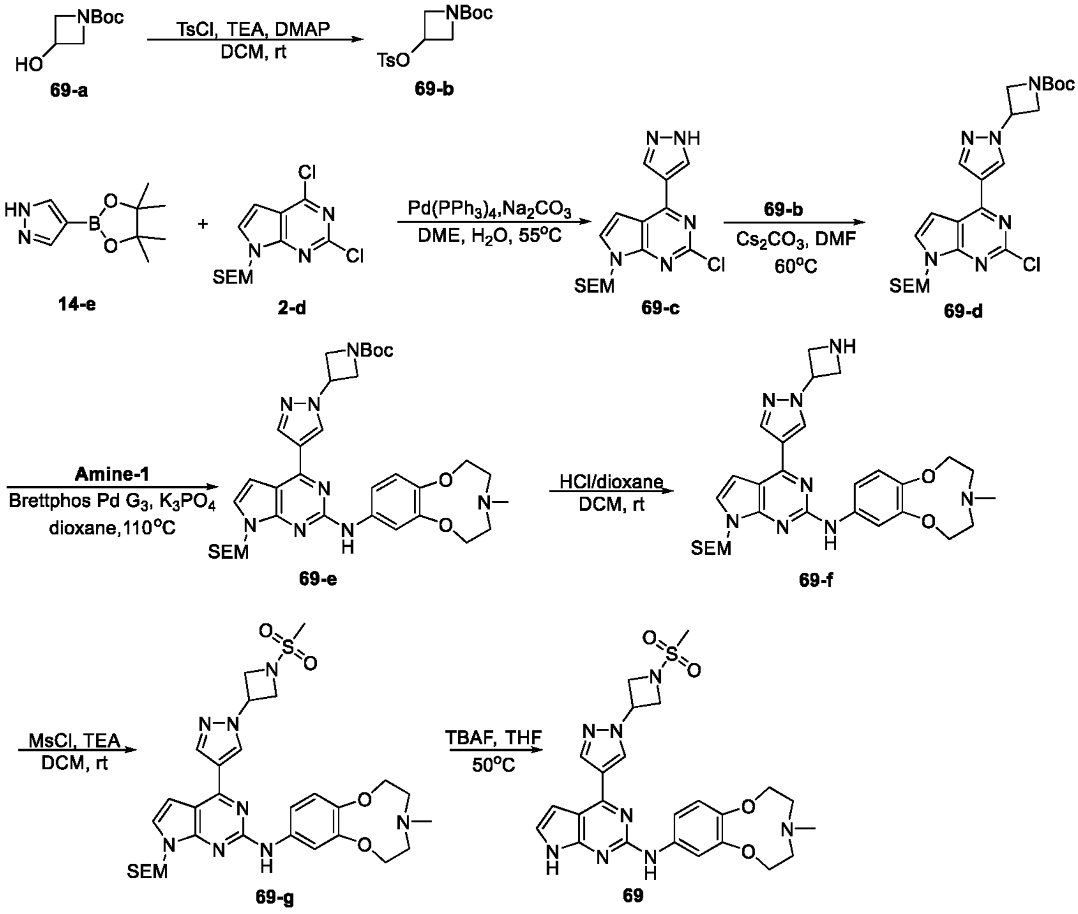


Step 1: synthesis of fragment **69-b** tert-butyl 3-(toluenesulfonyloxy)azetidin-1-carboxylate

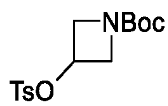

**69-b**

To a solution of tert-butyl 3-hydroxyazetidin-1-carboxylate (500 mg, 2.9 mmol), triethylamine (862.4 mg, 5.78 mmol), and 4-dimethylaminopyridine (36.7 mg, 0.3 mmol) in dichloromethane (5 ml) was added 4-toluenesulfonyl chloride (606.4 mg, 3.2 mmol) at 0°C, and the reaction mixture was stirred at room temperature for 12 hours. The completion of the reaction was monitored by TLC. The reaction mixture was partitioned in dichloromethane (20 ml) and water (20 ml). The organic layer was collected, washed with saturated sodium chloride solution (20 ml), dried over anhydrous sodium sulfate, and concentrated under reduced pressure. The obtained crude product was separated and purified by silica gel column chromatography (eluted with 30% ethyl acetate in n-hexane) to obtain tert-butyl 3-(toluenesulfonyloxy)azetidin-1-carboxylate as colorless oil (700 mg, 74%).

$^1$H NMR (400 MHz, $CDCl_3$): δ 7.78-7.80 (m, 2H), 7.37 (d, *J* = 8.0 Hz, 2H), 4.98-5.03 (m, 1H), 4.08-4.11 (m, 2H), 3.87-3.97 (m, 2H), 2.47 (s, 3H), 1.41 (s, 9H).

Step 2 : synthesis of fragment 69-c 2-chloro-4-(1H-pyrazol-4-yl)-7-((2-(trimethylsilyl)ethoxy)methyl)-*7H*-pyrrolo[2,3-d] pyrimidine

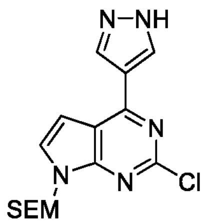

**69-c**

To a solution containing 2,4-dichloro-7-(((2-(trimethylsilyl)ethoxy)methyl)-7*H*-pyrrolo[2,3-d]pyrimidine (500 mg, 1.57 mmol), 4-(4,4,5,5-tetramethyl-1,3,2-dioxaborolan-2-yl)-1*H*-pyrazole (304.6 mg, 1.57 mmol), sodium carbonate (2 N, 1 ml) solution and acetonitrile (5 ml) was added tetrakis(triphenylphosphine)palladium (184.8 mg, 0.16 mmol) under nitrogen atmosphere at room temperature. The reaction mixture was replaced with nitrogen for three times and stirred at 55°C for 12 hours. The completion of the reaction was monitored by TLC. The reaction mixture was partitioned in ethyl acetate (20 ml) and water (20 ml). The organic layer was collected, washed with saturated sodium chloride solution (20 ml), dried over anhydrous sodium sulfate, and concentrated under reduced pressure. The obtained crude product was separated and purified by silica gel column chromatography (eluted with 50% ethyl acetate in n-hexane) to obtain 2-chloro-4-(1*H*-pyrazol-4-yl)-7-((2-(trimethylsilyl)ethoxy)methyl)-7*H*-pyrrolo[2,3-*d*] pyrimidine as a gray-white solid (200 mg, 36%).

LC_MS: (ES$^+$): m/z 350.4 [M+H]$^+$.

Step 3 : synthesis of fragment **69-d** tert-butyl 3-(4-(2-chloro-7-((2-(trimethylsilyl)ethoxy)methyl)-7*H*-pyrrolo[2,3-*d*]pyrimidin-4-yl)-1*H*-pyrazol-1-yl)azetidin-1-carboxylic acid

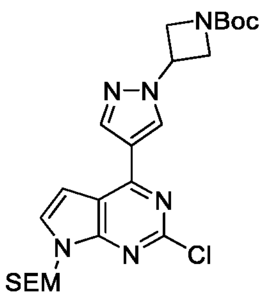

**69-d**

A solution of 2-chloro-4-(1*H*-pyrazol-4-yl)-7-((2-(trimethylsilyl)ethoxy)methyl)-7*H*-pyrrolo[2,3-*d*]pyrimidine (200 mg, 0.57 mmol), tert-butyl 3-(tosyloxy)azetidin-1-carboxylate (282 mg, 0.86 mmol), and cesium carbonate (371 mg, 1.14 mmol) in N,N-dimethylformamide (2 ml) was stirred at 60°C for 13 hours under nitrogen atmosphere. The completion of the reaction was monitored by TLC. The reaction mixture was cooled to room temperature, partitioned in water (20 ml), and extracted with ethyl acetate (20 ml×2). The organic layer was collected, washed with saturated sodium chloride solution

(20 ml), dried over anhydrous sodium sulfate, and concentrated under reduced pressure. The obtained crude product was separated and purified by silica gel column chromatography (eluted with 2% methanol in dichloromethane) to obtain tert-butyl 3-(4-(2-chloro-7-((2-(trimethylsilyl)ethoxy)methyl)-7*H*-pyrrolo[2,3-*d*]pyrimidin-4-yl)-1*H*-pyrazol-1-yl)azetidine-1-carboxylic acid as a white solid (200 mg, 65%).

Step 4 : Synthesis of fragment **69-e** tert-butyl 3-(4-(2-((4-methyl-3,4,5,6-tetrahydro-2*H*-benzo[b][1,4,7]dioxazolin-9-yl) amino)-7-(((2-(trimethy lsilyl)ethoxy)methyl)-7*H*-pyrrolo[2,3-*d*] pyrimidin-4-yl)-1*H*-pyrazol-1-yl)azetidin-1-carboxylic acid

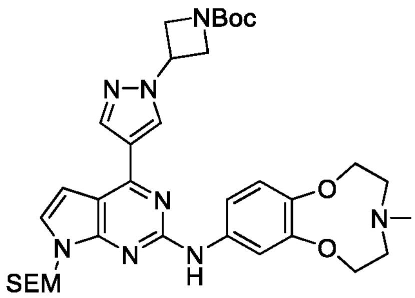

**69-e**

To a solution of 3-(4-(2-chloro-7-((2-(trimethylsilyl)ethoxy)methyl)-7*H*-pyrrolo[2,3-d]pyrimidin-4-yl)-1*H*-pyrazol-1-yl)azetidin-1-carboxylic acid (100 mg, 0.2 mmol), 4-methyl-3,4,5,6-tetrahydro-2*H*-benzo[*b*][1,4,7]dioxazolin-9-amine (42 mg, 0.2 mmol), potassium phosphate (127 mg, 0.6 mmol) in 1,4-dioxane (1 ml) was added BrettPhos Pd G3 (18 mg, 0.02 mmol) under nitrogen atmosphere at room temperature. The reaction mixture was replaced with nitrogen for three times and stirred at 110°C for 13 hours. The completion of the reaction was monitored by TLC. The reaction mixture was cooled to room temperature, partitioned in water (20 ml), and extracted with ethyl acetate (10 ml×2). The organic layer was collected, washed with saturated sodium chloride solution (20 ml), dried over anhydrous sodium sulfate, and concentrated under reduced pressure. The obtained crude product was separated and purified by preparative TLC (eluted with 4% methanol and 38% ethyl acetate in n-hexane) to obtain tert-butyl 3-(4-(2-((4-methyl-3,4,5,6-tetrahydro-2*H*-benzo[b][1,4,7]dioxazolin-9-yl)amino)-7-(((2-(trimethy lsilyl)ethoxy)methyl)-7*H*-pyrrolo[2,3-*d*]pyrimidin-4-yl)-1*H*-pyrazol-1-yl)azetidin-1-carboxylic acid as a yellow solid (100 mg, 74%).

LC_MS: (ES$^+$): m/z 677.7 [M+H]$^+$.

Step 5 : synthesis of fragment **69-f** N-(4-(1-(azetidin-3-yl)-1*H*-pyrazol-4-yl)-7-((2-(trimethylsilyl)ethoxy)methyl)-7*H*-pyrrolo[2,3-d] pyrimidin-2-yl)-4-methyl-3,4,5,6-tetrahydro-2H-benzo[b][1,4,7]dioxazolin-9-amine

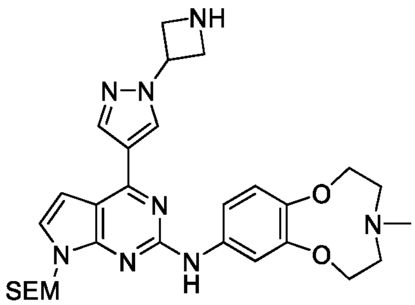

**69-f**

To a solution of tert-butyl 3-(4-(2-((4-methyl-3,4,5,6-tetrahydro-2*H* -benzo[b][1,4,7]dioxazolin-9-yl)amino)-7-(((2-(trimethylsilyl)ethoxy)methyl)-7*H*-pyrrolo[2,3-*d*] pyrimidin-4-yl)-1*H*-pyrazol-1-yl)azetidin-1-carboxylic acid (100 mg, 0.15 mmol) in dichloromethane (1 ml) was added hydrochloric acid/dioxane (4 M, 1 ml) solution at room temperature, and the reaction solution was stirred at room temperature for 1 hour. The completion of the reaction was monitored by TLC. The volatiles were removed under reduced pressure, and the residue was alkalized to pH=7-8 with

sodium carbonate solution and extracted with dichloromethane (10 ml×2). The organic layer was collected and washed with saturated sodium chloride solution (20 ml), dried over anhydrous sodium sulfate, and concentrated under reduced pressure. The obtained crude product was separated and purified by preparative TLC (eluted with 10% methanol in dichloromethane) to obtain *N*-(4-(1-(azetidin-3-yl)-1*H*-pyrazol-4-yl)-7-((2-(trimethylsilyl)ethoxy)methyl)-7*H*-pyrrolo [2,3-*d*] pyrimidin-2-yl)-4-methyl-3,4,5,6-tetrahydro-2*H*-benzo[*b*][1,4,7]dioxazolin-9-amine as a yellow solid (62 mg, 70%).
LC_MS: (ES$^+$): m/z 577.6 [M+H]$^+$.

Step 6 : synthesis of fragment **69-g** 4-methyl-*N*-(4-(1-(1-(1-(methylsulfonyl)azetidin-3-yl)-1*H*-pyrazol-4-yl)-7-(((2-(trimethylsilyl) ethoxy)methyl)-7*H*-pyrrolo[2,3-*d*]pyrimidin-2-yl)-3,4,5,6-tetrahydro-2*H*-benzo[*b*][1,4,7] dioxazolin-9-amine

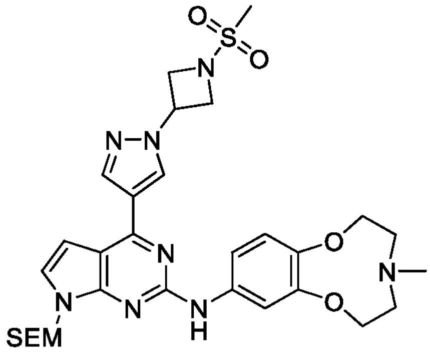

**69-g**

To a solution of *N*-(4-(1-(azetidin-3-yl)-1*H*-pyrazol-4-yl)-7-((2-(trimethylsilyl)ethoxy)methyl)-7*H*-pyrrolo[2,3-*d*] pyrimidin-2-yl)-4-methyl-3,4,5,6-tetrahydro-2*H*-benzo[*b*][1,4,7]dioxazolin-9-amine (62 mg, 0.11 mmol) and triethylamine (16.2 mg, 0.16 mmol) in dichloromethane (2 ml) was added methanesulfonyl chloride (12.6 mg, 0.11 mmol) at 0°C, and the resulting mixture was heated to room temperature and stirred for 3 hours. The completion of the reaction was monitored by TLC. The reaction mixture was partitioned in dichloromethane (10 ml) and water (10 ml). The organic layer was collected, washed with saturated sodium chloride solution (10 ml), dried over anhydrous sodium sulfate, and concentrated under reduced pressure. The obtained crude product was separated and purified by preparative TLC (eluted with 5% methanol in dichloromethane) to obtain 4-methyl-*N*-(4-(1-(1-(1-(methylsulfonyl)azetidin-3-yl)-1*H*-pyrazol-4-yl)-7-(((2-(trimethylsilyl) ethoxy)methyl)-7*H*-pyrrolo[2,3-*d*]pyrimidin-2-yl)-3,4,5,6-tetrahydro-2*H*-benzo[*b*][1,4,7] dioxazolin-9-amine as a yellow solid (60 mg, 80%).
LC_MS: (ES$^+$): m/z 655.3 [M+H]$^+$.
$^1$H NMR (400 MHz, DMSO-d6): δ 8.96 (s, 1H), 8.69 (s, 1H), 8.35 (s, 1H), 7.41 (d, *J*= 2.0 Hz, 1H), 7.33-7.34 (m, 1H), 7.28-7.30 (m, 1H), 6.87 (d, *J* = 3.6 Hz, 1H), 6.65 (d, *J* = 8.8 Hz, 1H), 5.50 (s, 2H), 5.39-5.46 (m, 1H), 4.32-4.39 (m, 4H), 4.13-4.14 (m, 2H), 3.51-3.57 (m, 4H), 3.27-3.38 (m, 4H), 3.27 (s, 3H), 3.16 (s, 3H), 0.84-0.89 (m, 2H), -0.11 (s, 9H).

Step 7 : synthesis of 4-methyl-*N*-(4-(1-(1-(methylsulfonyl)azetidin-3-yl)-1*H*-pyrazol-4-yl)-7H-pyrrolo[2,3-*d*] pyrimidin-2-yl)-3,4,5,6-tetrahydro-2H-benzo[b][1,4,7]dioxazolin-9-amine

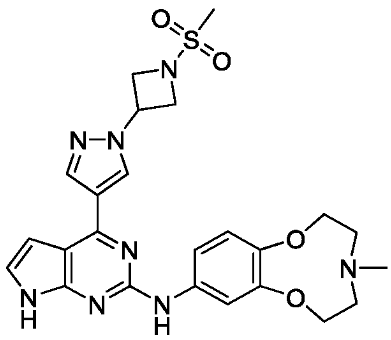

**69**

A solution of *N*-(4-(1-(azetidin-3-yl)-1*H*-pyrazol-4-yl)-7*H*-pyrrolo[2,3-*d*]pyrimidin-2-yl)-4-methyl-3,4,5,6-tetrahydro-2*H*-benzo[*b*][1,4,7]dioxazolin-9-amine (60 mg, 0.13 mmol) and tetrabutylammonium fluoride (0.5 ml, 1 M tetrahydrofuran solution) in tetrahydrofuran (0.5 ml) was stirred at 50°C for 48 hours. The completion of the reaction was monitored by TLC. The reaction mixture was cooled to room temperature and partitioned in water (10 ml) and ethyl acetate (10 ml). The organic layer was collected and the aqueous layer was extracted with ethyl acetate (10 ml×2). The organic layers were combined, washed with saturated sodium chloride solution (10 ml), dried over anhydrous sodium sulfate, and concentrated under reduced pressure. The obtained crude product was separated and purified by preparative TLC (eluted with 10% methanol and 1% ammonia in dichloromethane) to obtain 4-methyl-*N*-(4-(1-(1-(methylsulfonyl)azetidin-3-yl)-1*H*-pyrazol-4-yl)-7*H*-pyrrolo[2,3-*d*] pyrimidin-2-yl)-3,4,5,6-tetrahydro-2*H*-benzo[*b*][1,4,7]dioxazolin-9-amine (10 mg, 14%).

LC_MS: ($ES^+$): m/z 525.2 $[M+H]^+$.

$^1H$ NMR (400 MHz, $CDCl_3$): δ 8.83 (s, 1H), 8.28-7.32 (m, 2H), 6.92-7.02 (m, 2H), 6.59-6.66 (m, 2H), 5.19-5.22 (m, 1H), 4.43-4.51 (m, 4H), 4.24 (s, 2H), 3.60 (t, *J* = 5.6 Hz, 2H), 3.41-3.53 (m, 4H), 3.37 (s, 3H), 3.07 (s, 3H).

## Example 70

**1-(3-(4-(2-((4-methyl-3,4,5,6-tetrahydro-2*H*-benzo[*b*][1,4,7]dioxazolin-9-yl)amino)-7*H*-pyrrolo[2,3-*d*]pyrimidin-4-yl)-1*H*-pyrazol-1-yl)azetidin-1-yl)ethan-1-one**

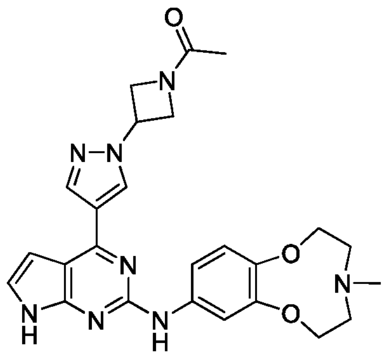

**70**

**Implementation method**

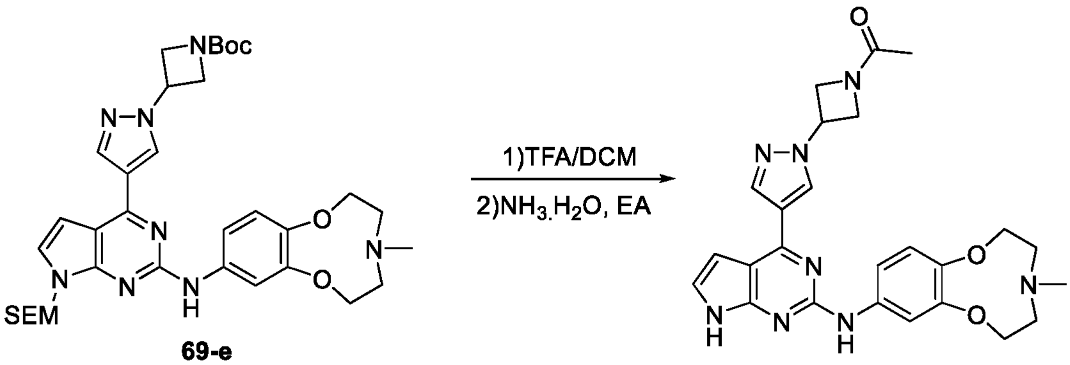


69-e

70

Step 1 : synthesis of 1-(3-(4-(2-((4-methyl-3,4,5,6-tetrahydro-2H-benzo[b][1,4,7] dioxazolin-9-yl)amino)-7*H*-pyrrolo [2,3-*d*]pyrimidin-4-yl)-11*H*-pyrazol-1-yl)azetidin-1-yl)ethanon e

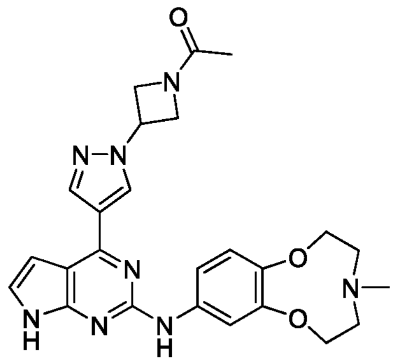

**70**

A solution of tert-butyl 3-(4-(2-((4-methyl-3,4,5,6-tetrahydro-2*H*-benzo[b][1,4,7] dioxazolin-9-yl)amino)-7-(((2-(trimethylsilyl)ethoxy)methyl)-7*H*-pyrrolo[2,3-d]pyrimidin-4-yl)-1 *H*-pyrazol-1-yl)azetidin-1-carboxylic acid (56 mg, 0.08 mmol) and trifluoroacetic acid (1 ml) in dichloromethane (2 ml) was stirred at room temperature for 4 hours. The completion of the reaction was monitored by TLC. The reaction mixture was concentrated under reduced pressure, and the residue was added to a solution of aqueous ammonia (3 ml) and ethyl acetate (2 ml), and the resulting mixture was stirred at 30°C for 16 hours. The completion of the reaction was monitored by TLC. The organic layer was collected and washed with saturated sodium chloride solution (10 ml), dried over anhydrous sodium sulfate, and concentrated under reduced pressure. The obtained crude product was separated and purified by preparative TLC (eluted with 10% methanol in dichloromethane) to obtain 1-(3-(4-(2-((4-methyl-3,4,5,6-tetrahydro-2*H*-benzo[*b*][1,4,7]dioxazolin-9-yl)amino)-7*H*-pyrrolo[ 2,3-*d*]pyrimidin-4-yl)-1*H*-pyrazol-1-yl)azetidin-1-yl)ethanone as a yellow solid (11 mg, 27%).

LC_MS: (ES$^+$): m/z 489.3 $[M+H]^+$.

$^1H$ NMR (400 MHz, $CDCl_3$): δ 8.81 (brs, 1H), 8.28 (s,2H), 7.28 (s, 1H), 6.93-7.02 (m, 2H), 6.57-6.67 (m, 2H), 5.17-5.23 (m, 1H), 4.59-4.67 (m, 2H), 4.45-4.56 (m, 2H), 4.24 (s, 2H), 3.58-3.61 (m, 2H), 3.37-3.44 (m, 6H), 1.97 (s, 3H).

**Example 71**

**(1*s*,3*s*)-3-amino-*N*-(3-((5-chloro-2-((4-methyl-3,4,5,6-tetrahydro-2*H*-benzo[*b*][1,4,7] dioxazolin-9-yl)amino)pyrimidin-4-yl)amino)phenyl)cyclobutan-1-carboxamide**

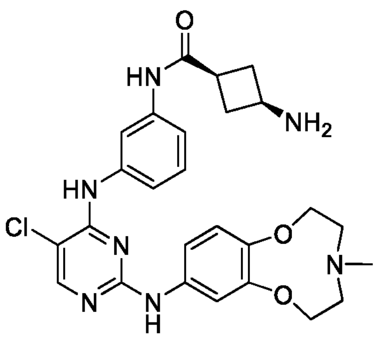

**71**

**Implementation method**

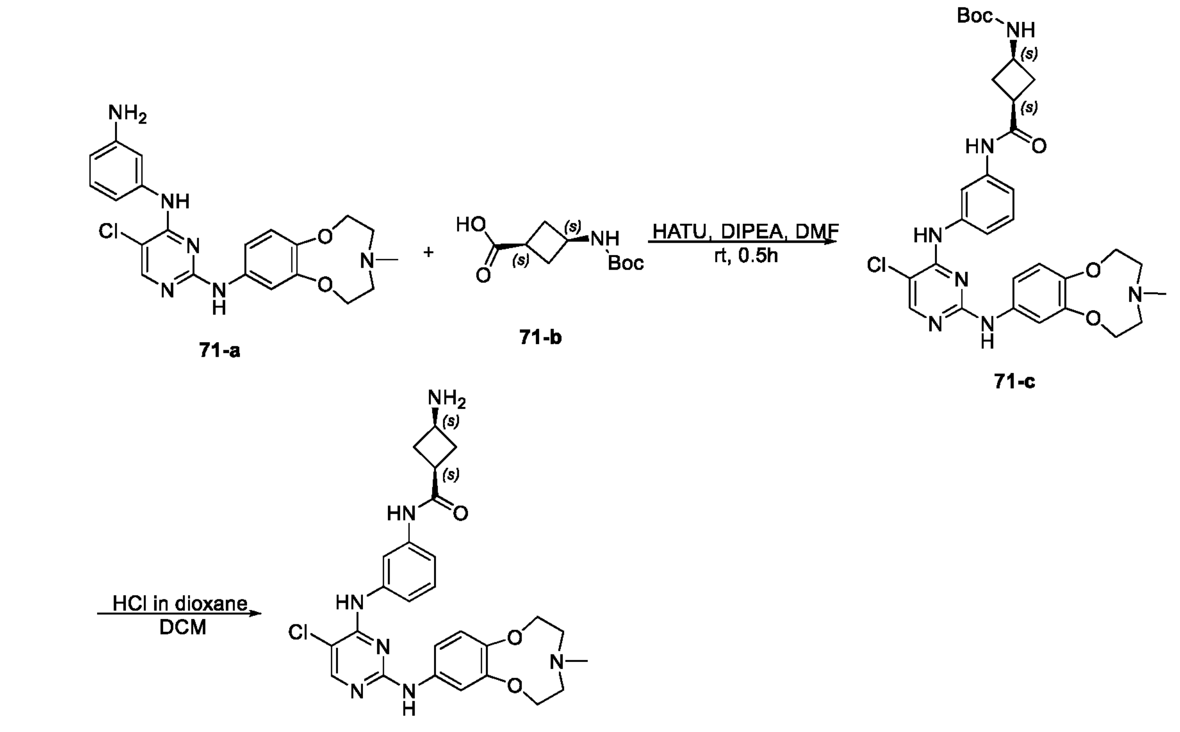


71-a 71-b 71-c

71

Step 1 : synthesis of fragment **71-c** tert-butyl ((1s,3s)-3-((3-((5-chloro-2-((4-methyl -3,4,5,6-tetrahydro-2*H*-benzo[*b*][1,4,7]dioxazolin-9-yl)amino)pyrimidin-4-yl)amino)phenyl) carbamoyl)cyclobutyl)carbamate

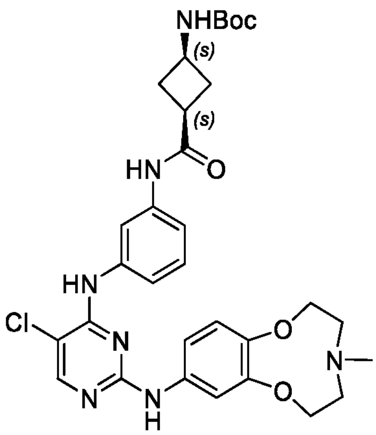

**71-c**

To a solution of $N^4$-(3-aminophenyl)-5-chloro-$N^2$-(4-methyl-3,4,5,6-tetrahydro-2*H*-benzo[*b*][1,4,7]dioxazolin-9-yl )pyrimidin-2,4-diamine(30 mg, 0.07 mmol), (1*s*,3*s*)-3-((tert-butyloxycarbonyl)amino) cyclobutanecarboxylic acid (15 mg, 0.07 mmol), *N,N*-diisopropylethylamine (36 mg, 0.28 mmol) in *N,N*-dimethylformamide (1 ml) was added 2-(7-azobenzotriazole)-*N,N,N',N'*-tetramethyluronium hexafluorophosphate (53 mg, 0.14 mmol) at 0°C; and the resulting mixture was stirred at room temperature for 30 minutes. The completion of the reaction was monitored by TLC. The reaction mixture was partitioned in water (10 ml) and ethyl acetate (10 ml). The organic layer was collected, washed with saturated sodium chloride solution (20 ml×2), dried over anhydrous sodium sulfate, and concentrated under reduced pressure. The obtained crude product was separated and purified by preparative TLC (eluted with 5% methanol in dichloromethane) to obtain tert-butyl ((1*s*,3*s*)-3-((3-((5-chloro-2-((4-methyl-3,4,5,6-tetrahydro-2*H*-benzo[*b*][1,4,7]dioxazolin-9-yl) amino)pyrimidin-4-yl)amino)phenyl)carbamoyl)cyclobutyl)carbamate as a white solid (35 mg, 79%) .

LC_MS: ($ES^+$): m/z 624.4 $[M+H]^+$.

Step 2 : synthesis of (1*s*,3*s*)-3-amino-N-(3-((5-chloro-2-((4-methyl-3,4,5,6-tetrahydro-2H-benzo[b][1,4,7]dioxazolin-9-yl)amino)pyrimidin-4-yl)amino)phenyl)cyclobutanecarboxamide

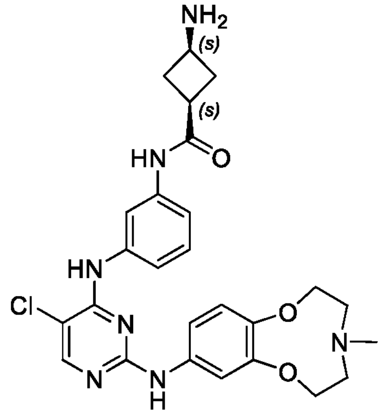

**71**

A solution of tert-butyl ((1*s*,3*s*)-3-((3-((5-chloro-2-((4-methyl-3,4,5,6-tetrahydro-2*H*-benzo[b][1,4,7]dioxazolin-9-yl) amino)pyrimidin-4-yl)amino)phenyl)carbamoyl)cyclobutyl)carbamate (35 mg, 0.056 mmol) in 4 M hydrochloric acid/dioxane (1 ml) and dichloromethane (1 ml) was stirred at room temperature for 3 hours. The completion of the reaction was monitored by TLC. The mixture was concentrated under reduced pressure, and the residue was adjusted to pH=8-9 with sodium carbonate solution , and then extracted with dichloromethane (10 ml×2). The organic layers were combined, washed with saturated sodium chloride solution (20 ml), dried over anhydrous sodium sulfate, and concentrated under reduced pressure. The obtained crude product was separated and purified by preparative TLC (eluted with 10% methanol and 1% ammonia in dichloromethane) to obtain (1*s*,3*s*)-3-amino-*N*-(3-((5-chloro-2-((4-methyl-3,4,5,6-tetrahydro-2*H*-benzo[*b*][1,4,7] dioxazolin-9-yl)amino)pyrimidin-4-yl)amino)phenyl)cyclobutanecarboxamide as a gray solid (20.4 mg, 69%).

LC_MS: ($ES^+$): m/z 524.2 $[M+H]^+$.

$^1H$ NMR (400 MHz, DMSO-d6): δ 9.90 (s, 1H), 8.94 (s, 1H), 8.71 (s, 1H), 8.06 (s, 1H), 7.86 (s, 1H), 7.34 (d, *J* = 8.0 Hz, 1H), 7.34 (d, *J* = 7.2 Hz, 1H), 7.21-7.25 (m, 1H), 7.00-7.03 (m, 2H), 6.49 (d, *J* = 8.4 Hz, 1H), 5.10(s, 2H), 4.08 (t, *J* = 4.0 Hz, 2H), 3.49 (t, *J* = 5.6 Hz, 2H), 3.34-3.41 (m, 4H), 3.27-3.29 (m, 1H), 3.26 (s, 3H), 2.82-2.86 (m, 1H), 2.32-2.39 (m, 2H), 1.98-2.06 (m, 2H).

**Example 72**

**(1*r*,3*r*)-3-amino-*N*-(3-((5-chloro-2-((4-methyl-3,4,5,6-tetrahydro-2*H*-benzo[*b*][1,4,7] dioxazolin-9-yl)amino)pyrimidin-4-yl)amino)phenyl)cyclobutan-1-carboxamide**

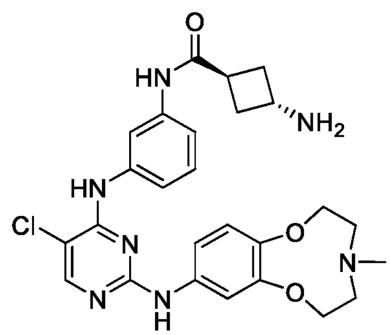

**72**

The implementation method was the same as Example 71, except that (1*r*,3*r*)-3-((tert-butyloxycarbonyl)amino) cyclobutanecarboxylic acid was used to replace fragment 71-b to obtain the title compound.

LC_MS: (ES+): m/z 524.7 [M+H]+.

1H NMR (400 MHz, DMSO-d6): δ 9.90 (s, 1H), 8.94 (s, 1H), 8.71 (s, 1H), 8.06 (s, 1H), 7.86 (s, 1H), 7.34 (d, *J* = 8.0 Hz, 1H), 7.34 (d, *J* = 7.2 Hz, 1H), 7.21-7.25 (m, 1H), 7.00-7.03 (m, 2H), 6.49 (d, *J* = 8.4 Hz, 1H), 5.10(s, 2H), 4.08 (t, *J* = 4.0 Hz, 2H), 3.49 (t, *J* = 5.6 Hz, 2H), 3.34-3.41 (m, 4H), 3.27-3.29 (m, 1H), 3.26 (s, 3H), 2.82-2.86 (m, 1H), 2.32-2.39 (m, 2H), 1.98-2.06 (m, 2H).

**Example 73**

**dimethyl(2-((2-((4-methyl-3,4,5,6-tetrahydro-2*H*-benzo[*b*][1,4,7]dioxazolin-9-yl) amino)-7*H*-pyrrolo[2,3-d]pyrimidin-4-yl)amino)phenyl)phosphine oxide**

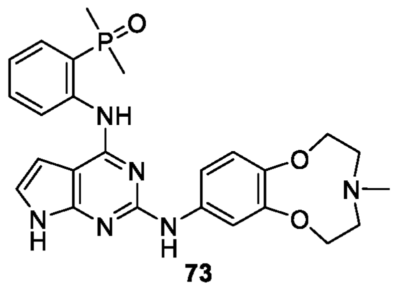

**73**

The implementation method was the same as method 4, except that fragment (2-aminophenyl)dimethylphosphine oxide was used to replace fragment 1-b, and fragment 2-d was used to replace fragment 4-a to obtain the title compound.

LC_MS: (ES+): m/z 493.4 [M+H]+.

1H NMR (400 MHz, DMSO-*d*6): δ 11.59 (s, 1H), 11.20 (s, 1H), 9.14-9.11 (m, 1H), 8.57 (s, 1H), 7.57-7.46 (m, 1H), 7.32 (s, 1H), 7.07-7.05 (m, 2H), 6.88 (s, 1H), 6.65-6.64 (m, 1H), 6.34 (s, 1H), 4.15 (s, 2H), 3.55-3.53 (m, 2H), 3.38-3.32 (m, 4H), 3.28 (s, 3H), 1.83 (d, *J* = 13.6 Hz, 6H).

**Example 74**

**(*R*)-3-cyclopentyl-3-(4-(2-((7-methyl-2,3,6,7,8,9-hexahydro-5*H*-benzo[*b*][1,4,7] trioxa[10]azacyclododecan-13-yl)amino)-7*H*-pyrrolo[2,3-*d*]pyrimidin-4-yl)-1*H*-pyrazol-1-yl) propionitrile**

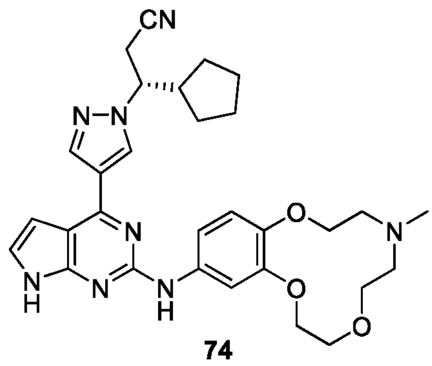

**74**

The implementation method was the same as method 2, except that fragment 74-a (R)-3-cyclopentyl-3-(4-(4,4,5,5-tetramethyl-1,3,2-dioxaborolan-2-yl)-1*H*-pyrazol-1-yl)propionitri le (Shanghai Bid Pharmaceutical Technology Co., Ltd.) was used to replace fragment 2-b, and fragment Amine-2 was used to replace fragment Amine-1 to obtain the title compound.

LC_MS: (ES+): m/z 557.7 [M+H]+.

1H NMR (400 MHz, $CDCl_3$): δ 9.31(s, 1H), 8.27(s, 2H), 7.49(s, 1H), 7.11-7.07(m, 2H), 6.95-6.92 (m, 2H), 4.27-4.25 (m, 1H), 4.19-4.14 (m, 4H), 3.80-3.75 (m, 4H), 3.16-3.10 (m, 1H), 2.97-2.92 (m, 1H), 2.82 (d, *J* = 8.0 Hz, 4H), 2.58

(m, 1H), 2.42 (s, 3H), 1.97 (m, 2H), 1.72 (m, 2H), 1.56 (m, 2H), 1.26 (m, 2H).

Example 75

**(*S*)-3-cyclopentyl-3-(4-(2-((7-methyl-2,3,6,7,8,9-hexahydro-5*H*-benzo[*b*][1,4,7] trioxa[10]azacyclododecan-13-yl) amino)-7*H*-pyrrolo[2,3-d]pyrimidin-4-yl)-1*H*-pyrazol-1-yl) propionitrile**

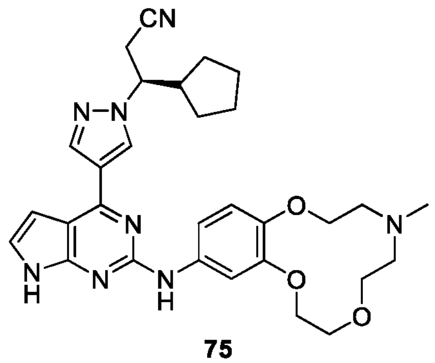

**75**

The implementation method was the same as method 2, except that fragment 75-a (*S*)-3-cyclopentyl-3-(4-(4,4,5,5-tetramethyl-1,3,2-dioxaborolan-2-yl)-1*H*-pyrazol-1-yl)propionitri le (Shanghai Bid Pharmaceutical Technology Co., Ltd.) was used to replace fragment 2-b, and fragment 1-a was used to replace fragment 2-d to obtain the title compound.

LC_MS: ($ES^+$): m/z 557.7 $[M+H]^+$.

$^1H$ NMR (400 MHz, $CDCl_3$): δ 9.06 (s, 1H), 8.27 (d, *J* = 1.8 Hz, 2H), 7.49 (s, 1H), 7.12-7.09 (m, 1H), 7.02 (s, 1H), 6.96-6.94 (m, 2H), 6.61 (m, 1H), 4.27-4.25 (m, 1H), 4.20-4.15 (m, 4H), 3.82-3.77 (m, 4H), 3.16-3.10 (m, 1H), 2.97-2.92 (m, 1H), 2.85 (d, *J* = 16.2 Hz, 4H), 2.60-2.58 (m, 1H), 2.44 (s, 3H), 1.98-1.96 (m, 1H), 1.73-1.68 (m, 2H), 1.58-1.56 (m, 2H), 1.33-1.26 (m, 2H).

**Example 76**

**(*R*)-3-cyclopentyl-3-(4-(2-((4-methyl-3,4,5,6-tetrahydro-2*H*-benzo[*b*][1,4,7] dioxazolin-9-yl)amino)-7*H*-pyrrolo [2,3-*d*]pyrimidin-4-yl)-1*H*-pyrazol-1-yl)propanenitrile**

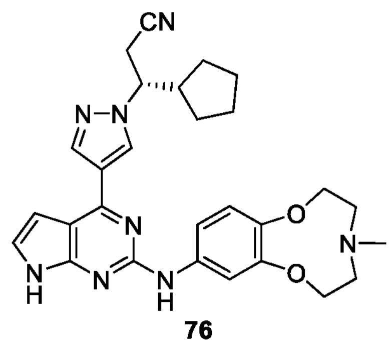

**76**

The implementation method was the same as method 2, except that fragment 74-a was used to replace fragment 2-b to obtain the title compound.

LC_MS: ($ES^+$): m/z 513.4 $[M+H]^+$.

$^1H$ NMR (400 MHz, DMSO-d6): δ 11.42 (s, 1H), 8.72 (s, 1H), 8.66 (s, 1H), 8.26 (s, 1H), 7.40 (d, *J* = 2.8 Hz, 1H), 7.20-7.15 (m, 2H), 6.75-6.74 (m, 1H), 6.65 (d, *J* = 8.8 Hz, 1H), 4.58-4.52 (m, 1H), 4.15 (d, *J* = 4.4 Hz, 2H), 3.53 (t, *J* = 5.6 Hz, 2H), 3.38 (t, *J* = 5.6 Hz, 2H), 3.29 (s, 2H), 3.27 (s, 3H), 3.22- 3.20 (m, 1H), 2.46-2.39 (m, 1H), 1.83-1.80 (m, 1H), 1.63-1.43 (m, 4H), 1.36-1.19 (m, 4H).

**Example 77**

**(*S*)-3-cyclopentyl-3-(4-(2-((4-methyl-3,4,5,6-tetrahydro-2*H*-benzo[*b*][1,4,7] dioxazolin-9-yl)amino)-7*H*-pyrrolo [2,3-*d*]pyrimidin-4-yl)-1*H*-pyrazol-1-yl)propanenitrile**

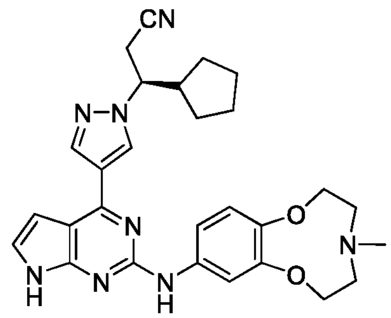

**77**

The implementation method was the same as method 2, except that fragment 75-a was used to replace fragment 2-b to obtain the title compound.

LC_MS: ($ES^+$): m/z 513.4 $[M+H]^+$.

$^1H$ NMR (400 MHz, DMSO-d6): δ 11.42 (s, 1H), 8.72 (s, 1H), 8.66 (s, 1H), 8.26 (s, 1H), 7.40-7.39 (m, 1H), 7.20-7.15 (m, 2H), 6.75-6.74 (m, 1H), 6.65 (d, *J* = 8.8 Hz, 1H), 4.57-4.52 (m, 1H), 4.15 (d, *J* = 4.0 Hz, 2H), 3.53 (t, *J* = 5.6 Hz, 2H), 3.38 (t, *J* = 5.6 Hz, 2H), 3.29 (s, 2H), 3.27 (s, 3H), 3.25-3.20 (m, 1H), 2.46-2.39 (m, 1H), 1.86-1.79 (m, 1H), 1.63-1.51 (m, 3H), 1.46-1.43 (m, 1H), 1.36-1.28 (m, 4H).

**Example 78**

**dimethyl(2-((2-((7-methyl-2,3,6,7,8,9-hexahydro-5*H*-benzo[*b*][1,4,7]trioxa[10] azacyclododecan-12-yl)amino)-5-(trifluoromethyl)pyrimidin-4-yl)amino)phenyl)phosphine oxide**

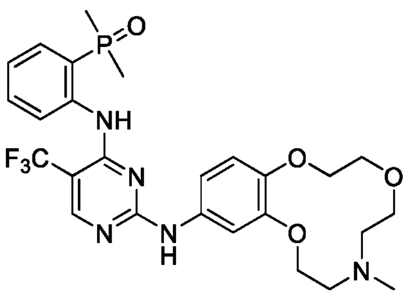

**78**

The implementation method was the same as method 7, except that fragment (2-aminophenyl)dimethylphosphine oxide was used to replace fragment 6-d to obtain the title compound.

LC_MS: ($ES^+$): m/z 566.20 $[M+H]^+$.

$^1H$ NMR (400 MHz, $CD_3OD$): δ 8.34 (s, 1H), 8.06-7.92 (m, 1H), 7.76-7.71 (m, 1H), 7.62 (t, *J* = 7.8 Hz, 1H), 7.42-7.31 (m, 2H), 7.12-7.09 (m, 1H), 6.94 (d, *J* = 8.4 Hz, 1H), 4.19-4.08 (m, 4H), 3.96-3.79 (m, 4H), 3.52-3.34 (m, 4H), 2.92 (s, 3H), 1.79 (d, *J* = 13.6 Hz, 6H).

**Example 79**

**cyclopropyl((1*R*,5*S*)-3-(2-((4-methyl-3,4,5,6-tetrahydro-2*H*-benzo[*b*][1,4,7]dioxazoli n-9-yl)amino)-7*H*-pyrrolo [2,3-*d*]pyrimidin-4-yl)-3,8-diazabicyclo[3.2.1]octan-8-yl)methanon e**

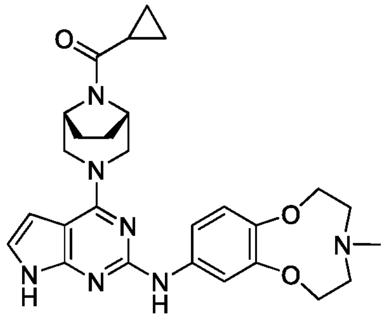

**79**

The implementation method was the same as method 4, except that fragment 79-d was used to replace fragment 1-b, and fragment 2-d was used to replace fragment 4-a to obtain the title compound.

LC_MS: ($ES^+$): m/z 504.4 $[M+H]^+$.

$^1H$ NMR (400 MHz, DMSO-d6): δ 11.31 (s, 1H), 8.32 (brs, 1H), 7.27 (s, 1H), 7.11-7.06 (m, 1H), 6.84 (s, 1H), 6.61 (brs, 1H), 6.44-6.43 (m, 1H), 4.76-4.63 (m, 2H), 4.54-4.41 (m, 2H), 4.13 (s, 2H), 3.52 (t, *J* = 5.2 Hz, 2H), 3.32 (brs, 5H), 3.26 (s, 3H), 3.21-3.16 (m, 1H), 2.04-1.96 (m, 2H), 1.80-1.77 (m, 2H), 1.71-1.69 (m, 1H), 0.79-0.72 (m, 4H).

The implementation method of fragment 79-d was as follows:

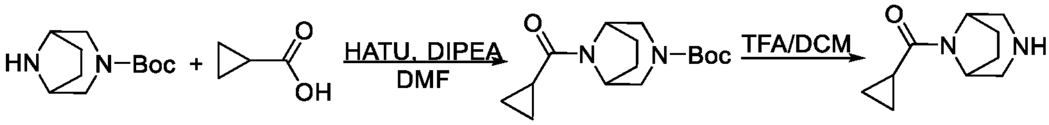


**79-a** **79-b** **79-c** **79-d**

Step 1 : synthesis of fragment **79-c** tert-butyl 8-(cyclopropanecarbonyl)-3,8-diazabicyclo[3.2.1]octan-3-carboxylate

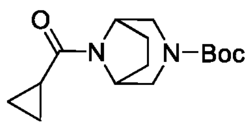

**79-c**

To a stirred reaction solution of tert-butyl 3,8-diazabicyclo[3.2.1]octan-3-carboxylate (500 mg, 2.35 mmol), cyclopropanecarboxylic acid (203 mg, 2.35 mmol), *N,N*-diisopropylethylamine (1.22 g, 9.44 mmol) in *N,N*-dimethylformamide was added 2-(7-azobenzotriazole)-*N,N,N',N'*-tetramethyluronium hexafluorophosphate (1.79 g, 4.72 mmol) at 0°C; the resulting mixture was heated to room temperature and stirred for 30 minutes. The completion of the reaction was monitored by TLC. The reaction mixture was partitioned in water (20 ml) and ethyl acetate (20 ml). The organic layer was collected, washed with saturated sodium chloride solution (10 ml×3), dried over anhydrous sodium sulfate, and concentrated under reduced pressure to obtain tert-butyl 8-(cyclopropanecarbonyl)-3,8-diazabicyclo[3.2.1]octan-3-carboxylate as yellow oil, (600 mg, crude product), which was used directly in the next step without purification.

Step 2 : synthesis of fragment **79-d** 3,8-diazabicyclo[3.2.1]octan-8-yl(cyclopropyl)methanone

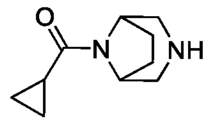

**79-d**

To a solution of tert-butyl 8-(cyclopropanecarbonyl)-3,8-diazabicyclo[3.2.1]octan-3-carboxylate (600 mg, 2.14 mmol) and trifluoroacetic acid (1.5 ml) in dichloromethane (3 ml) was stirred at room temperature for 5 hours. The completion of the reaction was monitored by TLC. The volatiles were removed under pressure, and the residue was adjusted to pH 7-8 with sodium carbonate solution, and then extracted with dichloromethane (10 ml×3). The organic layers were combined, washed with saturated sodium chloride solution (20 ml), dried over anhydrous sodium sulfate, and concentrated under reduced pressure to obtain 3,8-diazabicyclo[3.2.1]octan-8-yl(cyclopropyl)methanone as yellow oil (350 mg, crude product).

## Example 80

***N*⁴-(2-methoxy-3-(1-methyl-1*H*-1,2,4-triazol-3-yl)phenyl)-*N*2-(4-methyl-3,4,5,6-tetrahydro-2*H*-benzo[*b*][1,4,7]dioxazolin-9-yl)-7*H*-pyrrolo[2,3-*d*]pyrimidin-2,4-diamine**

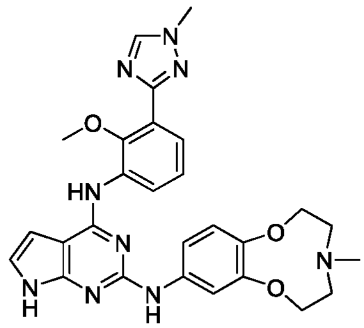

**80**

The implementation method was the same as method 4, except that fragment **80-f** 2-methoxy-3-(1-methyl-1*H*-1,2,4-triazol-3-yl)aniline was used to replace fragment 1-b, and fragment 2-d was used to replace fragment 4-a to obtain the title compound.

LC_MS: ($ES^+$): m/z 528.4 $[M+H]^+$.

$^1$H NMR (400 MHz, DMSO-d6): δ 11.14 (s, 1H), 8.54 (s, 1H), 8.34-8.38 (m, 1H), 8.26 (d, *J* = 4.0Hz, 1H), 7.55 (d, *J* = 3.8Hz, 1H), 7.27 (s, 1H), 7.19 (t, *J* = 8.0 Hz, 1H), 7.09 (s, 1H), 6.86 (s, 1H), 6.56-6.58 (m, 1H), 6.51 (s, 1H), 4.13 (s, 2H), 3.95 (s, 3H), 3.72 (s, 3H), 3.50-3.53 (m, 2H), 3.36 (s, 2H), 3.30 (s, 2H), 3.27 (s, 3H).

The implementation method of fragment **80-f** was as follows:

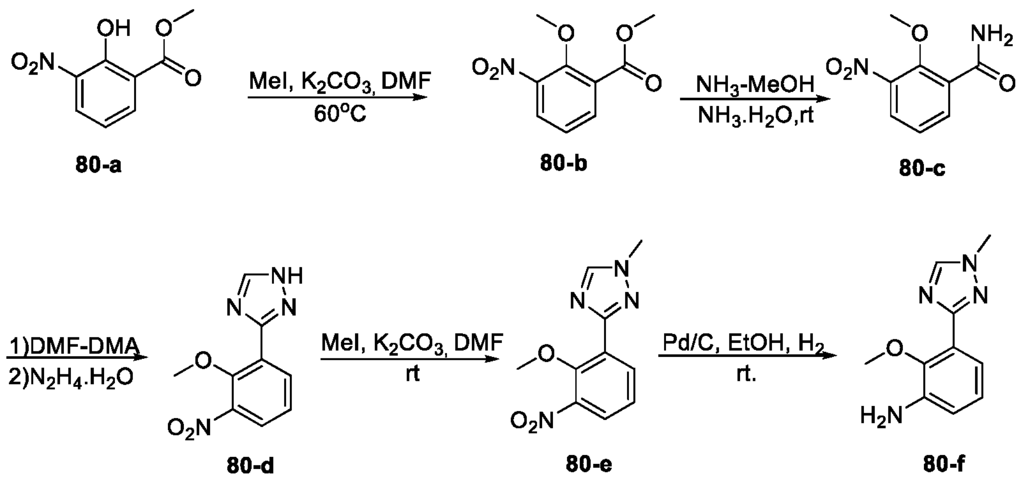

Step 1 : fragment **80-b** methyl 2-methoxy-3-nitrobenzoate

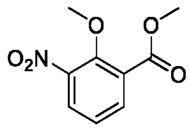

**80-b**

A solution of methyl 3-nitrosalicylate (2 g, 10.15 mmol), iodomethane (2.9 g, 20.29 mmol), and potassium carbonate (2.8 g, 20.29 mmol) in N,N-dimethylformamide (20 ml) was stirred at 60°C for 1 hour. The completion of the reaction was monitored by TLC. The reaction mixture was quenched with ice water (20 ml) and then extracted with ethyl acetate (10 ml×2). The organic layer was washed with saturated sodium chloride solution (30 ml), dried over anhydrous sodium sulfate, and concentrated under reduced pressure to obtain methyl 2-methoxy -3-nitrobenzoate (2.09 g, crude product) as a light yellow solid.

LC_MS: ($ES^+$): m/z 212.0 $[M+H]^+$.

$^1$H NMR (400 MHz, DMSO-d6): δ 8.11-8.14 (m, 1H), 8.03-8.05 (m, 1H), 7.44 (t, *J* = 4.0 Hz, 1H), 3.89 (d, *J* = 7.2 Hz, 6H).

Step 2 : Fragment **80-c** 2-methoxy-3-nitrobenzamide

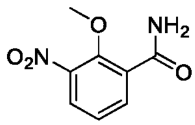

**80-c**

To a solution of methyl 2-methoxy-3-nitrobenzoate (2.09 g, 9.90 mmol) in ammonium hydroxide(20 ml) was added methylamine alcohol solution (35 ml) at 0°C; the reaction mixture was stirred at room temperature for 15 hours. The completion of the reaction was monitored by TLC. The reaction mixture was concentrated under reduced pressure to obtain 2-methoxy-3-nitrobenzamide as a yellow solid (2 g, crude product).

LC_MS: ($ES^+$): m/z 196.9 $[M+H]^+$.

$^1$H NMR (400 MHz, DMSO-d6): δ 7.95-7.98 (m, 2H), 7.76-7.78 (m, 2H), 7.37 (t, *J* = 4.0 Hz, 1H), 3.88 (s, 3H).

Step 3 : fragment **80-d** 3-(2-methoxy-3-nitrophenyl)-1H-1,2,4-triazole

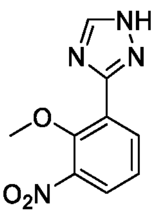

**80-d**

A solution of 2-methoxy-3-nitrobenzamide (2 g, 10 mmol) in N,N-dimethylformamide dimethyl acetal (11.9 g, 100 mmol) was stirred at 90 °C for 30 minutes of reaction. The completion of the reaction was monitored by TLC. A large amount of N,N-dimethylformamide dimethyl acetal was removed by concentration under reduced pressure, and the residue was dissolved in ethanol (10 ml). Then, a mixed solution of hydrazine hydrate (6.25 g, 100 mmol) in ethanol (50 ml) and acetic acid (12 ml) was added dropwise at 0°C. The resulting mixture was heated to room temperature and stirred for 4 hours. The completion of the reaction was monitored by TLC. The reaction mixture was concentrated and the residue was distributed in water (50 ml) and ethyl acetate (20 ml). The aqueous layer was washed with ethyl acetate (10 ml×3). The organic layers

were combined, washed with saturated sodium chloride solution (20 ml), dried over anhydrous sodium sulfate, and concentrated under reduced pressure. The obtained crude product was added to 30 ml of 2% ethyl acetate solution in petroleum ether at room temperature and stirred for 3 hours. The solid was collected by filtration and dried under vacuum to obtain 3-(2-methoxy-3-nitrophenyl)-1*H*-1,2,4- triazole as a yellow solid (1.56 g, 71%).
LC_MS: ($ES^+$): m/z 221.1 $[M+H]^+$.

Step 4 : fragment **80-e** 3-(2-methoxy-3-nitrophenyl)-1-methyl-1H-1,2,4-triazole

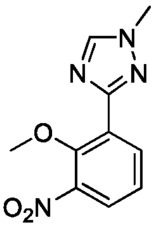

**80-e**

A solution of 3-(2-methoxy-3-nitrophenyl)-1*H*-1,2,4-triazole (1.56 g, 7.085 mmol), potassium carbonate (1.31 g, 9.49 mmol), iodomethane (1.31 g, 9.21 mmol) in N,N-dimethylformamide (10 ml) was stirred at room temperature for 3 hours. The completion of the reaction was monitored by TLC. The reaction mixture was partitioned in ethyl acetate (30 ml) and water (30 ml). The organic layer was collected, dried over anhydrous sodium sulfate, and concentrated under reduced pressure. The obtained crude product was added to ethanol (3 ml) and stirred and reacted at 45°C until the solid disappeared. The mixture was cooled to room temperature; the solid was collected by filtration, and the filter cake was washed with ethanol (2 ml) at -30°C and dried under vacuum to obtain 3-(2-methoxy-3-nitrophenyl)-1-methyl-1*H*-1,2,4-triazole (255 mg, 15%) as a yellow solid .
LC_MS: ($ES^+$): m/z 235.1 $[M+H]^+$.
$^1$H NMR (400 MHz, DMSO-d6): δ 8.53 (s, 1H), 8.12-8.14 (m, 1H), 7.86-7.88 (m, 1H), 7.40 (t, *J* = 4.0 Hz, 1H), 4.05 (s, 3H), 3.85 (s, 3H).

Step 5 : fragment **80-f** 2-methoxy-3-(1-methyl-1*H*-1,2,4-triazol-3-yl)aniline

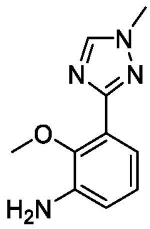

**80-f**

To a solution of 3-(2-methoxy-3-nitrophenyl)-1-methyl-1*H*-1,2,4-triazole (255 mg, 1.08 mmol) and palladium/-carbon (10%, 100 mg) in ethanol (5 ml) was stirred under hydrogen atmosphere (hydrogen balloon) at room temperature for 1 hour of reaction. The completion of the reaction was monitored by TLC. The palladium/carbon was removed by filtration and washed with ethanol (5 ml×2). The filtrates were combined and concentrated under reduced pressure. The obtained crude product was separated and purified by silica gel column chromatography (eluted with 3% methanol in dichloromethane) to obtain 2-methoxy-3-(1-methyl-1*H*-1,2,4-triazol-3-yl)aniline as a gray-white solid (140 mg, 63%).
LC_MS: ($ES^+$): m/z 205.2 $[M+H]^+$.
$^1$H NMR (400 MHz, DMSO-d6): δ 8.08 (s, 1H), 7.33-7.35 (m, 1H), 6.99 (t, *J* = 3.8 Hz, 1H), 6.80-6.82 (m, 1H), 3.99 (s, 3H), 3.77 (s, 3H).

**Example 81**

***N*⁴-ethyl-*N*²-(4-methyl-3,4,5,6-tetrahydro-2*H*-benzo[*b*][1,4,7]dioxazolin-9-yl)-5-(trifluoromethyl)pyrimidin-2,4-diamine**

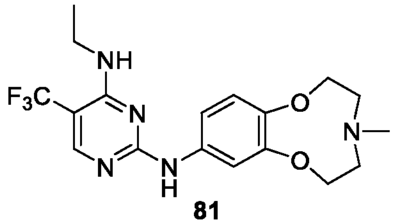

**81**

The implementation method was the same as method 8, except that fragment ethylamine hydrochloride was used to replace fragment 8-b to obtain the title compound.

LC_MS: (ES$^+$): m/z 398.3 [M+H]$^+$.

$^1$H NMR (400 MHz, DMSO-d6): δ 8.10-8.17 (m, 1H), 7.18-7.23 (m, 1H), 6.96-7.10 (m, 2H), 6.84-6.86 (m, 1H), 6.63-6.66 (m, 1H), 4.13 (t, *J* = 4.4 Hz, 2H), 3.52 (t, *J* = 5.6 Hz, 2H), 3.41 (t, *J* = 5.4 Hz, 2H), 3.34-3.36 (m, 2H), 3.26 (s, 3H), 3.10-3.26 (m, 2H), 1.05 (t, *J* = 7.0 Hz, 3H).

**Example 82**

***N*⁴-(2-(isopropylsulfonyl)phenyl)-*N*²-(4-methyl-3,4,5,6-tetrahydro-2*H*-benzo[*b*][1,4, 7]dioxazolin-9-yl)-5-(trifluoromethyl)pyrimidin-2,4-diamine**

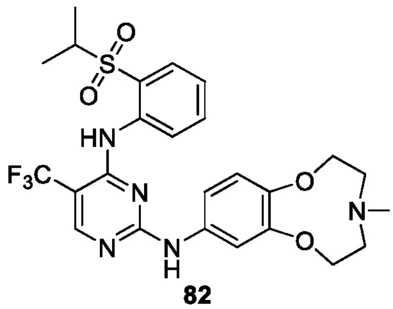

**82**

The implementation method was the same as method 8, except that fragment 2-(isopropylsulfonyl) aniline was used to replace fragment 8-b to obtain the title compound.

LC_MS: (ES$^+$): m/z 552.1 [M+H]$^+$.

$^1$H NMR (400 MHz, $CDCl_3$): δ 9.34(s, 1H). 8.42-8.44 (d, *J* = 4.2 Hz, 1H), 8.32 (s, 1H), 7.89-7.91 (d, *J* = 4.0 Hz, 1H), 7.58 (t, *J* = 3.8 Hz, 1H), 7.00 (s, 1H), 6.83-6.86 (d, *J* = 4.4 Hz, 1H), 6.59-6.61 (d, *J* = 4.2 Hz, 1H), 4.23 (t, *J* = 2.2 Hz, 2H), 3.60 (t, *J* = 3.0 Hz, 2H), 3.42-3.46 (m, 4H), 3.37 (s, 1H), 3.16-3.23 (m, 1H), 1.28-1.30 (d, *J* = 3.4 Hz, 6H).

**Example 83**

**dimethyl(2-((2-((4-methyl-3,4,5,6-tetrahydro-2*H*-benzo[*b*][1,4,7]dioxazolin-9-yl) amino)-5-(trifluoromethyl)pyrimidin-4-yl)amino)phenyl)phosphine oxide**

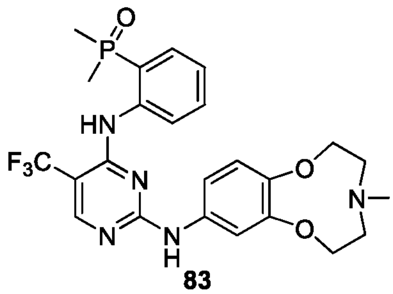

**83**

The implementation method was the same as method 8, except that fragment (2-aminophenyl)dimethylphosphine oxide was used to replace fragment 8-b to obtain the title compound.

LC_MS: ($ES^+$): m/z 522.30 $[M+H]^+$.

$^1$H NMR (400 MHz, DMSO-d6): δ 10.62 (s, 1H), 9.43 (s, 1H), 8.34 (s, 1H), 7.90-7.92 (m, 1H), 7.55-7.57 (m, 1H), 7.49 (s, 1H), 7.19 (t, *J* = 8.0 Hz, 1H), 6.93 (s, 2H), 6.59 (d, *J* = 8.8 Hz, 1H), 4.13 (s, 2H), 3.51 (t, *J* = 5.6 Hz, 2H), 3.39 (t, *J* = 5.6 Hz, 2H), 3.30 (s, 2H), 3.26 (s, 3H), 1.74 (d, *J* = 13.2 Hz, 6H),

**Example 84**

***N*$^2$-(4-methyl-3,4,5,6-tetrahydro-2*H*-benzo[*b*][1,4,7]dioxazolin-9-yl)-*N*$^4$-(2-(methyls ulfonyl)ethyl)-7*H*-pyrrolo [2,3-*d*]pyrimidin-2,4-diamine**

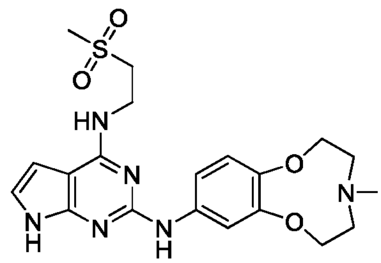

**84**

The implementation method was the same as method 4, except that fragment 2-(methylsulfonyl)ethan-1-amine was used to replace fragment 1-b, and fragment 2-d was used to replace fragment 4-a to obtain the title compound.

LC_MS: ($ES^+$): m/z 447.2 $[M+H]^+$.

$^1$H NMR (400 MHz, DMSO-d6): δ 10.96 (s, 1H), 8.25 (s, 1H), 7.41 (s, 1H), 7.30 (s, 1H), 7.11 (s, 1H), 6.74 (s, 1H), 6.61 (s, 1H), 6.34 (s, 1H), 4.12 (s, 2H), 3.81 (d, J = 5.2 Hz, 2H), 3.49 (d, J = 6.1 Hz, 5H), 3.26 (s, 6H), 3.02 (s, 3H).

Example **85**

**dimethyl(2-((2-((4-methyl-3,4,5,6-tetrahydro-2*H*-benzo[*b*][1,4,7]dioxazolin-9-yl)am ino)-7*H*-pyrrolo[2,3-*d*]pyrimidin-4-yl)amino)ethyl)phosphine oxide**

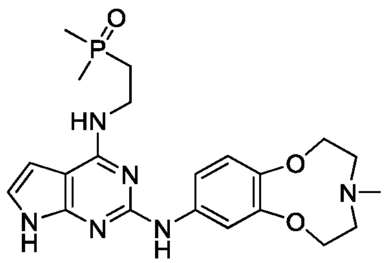

**85**

The implementation method was the same as method 4, except that fragment (2-aminoethyl)dimethylphosphine oxide was used to replace fragment 1-b, and fragment 2-d was used to replace fragment 4-a to obtain the title compound.

LC_MS: (ES$^+$): m/z 445.3 [M+H]$^+$.

$^1$H NMR (400 MHz, DMSO-d6): δ 10.92 (s, 1H), 8.23 (s, 1H), 7.39-7.27 (m, 2H), 7.10-7.08 (m, 1H), 6.76-6.69 (m, 1H), 6.61-6.58 (m, 1H), 6.35 (brs, 1H), 4.18-4.06 (m, 2H), 3.75-3.63 (m, 2H), 3.55-3.48 (m, 2H), 3.29-3.23 (m, 4H), 2.12-2.06 (m, 2H), 1.76 (s, 3H), 1.43 (d, *J* = 12.8 Hz, 6H).

**Example 86**

**dimethyl(2-((2-((4-methyl-3,4,5,6-tetrahydro-2*H*-benzo[*b*][1,4,7]dioxazolin-9-yl) amino)-5-(trifluoromethyl)pyrimidin-4-yl)amino)ethyl)phosphine oxide**

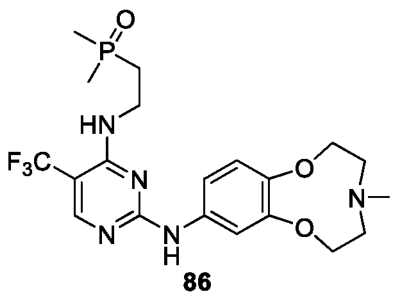

**86**

The implementation method was the same as method 8, except that fragment (2-aminoethyl)dimethylphosphine oxide was used to replace fragment 8-b to obtain the title compound.

LC_MS: (ES$^+$): m/z 474.2 [M+H]$^+$.

$^1$H NMR (400 MHz, DMSO-d6): δ 9.29 (s, 1H), 8.12 (s, 1H), 7.21 (s, 1H), 7.11-7.07 (m, 2H), 6.64 (d, *J* = 8.8 Hz, 1H), 4.12 (t, *J* = 4.4 Hz, 2H), 3.73-3.65 (m, 2H), 3.50 (t, *J* = 5.2 Hz, 2H), 3.37 (t, *J* = 5.6 Hz, 2H), 3.32-3.30 (m, 2H), 3.26 (s, 3H), 2.07-2.00 (m, 2H), 1.40 (d, *J* = 12.8 Hz, 6H).

**Example 87**

**(1*r*,4*r*)-1-methyl-4-((2-((7-methyl-2,3,6,7,8,9-hexahydro-5*H*-benzo[*b*][1,4,7]trioxa [10]azacyclododecan-12-yl) amino)-7*H*-pyrrolo[2,3-*d*]pyrimidin-4-yl)oxy)cyclohexan-1-ol**

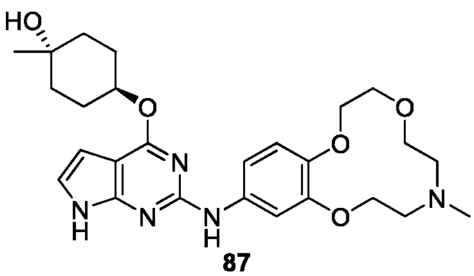

**87**

The implementation method was the same as method 6, except that fragment 6-d was used to replace fragment 6-e to obtain the title compound.

LC_MS: (ES$^+$): m/z 498.20 [M+H]$^+$.

$^1$H NMR(400 MHz, DMSO-d6): δ 11.31 (s, 1H), 8.87 (s, 1H), 7.60 (m, 1H), 7.32-7.30 (m, 1H), 6.95-6.91 (m, 1H), 6.27-6.26 (m, 1H), 5.37 (m, 1H), 4.25 (s, 2H), 4.10-4.06 (m, 4H), 3.68 (m, 4H), 3.00-2.64 (m, 4H), 2.35-2.33 (m, 3H), 2.03-1.96 (m, 2H), 1.76-1.63 (m, 4H), 1.51-1.45 (m, 2H), 1.19 (s, 3H).

**Example 88**

**(*S*)-*N*2-(7-methyl-2,3,6,7,8,9-hexahydro-5*H*-benzo[*b*][1,4,7]trioxa[10]azacyclododec an-12-yl)-*N*4-(oxetan-2-yl-methyl)-5-(trifluoromethyl)pyrimidin-2,4-diamine**

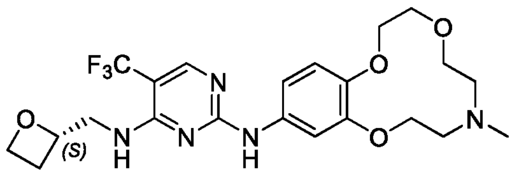

**88**

The implementation method was the same as method 8, except that fragment (S)-oxetan-2-ylmethylamine was used to replace fragment 8-b, and fragment Amine-3 was used to replace fragment Amine-1 to obtain the title compound.

LC_MS: ($ES^+$): m/z 484.30 $[M+H]^+$.

$^1H$ NMR (400 MHz, DMSO-d6): δ 9.60 (s, 1H), 8.95 (s, 1H), 8.19 (s, 1H), 7.62 (s, 1H), 7.37-7.27 (m, 2H), 7.16-7.05 (m, 2H), 4.95-4.92 (m, 1H), 4.52-4.41 (m, 2H), 4.29-4.02 (m, 4H), 3.85-3.62 (m, 6H), 3.51-3.44 (m, 1H), 3.33-3.18 (m, 1H), 2.88 (s, 3H), 2.63-2.57 (m, 1H), 2.41 (brs, 1H).

## Example 89

***N*2-(7-methyl-2,3,6,7,8,9-hexahydro-5*H*-benzo[*b*][1,4,7]trioxa[10]azacyclododecyl -12-yl)-*N*4-(2-(methylsulfonyl) ethyl)-5-(trifluoromethyl)pyrimidin-2,4-diamine**

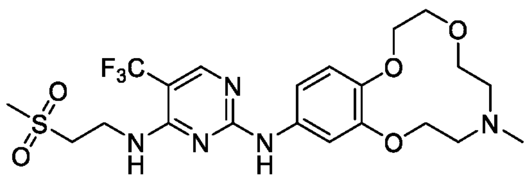

**89**

The implementation method was the same as method 8, except that fragment 2-(methylsulfonyl)ethan-1-amine was used to replace fragment 8-b, and fragment Amine-3 was used to replace fragment Amine-1 to obtain the title compound.

LC_MS: ($ES^+$): m/z 520.20 $[M+H]^+$.

$^1H$ NMR (400 MHz, DMSO-d6): δ 9.64 (s, 1H), 8.99 (s, 1H), 8.22 (s, 1H), 7.52 (s, 1H), 7.38 (brs, 1H), 7.25-7.08 (m, 1H), 4.31 (s, 2H), 4.16-3.83 (m, 6H), 3.70-3.47(m, 6H), 3.18 (brs, 2H), 3.02 (s, 3H), 2.90 (s, 3H).

## Example 90

**2-methyl-1-((2-((7-methyl-2,3,6,7,8,9-hexahydro-5*H*-benzo[*b*][1,4,7]trioxa[10]azacy clododecan-12-yl)amino)-7*H*-pyrrolo[2,3-*d*]pyrimidin-4-yl)amino)propan-2-ol**

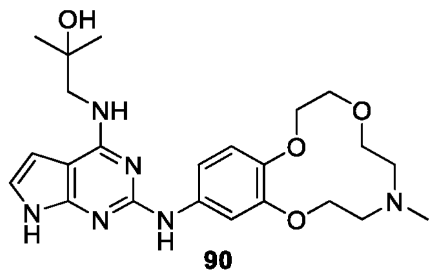

**90**

The implementation method was the same as method 3, except that fragment 1-amino-2-methylpropan-2-ol was used to replace fragment 3-a, and fragment Amine-3 was used to replace fragment Amine-1 to obtain the title compound.
LC_MS: ($ES^+$): m/z 457.25 $[M+H]^+$.
$^1H$ NMR (400 MHz, DMSO-d6): δ 10.93 (s, 1H), 8.43 (s, 1H), 7.70 (d, *J* = 2.4 Hz, 1H), 7.25-7.23 (m, 1H), 6.95 (brs, 1H), 6.86 (d, *J* = 8.8 Hz, 1H), 6.76 (t, *J* = 2.0 Hz, 1H), 6.50-6.49 (m, 1H), 4.69 (s, 1H), 4.08-4.03 (m, 4H), 3.67-3.62 (m, 4H), 3.50 (d, *J* = 5.6 Hz, 2H), 2.76 (t, *J* = 4.4 Hz, 2H), 2.61 (t, *J* = 4.4 Hz, 2H), 2.28 (s, 3H), 1.16 (s, 6H).

**Example 91**

***N*$^2$-(4-methyl-3,4,5,6-tetrahydro-2*H*-benzo[*b*][1,4,7]dioxazolin-9-yl)-*N*$^4$-phenyl-5-(trifluoromethyl)pyrimidin-2,4-diamine**

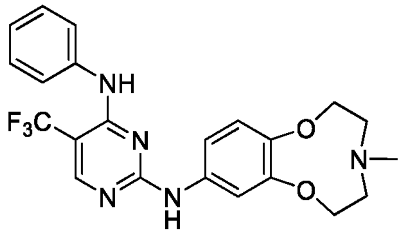

**91**

The implementation method was the same as method 7, except that fragment aniline was used to replace fragment 6-d, and fragment Amine-1 was used to replace fragment Amine-3 to obtain the title compound.
LC_MS: ($ES^+$): m/z 446.1 $[M+H]^+$.

**Example 92**

**(*S*)-*N*$^2$-(4-methyl-3,4,5,6-tetrahydro-2*H*-benzo[*b*][1,4,7]dioxazolin-9-yl)-*N*$^4$-(oxetan -2-ylmethyl)-5-(trifluoromethyl)pyrimidin-2,4-diamine**

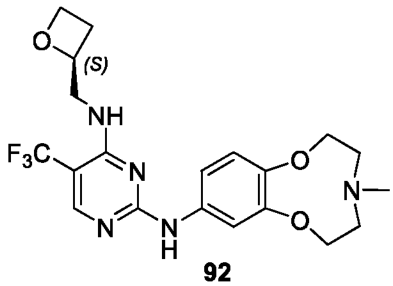

**92**

The implementation method was the same as method 8, except that fragment (S)-oxetan-2-ylmethylamine was used to replace fragment 8-b to obtain the title compound.
LC_MS: ($ES^+$): m/z 440.0 $[M+H]^+$.

**Example 93**

**(1*s*,4*s*)-1-methyl-4-((2-((4-methyl-3,4,5,6-tetrahydro-2*H*-benzo[*b*][1,4,7]dioxazolin-9-yl)amino)-5-(trifluoromethyl)pyrimidin-4-yl)oxy)cyclohexan-1-ol**

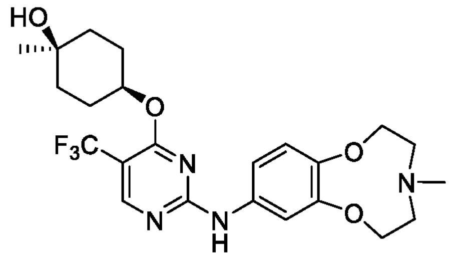

**93**

The implementation method was the same as method 7, except that fragment Amine-1 was used to replace fragment Amine-3 to obtain the title compound.

LC_MS: ($ES^+$): m/z 483.20 $[M+H]^+$.

$^1$H NMR (400 MHz, $CDCl_3$): δ 8.29 (s, 1H), 7.16 (s, 1H), 6.85 (d, *J* = 8.4 Hz, 1H), 6.62 (d, *J* = 8.8 Hz, 1H), 5.15-5.12 (m, 1H), 4.22 (t, *J* = 4.4 Hz, 2H), 3.59 (t, *J* = 5.6 Hz, 2H), 3.46-3.40 (m, 4H), 3.37 (s, 3H) 1.97-1.92 (m, 4H), 1.79-1.76 (m, 3H), 1.63-1.56 (m, 2H), 1.28 (s, 3H).

**Example 94**

***N*⁴-(2-(methoxymethyl)phenyl)-*N*²-(4-methyl-3,4,5,6-tetrahydro-2*H*-benzo[*b*][1,4,7] dioxazolin-9-yl)-5-(trifluoromethyl)pyrimidin-2,4-diamine**

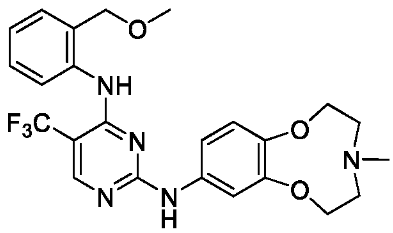

**94**

The implementation method was the same as method 7, except that fragment 2-(methoxymethyl)aniline was used to replace fragment 6-d, and fragment Amine-1 was used to replace fragment Amine-3 to obtain the title compound.

LC_MS: ($ES^+$): m/z 490.1 $[M+H]^+$.

$^1$H NMR (400 MHz, $CDCl_3$): δ 8.50 (s, 1H), 8.27 (s, 1H), 8.19 (d, *J* = 8.2 Hz, 1H), 7.33 (t, *J* = 7.8 Hz, 1H), 7.22 (dd, *J* = 7.6, 1.7 Hz, 1H), 7.08 (td, *J* = 7.5, 1.2 Hz, 1H), 7.00 (d, *J* = 2.5 Hz, 1H), 6.91 (dd, *J* = 8.6, 2.5 Hz, 2H), 6.58 (d, *J* = 8.7 Hz, 1H), 4.49 (s, 2H), 4.28 - 4.16 (m, 2H), 3.59 (t, *J* = 5.7 Hz, 2H), 3.47 - 3.39 (m, 7H), 3.36 (s, 3H).

**Example 95**

***N*⁴-(2-methoxyphenyl)-*N*²-(4-methyl-3,4,5,6-tetrahydro-2*H*-benzo[*b*][1,4,7]dioxazoli n-9-yl)-5-(trifluoromethyl) pyrimidin-2,4-diamine**

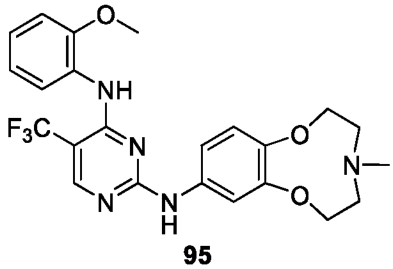

**95**

The implementation method was the same as method 7, except that fragment 2-methoxyaniline was used to replace fragment 6-d, and fragment Amine-1 was used to replace fragment Amine-3 to obtain the title compound.

LC_MS: ($ES^+$): m/z 476.1 $[M+H]^+$.

$^1H$ NMR (400 MHz, $CDCl_3$): δ 8.31 (d, *J* = 8.0 Hz, 1H), 8.16 (s, 1H), 7.14 - 7.05 (m, 1H), 7.01 (d, *J* = 2.5 Hz, 1H), 6.95 - 6.88 (m, 3H), 6.62 (d, *J* = 8.7 Hz, 1H), 4.27 - 4.21 (m, 2H), 3.92 (s, 3H), 3.61 (t, *J* = 5.6 Hz, 2H), 3.46 (dt, *J* = 8.8, 4.9 Hz, 4H), 3.38 (s, 3H).

**Example 96**

**1-(((2-((4-methyl-3,4,5,6-tetrahydro-2*H*-benzo[*b*][1,4,7]dioxazolin-9-yl)amino)-5-(trifluoromethyl)pyrimidin-4-yl) amino)methyl)cyclopropan-1-carbonitrile**

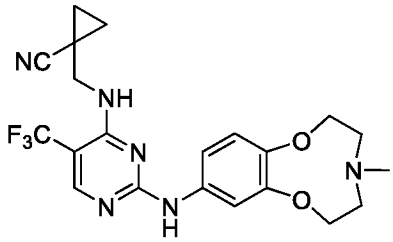

**96**

The implementation method was the same as method 8, except that fragment 1-(aminomethyl)cyclopropan-1-carbonitrile was used to replace fragment 8-b to obtain the title compound.

LC_MS: ($ES^+$): m/z 449.1 $[M+H]^+$.

$^1H$ NMR (400 MHz, $CDCl_3$): δ 8.11 (s, 1H), 7.00 (d, *J* = 2.5 Hz, 1H), 6.82 (d, *J* = 8.6 Hz, 1H), 6.61 (d, *J* = 8.7 Hz, 1H), 4.27 - 4.17 (m, 2H), 3.72 (d, *J* = 6.0 Hz, 2H), 3.58 (t, *J* = 5.6 Hz, 2H), 3.47 - 3.39 (m, 4H), 3.36 (s, 3H), 1.25 (d, *J* = 2.0 Hz, 2H), 1.05 (q, *J* = 5.1 Hz, 2H).

**Example 97**

***N*²-(2,3,6,7,8,9-hexahydro-5*H*-benzo[*b*][1,4,7]trioxa[10]azacyclododecan-13-yl)-*N*⁴-( 2-(isopropylsulfonyl)phenyl)-7*H*-pyrrolo[2,3-*d*]pyrimidin-2,4-diamine**

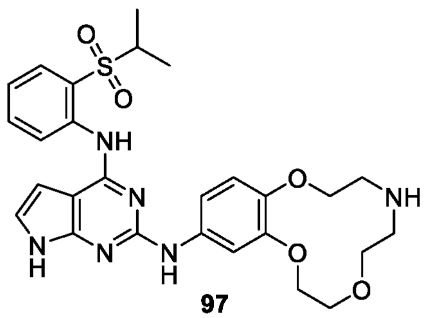

**97**

**Implementation method**

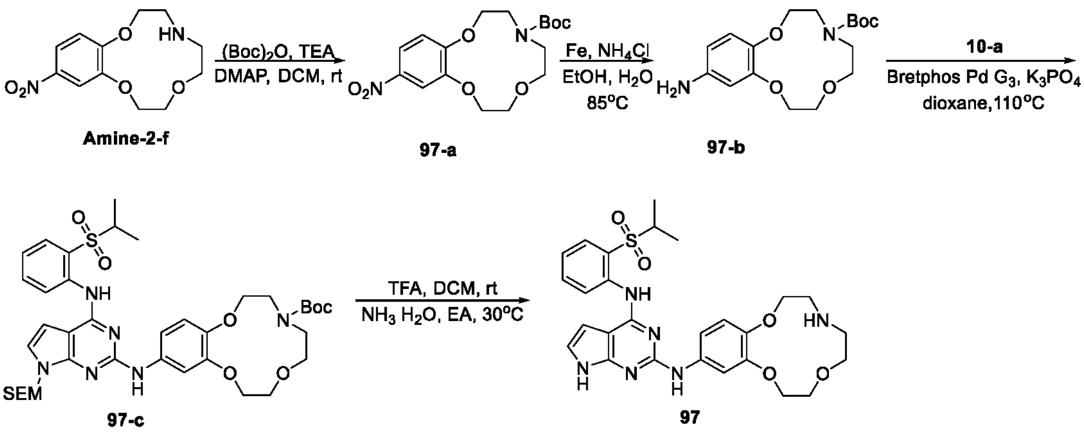

Amine-2-f
97-a
97-b
97-c
97

Step 1: synthesis of intermediate 97-a tert-butyl 13-nitro-2,3,5,6,8,9-hexahydro-7*H*-benzo[b][1,4,7]trioxa[10]azacyclododecan-7-carboxylate

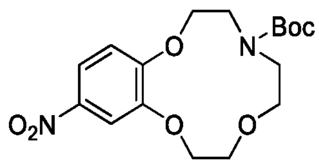

**97-a**

A solution of 13-nitro-2,3,6,7,8,9-hexahydro-5*H*-benzo[*b*][1,4,7]trioxa[10]azacyclododecane (140 mg, 0.52 mmol), di-tert-butyl dicarbonate (170 mg, 0.78 mmol), *N,N*-dimethylpyridin-4-amine (6 mg, 0.05 mmol) and triethylamine (105 mg, 1.0 mmol) in dichloromethane (2 ml) was stirred at room temperature for 12 hours. The completion of the reaction was monitored by TLC. The reaction mixture was partitioned in water (20 ml) and dichloromethane (10 ml). The organic layer was collected and the aqueous phase was extracted with dichloromethane (10 ml×2). The organic layers were combined, washed with saturated sodium chloride solution (20 ml), dried over anhydrous sodium sulfate, and concentrated under reduced pressure. The crude product was separated and purified by silica gel column chromatography (eluted with 1% methanol in dichloromethane) to obtain tert-butyl 13-nitro-2,3,5,6,8,9-hexahydro-7*H*-benzo[*b*][1,4,7]trioxa[10]azacyclododecan-7-carboxylate as a white solid (146 mg, 76%).

Step 2 : synthesis of intermediate **97-b** tert-butyl 13-amino-2,3,5,6,8,9-hexahydro-7*H*-benzo[*b*][1,4,7]trioxa[10]azacyclododecan-7-carboxylate

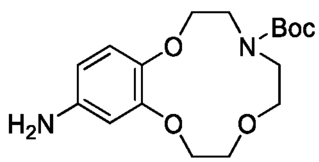

**97-b**

A solution of tert-butyl 13-nitro-2,3,5,6,8,9-hexahydro-7*H*-benzo[*b*][1,4,7]trioxa[10]azacyclododecan-7-carboxylate (146 mg, 0.4 mmol), iron powder (224 mg, 4.0 mmol), ammonium chloride (107 mg, 2.0 mmol) and water (0.2 ml) in ethanol (2 ml) was refluxed for 12 hours of reaction. The completion of the reaction was monitored by TLC. The reaction mixture was filtered and the filtrate was concentrated under reduced pressure to obtain tert-butyl 13-amino-2,3,5,6,8,9-hexahydro-7*H*-benzo[*b*][1,4,7]trioxa[10]azacyclododecan-7-carboxylate as black oil (100 mg, 73%), which was used directly in the next step without further purification.

Step 3: synthesis of intermediate **97-c** tert-butyl 13-((4-((2-(isopropylsulfonyl)phenyl)amino)-7-((2-(trimethylsilyl)ethoxy)methyl)-7*H*-pyrrolo[2, 3-*d*]pyrimidin-2-yl)amino)-2,3,5,6,8,9-hexahydro-7*H*-benzo[*b*][1,4,7]trioxa[10]azacyclododecan-7-carboxylate

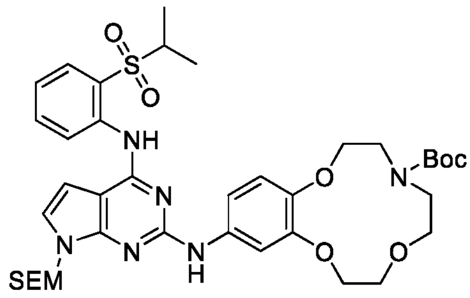

**97-c**

To a solution of 2-chloro-*N*-(2-(isopropylsulfonyl)phenyl)-7-((2-(trimethylsilyl)ethoxy)methyl)-7*H*-pyrrolo[2,3-*d*]pyrimidin-4-amine (71.1 mg, 0.15 mmol), tert-butyl 13-amino-2,3,5,6,8,9-hexahydro-7H-benzo[b][1,4,7]trioxa[10]azacyclododecan-7-carboxylate (50 mg, 0.15 mmol) and potassium phosphate (95.4 mg, 0.45 mmol) in 1,4-dioxane (1 ml) was added BrettPhos Pd G3 (13.6 mg, 0.015 mmol) under nitrogen atmosphere at room temperature. The reaction mixture was replaced with nitrogen gas for three times and stirred at 110°C for 12 hours. The completion of the reaction was monitored by TLC. The reaction mixture was cooled to room temperature and partitioned in water (20 ml) and ethyl acetate (10 ml). The organic layer was collected and the aqueous phase was extracted with ethyl acetate (10 ml×2). The organic layer was collected, washed with saturated sodium chloride solution (20 ml), dried over anhydrous sodium sulfate, and concentrated under reduced pressure. The obtained crude product was separated and purified by preparative TLC (eluted with 3% methanol in dichloromethane) to obtain tert-butyl 13-((4-((2-(isopropylsulfonyl)phenyl)amino)-7-((2-(trimethylsilyl)ethoxy)methyl)-7*H*-pyrrolo[2, 3-*d*]pyrimidin-2-yl)amino)-2,3,5,6,8,9-hexahydro-7*H*-benzo[*b*][1,4,7]trioxa[10]azacyclododecan-7-carboxylate as a yellow solid (70 mg, 59%).

LC_MS: ($ES^+$): m/z 783.7 $[M+H]^+$.

Step 4 : synthesis of *N*2-(2,3,6,7,8,9-hexahydro-5*H*-benzo[*b*][1,4,7]trioxa[10]azacyclododecan-13-yl-*N*-(2-(isopropyls ulfonyl)phenyl)-7*H*-pyrrolo[2,3-d]pyrimidin-2,4-diamine

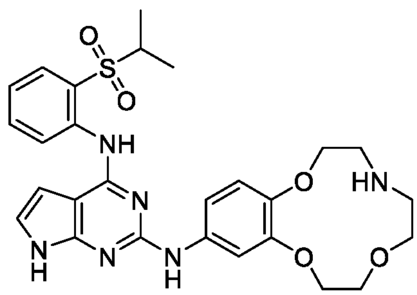

**97**

A solution of tert-butyl 13-((4-((2-(isopropylsulfonyl)phenyl)amino)-7-((2-(trimethylsilyl)ethoxy)methyl)-7*H*-pyrrolo[2, 3-*d*]pyrimidin-2-yl)amino)-2,3,5,6,8,9-hexahydro-7*H*-benzo[b][1,4,7]trioxa[10]azacyclododecan-7-carboxylate (70 mg, 0.09 mmol) and trifluoroacetic acid (2 ml) in dichloromethane ( 2 ml) was stirred at room temperature for 3 hours. The completion of the reaction was monitored by TLC. The reaction solution was concentrated and the residue was added to a mixed solution of ammonia hydroxide ( 2 ml) and ethyl acetate (4 ml). The reaction mixture was stirred at 30°C for 16 hours. The completion of the reaction was monitored by TLC. The organic layer was collected, washed with saturated sodium chloride solution (10 ml), dried over anhydrous sodium sulfate, and concentrated under reduced pressure. The obtained crude product was separated and purified by preparative TLC (eluted with 10% methanol in dichloromethane) to obtain *N*2-(2,3,6,7,8,9-hexahydro-5*H*-benzo[*b*][1,4,7]trioxa[10]azacyclododecan-13-yl-*N*4-(2-(isopropyls ulfonyl)phenyl)-7*H*-pyrrolo[2,3-*d*]pyrimidin-2,4-diamine as a yellow solid (18.7 mg , 37%).

LC_MS: ($ES^+$): m/z 553.9 $[M+H]^+$.

$^1H$ NMR (400 MHz, $CDCl_3$): δ 9.58 (s, 1H), 9.10 (s, 1H), 8.79-8.81 (m, 1H), 7.86-7.88 (m, 1H), 7.60-7.64 (m, 1H), 7.48-7.49 (m, 1H), 7.16-7.20 (m, 1H), 7.00-7.05 (m, 2H), 6.92 (s, 1H), 6.84-6.85 (d, *J* = 3.6 Hz, 1H), 6.44-6.45 (d, *J* = 3.6 Hz, 1H), 4.18-4.21 (m, 2H), 4.09-4.11 (m, 2H), 3.77-3.81 (m, 4H), 3.24-3.30 (m, 1H), 3.00-3.02 (m, 2H), 2.92-2.94 (m, 2H), 1.30-1.32 (d, *J* = 7.2 Hz, 6H).

**Example 98**

**1-(13-((4-((2-(isopropylsulfonyl)phenyl)amino)-7*H*-pyrrolo[2,3-*d*]pyrimidin-2-yl) amino)-2,3,5,6,8,9-hexahydro-7*H*-benzo[*b*][1,4,7]trioxa[10]azacyclododecan-7-yl)ethan-1-on e**

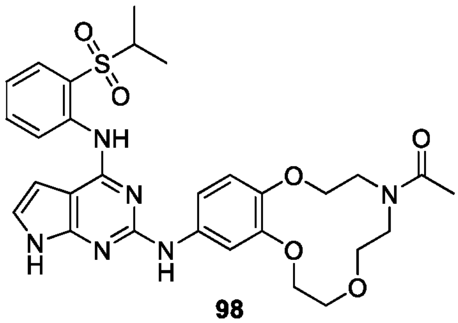

**98**

The implementation method was the same as Example 97, except that acetyl chloride was used to replace di-tert-butyl dicarbonate to obtain the title compound.

LC_MS: ($ES^+$): m/z 595.6 $[M+H]^+$.

$^1H$ NMR (400 MHz, $CDCl_3$): δ 9.60 (s, 1H), 8.74-8.80 (m, 2H), 7.85-7.88 (m, 1H), 7.60-7.65 (m, 1H), 7.41-7.47 (m, 1H), 7.09-7.20 (m, 2H), 6.84-6.97 (m, 3H), 6.45-6.46 (m, 1H), 4.24-4.33 (m, 2H), 4.12-4.15 (m, 1H), 4.06-4.10 (m, 2H), 3.80-3.82 (m, 1H), 3.72-3.76 (m, 4H), 3.55-3.63 (m, 2H), 3.22-3.29 (m, 1H), 2.17-2.23 (m, 3H), 1.29-1.31 (d, *J* = 6.8 Hz, 6H).

**Example 99**

***N*$^4$-(2-(isopropylsulfonyl)phenyl)-*N*$^2$-(7-(methylsulfonyl)-2,3,6,7,8,9-hexahydro-5*H*-benzo[b][1,4,7]trioxa[10]azacyclododecan-13-yl)-7*H*-pyrrolo[2,3-*d*]pyrimidin-2,4-diamine**

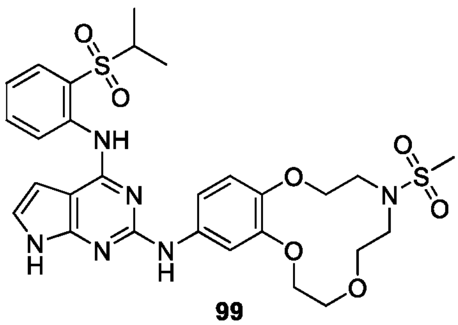

**99**

The implementation method was the same as Example 97, except that methanesulfonyl chloride was used to replace di-tert-butyl dicarbonate to obtain the title compound.

LC_MS: ($ES^+$): m/z 632.0 $[M+H]^+$.

$^1H$ NMR (400 MHz, DMSO-d6): δ 11.44 (s, 1H), 9.48(s, 1H), 8.88-8.93 (m, 2H), 7.81-7.84 (m, 1H), 7.72-7.76 (m, 1H), 7.62-7.63 (d, *J* = 2.4Hz, 1H), 7.28-7.34 (m, 2H), 7.03-7.04 (m, 1H), 6.92-6.94 (d, *J* = 8.4 Hz, 1H), 6.25-6.26 (m, 1H), 4.07-4.13 (m, 4H), 3.72-3.76 (m, 4H), 3.51-3.54 (t, *J* = 2.6 Hz, 2H), 3.44-3.49 (m, 1H), 3.38-3.41 (t, *J* = 2.4 Hz, 2H), 2.95 (s, 3H), 1.17-1.19 (d, *J* = 6.8 Hz, 6H).

**Example 100**

**(1*s*,4*s*)-4-((5-fluoro-2-((4-methyl-3,4,5,6-tetrahydro-2*H*-benzo[*b*][1,4,7]dioxazolin-9-yl)amino)pyrimidin-4-yl) oxy)-1-methylcyclohexan-1-ol**

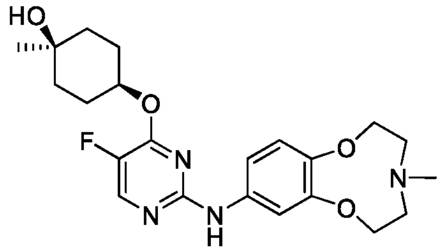

**100**

The implementation method was the same as method 1, except that fragment 6-e was used to replace fragment 1-b to obtain the title compound.

LC_MS: ($ES^+$): m/z 433.2 $[M+H]^+$.

$^1H$ NMR (400 MHz, $CDCl_3$): δ 8.07 - 7.88 (m, 1H), 7.23 - 7.09 (m, 1H), 7.23 - 7.09 (m, 1H), 7.09 - 6.33 (m, 3H), 5.07 (tt, *J* = 9.3, 5.2 Hz, 1H), 4.21 (t, *J* = 4.4 Hz, 2H), 3.59 (t, *J* = 5.5 Hz, 2H), 3.49 - 3.27 (m, 4H), 3.36 (s, 3H), 2.05 - 1.82 (m, 4H), 1.82 - 1.71 (m, 2H), 1.60 (ddd, *J* = 13.9, 10.7, 5.7 Hz, 2H), 1.28 (s, 3H).

**Example 101**

**(1*s*,4*s*)-1-methyl-4-((5-methyl-2-((4-methyl-3,4,5,6-tetrahydro-2*H*-benzo[*b*][1,4,7] dioxazolin-9-yl)amino)pyrimidin-4-yl)oxy)cyclohexan-1-ol**

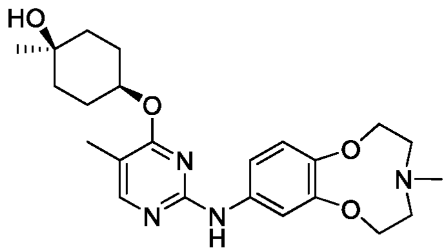

**101**

The implementation method was the same as method 1, except that fragment 2,4-dichloro-5-methylpyrimidine was used to replace fragment 1-a, and fragment 6-e was used to replace fragment 1-b to obtain the title compound.

LC_MS: ($ES^+$): m/z 429.2 $[M+H]^+$.

$^1H$ NMR (400 MHz, $CDCl_3$): δ 7.90 (s, 1H), 7.22 (d, *J* = 2.4 Hz, 1H), 7.01 - 6.74 (m, 2H), 6.60 (d, *J* = 8.6 Hz, 1H), 5.06 (tt, *J* = 9.2, 4.3 Hz, 1H), 4.21 (t, *J* = 4.4 Hz, 2H), 3.59 (t, *J* = 5.7 Hz, 2H), 3.50 - 3.37 (m, 4H), 3.36 (s, 3H), 1.96 - 1.82 (m, 4H), 1.77 (dd, *J* = 12.8, 3.9 Hz, 2H), 1.61 (ddd, *J* = 14.3, 11.5, 4.5 Hz, 2H), 1.28 (s, 3H).

**Example 102**

**(1*s*,4*s*)-4-((5-chloro-2-((4-methyl-3,4,5,6-tetrahydro-2*H*-benzo[*b*][1,4,7]dioxazolin-9 -yl)amino)pyrimidin-4-yl) oxy)-1-methylcyclohexan-1-ol**

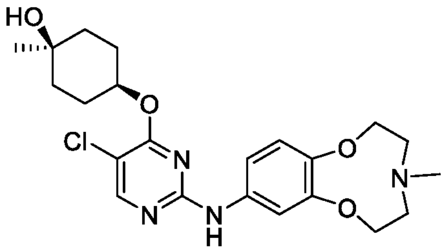

**102**

The implementation method was the same as method 1, except that fragment 2,4,5-trichloropyrimidine was used to replace fragment 1-a, and ragment 6-e was used to replace fragment 1-b to obtain the title compound.

LC_MS: (ES+): m/z 449.1 [M+H]+.

$^1$H NMR (400 MHz, $CDCl_3$): δ 8.06 (s, 1H), 7.16 (d, *J* = 2.5 Hz, 1H), 6.82 (dd, *J* = 8.6, 2.5 Hz, 2H), 6.61 (d, *J* = 8.7 Hz, 1H), 5.15 - 4.99 (m, 1H), 4.22 (dd, *J* = 5.2, 3.6 Hz, 2H), 3.59 (t, *J* = 5.7 Hz, 2H), 3.43 (t, *J* = 5.7 Hz, 2H), 3.41 - 3.37 (m, 2H), 3.36 (s, 3H), 1.95 (td, *J* = 10.2, 8.7, 4.4 Hz, 4H), 1.78 (dh, *J* = 9.6, 2.5 Hz, 2H), 1.62 - 1.55 (m, 2H), 1.28 (s, 3H).

**Example 103**

**(1*s*,4*s*)-4-((5-fluoro-2-((7-methyl-2,3,6,7,8,9-hexahydro-5*H*-benzo[*b*][1,4,7]trioxa[10] azacyclododecan-12-yl) amino)pyrimidin-4-yl)oxy)-1-methylcyclohexan-1-ol**

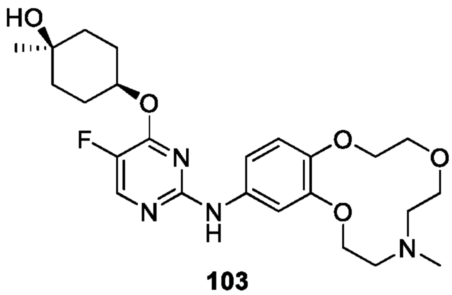

**103**

The implementation method was the same as method 1, except that fragment 6-e was used to replace fragment 1-b, and fragment Amine-3 was used to replace fragment Amine-1 to obtain the title compound.

LC_MS: (ES+): m/z 477.55 [M+H]+.

$^1$H NMR (400 MHz, DMSO-d6): δ 9.37 (s, 1H), 8.24 (d, *J* = 2.8 Hz, 1H), 7.48 (d, *J* = 2.4 Hz, 1H), 7.21-7.19 (m, 1H), 6.97 (d, *J* = 8.8 Hz, 1H), 5.08-5.01 (m, 1H), 4.25 (s, 1H), 4.12-4.06 (m, 4H), 3.70 (brs, 4H), 2.99-2.85 (m, 4H), 2.46 (s, 3H), 1.88-1.84 (m, 4H), 1.67-1.64 (m, 2H), 1.46-1.41 (m, 2H), 1.15 (s, 3H).

**Example 104**

**(1*s*,4*s*)-4-((5-chloro-2-((7-methyl-2,3,6,7,8,9-hexahydro-*5H*-benzo[*b*][1,4,7]trioxa[10] azacyclododecan-12-yl) amino)pyrimidin-4-yl)oxy)-1-methylcyclohexan-1-ol**

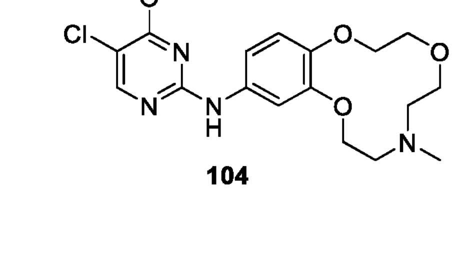

**104**

The implementation method was the same as method 1, except that fragment 2,4,5-trichloropyrimidine was used to replace fragment 1-a, fragment 6-e was used to replace fragment 1-b, and fragment Amine-3 was used to replace fragment Amine-1 to obtain the title compound.
LC_MS: ($ES^+$): m/z 493.40 $[M+H]^+$.
$^1$H NMR (400 MHz, DMSO-d6): δ 9.54 (s, 1H), 8.26 (s, 1H), 7.48 (d, *J* = 1.2 Hz, 1H), 7.25-7.23 (m, 1H), 7.01 (d, *J* = 8.8 Hz, 1H), 5.09-5.02 (m, 1H), 4.26 (s, 1H), 4.17-4.08 (m, 4H), 3.72 (brs, 4H), 3.07-2.87 (m, 4H), 2.57 (s, 3H), 1.85-1.84 (m, 4H), 1.68-1.64 (m, 2H), 1.46-1.40 (m, 2H), 1.16 (s, 3H).

**Example 105**

**(1*s*,4*s*)-1-methyl-4-((5-methyl-2-((7-methyl-2,3,6,7,8,9-hexahydro-5*H*-benzo[*b*][1,4, 7]trioxa[10]azacyclododecan-12-yl)amino)pyrimidin-4-yl)oxy)cyclohexan-1-ol**

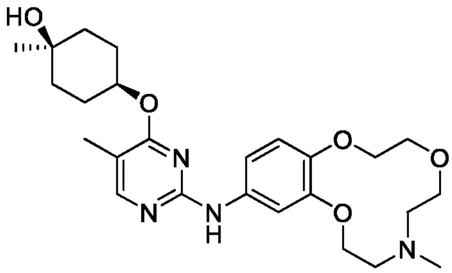

**105**

The implementation method was the same as method 1, except that fragment 2,4-dichloro-5-methylpyrimidine was used to replace fragment 1-a, fragment 6-e was used to replace fragment 1-b, and fragment Amine-3 was used to replace fragment Amine-1 to obtain the title compound.
LC_MS: (ES+): m/z 473.3 $[M+H]^+$.
$^1$HNMR (400 MHz, DMSO-d6): δ 9.15 (s, 1H), 8.00 (s, 1H), 7.55 (d, *J* = 2.0 Hz, 1H), 7.29-7.27 (m, 1H), 6.99 (d, *J* = 8.8 Hz, 1H), 5.06-4.99 (m, 1H), 4.22-4.08 (m, 5H), 3.78-3.67 (m, 4H), 3.10-2.58 (m, 4H), 2.50 (s, 3H), 1.96 (s, 3H), 1.83-1.80 (m, 4H), 1.70-1.62 (m, 2H), 1.46-1.39 (m, 2H), 1.16 (s, 3H).

**Example 106**

**cyclopropyl((1*R*,5*S*)-3-(5-fluoro-2-((4-methyl-3,4,5,6-tetrahydro-2*H*-benzo[*b*][1,4,7] dioxazolin-9-yl)amino)pyrimidin-4-yl)-3,8-diazabicyclo[3.2.1]octan-8-yl)methanone**

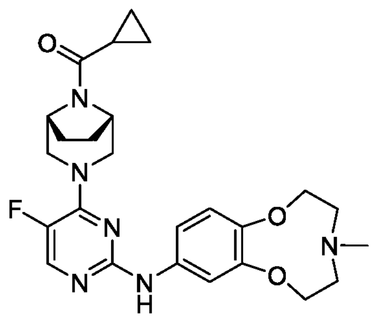

**106**

The implementation method was the same as method 1, except that fragment 79-d was used to replace fragment 1-b to obtain the title compound.
LC_MS: ($ES^+$): m/z 483.4 $[M+H]^+$.
$^1$H NMR (400 MHz, DMSO-d6): δ 8.77 (s, 1H), 7.94 (d, *J* = 6.8 Hz, 1H), 7.01-7.06 (m, 2H), 6.61 (d, *J* = 8.8 Hz, 1H), 4.73-4.74 (m, 1H), 4.57-4.58 (m, 1H), 4.10-4.22 (m, 4H), 3.56 (t, *J* = 5.6 Hz, 2H), 3.35 (t, *J* = 5.6 Hz, 2H), 3.29 (s, 2H), 3.26 (s,

3H), 3.21 (d, $J$ = 12.4 Hz, 1H), 3.09 (d, $J$ = 12.4 Hz, 1H), 1.96-2.01 (m, 2H), 1.70-1.82 (m, 2H), 1.26-1.34 (m, 1H), 0.70-0.79 (m, 4H).

**Example 107**

**(1*s*,4*s*)-1-methyl-4-((2-((7-methyl-2,3,6,7,8,9-hexahydro-5*H*-benzo[*b*][1,4,7]trioxa[1 0]azacyclododecan-12-yl) amino)-6-(methylamino)pyrimidin-4-yl)oxy)cyclohexan-1-ol**

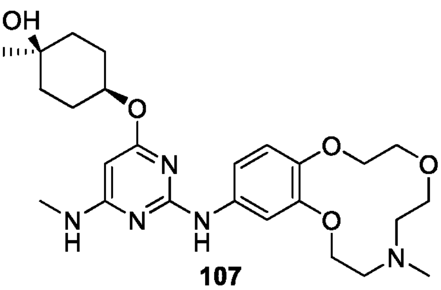

**107**

The implementation method was the same as method 7, except that fragment 2,6-dichloro-N-methylpyrimidin-4-amine was used to replace fragment 7-a to obtain the title compound.

LC_MS: (ES$^+$): m/z 488.35 [M+H] $^+$.

$^1$H NMR (400 MHz, DMSO-d6): δ 8.71 (s, 1H), 7.66 (s, 1H), 7.17 (d, $J$ = 8.4 Hz, 1H), 6.89 (d, $J$ = 8.8 Hz, 1H), 6.71 (d, $J$ = 8.4 Hz, 1H), 6.24 (d, $J$ = 2.0 Hz, 1H), 5.16 (s, 1H), 4.89-4.85 (m, 1H), 4.06-4.05(m, 2H), 3.98-3.94 (m, 2H), 3.66-3.62 (m, 4H), 2.84-2.67 (m, 7H), 2.37-2.33 (m, 3H), 1.75-1.60 (m, 6H), 1.42-1.36 (m, 2H), 1.13 (s, 3H).

**Example 108**

**(1*s*,4*s*)-1-methyl-4-((2-((4-methyl-3,4,5,6-tetrahydro-2*H*-benzo[*b*][1,4,7]dioxazolin-9-yl)amino)-6-(methylamino) pyrimidin-4-yl)oxy)cyclohexan-1-ol**

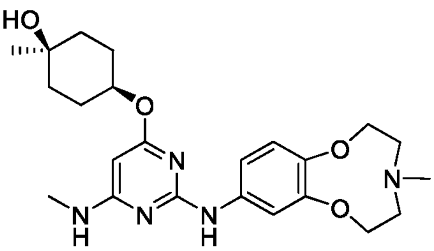

**108**

The implementation method was the same as method 7, except that fragment 2,6-dichloro-*N*-methylpyrimidine-4-amine was used to replace fragment 7-a, and fragment Amine-1 was used to replace fragment Amine-3 to obtain the title compound.

LC_MS: (ES$^+$): m/z 444.55 [M+H] $^+$.

$^1$H NMR (400 MHz, $CDCl_3$): δ 7.24 (d, $J$ = 2.4 Hz, 1H), 6.86-6.84 (m, 1H), 6.75 (brs, 1H), 6.59 (d, $J$ = 8.8 Hz, 1H), 5.18 (s, 1H), 4.99-4.94 (m, 1H), 4.84 (brs, 1H), 4.22-4.20 (m, 2H), 3.59 (t, $J$ = 5.6 Hz, 2H), 3.43-3.37 (m, 4H), 3.36 (s, 3H), 2.85 (d, $J$ = 5.2 Hz, 3H), 1.95-1.91 (m, 2H), 1.88-1.81 (m, 2H), 1.78-1.74 (m, 2H), 1.63-1.55 (m, 2H), 1.27 (s, 3H).

**Example 109**

**(1s,4s)-1-methyl-4-((2-((7-methyl-2,3,6,7,8,9-hexahydro-5H-benzo[b][1,4,7]trioxa[1 0]azacyclododecan-12-yl) amino)quinazolin-4-yl)oxy)cyclohexan-1-ol**

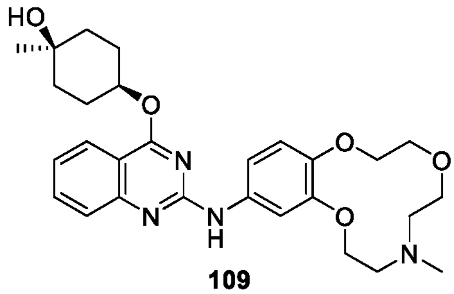

**109**

The implementation method was the same as method 7, except that fragment 2,4-dichloroquinazoline was used to replace fragment 7-a to obtain the title compound.

LC_MS: ($ES^+$): m/z 509.40 $[M+H]^+$.

$^1$H NMR (400 MHz, DMSO-d6): δ 9.35 (s, 1H), 7.93 (d, *J* = 8.0 Hz, 1H), 7.77-7.69 (m, 2H), 7.52 (d, *J* = 8.4 Hz, 1H), 7.37 (d, *J* = 8.4 Hz, 1H), 7.26 (t, *J* = 7.6 Hz, 1H), 6.98 (t, *J* = 8.8 Hz, 1H), 5.29-5.27 (m, 1H), 4.30 (s, 1H), 4.13-4.08 (m, 4H), 3.68-3.66 (m, 4H), 2.86-2.67 (m, 4H), 2.35 (s, 3H), 1.93-1.91 (m, 4H), 1.73-1.70 (m, 2H), 1.50-1.43 (m, 2H), 1.17 (s, 3H).

**Example 110**

**(1*s*,4*s*)-1-methyl-4-((2-((7-methyl-2,3,6,7,8,9-hexahydro-5*H*-benzo[*b*][1,4,7]trioxa[1 0]azacyclododecan-12-yl) amino)quinolin-4-yl)oxy)cyclohexan-1-ol**

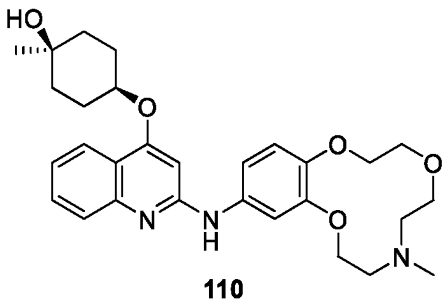

**110**

The implementation method was the same as method 7, except that fragment 2,4-dichloroquinoline was used to replace fragment 7-a to obtain the title compound.

LC_MS: ($ES^+$): m/z 508.35 $[M+H]^+$.

$^1$H NMR (400 MHz, DMSO-d6): δ 9.16 (s, 1H), 7.96 (s, 1H), 7.90 (d, *J* = 8.4 Hz, 1H), 7.60-7.52 (m, 2H), 7.30-7.21 (m, 2H), 7.00 (d, *J* = 8.8 Hz, 1H), 6.50 (s, 1H), 4.48-4.42 (m, 1H), 4.31 (s, 1H), 4.20 (brs, 2H), 4.12-4.09 (m, 2H), 3.70 (brs, 4H), 3.03-2.77 (m, 4H), 2.49 (s, 3H), 1.93-1.89 (m, 4H), 1.73-1.69 (m, 2H), 1.51-1.44 (m, 2H), 1.18 (s, 3H).

**Example 111**

***N*-(5-methyl-2-((2-((4-methyl-3,4,5,6-tetrahydro-*2H*-benzo[*b*][1,4,7]dioxazolin-9-yl) amino)-7*H*-pyrrolo[2,3-*d*]pyrimidin-4-yl)amino)phenyl)acrylamide**

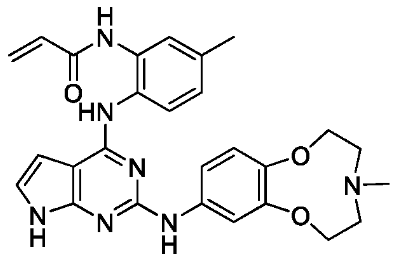

**111**

The implementation method was the same as method 3, except that fragment tert-butyl (2-amino-5-methylphenyl)carbamate was used to replace fragment 3-a, and fragment Amine-3 was used to replace fragment Amine-1 to obtain the title compound.

LC_MS: ($ES^+$): m/z 500.2[M+H]$^+$.

$^1$H NMR (400 MHz, $CDCl_3$) δ 8.95 (s, 1H), 8.57 (s, 1H), 7.74 (s, 1H), 6.95 (dd, *J* = 48.3, 41.0 Hz, 2H), 6.80 (d, *J* = 26.0 Hz, 1H), 6.60 (d, *J* = 33.7 Hz, 2H), 6.30 (d, *J* = 16.8 Hz, 1H), 6.11 (dd, *J* = 29.9, 19.3 Hz, 1H), 5.94 (s, 1H), 5.64 (d, *J* = 9.9 Hz, 1H), 5.34 (s, 1H), 4.12 (dd, *J* = 21.4, 14.4 Hz, 2H), 3.59 (d, *J* = 30.4 Hz, 2H), 3.35 (s, 5H), 2.33 (d, *J* = 17.2 Hz, 2H), 1.75 (s, 3H).

**Example 112**

***N*-(5-methyl-2-((2-((4-methyl-3,4,5,6-tetrahydro-*2H*-benzo[*b*][1,4,7]dioxazolin-9-yl) amino)-5-(trifluoromethyl) pyrimidin-4-yl)amino)phenyl)acrylamide**

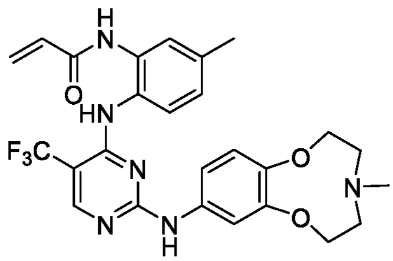

**112**

The implementation method was the same as method 3, except that fragment tert-butyl (2-amino-5-methylphenyl)carbamate was used to replace fragment 3-a, and fragment 7-a was used to replace fragment 2-d to obtain the title compound.

LC_MS: ($ES^+$): m/z 529.0 [M+H]$^+$.

1H NMR (400 MHz, MeOD) δ 8.13 (s, 1H), 7.53 (d, J = 8.0 Hz, 1H), 7.15 (d, J = 8.5 Hz, 2H), 6.77 (ddd, J = 18.1, 15.6, 11.2 Hz, 3H), 6.37 (dd, J = 5.9, 3.9 Hz, 2H), 5.77 (dd, J = 8.4, 3.5 Hz, 1H), 4.17 - 4.12 (m, 2H), 3.58 (t, J = 5.6 Hz, 2H), 3.49 - 3.36 (m, 4H), 3.35 (s, 3H), 2.39 (s, 3H).

**Example 113**

***N*-(2-((2-((4-methyl-3,4,5,6-tetrahydro-*2H*-benzo[*b*][1,4,7]dioxazolin-9-yl)amino)-5-(trifluoromethyl)pyrimidin-4-yl)amino)phenyl)methanesulfonamide**

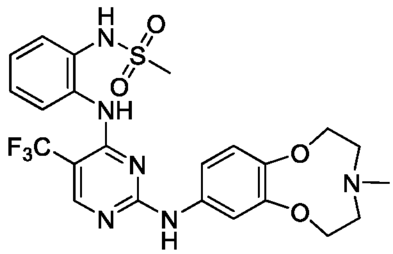

113

The implementation method was the same as method 7, except that fragment N-(2-aminophenyl)methanesulfonamide was used to replace fragment 6-d, and fragment Amine-1 was used to replace fragment Amine-3 to obtain the title compound.

LC_MS: (ES$^+$): m/z 539.20 [M+H]$^+$.

$^1$H NMR (500 MHz, DMSO-d6): δ 9.14 (s, 1H), 8.60 (s, 1H), 8.51 (s, 1H), 8.28 (s, 1H), 7.82 (d, *J* = 7.0 Hz, 1H), 7.28 (dd, *J*= 7.6, 1.6 Hz, 1H), 7.11 - 7.03 (m, 2H), 6.82 - 6.74 (m, 2H), 6.67 (d, *J* = 8.7 Hz, 1H), 4.19 - 4.13 (m, 2H), 3.54 (t, *J* = 5.5 Hz, 2H), 3.45 (t, *J* = 5.4 Hz, 2H), 3.41 - 3.37 (m, 2H), 3.28 (s, 3H), 2.89 (s, 3H).

**Example 114**

***N*-(5-fluoro-4-(5-fluoro-2-methoxyphenyl)pyrimidin-2-yl)-7-methyl-2,3,6,7,8,9-hexahydro-*5H*-benzo[*b*][1,4,7] trioxa[10]azacyclododecan-12-amine**

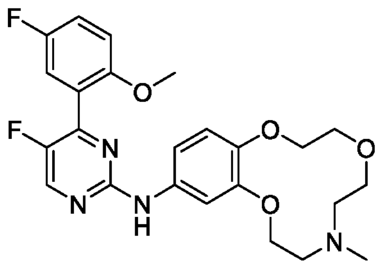

114

The implementation method was the same as method 2, except that fragment 5-fluoro-2-methoxyphenylboronic acid was used to replace fragment 2-b, fragment 1-a was used to replace fragment 2-d, and fragment Amine-3 was used to replace fragment Amine-1 to obtain the title compound.

LC_MS: (ES$^+$): m/z 473.5 [M+H]$^+$.

$^1$H NMR (400 MHz, DMSO-d6): δ 9.66 (s, 1H), 8.54 (d, *J* =2.0 Hz, 1H), 7.58 (brs, 1H), 7.39-7.36 (m, 2H), 7.23-7.19 (m, 2H), 6.97-6.95 (m, 1H), 4.08 (brs, 4H), 3.80 (s, 3H), 3.73-3.69 (m, 4H), 3.29 (s, 3H), 2.89-2.52 (m, 4H).

**Example 115**

***N*-(5-fluoro-4-(5-fluoro-2-methoxyphenyl)pyrimidin-2-yl)-7-methyl-2,3,6,7,8,9-hexahydro-*5H*-benzo[*b*][1,4,7] trioxa[10]azacyclododecan-13-amine**

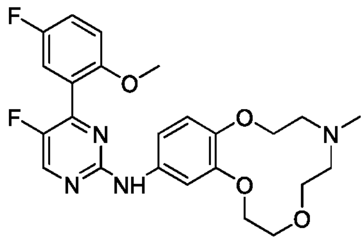

**115**

The implementation method was the same as method 2, except that fragment 5-fluoro-2-methoxyphenylboronic acid was used to replace fragment 2-b, fragment 1-a was used to replace fragment 2-d, and fragment Amine-2 was used to replace fragment Amine-1 to obtain the title compound.

LC_MS: ($ES^+$): m/z 473.6 $[M+H]^+$.

$^1H$ NMR (400 MHz, $CDCl_3$): δ 9.67 (s, 1H), 8.55 (d, *J* = 2.0 Hz, 1H), 7.57 (d, *J* = 2.4 Hz, 1H), 7.41-7.36 (m, 2H), 7.23-7.20 (m, 2H), 6.99 (d, *J* = 8.8 Hz, 1H), 4.13-4.07 (m, 4H), 3.80 (s, 3H), 3.70-3.62 (m, 4H), 3.30 (s, 3H), 3.08-2.75 (m, 4H).

**Example 116**

***N*$^4$-(2-(methoxymethyl)phenyl)-*N*$^2$-(7-methyl-2,3,6,7,8,9-hexahydro-5*H*-benzo[*b*][1, 4,7]trioxa[10]azacyclododecan-12-yl)-5-(trifluoromethyl)pyrimidin-2,4-diamine**

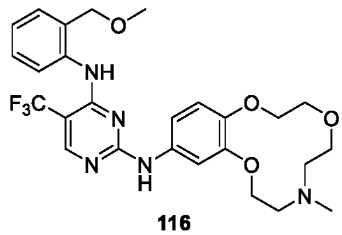

**116**

The implementation method was the same as method 7, except that fragment 2-(methoxymethyl)aniline was used to replace fragment 6-d to obtain the title compound.

LC_MS: ($ES^+$): m/z 534.75 $[M+H]^+$.

$^1H$ NMR (400 MHz, $CD_3OD$): δ 8.28 (s, 1H), 7.94-7.91 (m, 1H), 7.85-7.79 (m, 1H), 7.47-7.33 (m, 4H), 7.21-7.19 (m, 1H), 7.08-7.06 (m, 1H), 6.92-6.90 (m, 1H), 4.50 (s, 2H), 4.15-4.13 (m, 2H) , 3.98-3.95 (m, 2H), 3.81-3.78 (m, 4H), 3.39 (s, 3H), 3.09-2.92 (m, 4H), 2.58 (s, 3H).

**Example 117: measurement of the solubility of compounds in water and diluted hydrochloric acid**

Preparation of standard samples

The compounds were dissolved in acetonitrile (LCMS pure) and prepared into standard samples having the concentration gradient of 1000 μg/mL, 500 μg/mL, 250 μg/mL, 125 μg/mL, 62.5 μg/mL, and 31.25 μg/mL.

Preparation of the sampled to be tested in water

About 1.0 mg of the powder of the compound to be tested was weighed and added to 0.3 mL of ultrapure water (pH-6), the resulting mixture was shaked thoroughly at room temperature overnight and centrifuged, and the supernatant was taken and diluted 10 times with acetonitrile. The sample was analyzed after mixing evenly.

Preparation of samples to be tested in diluted hydrochloric acid

153.8 μL of 6 M HCl was pipetted, 20 mL of water was added, and mixed evenly to prepare diluted hydrochloric acid solution A (pH-1.5). About 1.0 mg of the powder of the compound to be tested was weighed and added to 0.3 mL of diluted hydrochloric acid solution A, the resulting mixture was shaked thoroughly at room temperature for 1 hour and

centrifuged, and the supernatant was taken and diluted 10 times with acetonitrile. The sample was analyzed after mixing evenly.

Analytical method

The standard sample and the sample to be tested were analyzed by Shimadzu LCMS-2020, where chromatographic column was Skim-psck GIST 5 μm C18 2.1*50 nm; mobile phase A was water and mobile phase B was methanol; and flow rate was 0.3 mL/min. The gradient of B was as the following: 0 min: 10%; 0-1.0 min: 10%; 1.0-2.5 min: 95%; 3.0-4.0 min: 95%; 4.0-4.1 min: 10%; 4.1-6.0 min: 10%.

Data processing

The relationship between the concentration and peak area of the standard sample was ploted into a standard curve, and the concentration of the sample to be tested was calibrated using this standard curve.

$$\text{Compound solubility}=\text{concentration of the sample to be tested}\times 10$$

The solubilitys of the compounds are shown in table below.

| compound | solubility in water | solubility in diluted hydrochloric acid |
|---|---|---|
| **6** | 368 μg/mL | >3070 μg/mL |
| **7** | 680 μg/mL | >3770 μg/mL |
| **54** | 886 μg/mL | >3730 μg/mL |
| **75** | 120 μg/mL | >3500 μg/mL |
| **86** | 688 μg/mL | >3830 μg/mL |
| **97** | 180 μg/mL | >4330 μg/mL |

As can be seen from the above results, the compounds of the present disclosure have good solubility in water, especially in diluted hydrochloric acid simulating gastric acid.

**Examples of biological activity test**

**Example-1 of biological test: experiment of inhibiting kinase activity**

The inhibition rates of tested compounds on JAK1, JAK2, JAK3, TYK2, CDK7, CDK9, EGFR (T790M) and LRRK2 kinases were measured. The test concentrations of the compounds were 10 nM and 100 nM, and the detection was performed by replicate wells. Alternatively, compounds were 3-fold serial diluted from 10 μM for 10 concentrations in replicate wells.

The inhibitory effects of the compounds on JAK1, JAK2, JAK3, CDK7 and CDK9 kinases were detected by the method of Caliper mobility shift assay. 250 nL of the compound at 100-fold final concentration was transferred to a destination plate 384-well-plate by using a liquid handler Echo 550. A kinase solution at 2.5-fold final concentration was prepared with 1×kinase buffer. 10 μL of the kinase solution with 2.5-fold final concentration was added to compound wells and positive control wells; and 10 μL of 1×kinase buffer was added to negative control wells. The mixture was centrifuged at 1000 rpm for 30 seconds and then incubated at room temperature for 10 minutes. A mixed solution of ATP and kinase substrate at 5/3-fold final concentration was prepared with 1×kinase buffer, and 15 μL of the mixed solution of ATP and substrate at 5/3-fold final concentration was added to start the reaction. The 384-well plate was centrifuged at 1000 rpm for 30 seconds, shaked and mixed evenly, then incubated at room temperature for a corresponding time. 30 μL of stop solution was added to stop the kinase reaction, the mixture was centrifuged at 1000 rpm for 30 seconds, and shaked and mixed evenly. The conversion rate was read by Caliper EZ Reader. The conversion inhibition rate%=(average positive control conversion rate%-sample conversion rate%)/(average positive control conversion rate%-average negative control conversion rate%). Where negative control wells represent the conversion readings of the background well of kinase buffer; and positive control wells represent the conversion readings of wells without compound inhibition. With the log value of concentration as the x axis and the percentage inhibition rate as the y axis, the dose-response curve was fitted by

the log (inhibitor) vs. response-variable slope of the analyzing softwar GraphPad Prism 5, and the IC50 values of each compound on enzyme activity were obtained. Calculation formula is as follows: y=Bottom+(Top-Bottom)/(1+10^((LogIC50-x)×hillslope)), where x is logarithm of inhibitor concentration; and Y is % inhibition rate.

The inhibition rates of some compounds on some kinase activities at 100 nM are provided in Table 1 below.

(inhibition ranges: A>80%; B: 40-79%; C: 25-39%)

| Compound | JAK1 | JAK2 | JAK3 | CDK7 | CDK9 | LRRK2 | TYK2 |
|---|---|---|---|---|---|---|---|
| 1 | C | A | C | | | | |
| 2 | A | A | A | C | | A | A |
| 5 | | | | C | | | |
| 9 | C | A | B | | | B | B |
| 10 | C | A | A | A | B | A | A |
| 11 | | A | A | B | | B | A |
| 12 | | B | C | A | | A | |
| 13 | | B | | | | B | C |
| 15 | B | A | | | B | B | A |
| 16 | B | A | A | A | A | B | A |
| 19 | | A | B | B | | A | C |
| 20 | | A | B | B | | B | C |
| 21 | | B | | | | B | |
| 23 | | A | B | B | | B | C |
| 24 | | B | | B | | C | |
| 25 | B | A | B | B | | B | A |
| 26 | A | A | A | B | | A | A |
| 27 | A | A | A | | | A | A |
| 28 | A | A | | | | A | A |
| 29 | A | A | | | | B | A |
| 30 | C | A | A | B | B | C | B |
| 31 | | A | A | B | B | A | B |
| 32 | | A | A | B | C | A | B |
| 33 | | B | A | B | | A | |
| 35 | B | A | A | B | | A | B |
| 36 | | B | C | B | | A | |
| 37 | B | A | A | C | | C | A |
| 38 | B | A | C | | | B | B |
| 39 | | B | | C | | B | |
| 40 | C | A | C | | | C | B |
| 44 | A | A | B | B | A | B | A |
| 60 | | A | A | A | B | A | B |
| 64 | | C | | | | A | |
| 67 | | B | C | B | | A | |
| 97 | B | A | A | A | B | A | B |
| 99 | B | A | A | A | C | A | A |

(continued)

| Compound | JAK1 | JAK2 | JAK3 | CDK7 | CDK9 | LRRK2 | TYK2 |
|---|---|---|---|---|---|---|---|
| 85 | | | | | A | | |

The inhibition rates of some compounds on some kinase activities at 10 nM are provided in table 2 below.

(inhibition ranges: A>80%; B: 40-79%; C: 25-39%)

| Compound | JAK1 | JAK2 | JAK3 | CDK7 | CDK9 | LRRK2 | TYK2 | EGFR(T790M) |
|---|---|---|---|---|---|---|---|---|
| 1 | | B | | | | | | |
| 2 | | B | | | | | | |
| 9 | | A | | | | | | |
| 10 | | A | B | B | | A | | |
| 11 | | B | B | | | | C | |
| 12 | | | | B | | C | | |
| 15 | | B | | | | | C | |
| 16 | | B | B | C | B | | B | |
| 17 | B | A | B | | | B | B | |
| 19 | | B | | | | B | | |
| 25 | | B | | | | | B | |
| 26 | A | A | A | | | A | A | |
| 27 | B | A | B | | | | B | |
| 28 | A | A | | | | B | A | |
| 30 | | B | C | | | | | |
| 31 | | B | B | | C | B | | |
| 32 | | B | A | | | A | C | |
| 33 | | | C | | | A | | |
| 35 | | B | C | | | A | | |
| 37 | | B | B | | | | B | |
| 40 | | A | | | | | | |
| 41 | | B | C | | | | | |
| 42 | | A | | C | | | A | |
| 43 | | A | C | | | C | B | |
| 44 | C | A | | | B | | B | |
| 48 | A | A | A | | | A | A | |
| 49 | A | A | A | | | B | A | |
| 50 | | A | | | | | C | |
| 51 | B | A | B | | | C | C | |
| 53 | | B | B | A | | A | | |
| 54 | | | | | | | | A |
| 55 | | C | A | | | | | |
| 56 | | | | | | | | A |
| 60 | | B | B | A | | B | | |

(continued)

| Compound | JAK1 | JAK2 | JAK3 | CDK7 | CDK9 | LRRK2 | TYK2 | EGFR(T790M) |
|---|---|---|---|---|---|---|---|---|
| 61 | | B | B | | | A | | |
| 63 | | B | | | | B | | |
| 69 | | B | C | | | C | | |
| 72 | C | B | | | | | | |
| 73 | | A | A | | | A | B | |
| 74 | C | A | A | | | B | A | |
| 75 | A | A | A | | | A | A | |
| 76 | B | A | B | | | B | A | |
| 77 | A | A | A | | | A | A | |
| 79 | B | A | | | | B | A | |
| 82 | | B | | | | A | B | |
| 83 | C | A | | | | A | B | |
| 84 | | | | | | B | | |
| 97 | | A | B | B | | A | | |
| 98 | | B | B | C | | A | | |
| 99 | C | B | B | B | | A | C | |
| 111 | | | | | | | | |
| 112 | | | | B | | | | |
| 114 | | | | | B | | | |
| 115 | | | | | B | | | |

$IC_{50}$ ranges of some compounds in some kinase activity assays are provided in Table 3 below.

($IC_{50}$ ranges: A<0.05 μM; B: 0.05-0.2 μM; C: 0.2-1 μM).

| Compound | LRRK2 | CDK9 | CDK7 | JAK1 | JAK2 | JAK3 |
|---|---|---|---|---|---|---|
| 1 | | | | | A | |
| 2 | A | | | A | A | A |
| 6 | A | | | | | |
| 9 | | | | | A | |
| 10 | A | | | | | |
| 12 | A | | A | | | |
| 13 | B | | | | A | B |
| 15 | B | | | A | A | A |
| 16 | B | A | | B | A | A |
| 19 | A | | A | | A | |
| 21 | B | | | | A | |
| 25 | B | | | | A | A |
| 27 | A | | | A | A | A |
| 28 | A | | | A | A | A |
| 29 | B | | | A | A | A |

(continued)

| Compound | LRRK2 | CDK9 | CDK7 | JAK1 | JAK2 | JAK3 |
|---|---|---|---|---|---|---|
| 32 | A | | | | | |
| 33 | A | | | | | |
| 35 | A | | A | B | A | A |
| 36 | A | | | | | |
| 37 | | | | | A | A |
| 38 | B | | | | A | |
| 39 | A | | | | | |
| 44 | | A | | | | |
| 61 | A | | | | | |
| 73 | A | | | | | |
| 82 | A | | | | | |
| 83 | A | | | | | |
| 84 | A | | | | | |
| 85 | A | | | | | |
| 98 | A | | | | | |
| 114 | | A | | | | |
| 115 | | A | | | | |

**Example-2 of Biological test: experiment of inhibiting LRRK2 kinase activity**

The inhibitory effect of the compounds on the activity of LRRK2 kinase was detected by ADP-Glo method. The diluted solutions of the compounds were transfered to a 384-well plate (784075, Greiner), the plate was sealed and centrifuged at a speed of 1000 g for 1 minute. A kinase solution at 2-fold final concentration was prepared with 1×kinase buffer, 2.5 μl of the kinase solution with 2-fold final concentration was added to the wells, the plate was centrifuged at 1000 g for 30 seconds, and left at room temperature for 10 minutes. A mixed solution of ATP and kinase substrate at 2-fold final concentration was prepared with 1×kinase buffer, and 2.5 μL of mixed solution of ATP and substrate at 2-fold final concentration was added to start the reaction. The plate was centrifuged at 1000 g for 30 seconds, sealed and left at room temperature for 2 hours. 4 μL of ADP-Glo reagent was added and incubated at room temperature for 40 minutes; 8 μL kinase detection reagent was added and incubated at room temperature for another 40 minutes. The luminescence signal value was detected on Envision 2104 microplate reader. The conversion inhibition rate%=100-(compound signal value-positive control signal value)/(blank control signal value-positive control signal value)×100.

The IC50 was calculated with GraphPad 6.0 and the dose-responsive curve of the compounds was plotted as follows: y=Bottom+(Top-Bottom)/(1+10^((logic50-x)×hillslope)), where x was logarithm of inhibitor concentration; and y was % inhibition rate.

The $IC_{50}$ ranges of some compounds in the mesurement of LRRK2 kinase activity are provided in Table 4 below.

($IC_{50}$ ranges: A<0.1 μm; B: 0.1-0.5 μM; C: 0.5-1 μM).

| Compound | $IC_{50}$ |
|---|---|
| 7 | A |
| 86 | A |
| 87 | A |
| 91 | A |
| 92 | A |
| 93 | A |
| 95 | A |

(continued)

| Compound | $IC_{50}$ |
|---|---|
| 100 | B |
| 102 | A |
| 103 | B |
| 104 | A |
| 105 | A |
| 108 | C |
| 109 | B |
| 116 | A |

**Example-3 of biological test: experiment of inhibiting EGFR kinase activity**

The inhibitory effect of the compounds on EGFR kinase activity was measured by TR-FRET method. The diluted solutions of the compounds were transfered to a 384-well plate (784075, Greiner), the plate was sealed and centrifuged at a speed of 1000 g for 1 minute. Wild-type EGFR kinase protein at 2-fold final concentration was prepared in 1×kinase buffer, 5 μL of wild-type EGFR at 2-fold final concentration was added to 384-well plate, the well plate was centrifuged at 1000 g for 30 seconds, and left at room temperature for 10 minutes. A mixture of 2-fold TK-substrate-biotin (2 μM) and ATP was prepared in 1×kinase buffer, and 5 μL of the mixture of 2×TK-substrate-biotin (2 μM) and ATP was added to start the reaction. The well plate was centrifuged at 1000 g for 30 seconds, sealed and left at room temperature for 40 minutes. 4×Sa-XL 665 was prepared in HTRF detection buffer, 5 μL of Sa-XL 665 and 5 μL of TK-antibody-Cryptate were added to the well plate, the well plate was centrifuged at the speed of 1000 g for 30 seconds, and left at room temperature for 1 hour. The fluorescence signal values at 615 nm (Cryptate) and 665 nm (XL665) were detected by using Envision 2104 microplate reader. The conversion inhibition rate%=100-(compound signal value-positive control signal value)/(blank control signal value-positive control signal value)×100. The IC50 was calculated with GraphPad 8.0 and the dose-reponse curve of the compound was plotted as follows: y=Bottom+(Top-Bottom)/(1+10^((logic50-x)×hillslope)), where x was logarithm of inhibitor concentration; and y was % inhibition rate.

The $IC_{50}$ ranges of some compounds in the mesurement of EGFR kinase activity are provided in Table 5 below.

($IC_{50}$ ranges: A<0.05 μM; B: 0.05-1 μM; C: 1-5 μM).

| Compound | EGFR $IC_{50}$ |
|---|---|
| 54 | A |
| 56 | A |

**Example-4 of biological test**

CDK9 cell activity test

In these experiments, the cell line MV-4-11 was cultured in IMDM culture solution (containing 100 U/mL penicillin and 0.1 g/L streptomycin), the cell line HCC1187 was cultured in RPMI-1640 culture solution (containing 100 U/mL penicillin and 0.1 g/L streptomycin) containing 10% fetal bovine serum, the cell line Mia Paca-2 was cultured in DMEM (containing 100 U/mL penicillin and 0.1 g/L streptomycin) containing 10% fetal bovine serum and 2.5% horse serum, and the cell line A375 was cultured in RPMI-1640 culture solution (containing 100 U/mL penicillin and 0.1 g/L streptomycin) containing 10% fetal bovine serum. All cells were cultured in a saturated humidity incubator with 37 °C and 5% $CO_2$.

When MV-4-11 cells, HCC-1187 cells, Mia Paca-2 cells and A375 cells grew to 80% -90% confleuncy, they were digested with trypsin, resuspended, counted and diluted to a certain cell density with complete medium, and inoculated into 96-well plate at 100 μL/well. With complete medium as background control, they were cultured overnight in a saturated humidity incubator with 37°C and 5% $CO_2$. The compound was diluted to 200-fold of the final concentration using DMSO, 3 μL of DMSO storage solution of the above compound was added to 197 μL of complete medium, 50 μL of compound diluted in medium was added to cells in each well, and cultivated in a saturated humidity incubator with 37°C and 5% $CO_2$ for 72 hours. After 72 hours, the culture plate was placed at room temperature for equilibrium, and 40 μl of Cell Titer-GLO® reagent was added to each well. The cells were lysed by mixing evenly for 2 minutes using a shaker, incubated at room

temperature for 60 minutes to stabilize the chemiluminescence signal, and the chemiluminescence was read by PE EnVision microtiter plate reader. The cell growth inhibition rate Inh%=(DMSO well - compound treatment well)/(DMSO well - background well)×100 was calculated, and the cell growth IC50 value was obtained by fitting with GraphPad Prism 5 .

$IC_{50}$ ranges of some compounds in cell activity measurement are provided in Table 6 below.

($IC_{50}$ ranges: A<0.1 μM; B: 0.1-1 μM; C: 1-5 μM), NT: not tested.

| Compound | MV-4-11 | HCC1187 | Mia-Paca-2 | A375 |
|---|---|---|---|---|
| | $IC_{50}$ (nM) | | | |
| 16 | A | C | B | B |
| 44 | A | NT | NT | NT |
| 114 | B | NT | NT | NT |

**Example-5 of biological test**

JAKs cell activity test

Detection of pSTAT6 in THP1 cells induced by JAK1_IL-4

THP1 cells were collected by centrifugation, resuspended with 1×HBSS, counted and diluted to a certain cell density. They were inoculated in a 384-well plate and cultured in a saturated humidity incubator with 37 °C and 5% $CO_2$. The compound (DMSO final volume of 0.3%) was added to THP1 cells (10 doses in duplicates) and cultured in a saturated humidity incubator with 37 °C and 5% $CO_2$. hIL-4 was added to stimulate cells and cultured in a saturated humidity incubator with 37 °C and 5% $CO_2$. The lysis solution was added and incubated at room temperature. According to the instructions of AlphaLISA SureFire Ultra p-STAT6 detection kit, the lysis solution was transferred to pSTAT6 detection system, and the ALPHA signal was read by PE Envision microplate reader. The inhibition rate Inh%=((DMSO-sample)/(DMSO-background))×100 was calculated. IC50 was obtained by fitting with GraphPad Prism non-linear regression equation, and y=Bottom+(Top-Bottom)/(1+10^((LogIC50-x)×hillslope)), where x was the concentration of the compound and y was the inhibition rate.

JAK2_pSTAT5 was tesed in IL-3-induced BAF3 cells according to the above steps, and JAK3_pSTAT5 was tested in IL-2-induced CTLL-2 cells (without serum stimulation) according to the above steps.

IC50 ranges of some compounds in cellular activity measurement are provided in Table 7 below.

($IC_{50}$ ranges: A<0.05 μM; B: 0.05-0.5 μM; C: 0.5-5 μM), NT: not tested.

| Compound | JAK1, EC50 | JAK2, EC50 | JAK3, EC50 |
|---|---|---|---|
| 2 | A | NT | NT |
| 16 | B | | NT |
| 26 | A | C | C |
| 27 | A | B | A |
| 28 | A | B | A |
| 54 | | | B |
| 55 | | | B |

**Example-6 of biological test**

**Detection of phosphorylated S935 in HEK293T Overexpressing full-length LRRK2 (G2019S) or wild-type LRRK2**

HEK293T cells was inoculated into a 6-well plate and cultured overnight. The next day, they were transiently transfected with plasmid pcDNA5-FRT/TO-FLAG LRRK2 (G2019S, full-length) or pCMV-flag-WT LRRK2 (full-length) by using Superfect transfection reagent and cultured at 37°C and 5% CO2 for 20-24 hours. The next day, cells were collected, resuspended at a density of 0.48×10^6 cells/ml, and inoculated into a 384-well plate at 50 microliters/well. Compound or

DMSO solutions (volume ratio of 0.5%) were added by Tecan, and cultured at 37°C and 5% CO2 for 1.5 hours. After 1.5 hours, the supernatant was discarded, 50 microliters of 8% PFA were added in each well to fix the cells, and the cells were incubated at room temperature for 1 hour. The plate was washed with EL406 plate washer for three times, with 115 microliters of PBST per well, and the culture plate was patted to dryness. The cells were blocked with 50 microliters/well of Licor Odyssey blocking solution (0.1% Tween 20) at room temperature for 1.5 hours. The blocking solution was discarded and the culture plate was patted to dryness. 20 microliters of primary antibody (1:1000(pS935), diluted with Licor Odyssey buffer (0.1% Tween20)) was added into each well, and incubated at 4 °C overnight. The next day, the first antibody was discarded and washing was performed with PBST for five times. 20 microliters of secondary antibody (diluted with Licor Odyssey buffer (0.1% Tween20) at 1:500 and stained with DNA at 1:2000) were added into each well, and incubated at room temperature for 1 hour. The second antibody was discarded, and washing was performed for three times with PBST. The fluorescence intensity was measured by LICOR Odyssey imager Scanner, and the data was analyzed.

The $IC_{50}$ ranges of some compounds in the activity test of LRRK2 (G2019S) or wild-type LRRK2 cells are provided in Table 8 below.

(IC50 ranges: A<0.5 μM; B: 0.5-1 μM; C: 1-5 μM).

| Compound | $EC_{50}$ WT | $EC_{50}$ G2019S |
|---|---|---|
| 2 | A | NT |
| 6 | A | A |
| 28 | A | NT |
| 73 | A | A |
| 82 | A | A |
| 83 | A | A |
| 84 | C | NT |

**Example-7 of biological test**

**Detection of endogenous phosphorylated LRRK2 (S935) in A549 cells**

A549 cells were resuspended in serum-free 1640 medium at a density of 1×10^6 cells/ml, and inoculated into a 6-well plate at 1 ml/well, and cultured at 37°C and 5% CO2 for 6-8 hours. 1 microliter of compound solution in DMSO or DMSO (volume ratio of 0.1%) was added, mixed evenly, and cultivated at 37°C and 5% CO2 for 15 hours. Cells were collected, 50 microliters of RIPA III lysis solution were added to each sample to lyse cells on ice for 15 minutes, centrifuged at 4°C and 13000 rpm for 30 minutes, the supernatant was collected, the protein concentration of cell lysis solution was measured by BCA, and the protein concentrations of all samples were normalized. 5×sample loading buffer were added and kept at 95°C for 10 minutes. The content of phosphorylated S935 of endogenous LRRK2 was detected by Western blot, using antibody against phosphorylated LRRK2 (S935) (Abcam#133450). The signal intensity of phosphorylated LRRK2 (S935) was counted by Image J, the signal of phosphorylated LRRK2 (S935) in a sample was normalized by β-actin, and the data was analyzed.

The inhibition rates of some compounds at 2 μM on LRRK2 (S935) activity in A549 cells are provided in Table 9 below.

(Inhibition range: A>80%; B: 40-79%; C: 25-39%;)

| Compound | Inhibition rate |
|---|---|
| 7 | A |
| 8 | A |
| 88 | A |
| 89 | B |
| 90 | A |
| 92 | B |

The inhibition rates of some compounds at 1 μM on LRRK2 (S935) activity in A549 cells are provided in Table 10

below.

(inhibition range: A> 0%; B: 40-79%; C: 25-39%;)

| Compound | Inhibition rate |
|---|---|
| 94 | A |
| 96 | A |
| 100 | B |
| 101 | B |
| 102 | A |

$IC_{50}$ ranges of inhibitory activity of some compounds on LRRK2 (S935) activity in A549 cells are provided in Table 11 below.

($IC_{50}$ ranges: A<0.1 μM; B: 0.1-0.5 μM; C: 0.5-5 μM).

| Compound | Inhibition rate |
|---|---|
| 91 | A |
| 93 | B |
| 103 | A |

## Claims

**1.** A compound represented by formula (I),

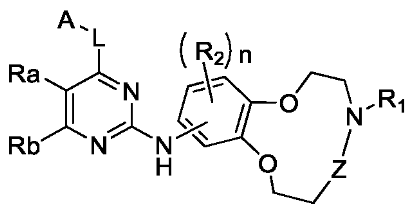

(I)

wherein,

Z represents absence (covalent bond) or $-OCH_2CH_2-$;

L represents absence (covalent bond), -O-, -NH- or -N($C_{1-4}$ alkyl)-;

A is $C_{6-14}$ aryl, 5-12 membered heteroaryl, $C_{1-10}$ alkyl, $C_{3-8}$ cycloalkyl, 3-8 membered heterocyclyl or 5-18 membered bridged ring group, optionally substituted with one or two substituents, wherein the substituent is selected from the group consisting of deuterium, $C_{1-4}$ alkyl, $C_{1-10}$ alkoxy, $C_{3-6}$ cycloalkyl, 3-8 membered heterocyclyl, $C_{1-4}$ alkylphosphoryl, $C_{1-4}$ alkylsulfonyl, aminosulfonyl, $C_{1-4}$ alkylaminosulfonyl, cyano $C_{3-6}$ cycloalkyl, $C_{2-4}$ alkenylformyl, $C_{2-4}$ alkenylformamido, cyano $C_{1-4}$ alkylcarbamoyl, cyano $C_{1-4}$ alkyl, $C_{1-4}$ alkylformyl-3-8 membered heterocyclyl, $C_{3-6}$ cycloalkylformyl, 3-8 membered heterocyclyl-sulfonyl, $C_{1-4}$ alkylsulfonyl-3-8 membered heterocyclyl, amino $C_{3-6}$ cycloalkylformylamino, $C_{1-4}$ alkylphosphoramido, $C_{1-4}$ alkylsulfonamido, cyano, hydroxyl, oxo, mercapto, amino, $C_{2-4}$ alkenyl and halogen;

$R_1$ is hydrogen, or optionally substituted $C_{1-4}$ alkyl, $C_{3-6}$ cycloalkyl, $C_{1-4}$ alkyl-CO-, -CHO or $C_{1-4}$ alkylsulfonyl, wherein the substituent is selected from the group consisting of deuterium, $C_{1-4}$ alkyl, $C_{1-10}$ alkoxy, $C_{3-6}$ cycloalkyl, oxo, cyano, hydroxyl, amino, dimethylamino, and hydroxylamino;

$R_2$ is independently selected from the group consisting of hydrogen, $C_{1-3}$ alkyl, trifluoromethyl, $C_{1-3}$ alkoxy, cyano and halogen;

n is 1 or 2;

Ra is selected from the group consisting of hydrogen, amino, trifluoromethyl, halogen, cyano, $C_{1-3}$ alkyl, acetyl, $C_{1-3}$ alkylphosphoryl and $C_{1-3}$ alkylsulfonyl;

Rb is selected from the group consisting of hydrogen, amino and $C_{1-3}$ alkylamino; and
Ra, Rb and the carbon atom to which they are linked can together form a 5-6 membered aromatic ring, 5-12 membered heteroaromatic ring or 5-8 membered heterocyclic ring;
or a stereoisomer, tautomer or pharmaceutically acceptable salt thereof.

**2.** The compound represented by formula (I) or the stereoisomer, tautomer or pharmaceutically acceptable salt thereof according to claim 1, wherein the compound represented by formula (I) has a structure represented by formula (II),

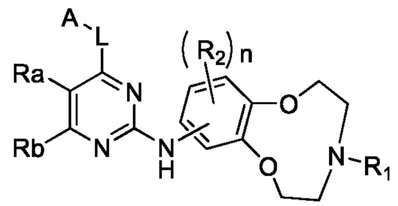

(II)

wherein L, A, $R_1$, $R_2$, Ra, Rb and n have the definitions as in claim 1.

**3.** The compound represented by formula (I) or the stereoisomer, tautomer or pharmaceutically acceptable salt thereof according to claim 1, wherein the compound represented by formula (I) has a structure represented by formula (III),

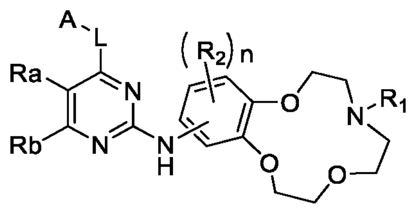

(III)

wherein L, A, $R_1$, $R_2$, Ra, Rb and n have the definitions as in claim 1.

**4.** The compound represented by formula (I) or the stereoisomer, tautomer or pharmaceutically acceptable salt thereof according to claim 1, wherein the compound represented by formula (I) has a structure represented by formula (IV),

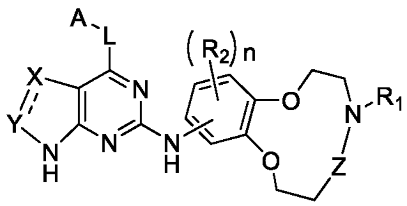

(IV)

wherein L, A, $R_1$, $R_2$ and n have the definitions as in claim 1;
X and Y are each independently selected from the group consisting of C and N; and
Z represents absence (covalent bond) or $-OCH_2CH_2-$.

**5.** The compound or the stereoisomer, tautomer or pharmaceutically acceptable salt thereof according to any one of claims 1 to 4, wherein A is selected from the group consisting of:

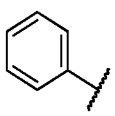 , 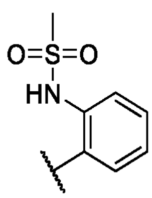 , 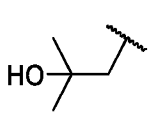 , 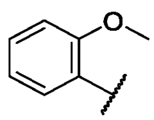 , 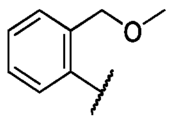 , 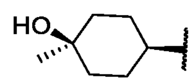 ,

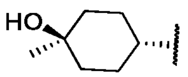, 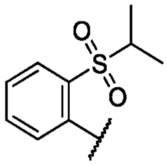, 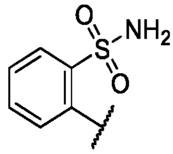, 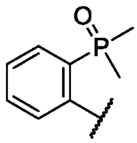, 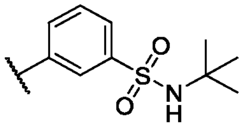, 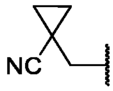,

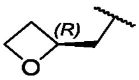, 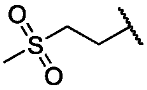, 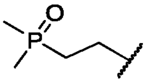, 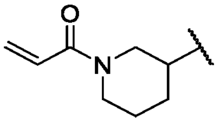, 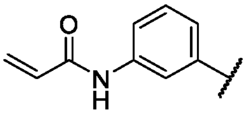,

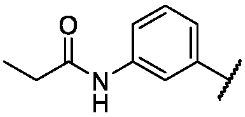, 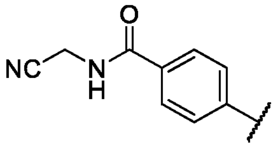, 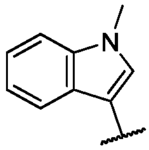, 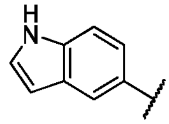, 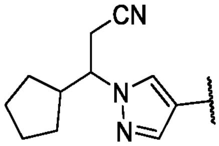,

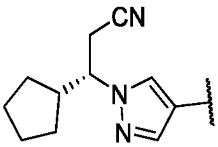, 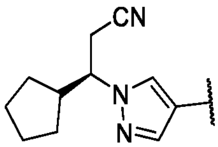, 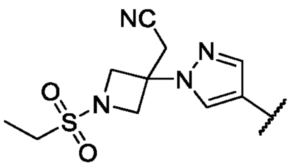, 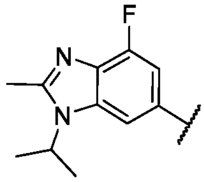,

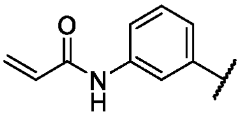, 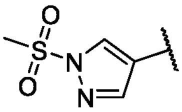, 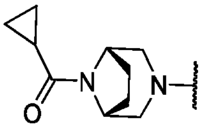, 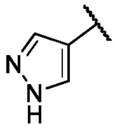, 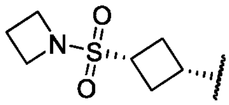,

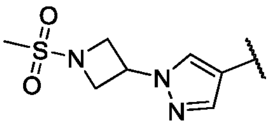, 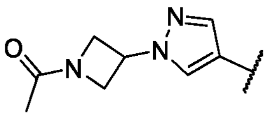, 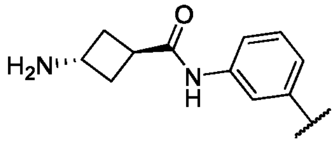,

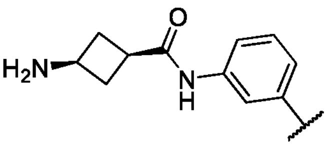, 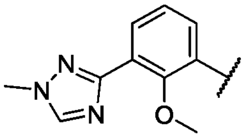, 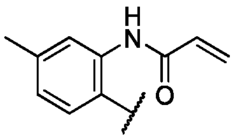, 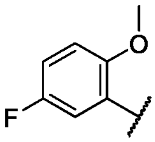,

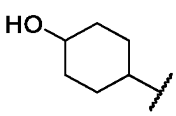, and 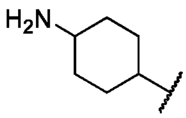.

6. The compound or the stereoisomer, tautomer or pharmaceutically acceptable salt thereof according to any one of claims 1 to 5, wherein the compound is selected from the group consisting of:

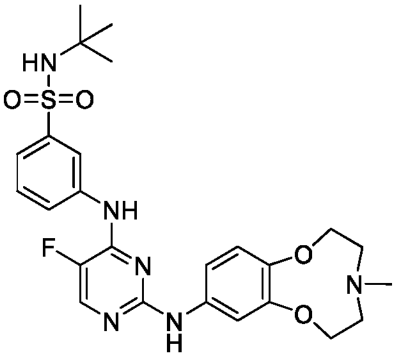

**1**

,

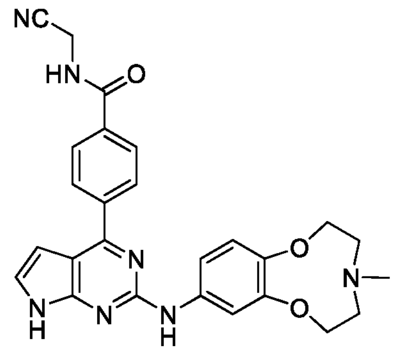

**2**

,

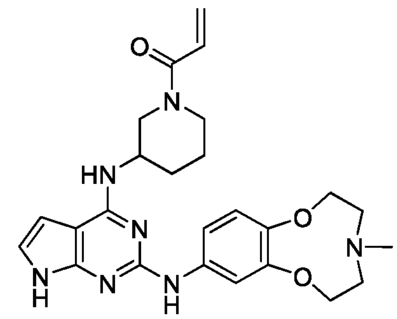

**3**

,

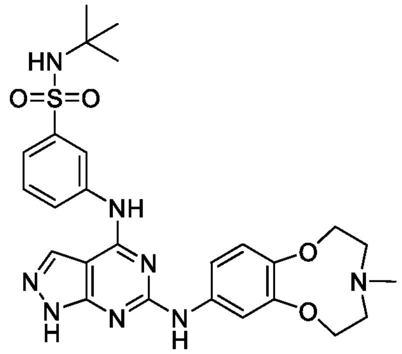

**4**

,

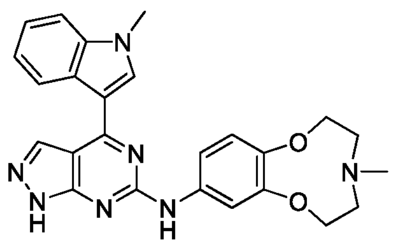

**5**

,

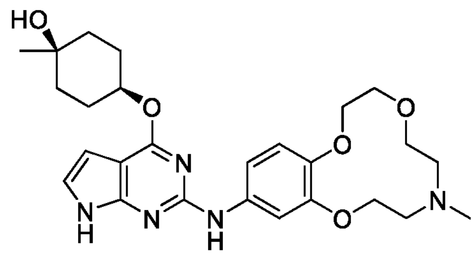

**6**

,

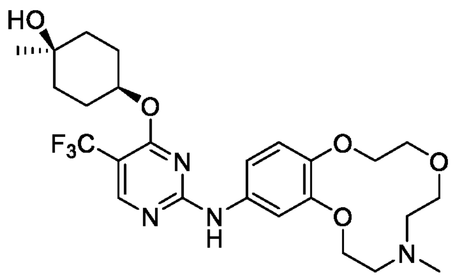

**7**

,

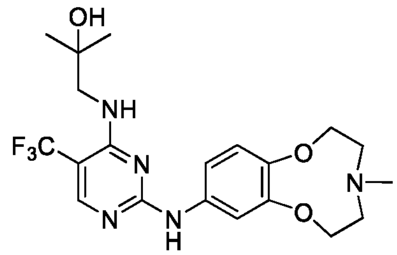

**8**

,

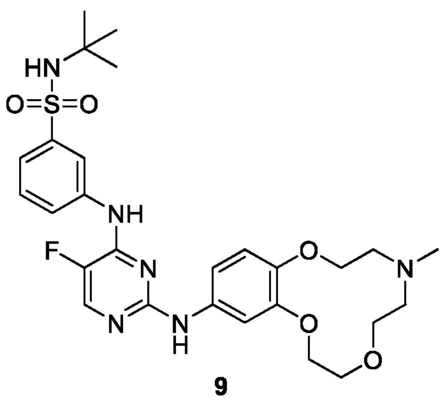

9

,

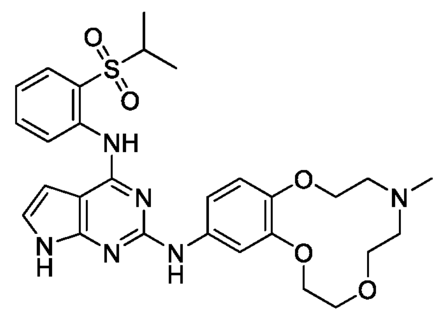

10

,

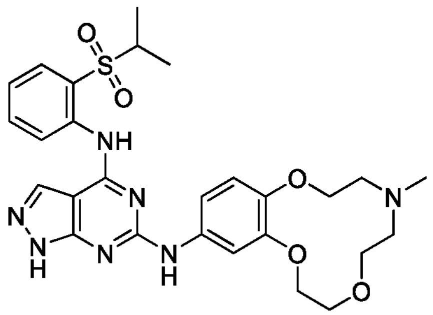

11

,

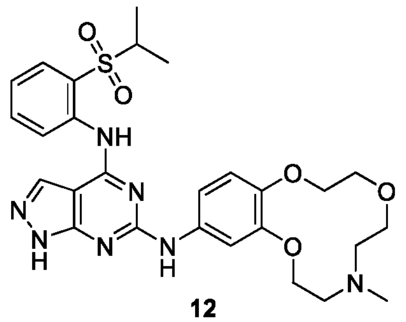

12

,

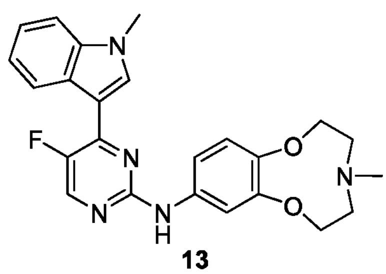

13

,

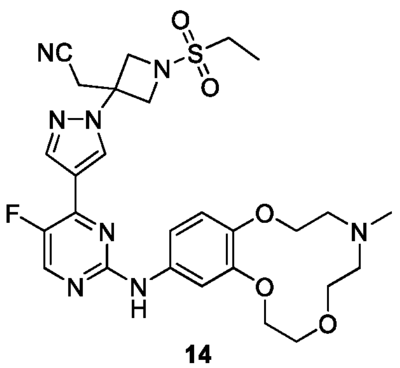

14

,

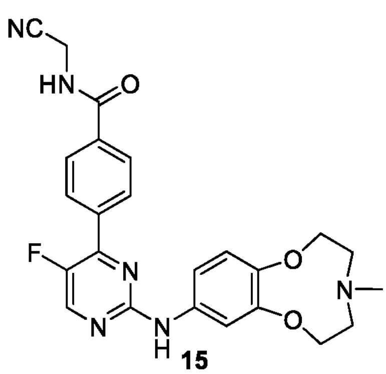

15

,

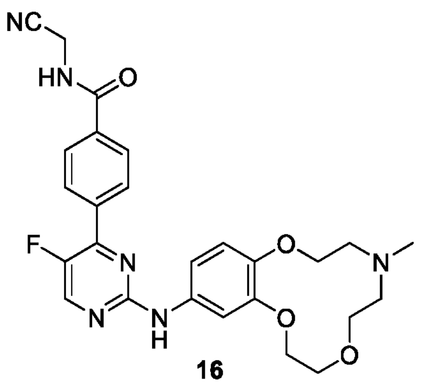

16

,

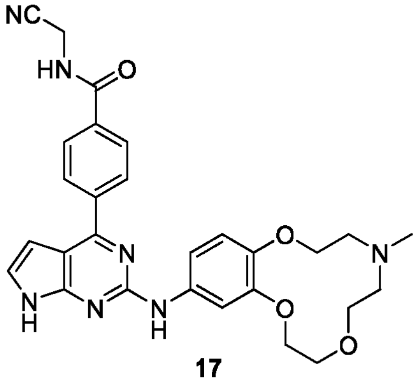

17

,

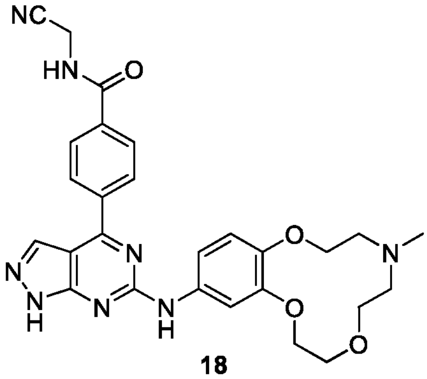

18

,

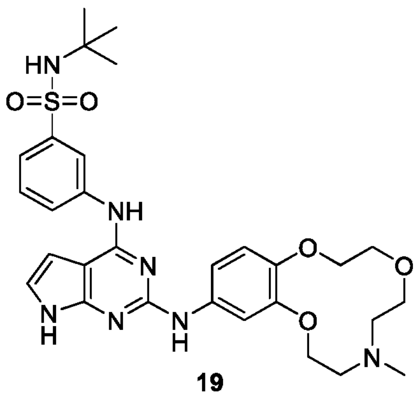

19

,

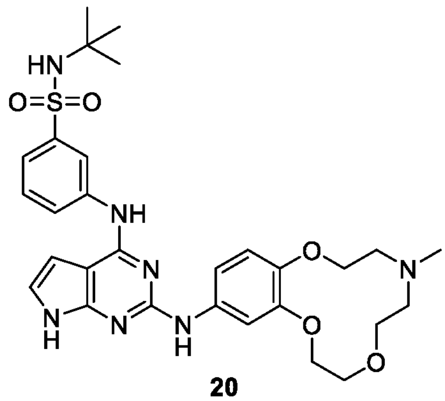

20

,

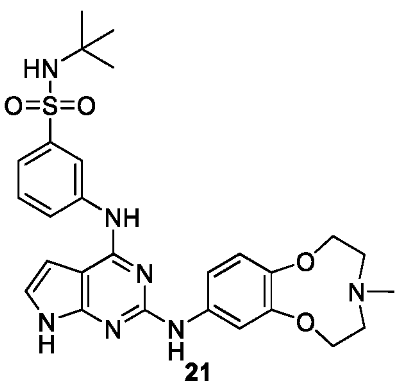

21

,

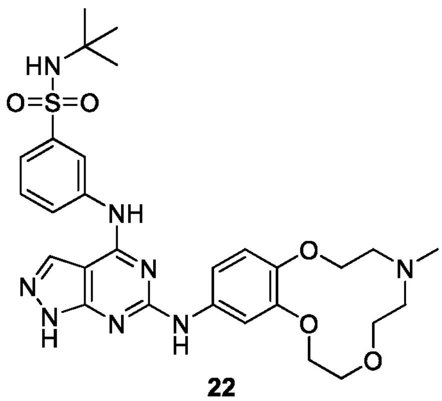

22

,

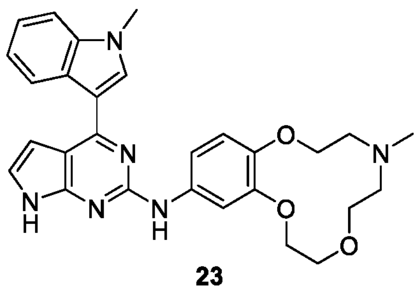

23

,

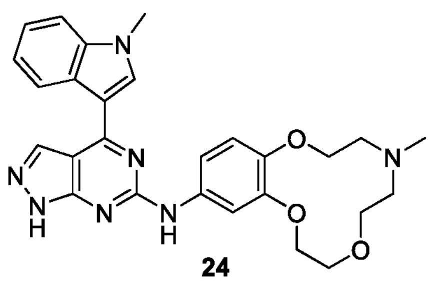

24

,

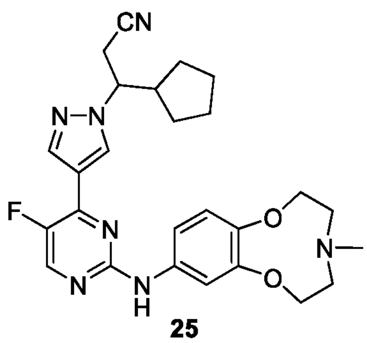

**25**

,

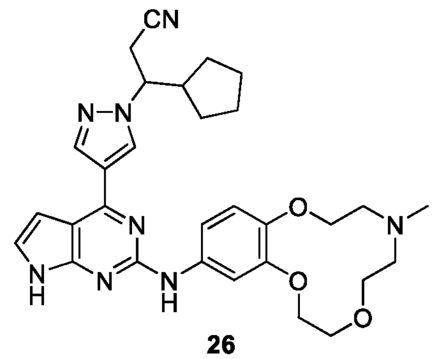

**26**

,

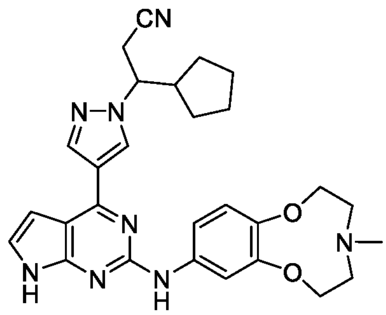

**27**

,

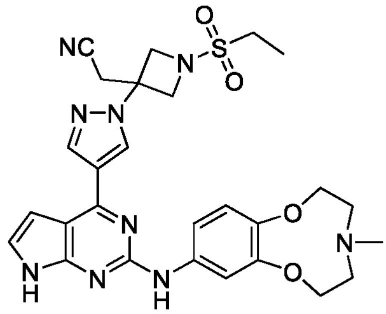

**28**

,

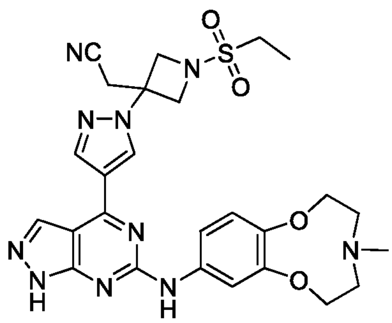

**29**

,

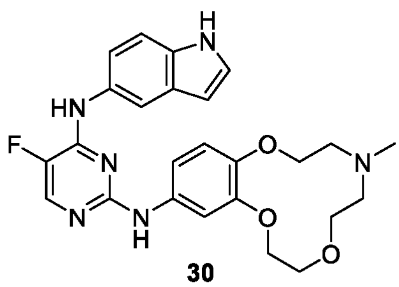

**30**

,

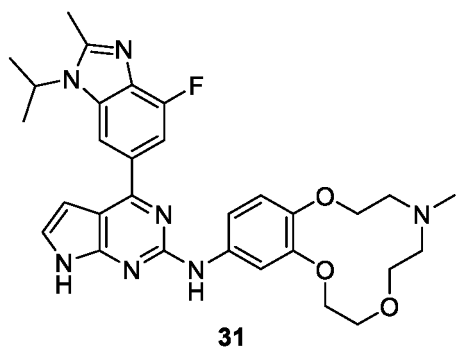

**31**

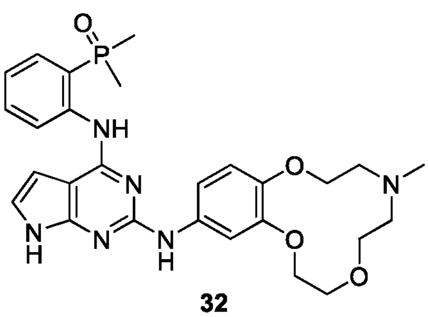

**32**

, ,

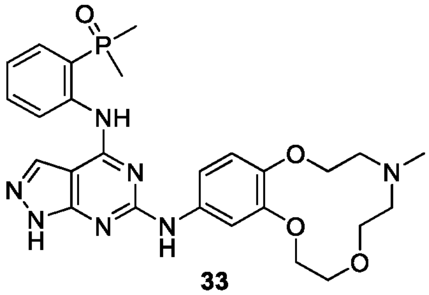

**33**

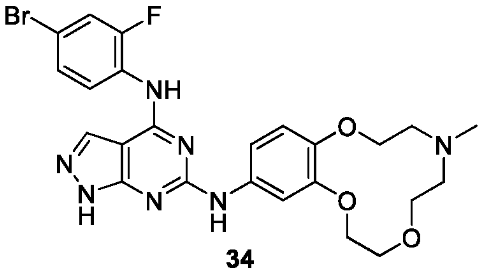

**34**

, ,

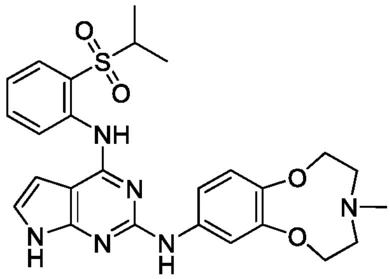

**35**

,

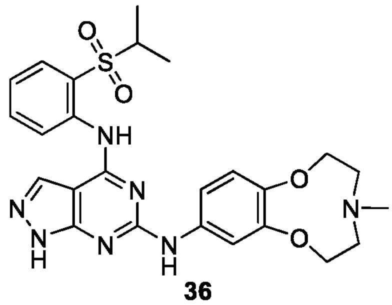

**36**

,

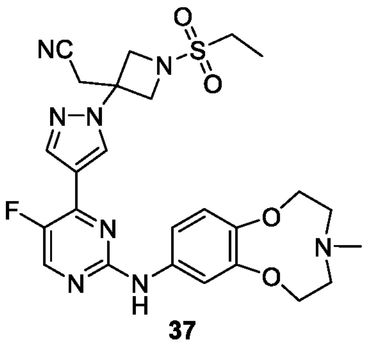

**37**

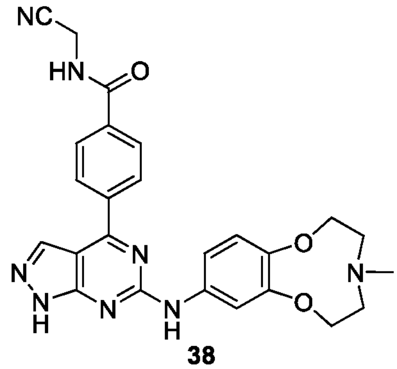

**38**

, ,

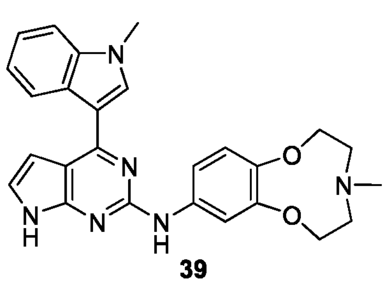

**39**

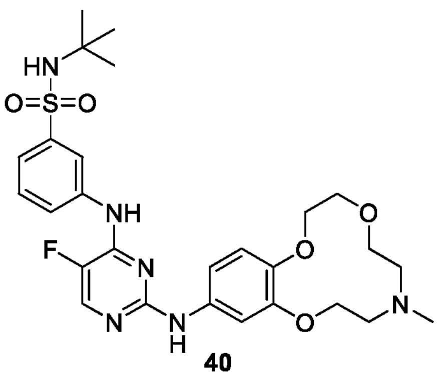

**40**

,

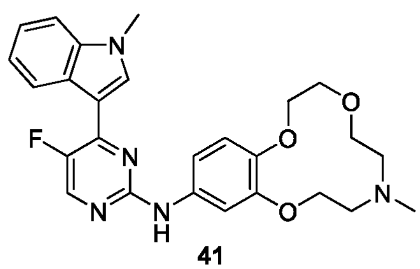

41

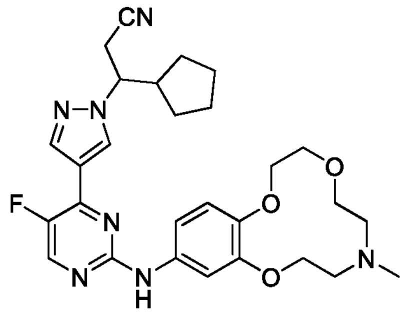

42

,

,

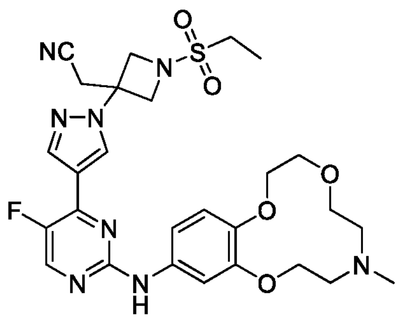

43

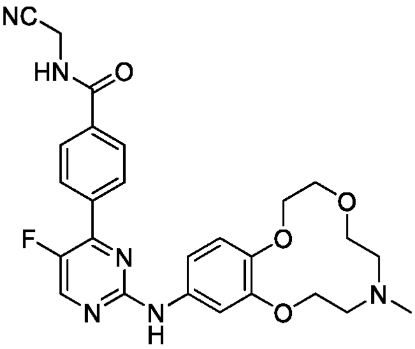

44

,

,

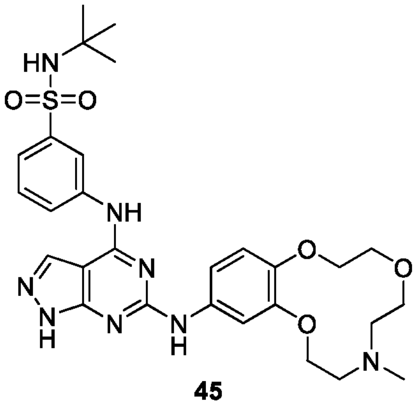

45

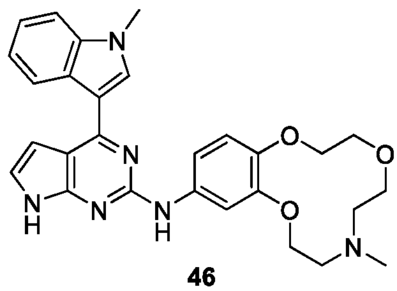

46

,

,

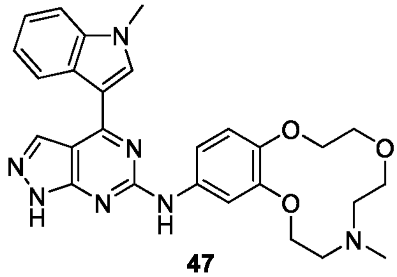

47

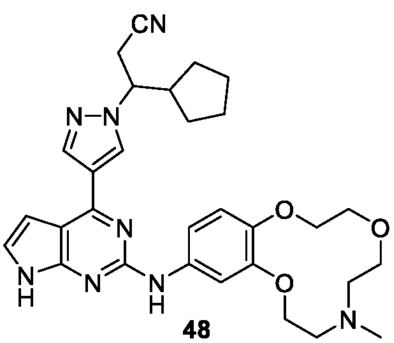

48

, ,

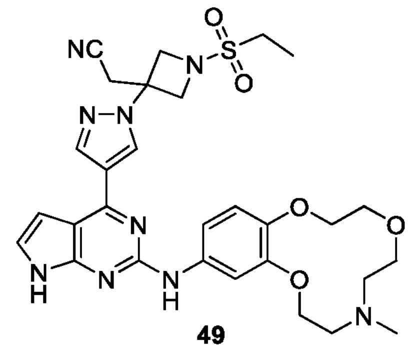

49

,

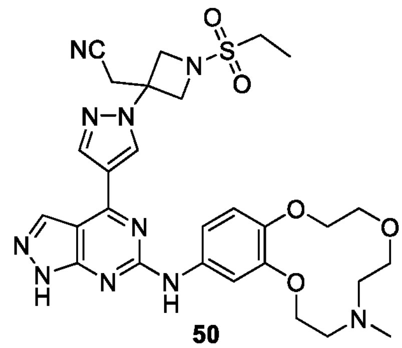

50

,

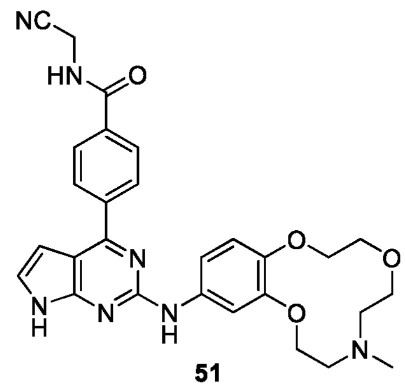

51

,

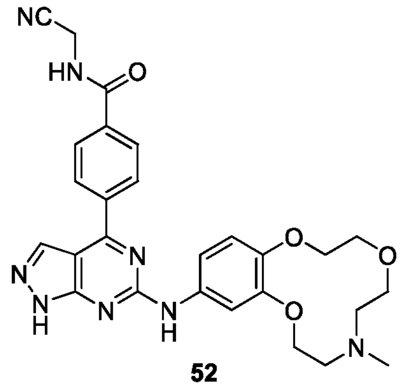

52

,

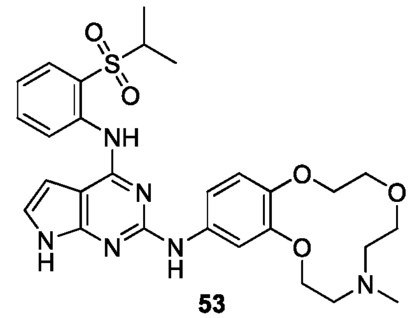

53

,

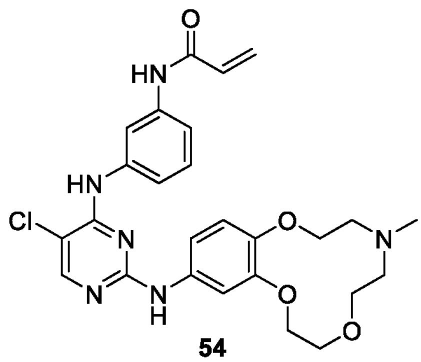

54

,

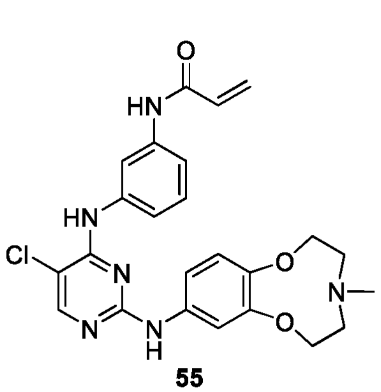

55

,

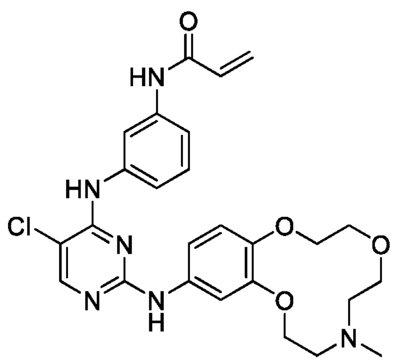

56

,

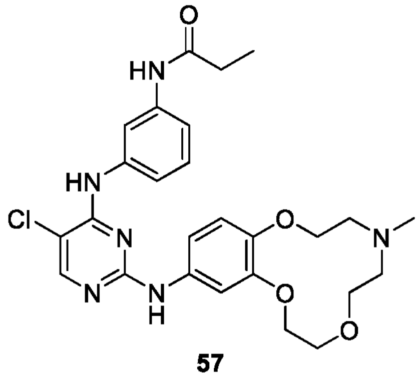

**57**

,

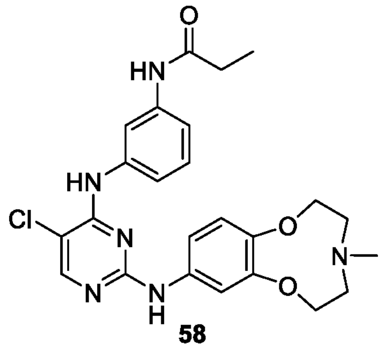

**58**

,

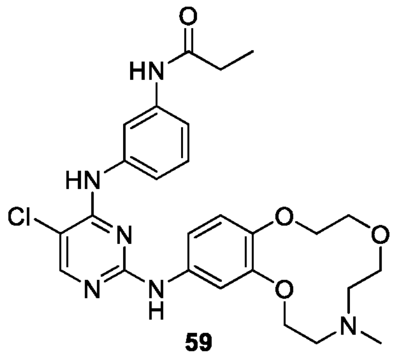

**59**

,

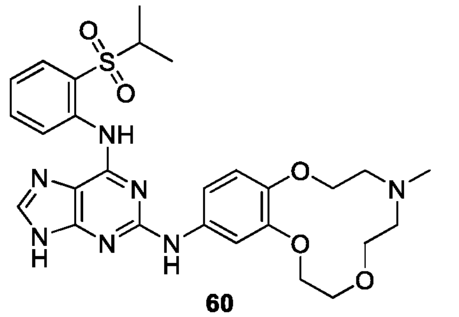

**60**

,

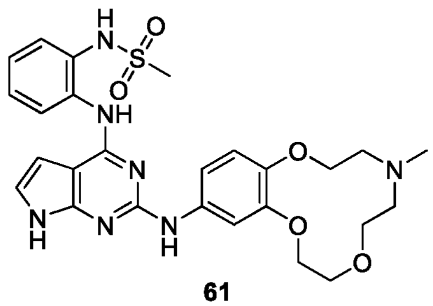

**61**

,

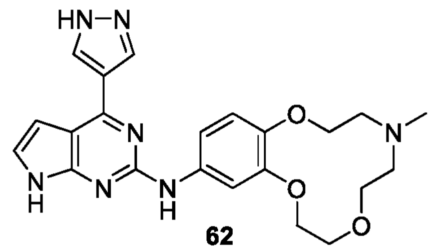

**62**

,

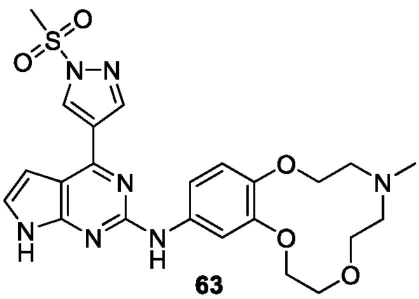

**63**

,

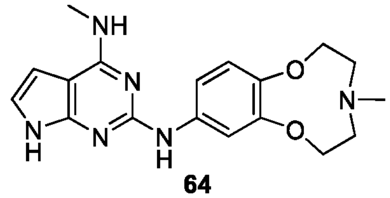

**64**

,

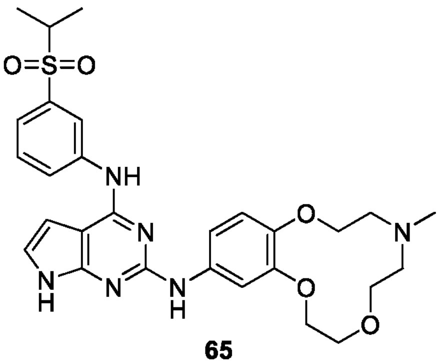

**65**

,

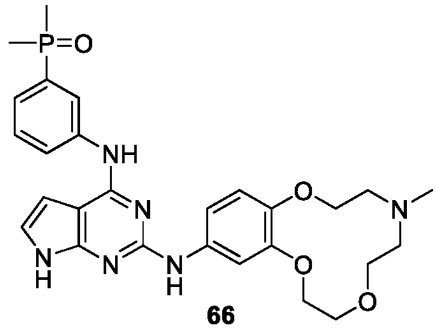

**66**

,

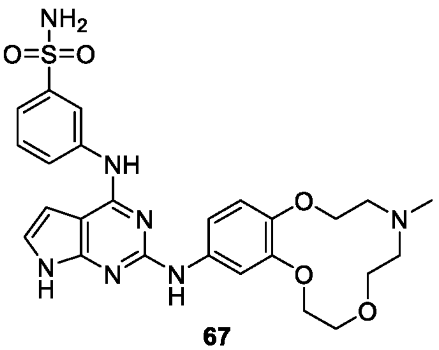

**67**

,

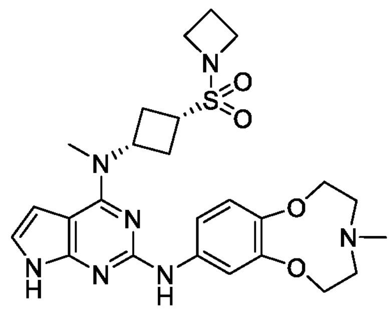

**68**

,

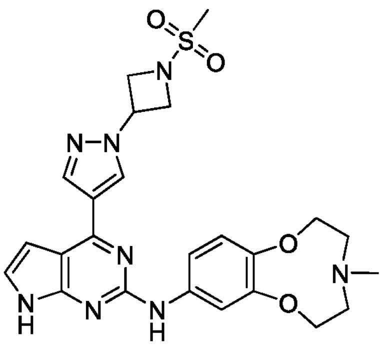

**69**

,

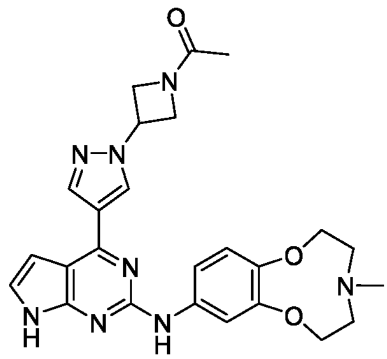

**70**

,

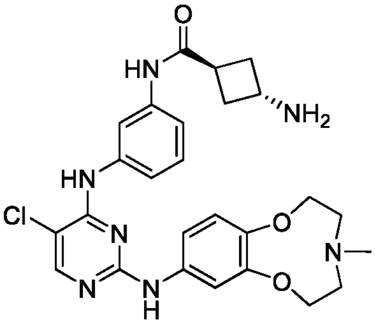

**71**

,

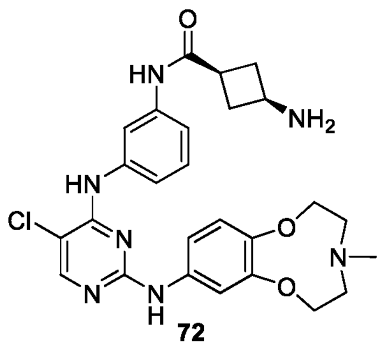

**72**

,

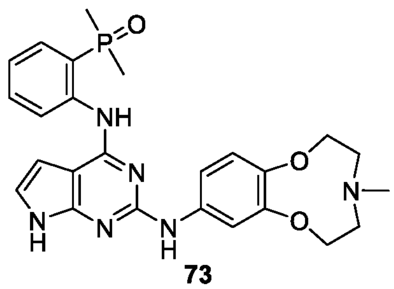

73

,

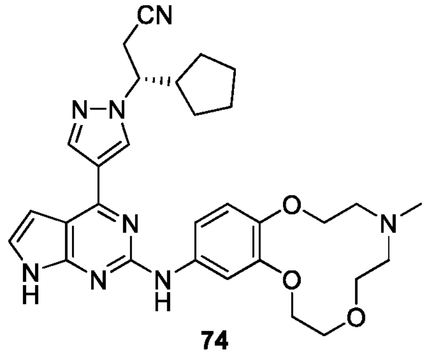

74

,

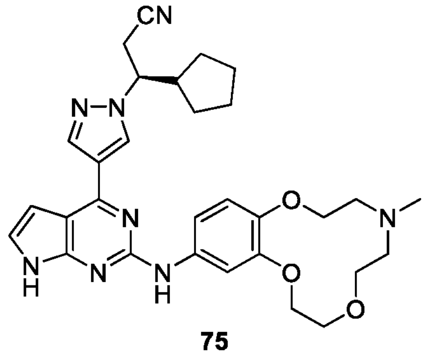

75

,

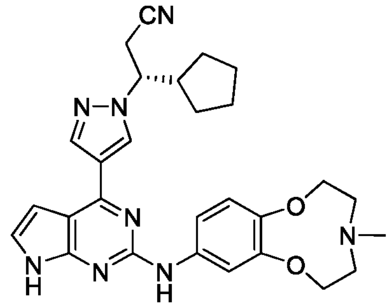

76

,

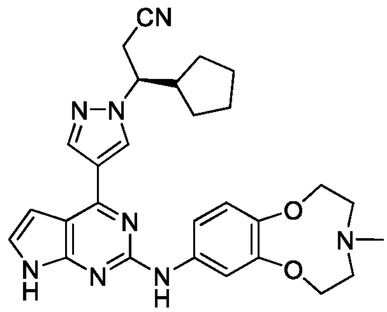

77

,

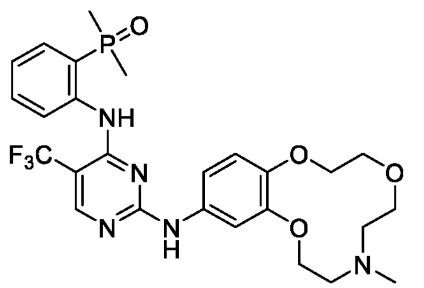

78

,

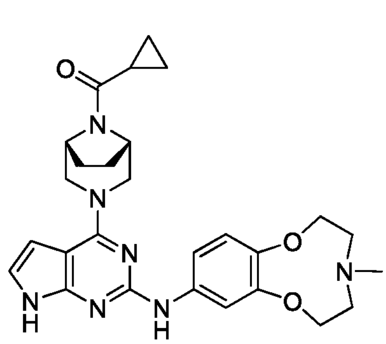

79

,

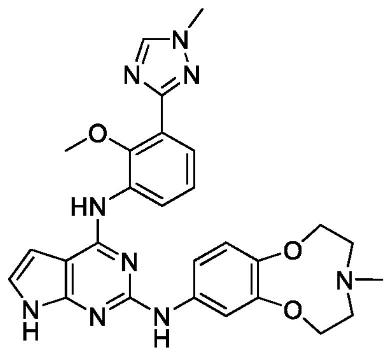

80

,

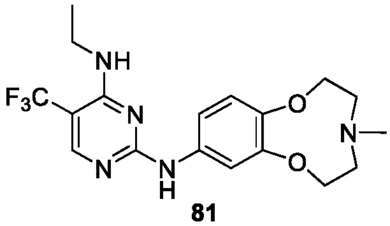

81

,

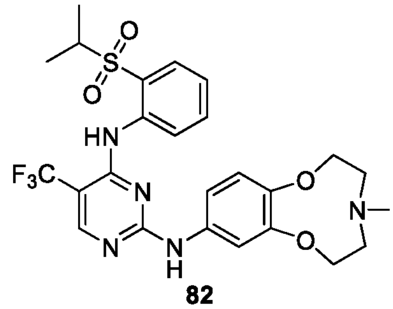

82

,

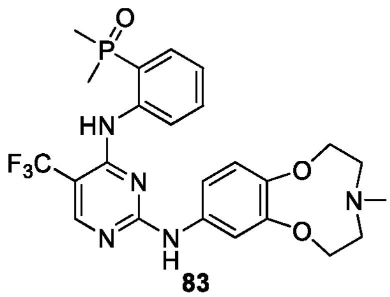

83

,

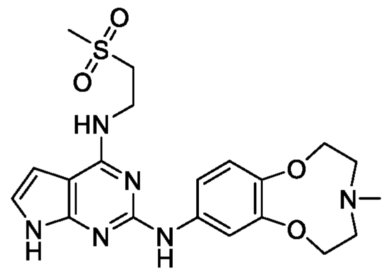

84

,

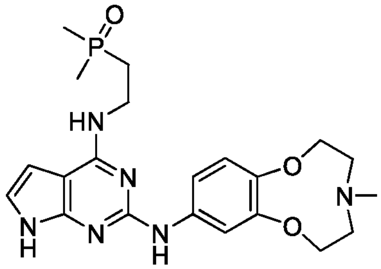

85

,

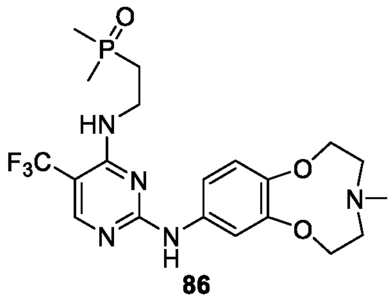

86

,

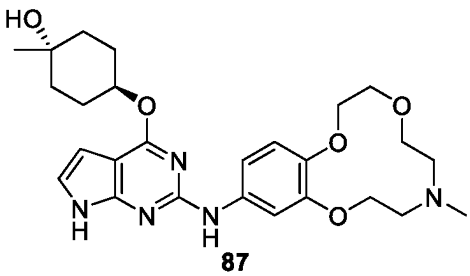

87

,

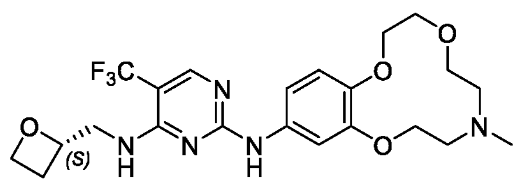

88

,

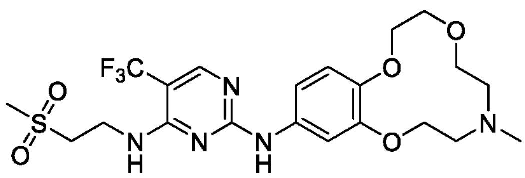

89

,

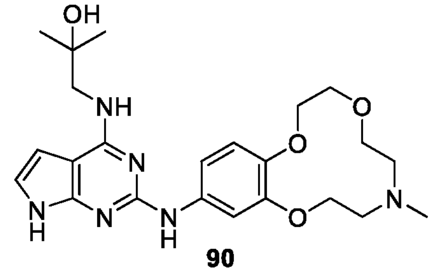

90

,

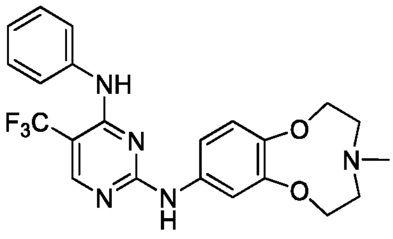
91
,

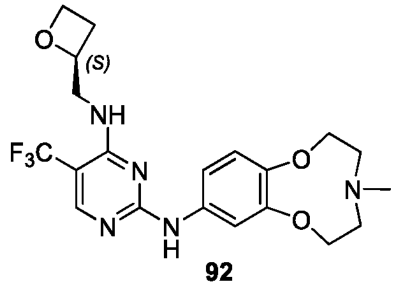
92
,

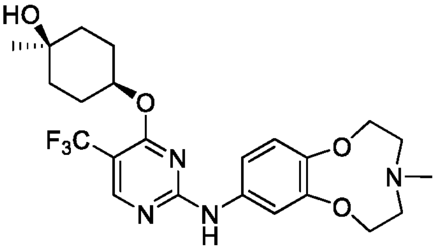
93
,

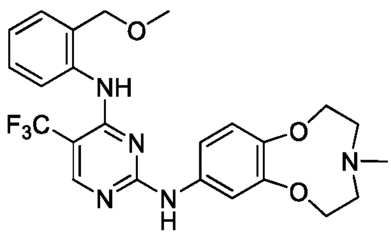
94
,

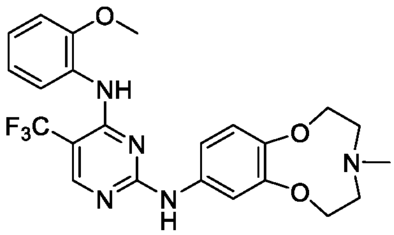
95
,

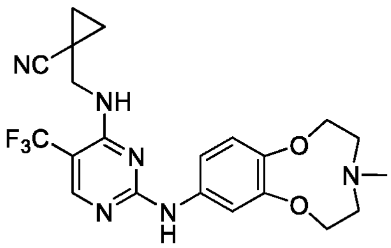
96
,

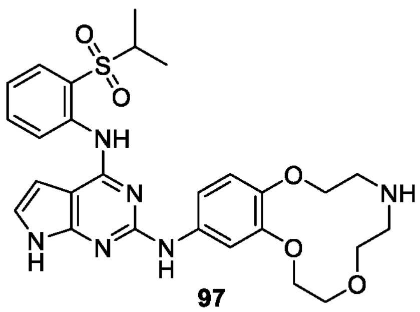
97
,

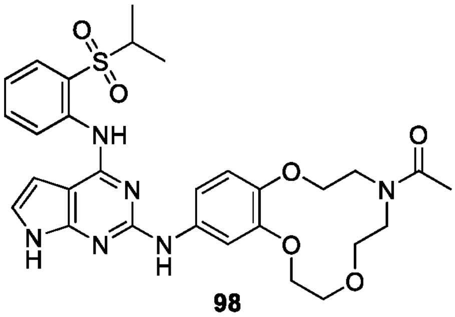
98
,

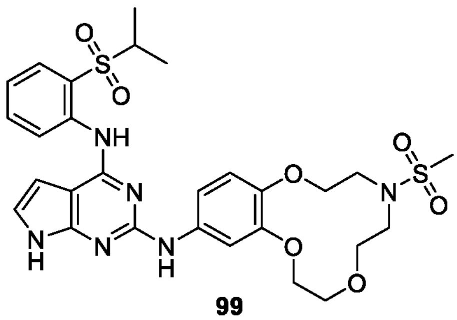
99
,

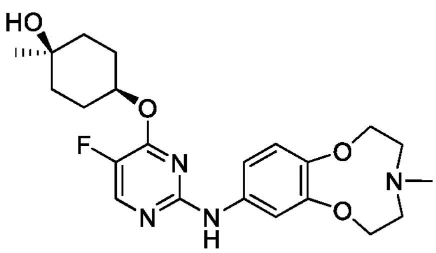
100
,

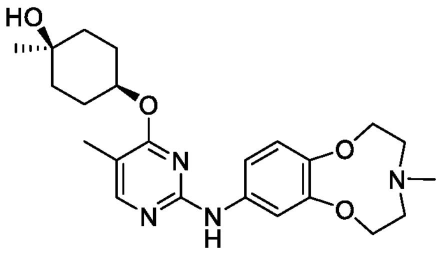

101 ,

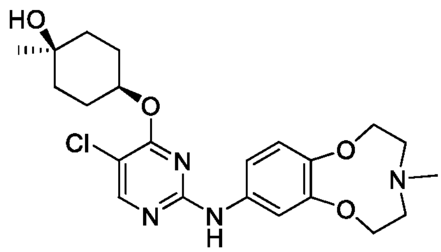

102 ,

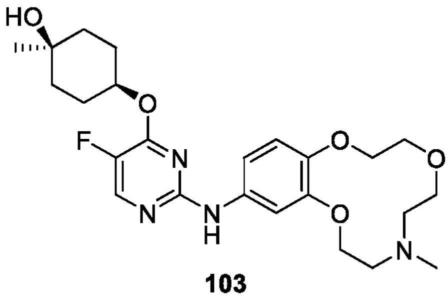

103 ,

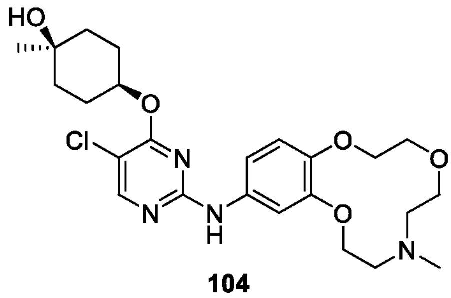

104 ,

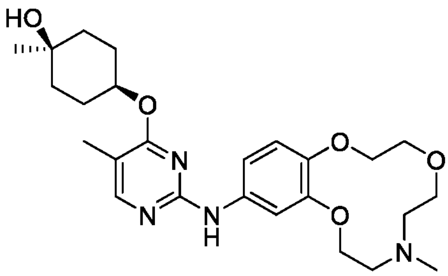

105 ,

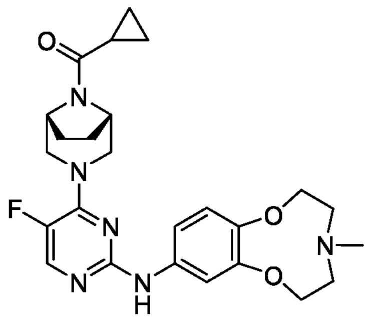

106 ,

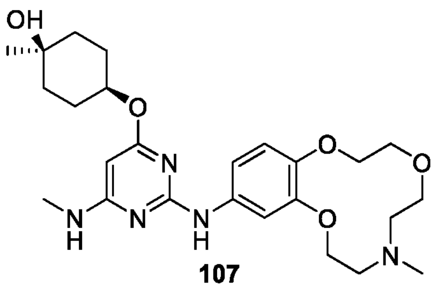

107 ,

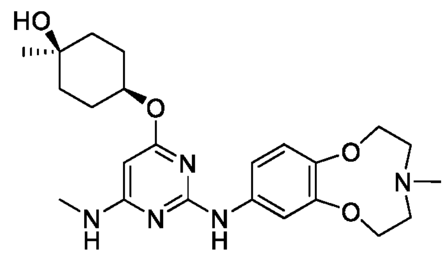

108 ,

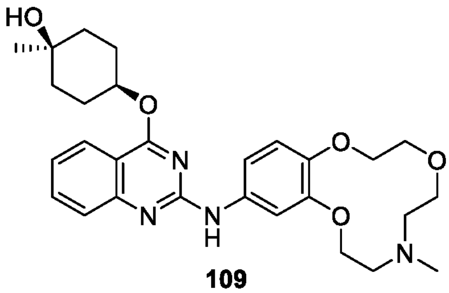

109 ,

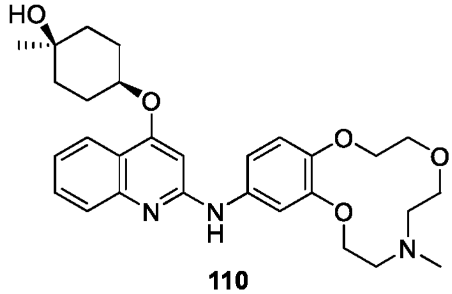

110 ,

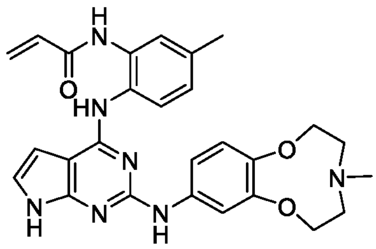

111

,

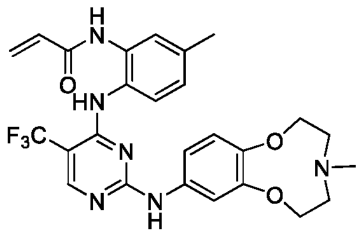

112

,

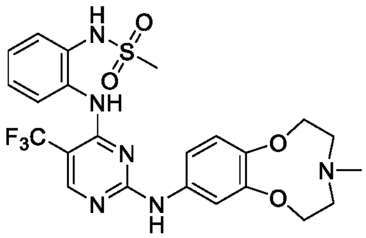

113

,

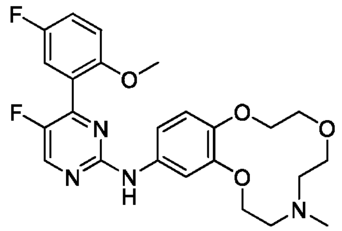

114

,

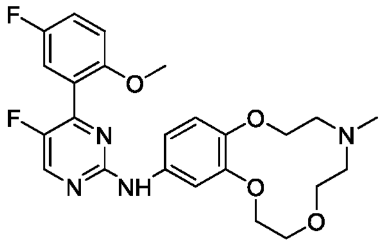

115

, and

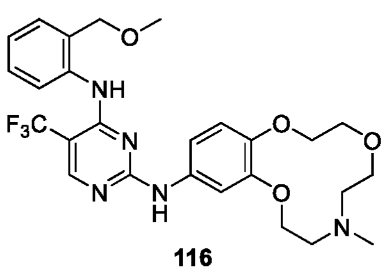

116

.

7. A pharmaceutical composition comprising the compound or the stereoisomer, tautomer or pharmaceutically acceptable salt thereof according to any one of claims 1 to 6, and an optional pharmaceutically acceptable carrier.

8. Use of the compound or the stereoisomer, tautomer or pharmaceutically acceptable salt thereof according to any one of claims 1 to 7 in the manufacture of a medicament as a protein kinase inhibitor.

9. Use of the compound or the stereoisomer, tautomer or pharmaceutically acceptable salt thereof according to any one of claims 1 to 7 in the manufacture of a medicament for the treatment or prevention of a disease related to a protein kinase.

10. The use according to claim 9, wherein the disease related to a protein kinase is a disease in which development or symptom of disease is related to signal transmission, mediation, regulation or adjustment of protein kinase.

11. The use according to any one of claims 8-10, wherein the protein kinase is selected from the group consisting of LRRK2, JAK1, JAK2, JAK3, EGFR and CDK9.

12. The use according to any one of claims 9 to 11, wherein the disease is selected from the group consisting of a neurodegenerative disease, an autoimmune disease and a tumor.

13. The use according to any one of claims 8 to 11, wherein the disease is selected from the group consisting of Parkinson's disease, asthma, dermatitis, non-small cell lung cancer, acute myeloid leukemia (AML) and liver cancer.

**INTERNATIONAL SEARCH REPORT**

International application No. **PCT/CN2023/119030**

**A. CLASSIFICATION OF SUBJECT MATTER**

C07D413/12(2006.01)i; C07D487/04(2006.01)i; C07D413/14(2006.01)i; C07F9/6561(2006.01)i; C07F9/6558(2006.01)i; C07D473/16(2006.01)i; C07D519/00(2006.01)i; C07D487/08(2006.01)i; A61K31/506(2006.01)i; A61K31/519(2006.01)i; A61K31/52(2006.01)i; A61K31/517(2006.01)i; A61K31/675(2006.01)i; A61K31/4709(2006.01)i; A61P25/00(2006.01)i; A61P37/02(2006.01)i; A61P35/00(2006.01)i; A61P35/02(2006.01)i; A61P25/16(2006.01)i; A61P11/06(2006.01)i; A61P17/00(2006.01)i

According to International Patent Classification (IPC) or to both national classification and IPC

**B. FIELDS SEARCHED**

Minimum documentation searched (classification system followed by classification symbols)

IPC: C07D, C07F, A61K, A61P

Documentation searched other than minimum documentation to the extent that such documents are included in the fields searched

Electronic data base consulted during the international search (name of data base and, where practicable, search terms used)

CNTXT, ENTXTC, DWPI, VEN, 万方, WANFANG, CNKI, Web of Science, 百度学术, BAIDU SCHOLAR, Registry, Caplus, Marpat: 科辉智药, 朱振东, 牛德强, 凡明, 李旭珂, 含氮冠醚, 氮 5d 冠醚, 嘧啶, 蛋白激酶, 癌, 肿瘤, Nitrogen+ 5d Crown ether, Pyrimidine, Protein kinase, LRRK2, JAK1, JAK2, JAK3, EGFR, CDK9, cancer, tumor, AML, 结构检索, structural search.

**C. DOCUMENTS CONSIDERED TO BE RELEVANT**

| Category* | Citation of document, with indication, where appropriate, of the relevant passages | Relevant to claim No. |
|---|---|---|
| X | CN 111683662 A (CHEN ZHIHONG) 18 September 2020 (2020-09-18) description, paragraphs 0007-0013, 0017-0018, 0113, and 0345, and tables 1 and 3 | 1-13 |
| A | WO 2017118438 A1 (HANGZHOU INNOGATE PHARMA CO., LTD.) 13 July 2017 (2017-07-13) entire document | 1-13 |
| A | WO 2016197987 A1 (HANGZHOU INNOGATE PHARMA CO., LTD.) 15 December 2016 (2016-12-15) entire document | 1-13 |
| A | US 2017342088 A1 (SALK INSTITUTE FOR BIOLOGICAL STUDIES et al.) 30 November 2017 (2017-11-30) entire document | 1-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **2023年12月12日** | **14 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/119030**

**C. DOCUMENTS CONSIDERED TO BE RELEVANT**

| Category* | Citation of document, with indication, where appropriate, of the relevant passages | Relevant to claim No. |
|---|---|---|
| A | CN 1486312 A (VERTEX PHARMACEUTICALS INC.) 31 March 2004 (2004-03-31) entire document | 1-13 |
| A | CN 102260248 A (NOVARTIS AG) 30 November 2011 (2011-11-30) entire document | 1-13 |

Form PCT/ISA/210 (second sheet) (July 2022)

**INTERNATIONAL SEARCH REPORT**
**Information on patent family members**

International application No.
**PCT/CN2023/119030**

| Patent document cited in search report | | | Publication date (day/month/year) | Patent family member(s) | | | Publication date (day/month/year) |
|---|---|---|---|---|---|---|---|
| CN | 111683662 | A | 18 September 2020 | EP | 3675860 | A1 | 08 July 2020 |
| | | | | EP | 3675860 | A4 | 14 April 2021 |
| | | | | EP | 3675860 | B1 | 08 March 2023 |
| | | | | US | 2020190068 | A1 | 18 June 2020 |
| | | | | US | 11440904 | B2 | 13 September 2022 |
| | | | | US | 2023109688 | A1 | 13 April 2023 |
| | | | | WO | 2019046163 | A1 | 07 March 2019 |
| WO | 2017118438 | A1 | 13 July 2017 | US | 2019100531 | A1 | 04 April 2019 |
| | | | | US | 10934311 | B2 | 02 March 2021 |
| WO | 2016197987 | A1 | 15 December 2016 | CN | 107709336 | A | 16 February 2018 |
| US | 2017342088 | A1 | 30 November 2017 | JP | 2017526674 | A | 14 September 2017 |
| | | | | JP | 6684780 | B2 | 22 April 2020 |
| | | | | WO | 2016033100 | A1 | 03 March 2016 |
| | | | | CA | 2959347 | A1 | 03 March 2016 |
| | | | | CA | 2959347 | C | 07 March 2023 |
| | | | | US | 2019284207 | A1 | 19 September 2019 |
| | | | | EP | 3185868 | A1 | 05 July 2017 |
| | | | | EP | 3185868 | A4 | 10 January 2018 |
| | | | | EP | 3185868 | B1 | 06 April 2022 |
| | | | | ES | 2914099 | T3 | 07 June 2022 |
| | | | | US | 2019152989 | A1 | 23 May 2019 |
| | | | | US | 10689397 | B2 | 23 June 2020 |
| | | | | EP | 4070795 | A1 | 12 October 2022 |
| | | | | JP | 2022017384 | A | 25 January 2022 |
| | | | | JP | 2020059731 | A | 16 April 2020 |
| | | | | JP | 6968141 | B2 | 17 November 2021 |
| | | | | US | 10266549 | B2 | 23 April 2019 |
| | | | | DK | 3185868 | T3 | 23 May 2022 |
| | | | | US | 2019248806 | A1 | 15 August 2019 |
| | | | | US | 10774092 | B2 | 15 September 2020 |
| CN | 1486312 | A | 31 March 2004 | AU | 2001297619 | B2 | 08 June 2006 |
| | | | | ATE | 326463 | T1 | 15 June 2006 |
| | | | | ATE | 326461 | T1 | 15 June 2006 |
| | | | | ES | 2265450 | T3 | 16 February 2007 |
| | | | | CA | 2432872 | A1 | 27 June 2002 |
| | | | | CA | 2432872 | C | 23 September 2008 |
| | | | | JP | 2004517894 | A | 17 June 2004 |
| | | | | JP | 4210520 | B2 | 21 January 2009 |
| | | | | MXPA | 03005605 | A | 06 October 2003 |
| | | | | KR | 20030061858 | A | 22 July 2003 |
| | | | | NZ | 526471 | A | 26 August 2005 |
| | | | | CA | 2432223 | A1 | 06 September 2002 |
| | | | | CA | 2432223 | C | 20 May 2008 |
| | | | | MXPA | 03005607 | A | 06 October 2003 |
| | | | | HU | 0400638 | A2 | 28 June 2004 |
| | | | | HK | 1061389 | A1 | 17 September 2004 |
| | | | | US | 2005038023 | A1 | 17 February 2005 |
| | | | | US | 7531536 | B2 | 12 May 2009 |
| | | | | US | 2003004164 | A1 | 02 January 2003 |
| | | | | US | 6656939 | B2 | 02 December 2003 |

Form PCT/ISA/210 (patent family annex) (July 2022)

**INTERNATIONAL SEARCH REPORT**
**Information on patent family members**

International application No.
**PCT/CN2023/119030**

| Patent document cited in search report | Publication date (day/month/year) | Patent family member(s) | | | Publication date (day/month/year) |
|---|---|---|---|---|---|
| | | EP | 1345925 | A2 | 24 September 2003 |
| | | EP | 1345925 | B1 | 17 May 2006 |
| | | JP | 2005097322 | A | 14 April 2005 |
| | | HK | 1060349 | A1 | 06 August 2004 |
| | | MXPA | 03005609 | A | 06 October 2003 |
| | | AR | 042398 | A1 | 22 June 2005 |
| | | ES | 2265446 | T3 | 16 February 2007 |
| | | AU | 2002231166 | B2 | 21 February 2008 |
| | | JP | 2004517927 | A | 17 June 2004 |
| | | WO | 02059112 | A2 | 01 August 2002 |
| | | WO | 02059112 | A3 | 06 February 2003 |
| | | NZ | 526474 | A | 28 October 2005 |
| | | NO | 20032670 | D0 | 12 June 2003 |
| | | NO | 20032670 | L | 15 August 2003 |
| | | NO | 328537 | B1 | 15 March 2010 |
| | | MXPA | 03005611 | A | 06 October 2003 |
| | | SI | 1353916 | T1 | 28 February 2007 |
| | | AR | 040925 | A1 | 27 April 2005 |
| | | US | 2003078275 | A1 | 24 April 2003 |
| | | US | 6653301 | B2 | 25 November 2003 |
| | | DE | 60126658 | D1 | 29 March 2007 |
| | | DE | 60126658 | T2 | 22 November 2007 |
| | | EP | 1345928 | A2 | 24 September 2003 |
| | | EP | 1345928 | B1 | 14 February 2007 |
| | | JP | 2004516292 | A | 03 June 2004 |
| | | IL | 209827 | A0 | 28 February 2011 |
| | | HU | 0400842 | A2 | 28 July 2004 |
| | | US | 2013096128 | A1 | 18 April 2013 |
| | | US | 8697698 | B2 | 15 April 2014 |
| | | DE | 60126828 | D1 | 05 April 2007 |
| | | DE | 60126828 | T2 | 08 November 2007 |
| | | JP | 2008260767 | A | 30 October 2008 |
| | | NZ | 526469 | A | 28 October 2005 |
| | | AU | 2002246754 | B2 | 08 June 2006 |
| | | PT | 1353916 | E | 31 January 2007 |
| | | US | 2003022885 | A1 | 30 January 2003 |
| | | US | 6727251 | B2 | 27 April 2004 |
| | | DE | 60119774 | D1 | 22 June 2006 |
| | | DE | 60119774 | T2 | 16 May 2007 |
| | | JP | 2008247921 | A | 16 October 2008 |
| | | HK | 1062433 | A1 | 05 November 2004 |
| | | KR | 20030061465 | A | 18 July 2003 |
| | | KR | 100889246 | B1 | 19 March 2009 |
| | | KR | 20030061463 | A | 18 July 2003 |
| | | AU | 3116602 | A | 01 July 2002 |
| | | KR | 20030061468 | A | 18 July 2003 |
| | | KR | 100875091 | B1 | 22 December 2008 |
| | | ATE | 528303 | T1 | 15 October 2011 |
| | | AP | 200302825 | A0 | 30 September 2003 |
| | | DE | 60123283 | D1 | 02 November 2006 |

Form PCT/ISA/210 (patent family annex) (July 2022)

**INTERNATIONAL SEARCH REPORT**
**Information on patent family members**

International application No.
**PCT/CN2023/119030**

| Patent document cited in search report | Publication date (day/month/year) | Patent family member(s) | | | Publication date (day/month/year) |
|---|---|---|---|---|---|
| | | DE | 60123283 | T2 | 12 April 2007 |
| | | DE | 60119776 | D1 | 22 June 2006 |
| | | DE | 60119776 | T2 | 16 May 2007 |
| | | WO | 02068415 | A1 | 06 September 2002 |
| | | PL | 363244 | A1 | 15 November 2004 |
| | | PL | 210414 | B1 | 31 January 2012 |
| | | KR | 20030061464 | A | 18 July 2003 |
| | | KR | 20030061466 | A | 18 July 2003 |
| | | KR | 100843114 | B1 | 02 July 2008 |
| | | IL | 156369 | A | 28 February 2011 |
| | | ATE | 326462 | T1 | 15 June 2006 |
| | | AU | 2006201267 | A1 | 27 April 2006 |
| | | AU | 2006201267 | B2 | 29 July 2010 |
| | | AU | 2002255452 | B2 | 08 June 2006 |
| | | NO | 20032704 | D0 | 13 June 2003 |
| | | NO | 20032704 | L | 21 August 2003 |
| | | NO | 330527 | B1 | 09 May 2011 |
| | | ES | 2280313 | T3 | 16 September 2007 |
| | | CA | 2432132 | A1 | 01 August 2002 |
| | | CA | 2432132 | C | 29 July 2008 |
| | | US | 2003055068 | A1 | 20 March 2003 |
| | | US | 6989385 | B2 | 24 January 2006 |
| | | DE | 60120219 | D1 | 06 July 2006 |
| | | DE | 60120219 | T2 | 12 April 2007 |
| | | JP | 2008201808 | A | 04 September 2008 |
| | | JP | 2004518743 | A | 24 June 2004 |
| | | JP | 2013213068 | A | 17 October 2013 |
| | | ATE | 340172 | T1 | 15 October 2006 |
| | | HK | 1060346 | A1 | 06 August 2004 |
| | | NZ | 526472 | A | 30 April 2004 |
| | | IL | 156369 | A0 | 04 January 2004 |
| | | EP | 1355905 | A1 | 29 October 2003 |
| | | EP | 1355905 | B1 | 21 February 2007 |
| | | ATE | 327989 | T1 | 15 June 2006 |
| | | MY | 140377 | A | 31 December 2009 |
| | | HK | 1059776 | A1 | 16 July 2004 |
| | | US | 2004214814 | A1 | 28 October 2004 |
| | | US | 7625913 | B2 | 01 December 2009 |
| | | WO | 02066461 | A1 | 29 August 2002 |
| | | HU | 0400641 | A2 | 28 June 2004 |
| | | HU | 0400641 | A3 | 29 March 2010 |
| | | US | 2004157893 | A1 | 12 August 2004 |
| | | CA | 2432222 | A1 | 15 August 2002 |
| | | CA | 2432222 | C | 29 July 2008 |
| | | JP | 2009155352 | A | 16 July 2009 |
| | | JP | 5249842 | B2 | 31 July 2013 |
| | | WO | 0250065 | A2 | 27 June 2002 |
| | | WO | 0250065 | A3 | 24 October 2002 |
| | | EP | 1353916 | A2 | 22 October 2003 |
| | | EP | 1353916 | B1 | 20 September 2006 |

Form PCT/ISA/210 (patent family annex) (July 2022)

**INTERNATIONAL SEARCH REPORT**
**Information on patent family members**

International application No.
**PCT/CN2023/119030**

| Patent document cited in search report | Publication date (day/month/year) | Patent family member(s) | | | Publication date (day/month/year) |
|---|---|---|---|---|---|
| | | ES | 2272567 | T3 | 01 May 2007 |
| | | MXPA | 03005606 | A | 06 October 2003 |
| | | IL | 156408 | A0 | 04 January 2004 |
| | | NZ | 526475 | A | 24 June 2005 |
| | | HU | 0400639 | A2 | 28 June 2004 |
| | | HU | 0400639 | A3 | 29 March 2010 |
| | | US | 2003004161 | A1 | 02 January 2003 |
| | | US | 6653300 | B2 | 25 November 2003 |
| | | US | 2014187772 | A1 | 03 July 2014 |
| | | EP | 1345927 | A1 | 24 September 2003 |
| | | EP | 1345927 | B1 | 17 May 2006 |
| | | WO | 02062789 | A1 | 15 August 2002 |
| | | DK | 1355905 | T3 | 18 June 2007 |
| | | ES | 2265452 | T3 | 16 February 2007 |
| | | PL | 363246 | A1 | 15 November 2004 |
| | | PL | 210066 | B1 | 30 November 2011 |
| | | PT | 1345922 | E | 29 September 2006 |
| | | NO | 20032736 | D0 | 16 June 2003 |
| | | NO | 20032736 | L | 18 August 2003 |
| | | AP | 200302816 | A0 | 30 June 2003 |
| | | AP | 2003002816 | A0 | 06 March 2006 |
| | | MXPA | 03005608 | A | 06 October 2003 |
| | | AR | 042397 | A1 | 22 June 2005 |
| | | SI | 1355905 | T1 | 31 August 2007 |
| | | AU | 3404702 | A | 01 July 2002 |
| | | KR | 20030061467 | A | 18 July 2003 |
| | | KR | 100947185 | B1 | 15 March 2010 |
| | | EP | 2264028 | A1 | 22 December 2010 |
| | | EP | 1702920 | A1 | 20 September 2006 |
| | | EP | 1702920 | B1 | 12 October 2011 |
| | | JP | 2004517926 | A | 17 June 2004 |
| | | JP | 4160392 | B2 | 01 October 2008 |
| | | ES | 2375491 | T3 | 01 March 2012 |
| | | HU | 0400908 | A2 | 28 July 2004 |
| | | HU | 0400908 | A3 | 29 March 2010 |
| | | NO | 20032671 | D0 | 12 June 2003 |
| | | NO | 20032671 | L | 18 August 2003 |
| | | AR | 042169 | A1 | 15 June 2005 |
| | | NZ | 526470 | A | 31 March 2006 |
| | | MXPA | 03005610 | A | 06 October 2003 |
| | | DK | 1353916 | T3 | 29 January 2007 |
| | | US | 2009312543 | A1 | 17 December 2009 |
| | | US | 7982037 | B2 | 19 July 2011 |
| | | JP | 2008189687 | A | 21 August 2008 |
| | | JP | 2004516291 | A | 03 June 2004 |
| | | JP | 4160389 | B2 | 01 October 2008 |
| | | MXPA | 03005612 | A | 06 October 2003 |
| | | ES | 2266095 | T3 | 01 March 2007 |
| | | US | 2008287444 | A1 | 20 November 2008 |
| | | US | 8304414 | B2 | 06 November 2012 |

Form PCT/ISA/210 (patent family annex) (July 2022)

**INTERNATIONAL SEARCH REPORT**
**Information on patent family members**

International application No.
**PCT/CN2023/119030**

| Patent document cited in search report | Publication date (day/month/year) | Patent family member(s) | | | Publication date (day/month/year) |
|---|---|---|---|---|---|
| | | WO | 0250066 | A2 | 27 June 2002 |
| | | WO | 0250066 | A3 | 23 January 2003 |
| | | IL | 156389 | A | 31 January 2011 |
| | | ATE | 353890 | T1 | 15 March 2007 |
| | | CA | 2432799 | A1 | 27 June 2002 |
| | | CA | 2432799 | C | 19 August 2008 |
| | | US | 2004167141 | A1 | 26 August 2004 |
| | | US | 7427681 | B2 | 23 September 2008 |
| | | EP | 1345922 | A1 | 24 September 2003 |
| | | EP | 1345922 | B1 | 31 May 2006 |
| | | JP | 2008222719 | A | 25 September 2008 |
| | | TWI | 290551 | B | 01 December 2007 |
| | | IL | 156368 | A0 | 04 January 2004 |
| | | TW | 200716617 | A | 01 May 2007 |
| | | TWI | 313269 | B | 11 August 2009 |
| | | HK | 1062565 | A1 | 12 November 2004 |
| | | ATE | 354573 | T1 | 15 March 2007 |
| | | NZ | 526473 | A | 24 June 2005 |
| | | HK | 1060557 | A1 | 13 August 2004 |
| | | IL | 156389 | A0 | 04 January 2004 |
| | | CA | 2432129 | A1 | 25 July 2002 |
| | | CA | 2432129 | C | 26 August 2008 |
| | | DE | 60119775 | D1 | 22 June 2006 |
| | | DE | 60119775 | T2 | 10 May 2007 |
| | | JP | 2008189682 | A | 21 August 2008 |
| | | WO | 02059111 | A2 | 01 August 2002 |
| | | WO | 02059111 | A3 | 09 January 2003 |
| | | ATE | 326460 | T1 | 15 June 2006 |
| | | BR | 0116411 | A | 11 November 2003 |
| | | RU | 2003122209 | A | 10 February 2005 |
| | | RU | 2355688 | C2 | 20 May 2009 |
| | | CA | 2432131 | A1 | 01 August 2002 |
| | | CA | 2432131 | C | 08 July 2008 |
| | | DE | 60119777 | D1 | 22 June 2006 |
| | | DE | 60119777 | T2 | 03 May 2007 |
| | | US | 2003105090 | A1 | 05 June 2003 |
| | | JP | 2008247920 | A | 16 October 2008 |
| | | JP | 2004519479 | A | 02 July 2004 |
| | | JP | 4234435 | B2 | 04 March 2009 |
| | | BR | 0116493 | A | 30 September 2003 |
| | | NZ | 526468 | A | 26 March 2004 |
| | | NO | 20032703 | D0 | 13 June 2003 |
| | | NO | 20032703 | L | 19 August 2003 |
| | | NO | 328501 | B1 | 01 March 2010 |
| | | KR | 20030061857 | A | 22 July 2003 |
| | | KR | 100909665 | B1 | 29 July 2009 |
| | | JP | 2004518703 | A | 24 June 2004 |
| | | JP | 4160395 | B2 | 01 October 2008 |
| | | JP | 2009286805 | A | 10 December 2009 |
| | | HK | 1060347 | A1 | 06 August 2004 |

Form PCT/ISA/210 (patent family annex) (July 2022)

**INTERNATIONAL SEARCH REPORT**
**Information on patent family members**

International application No.
**PCT/CN2023/119030**

| Patent document cited in search report | Publication date (day/month/year) | Patent family member(s) | Publication date (day/month/year) |
|---|---|---|---|
| | | PT 1355905 E | 31 May 2007 |
| | | IL 156407 A0 | 04 January 2004 |
| | | DK 1345922 T3 | 18 September 2006 |
| | | US 2004132781 A1 | 08 July 2004 |
| | | US 7087603 B2 | 08 August 2006 |
| | | CA 2432303 A1 | 29 August 2002 |
| | | CA 2432303 C | 13 April 2010 |
| | | US 2003036543 A1 | 20 February 2003 |
| | | US 6664247 B2 | 16 December 2003 |
| | | EP 1345926 A2 | 24 September 2003 |
| | | EP 1345926 B1 | 17 May 2006 |
| | | WO 02057259 A2 | 25 July 2002 |
| | | WO 02057259 A3 | 24 April 2003 |
| | | EP 1345929 A2 | 24 September 2003 |
| | | EP 1345929 B1 | 17 May 2006 |
| CN 102260248 A | 30 November 2011 | SG 132676 A1 | 28 June 2007 |
| | | NO 20061656 L | 16 June 2006 |
| | | ZA 200601963 B | 30 May 2007 |
| | | AU 2004272283 A1 | 24 March 2005 |
| | | AU 2004272283 B2 | 02 October 2008 |
| | | AU 2004272283 B9 | 23 October 2008 |
| | | IS 8402 A | 07 April 2006 |
| | | BRPI 0414480 A | 14 November 2006 |
| | | ES 2487534 T3 | 21 August 2014 |
| | | WO 2005026158 A1 | 24 March 2005 |
| | | JP 2007505856 A | 15 March 2007 |
| | | JP 4504375 B2 | 14 July 2010 |
| | | TNSN 06083 A1 | 03 October 2007 |
| | | EP 2266977 A1 | 29 December 2010 |
| | | EP 2266977 B1 | 14 May 2014 |
| | | EP 1664035 A1 | 07 June 2006 |
| | | EP 1664035 B1 | 16 February 2011 |
| | | GB 0321710 D0 | 15 October 2003 |
| | | TW 200521118 A | 01 July 2005 |
| | | MA 28076 A1 | 01 August 2006 |
| | | GB 0414440 D0 | 28 July 2004 |
| | | DE 602004031436 D1 | 31 March 2011 |
| | | AR 045747 A1 | 09 November 2005 |
| | | PT 1664035 E | 03 May 2011 |
| | | US 2006247262 A1 | 02 November 2006 |
| | | US 7671063 B2 | 02 March 2010 |
| | | PE 20050429 A1 | 14 July 2005 |
| | | ES 2361412 T3 | 16 June 2011 |
| | | ATE 498623 T1 | 15 March 2011 |
| | | US 2010152182 A1 | 17 June 2010 |
| | | US 8283356 B2 | 09 October 2012 |
| | | CA 2538909 A1 | 24 March 2005 |
| | | ECSP 066424 A | 18 September 2006 |
| | | MXPA 06002966 A | 31 May 2006 |
| | | KR 20060056388 A | 24 May 2006 |

Form PCT/ISA/210 (patent family annex) (July 2022)

**INTERNATIONAL SEARCH REPORT**
**Information on patent family members**

International application No.
**PCT/CN2023/119030**

| Patent document cited in search report | Publication date (day/month/year) | Patent family member(s) | Publication date (day/month/year) |
|---|---|---|---|
| | | KR 100813384 B1 | 12 March 2008 |
| | | RU 2006112593 A | 10 November 2007 |
| | | RU 2403251 C2 | 10 November 2010 |
| | | IL 174217 A0 | 01 August 2006 |
| | | PL 1664035 T3 | 29 July 2011 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202211155875X